# EUROPEAN PATENT APPLICATION

(11) **EP 4 644 403 A1**
(43) Date of publication of application: **05.11.2025**
(21) Application number: 24784363.4
(22) Date of filing: 03.04.2024
(51) Int. Cl.: C07H 19/20, C07H 21/02, A61K 31/7125, C12N 15/10

(54) **HALOGEN-CONTAINING COMPOUNDS USED FOR CAPPING THE 5' END OF NUCLEIC ACID AND USE THEREOF**

(30) Priority: 04.04.2023 CN 202310391954
(71) Applicant: Shenzhen Rhegen Biotechnology Co., Ltd., Shenzhen City, Guangdong 518057 (CN); Wuhan Rhegen Biotechnology Co., Ltd., Wuhan, Hubei 430205 (CN)
(72) Inventor: HU, Yong, Shenzhen, Guangdong 518057 (CN); HU, Zhaoyu, Wuhan, Hubei 430205 (CN); YAO, Jun, Wuhan, Hubei 430205 (CN)
(74) Representative: Patentanwälte Olbricht Buchhold Keulertz
(86) International application number: PCT/CN2024/085861
(87) International publication number: WO 2024/208281

(57) **Abstract**

The present invention provides a halogen-containing compound for capping 5' end of nucleic acid and a use thereof. The compound is shown in the following formula (I). The present invention also provides uses and effects of the compound in nucleic acid transcription and expression.

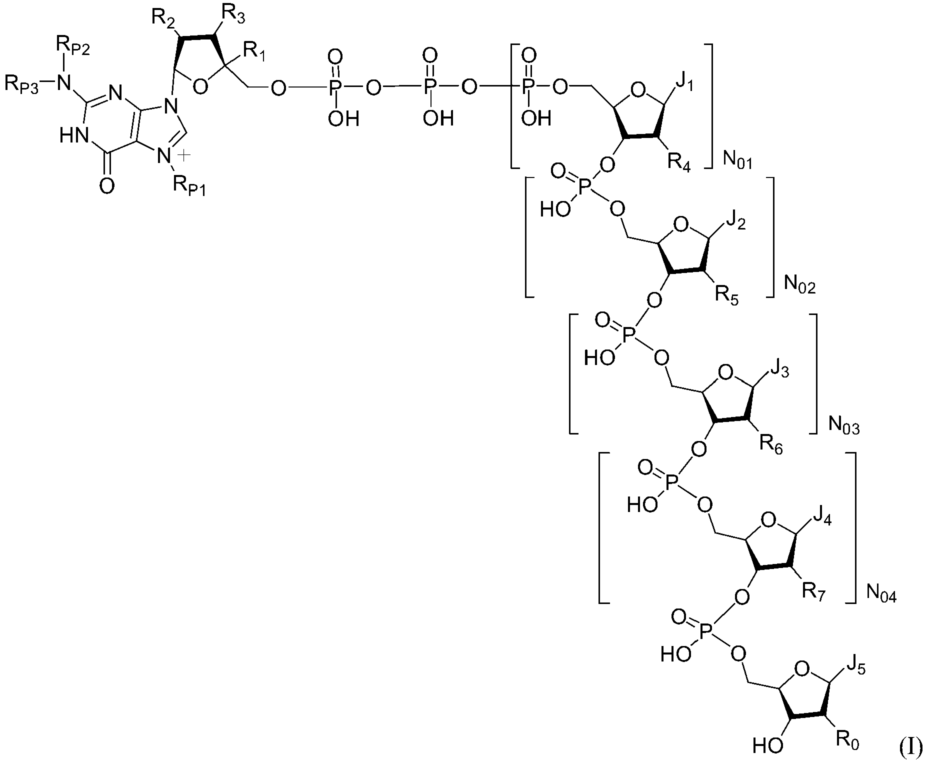

## Description

### Technical Field

The present invention relates to the fields of biotechnology and synthetic chemistry, in particular to a class of nucleoside compounds, and in particular to a halogen-containing compound for capping 5' end of a nucleic acid (RNA) and a use of the halogen-containing compound.

### Background Art

In recent years, as the global COVID-19 situation has changed, messenger RNA (mRNA) technology and vaccine drugs thereof have achieved breakthroughs from laboratory to clinical application. With the unprecedented success of the COVID-19 vaccine, market expectations for mRNA technology have risen sharply. The application scenarios of mRNA technology have great development potential not only in the field of infectious diseases such as COVID-19, but also in preventive vaccines, therapeutic drugs, and even cell programming and regenerative therapies.

The cap structure is a RNA modification widely present in cells, which plays a key role in maintaining the stability of mRNA and regulating protein translation. Currently, many studies are devoted to further chemically modifying the cap structure to further enhance the translation efficiency of mRNA while reducing immunogenicity thereof.

### Summary of the Invention

The present invention provides a halogen-containing compound for capping ***5*'** end of a nucleic acid (RNA), a pharmaceutically acceptable, solvate, and stereoisomer thereof, and a use thereof. This compound has various modified or unmodified 7-methylguanine bases on the nucleoside at one end and a halogen substituent at the 2' position of the nucleoside at the other end. The use of this compound in a reaction to cap 5' end of mRNA has a high capping rate and good *in vitro* transcription efficiency. At the same time, it has a high translation expression efficiency at the cellular level and *in vivo,* such as in mice.

The present invention provides a halogen-containing compound for capping 5' end of a nucleic acid, or a pharmaceutically acceptable salt, solvate, or stereoisomer thereof, wherein the compound has a structure of Formula (I): 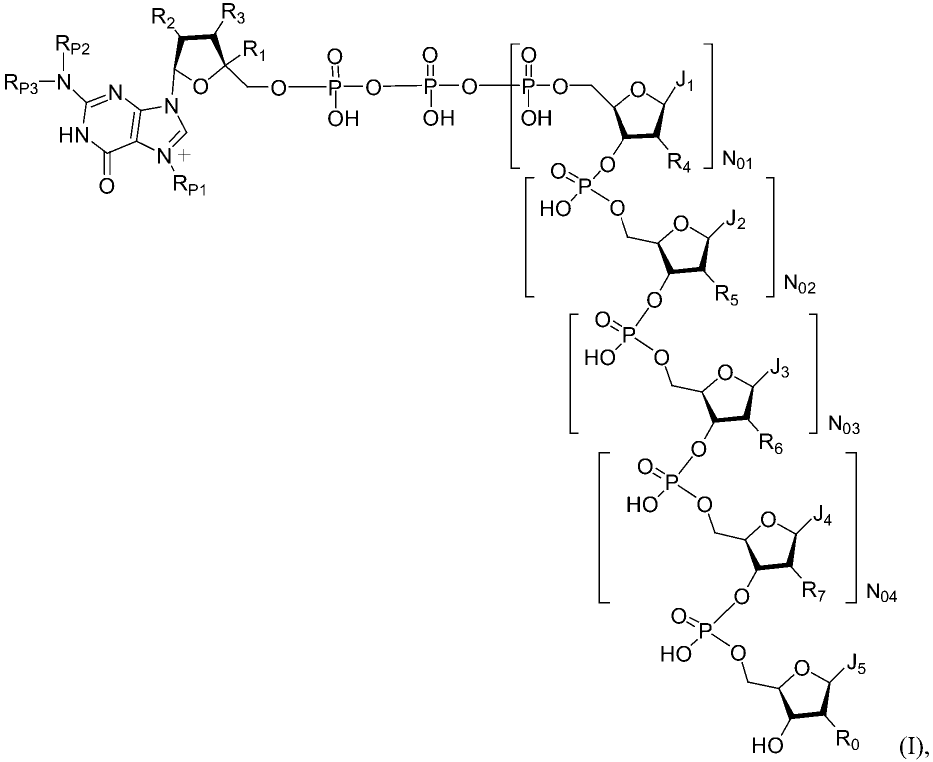 in which
R₀ is any one selected from the group consisting of F, Cl, Br and I,
R₁ is a group selected from the group consisting of -H, -OH, C₁₋₄ alkyl and C₁₋₄ alkoxy,
R₂ is any one selected from the group consisting of -H, -OH, C₁₋₆ alkyl and C₁₋₆ alkoxy,
optionally, R₁ and R₂ are connected to form a ring by a chemical bond, and -R₁-R₂- is any one of -(CH₂)_{q}-O-, -O-(CH₂)_{q}- and -(CH₂)ₘ-O-(CH₂)ₙ-, wherein q, m, n are each independently 1, 2 or 3,
R₃ is any one of H, -OH, -SH, -N₃, -NH₂, halogen, -CN, C₁₋₆ alkoxy, -O(CH₂)ₛCN, -SR₃ₐ, - O(CH₂)ₚR_{3b}, OCOR_{3c}, O(CH₂)ₚCOR_{3c}, -O(CH₂)ₜSH, -O(CH₂)ₚOH, -O(CH₂)ₚN₃, and -O(CH₂)ₚNH₂, wherein t, p and s are each independently any integer from 1 to 6, R₃ₐ is C₁₋₆ alkyl, R_{3b} is C₆₋₁₂ aryl optionally substituted with one or more R_{3d} or C₅₋₁₂ heteroaryl optionally substituted with one or more R_{3d}, R_{3c} is C₁₋₁₀ alkyl optionally substituted with one or more R_{3d}, C₁₋₁₀ alkenyl optionally substituted with one or more R_{3d}, C₅₋₁₂ cycloalkyl optionally substituted with one or more R_{3d}, or C₅₋₁₂ cycloalkenyl optionally substituted with one or more R_{3d}, wherein R₃ is optionally substituted with one or more R₃ₑ, and R_{3d} and R₃ₑ are selected from the group consisting of alkyl, alkenyl, alkoxy, halogen, cyano, amino, nitro, -OH, and -SH,
R₄, R₅, R₆, and R₇ are each independently any one selected from the group consisting of -H, - OH, -OCH₃, halogen, -CN, and -SH,
N₀₁, N₀₂, N₀₃, and N₀₄ are each independently selected from 0 or 1,
J₁, J₂, J₃, J₄, and J₅ are each independently selected from natural or modified pyrimidine nucleotide bases, or natural or modified purine nucleotide bases,
R_{P1} is C₁ to C₆ alkyl, preferably C₁ to C₃ alkyl, which is optionally substituted with -SH, -N₃, C₂ to C₆ alkenyl or C₂ to C₆ alkynyl,
R_{P2} and R_{P3} are each independently selected from the group consisting of H, C₁ to C₆ alkyl, C₂ to C₆ alkenyl, C₂ to C₆ alkynyl, cycloalkyl, cycloalkenyl, cycloalkynyl, PEG, COR_{P4}, and SO₂R_{P4}, wherein these groups are each optionally substituted with -CN, -N₃, -SH, or alkynyl, R_{P4} is selected from the group consisting of H and C₁ to C₆ alkyl, and R_{P2} and R_{P3} are optionally connected to form a ring, and
with the proviso that when N₀₁, N₀₂, N₀₃, and N₀₄ are all 0, J₅ is a guanine base, and when R₂ is -OH, R₃ is not a methoxy group.

In a preferred embodiment, the compound of the present invention has a structure of Formula (I'): 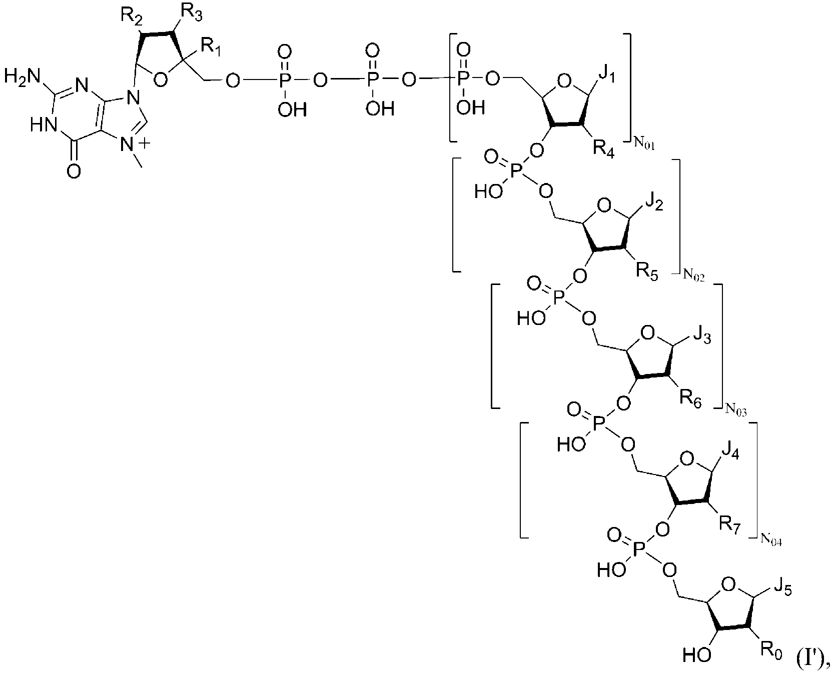 in which
each group in Formula (I') has the same meaning as that described above with respect to Formula (I).

In a preferred embodiment, at least one of J₁, J₂, J₃, J₄, and J₅ is a modified nucleotide base, preferably a modified purine nucleotide base, more preferably a methyl-modified purine nucleotide base, and still more preferably 6-N-methyladenine.

In a preferred embodiment, R₃ is any one of -H, -OH, -SH, -N₃, -NH₂, halogen, -CN, C₁₋₆ alkoxy, -O(CH₂)ₚCN, -SR₃ₐ, -O(CH₂)ₚR_{3b}, OCOR_{3c}, O(CH₂)ₚCOR_{3c}, -O(CH₂)ₚSH, -O(CH₂)ₚOH, - O(CH₂)ₚN₃, and -O(CH₂)ₚNH₂, wherein t, p and s are each independently any integer from 1 to 6, preferably 1 to 4, R₃ₐ is C₁₋₄ alkyl, R_{3b} is C₆₋₁₀ aryl optionally substituted with one or more R_{3d}, or C₅₋₁₀ heteroaryl optionally substituted with one or more R_{3d}, R_{3c} is C₅₋₁₀ cycloalkyl optionally substituted with one or more R_{3d}, or C₅₋₁₀ cycloalkenyl optionally substituted with one or more R_{3d}, wherein R₃ is optionally substituted with one or more R₃ₑ, and R_{3d} and R₃ₑ are selected from the group consisting of C₁₋₄ alkyl, C₂₋₄ alkenyl, C₁₋₄ alkoxy, halogen, cyano, amino, nitro, -OH, and -SH.

In another preferred embodiment, R and R₃ are any one of -H, -OH, -SH, -N₃, -NH₂, halogen, - CN, C₁₋₃ alkoxy, -O(CH₂)ₚCN, -SR₃ₐ, -O(CH₂)ₚR_{3b}, OCOR_{3c}, O(CH₂)ₚCOR_{3c}, -O(CH₂)ₚSH, - O(CH₂)ₚOH, -O(CH₂)ₚN₃, and -O(CH₂)ₚNH₂, wherein p and s are each independently any integer from 1 to 3, t is any integer from 1 to 4, R₃ₐ is methyl or ethyl, R_{3b} is C₅₋₁₀ heteroaryl optionally substituted with one or two R_{3d}, R_{3c} is C₅₋₁₀ cycloalkyl optionally substituted with one or two R_{3d}, or C₅₋₁₀ cycloalkenyl optionally substituted with one or two R_{3d}, wherein R₃ is optionally substituted with one or more R₃ₑ, and R_{3d} and R₃ₑ are selected from the group consisting of C₁₋₄ alkyl, C₂₋₄ alkenyl, C₁₋₄ alkoxy, halogen, cyano, amino, nitro, -OH, and -SH.

In another preferred embodiment, R_{3b} is C₅ or C₆ heteroaryl optionally substituted with C₁₋₄ alkyl, for example, tetrazinyl optionally substituted with C₁₋₄ alkyl, and R_{3c} is C₅₋₁₀ cycloalkenyl optionally substituted with C₁₋₄ alkyl, halogen, cyano, amino, or nitro, for example, norbornenyl or cyclooctenyl optionally substituted with C₁₋₄ alkyl, halogen, cyano, amino, or nitro, and for example, unsubstituted norbornenyl or cyclooctenyl.

In a preferred embodiment, the compound has a structure of Formula (Ia), Formula (Ib) or Formula (Ic): 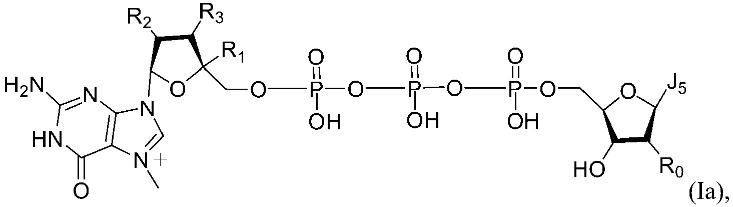 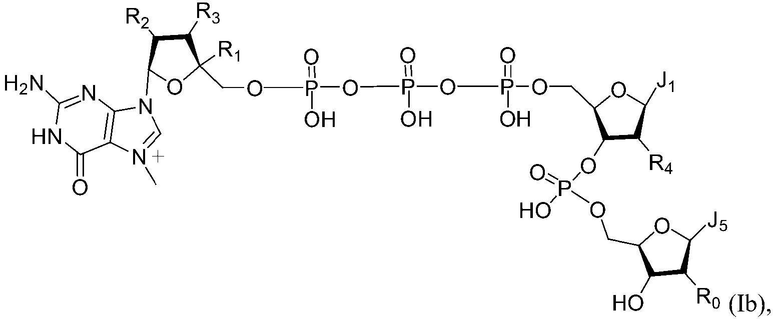 or

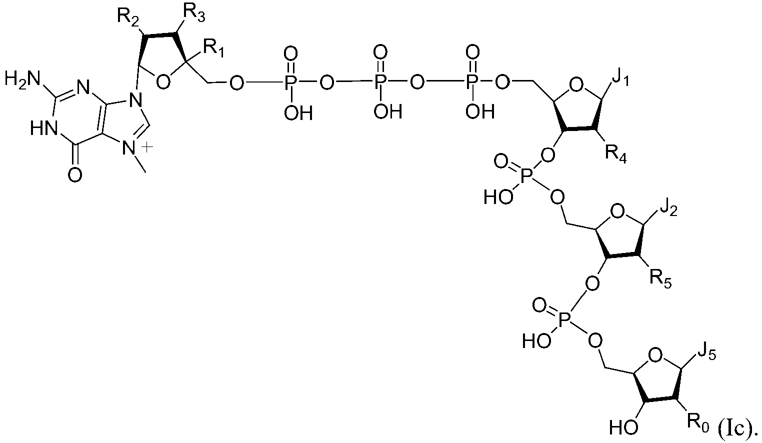

In a preferred embodiment, R₀ is -F or -Cl, and/or
R₄ and R₅ are each independently any one selected from the group consisting of H, OH, OCH₃, F, Cl, -CN, and -SH, and preferably any one selected from the group consisting of H, OH, OCH₃, and F.

In another preferred embodiment, the compound has a structure of Formula (Id): 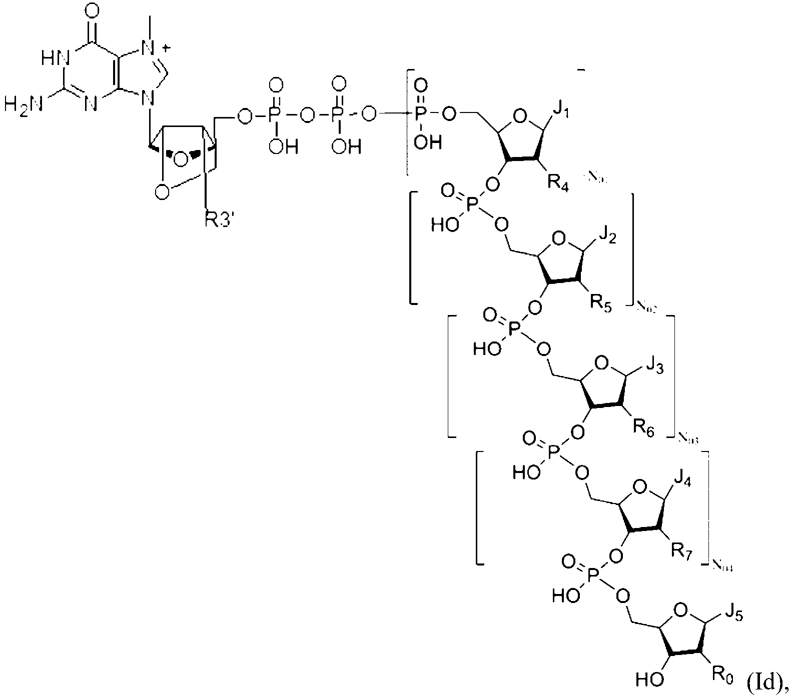 in which
R₃' has a meaning the same as that defined above with respect to R₃, and
the remaining groups have the meanings same as those defined above.

In a preferred embodiment, the compound has a structure of Formula (Ie), Formula (If) or Formula (Ig): 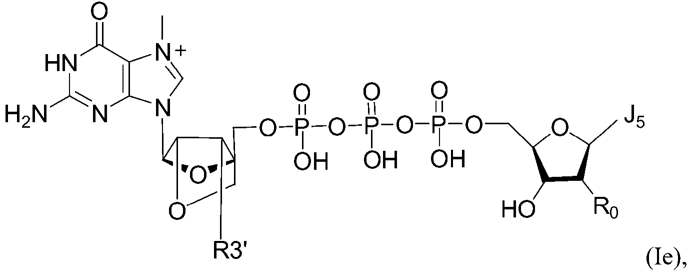 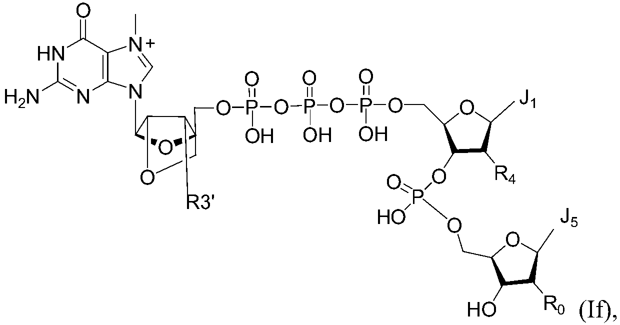 or

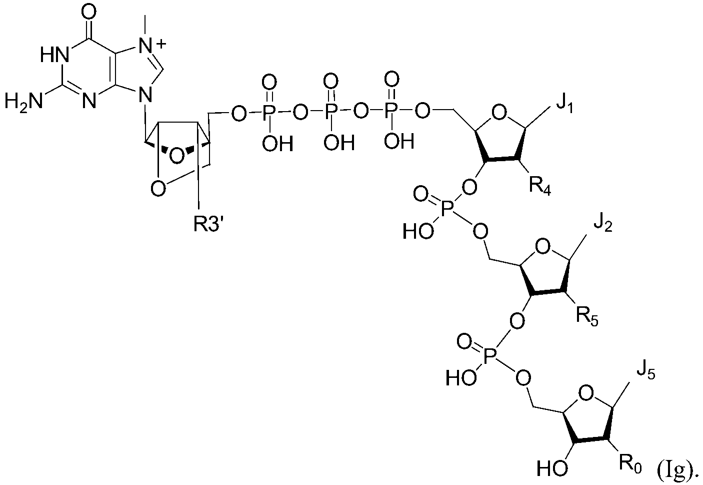

In a preferred embodiment, R₀ is -F or -Cl, and/or
R₄ and R₅ are each independently any one selected from the group consisting of H, OH, OCH₃, F, Cl, -CN, and -SH, and preferably any one selected from the group consisting of H, OH, OCH₃, and F.

In a most preferred embodiment, the compound has one of the structures shown in Table 1.

In a preferred embodiment, the compound of the present invention is present in the form of a pharmaceutically acceptable salt, and preferably in the form of a triethylamine salt, a sodium salt, a potassium salt, an ammonium salt, or tris(hydroxymethyl)aminomethane hydrochloride.

Another aspect of the present invention relates to a use of the compound as described above as an *in vitro* co-transcriptional RNA capping reagent.

Yet another aspect of the present invention relates to an RNA molecule including the compound as described above as a cap structure or a cap structure fragment.

Still another aspect of the present invention relates to a pharmaceutical composition including the RNA molecule as described above, and a pharmaceutically acceptable carrier.

The present invention also relates to a method for synthesizing an RNA molecule, which includes incubating the compound as described above with a polynucleotide template in order to perform a template-based transcription.

The present invention further relates to a transcriptional RNA-capping reaction system which includes a polynucleotide template, the compound as described above, NTPs, and a RNA polymerase.

According to some embodiments of the present invention, the compound (or cap analog) has one of the structures shown in Table 1 below:

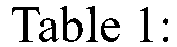

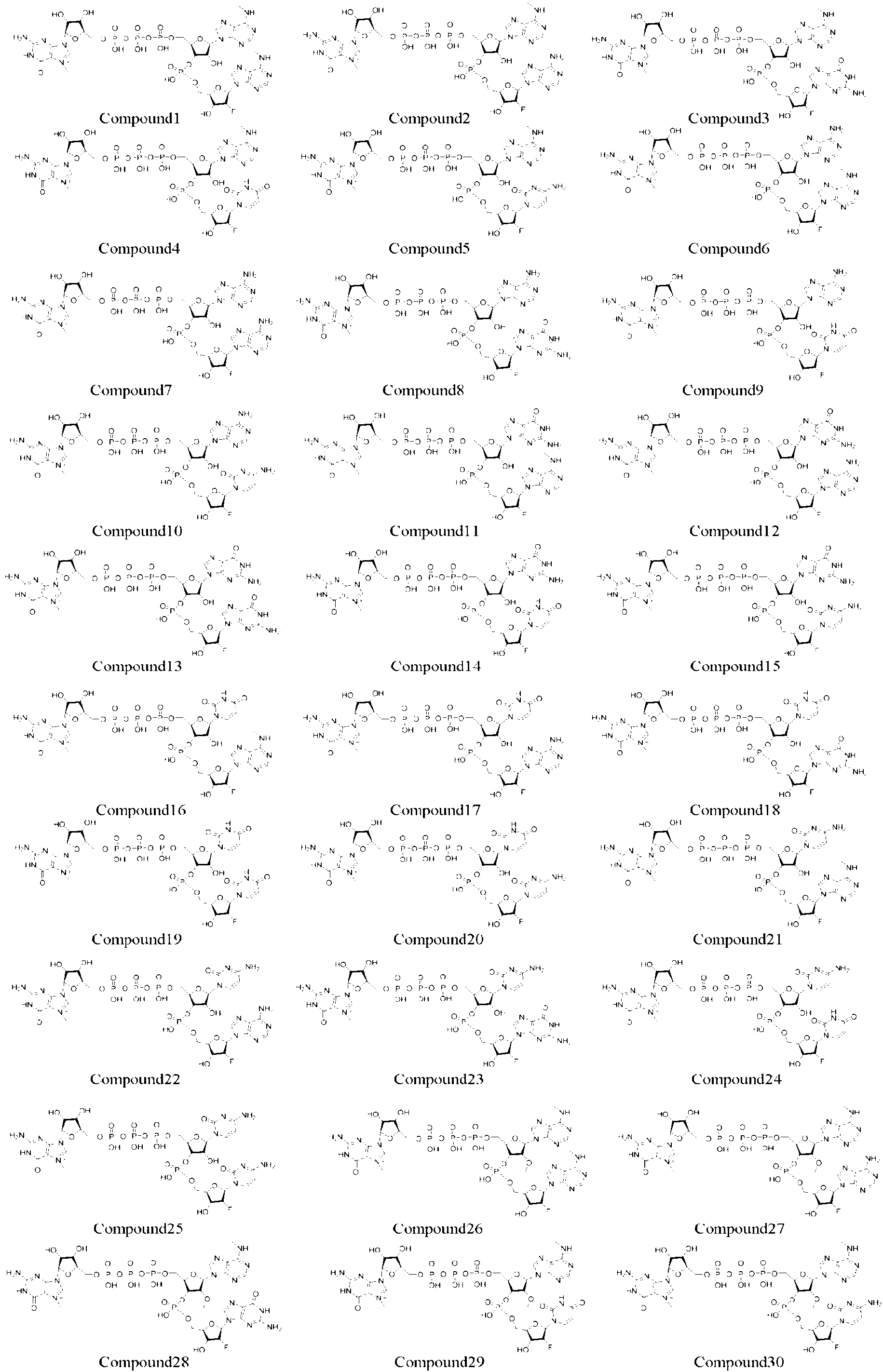

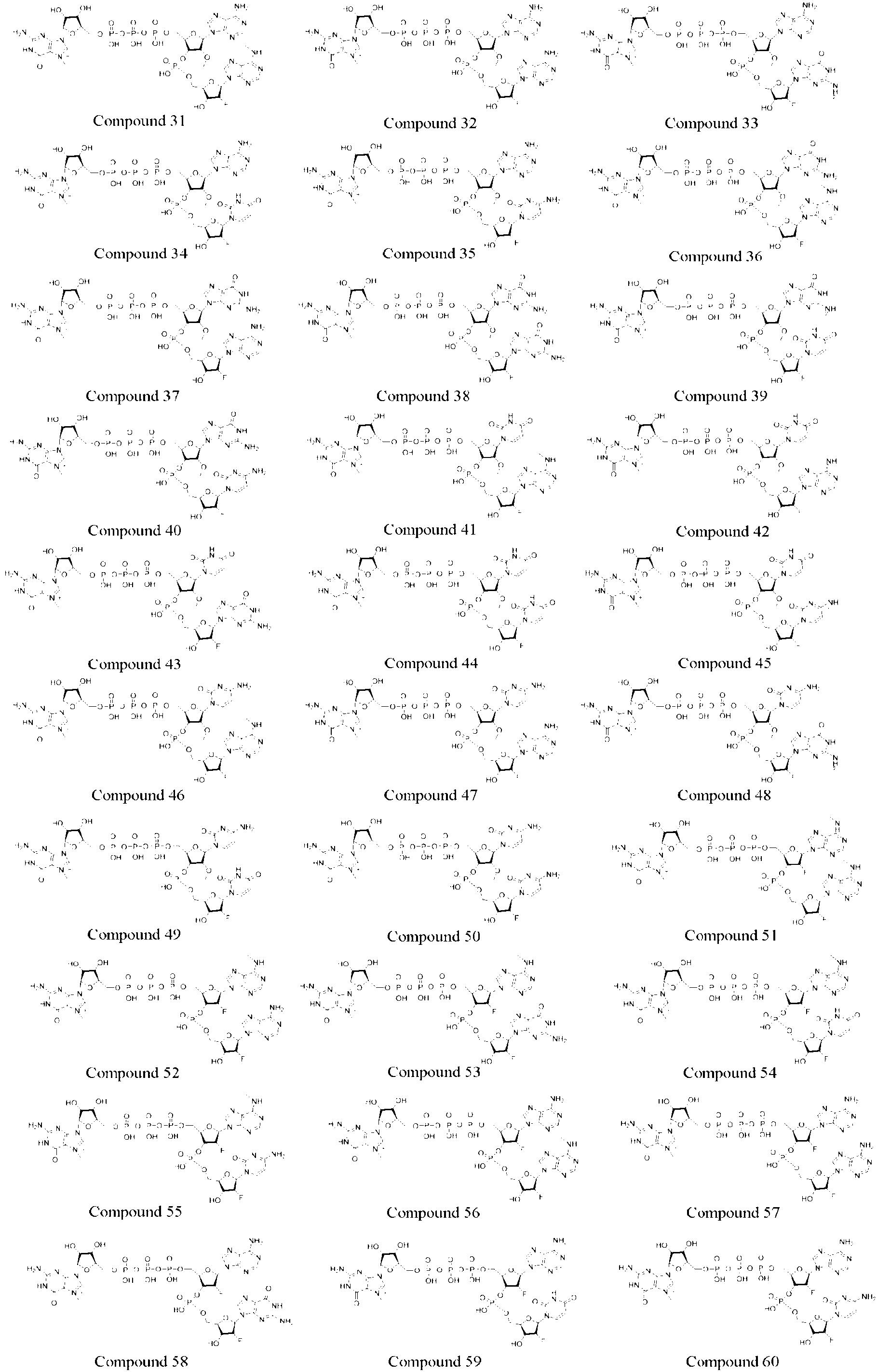

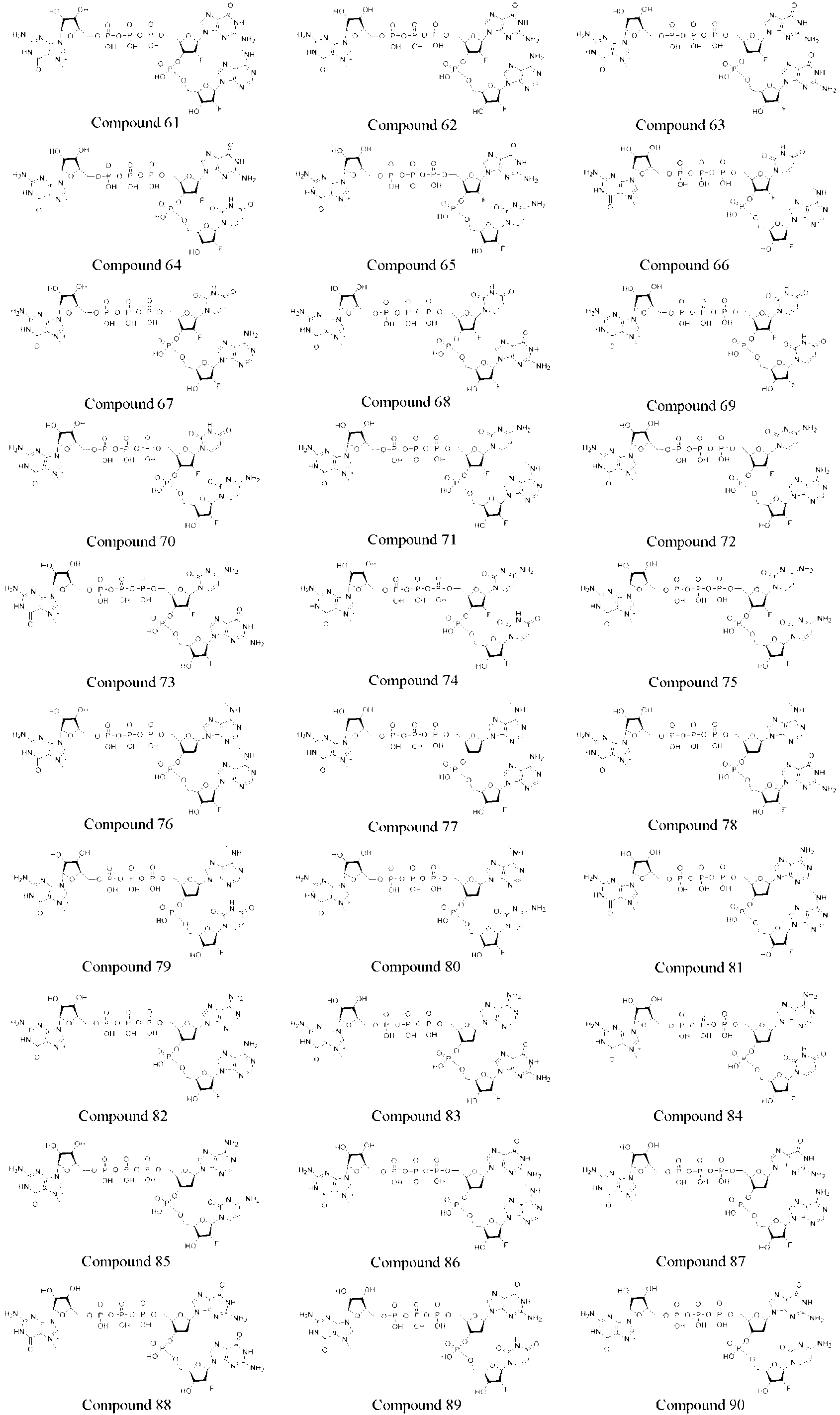

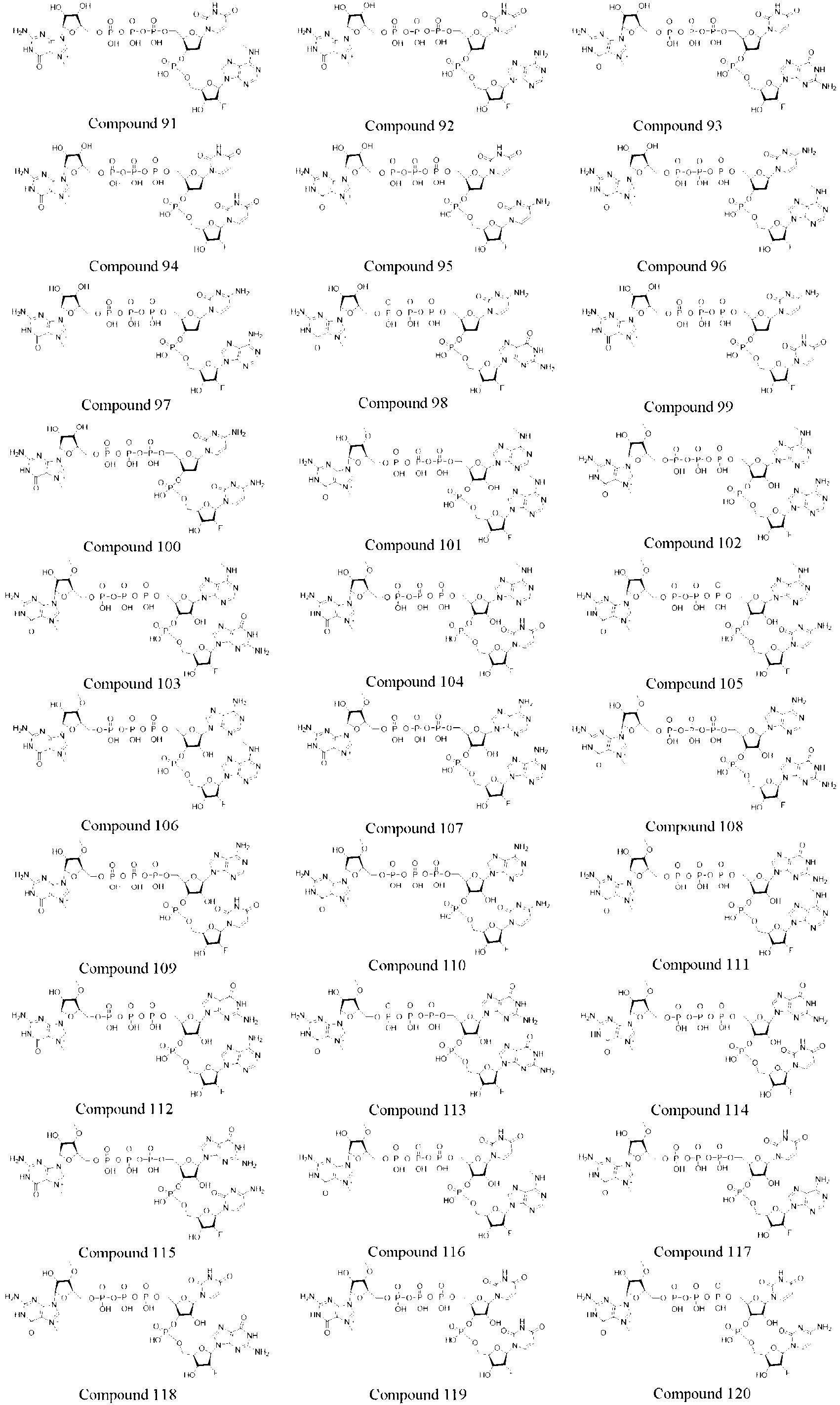

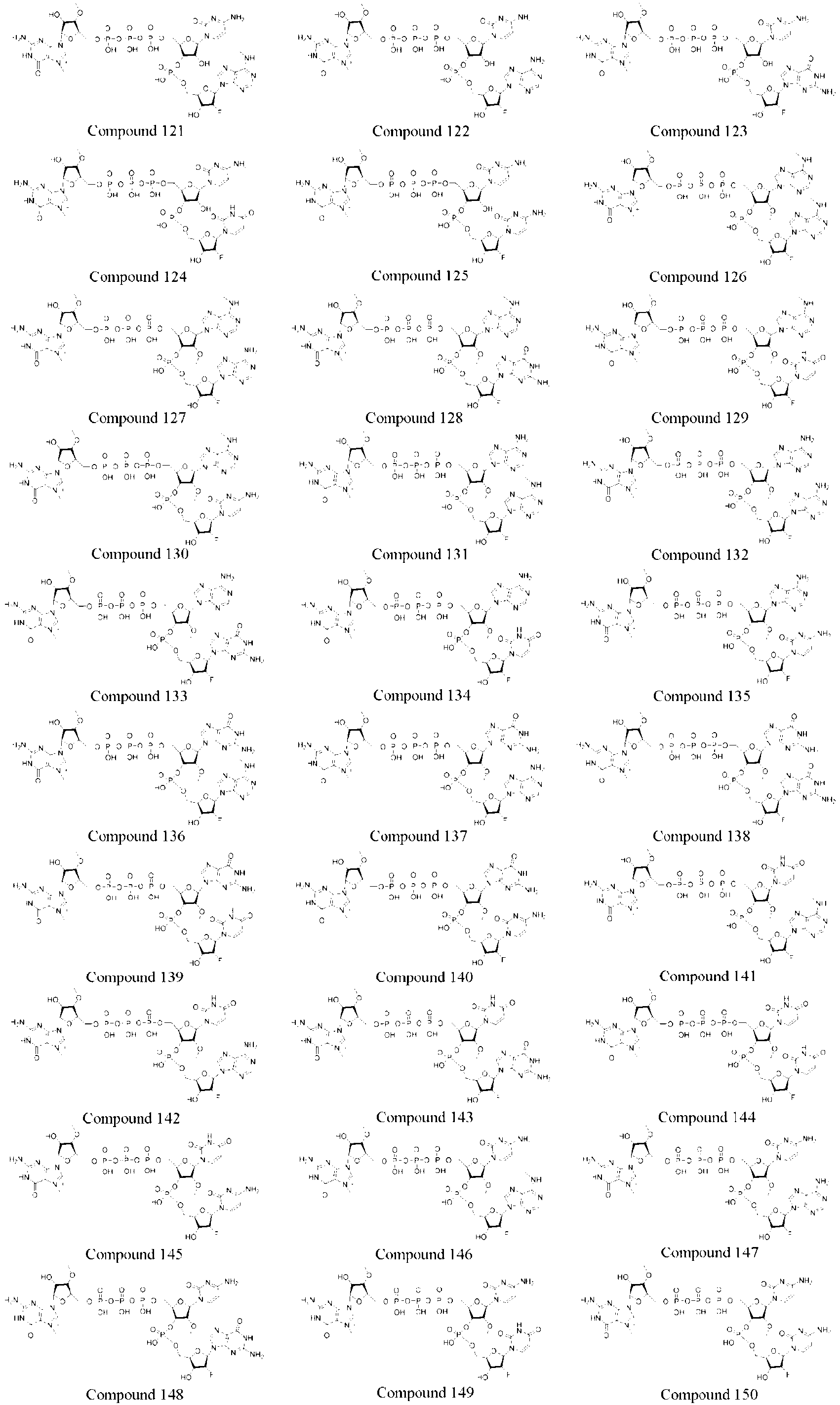

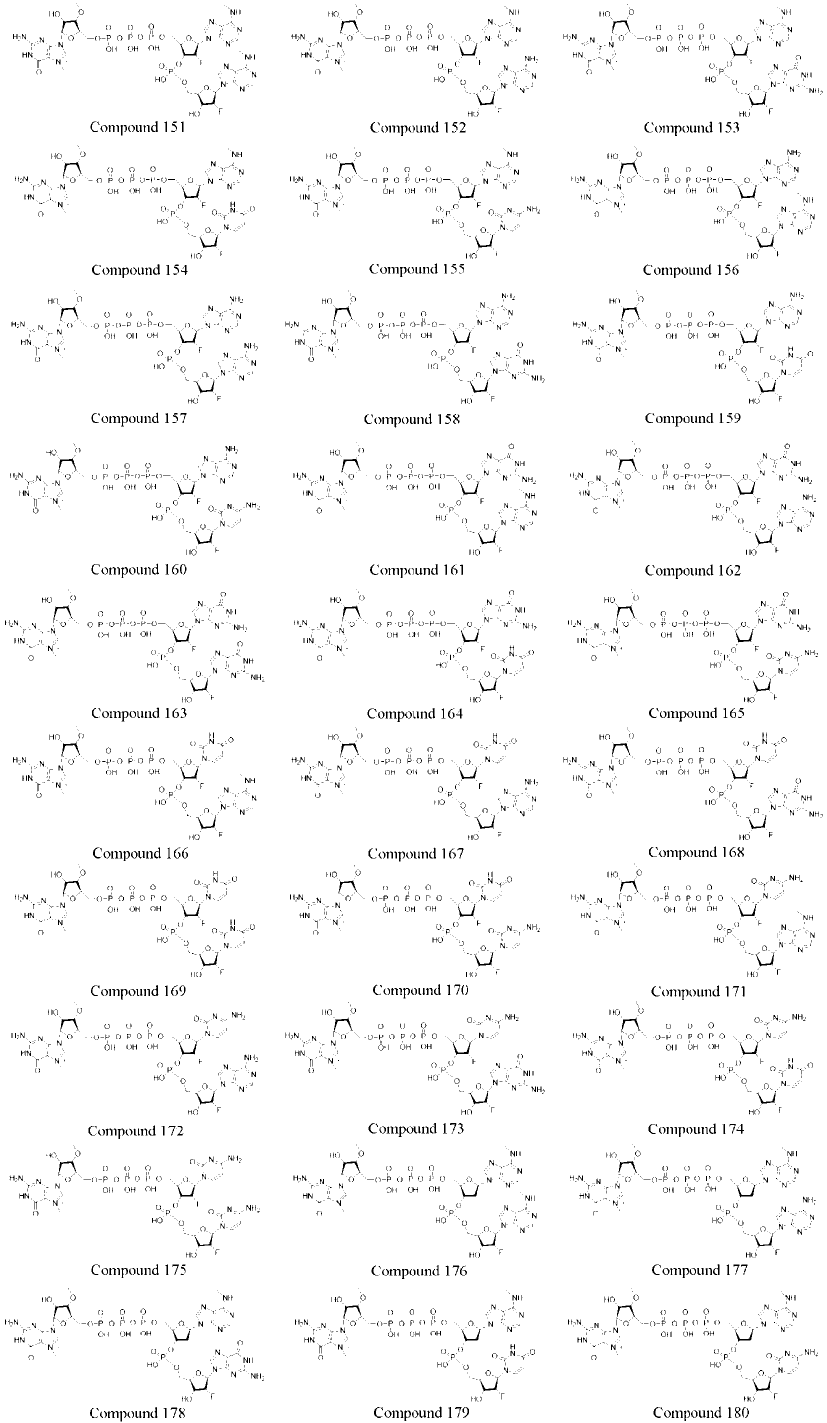

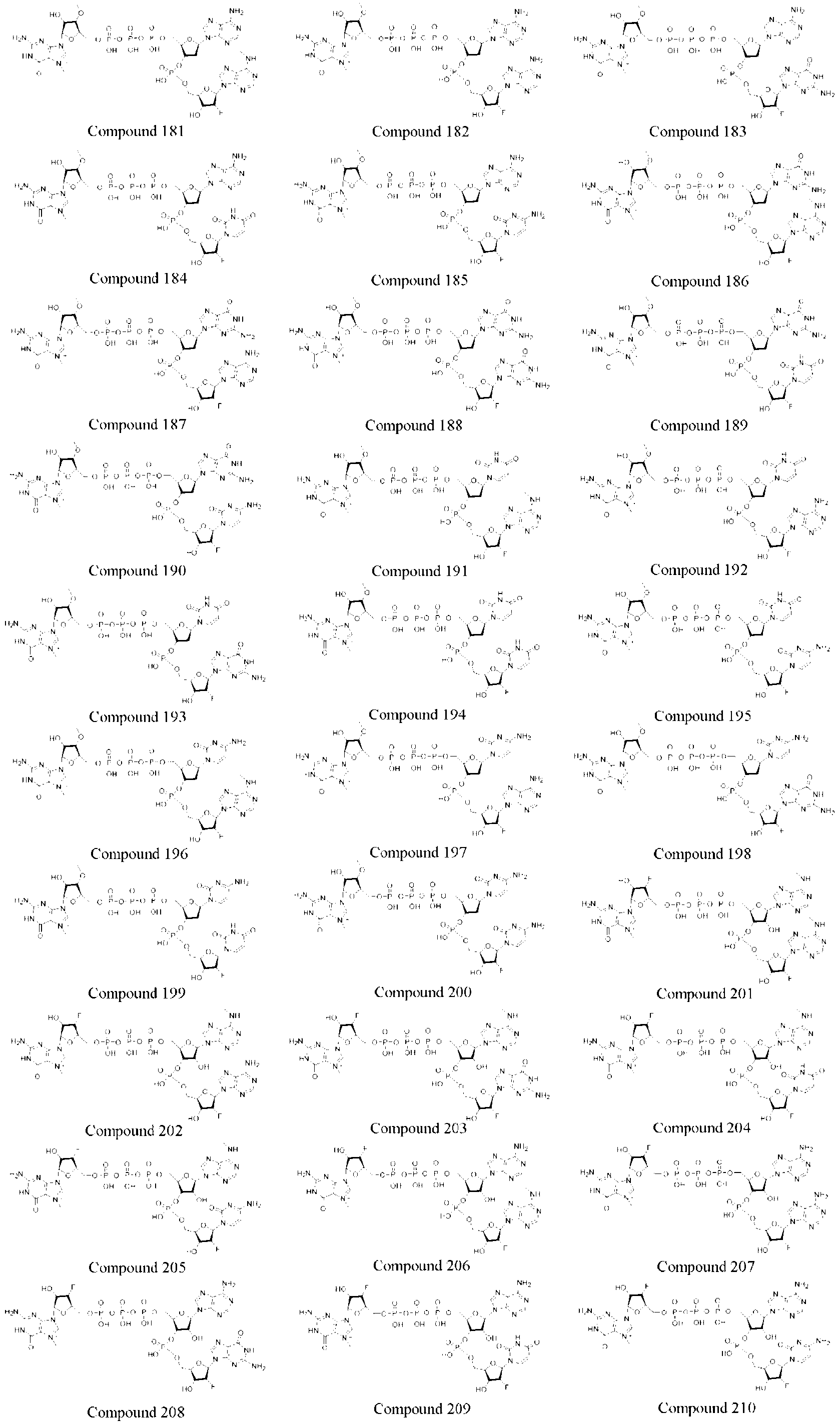

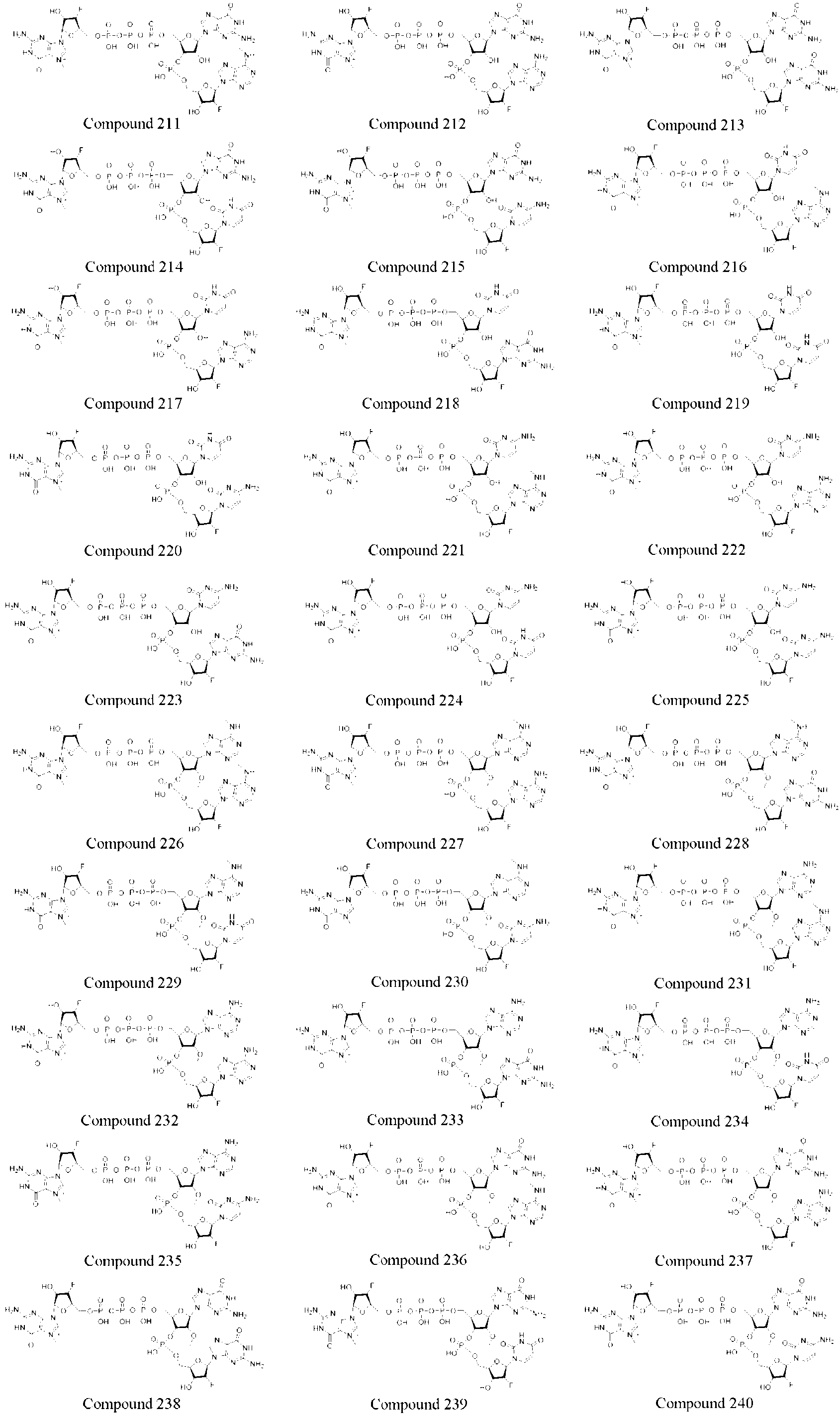

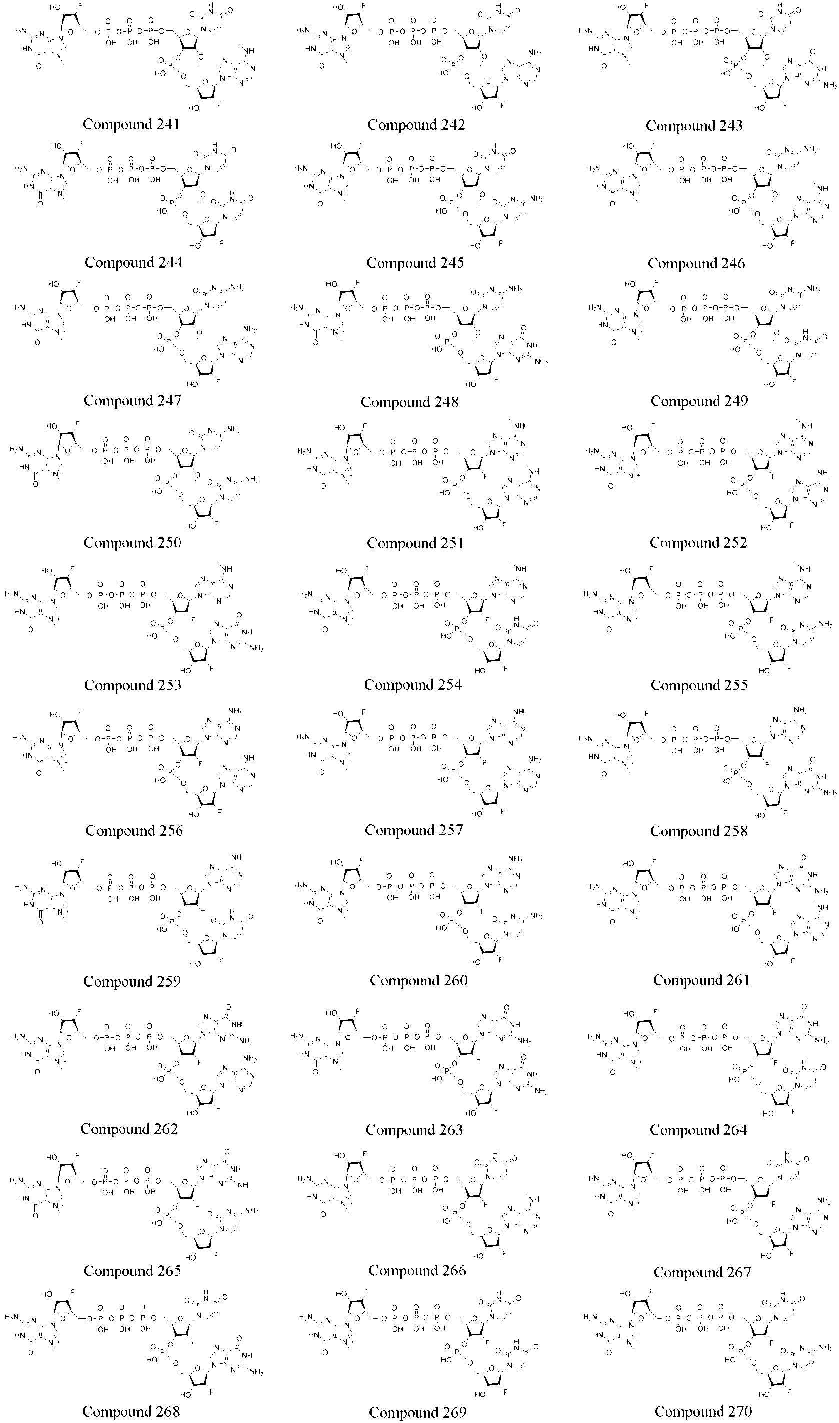

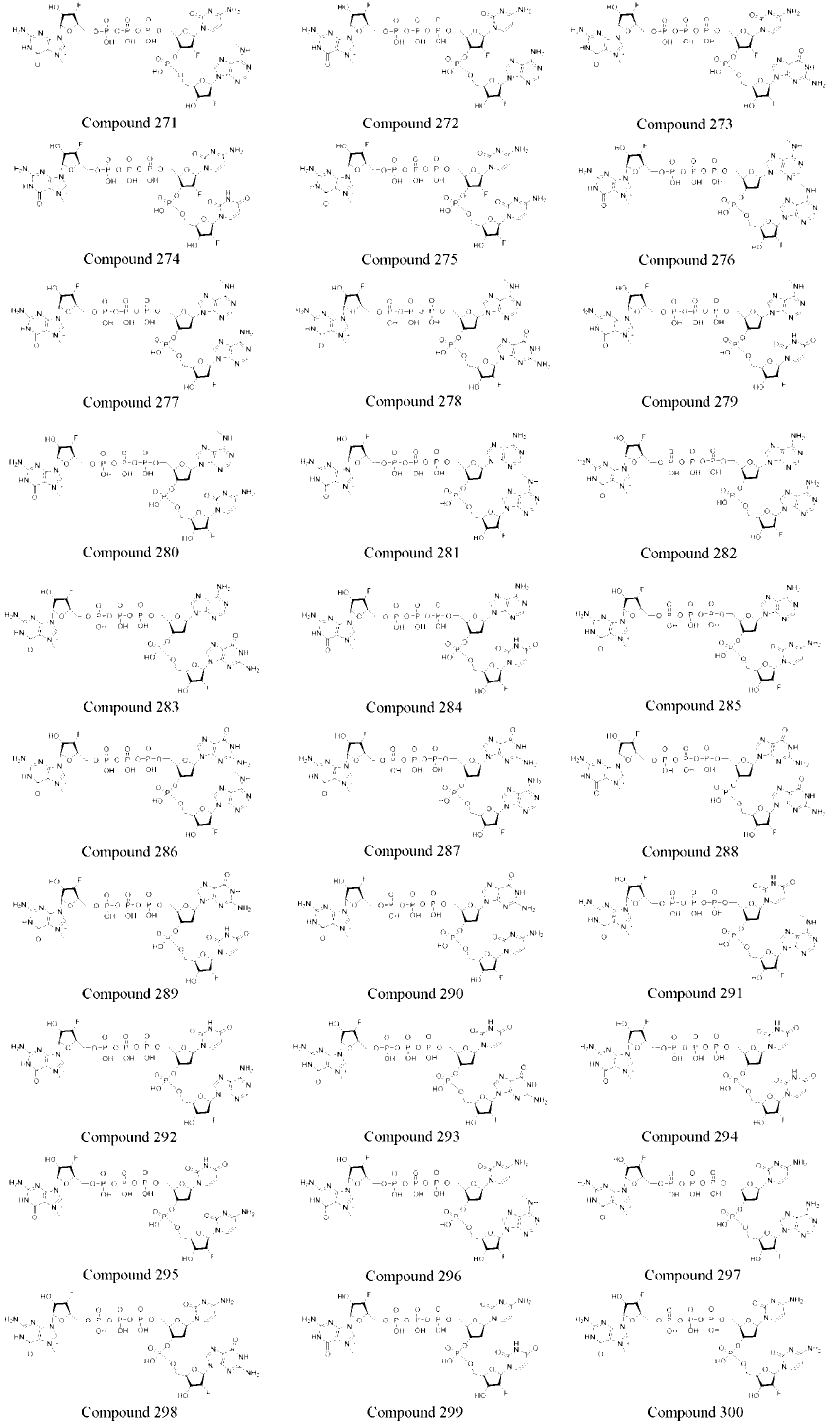

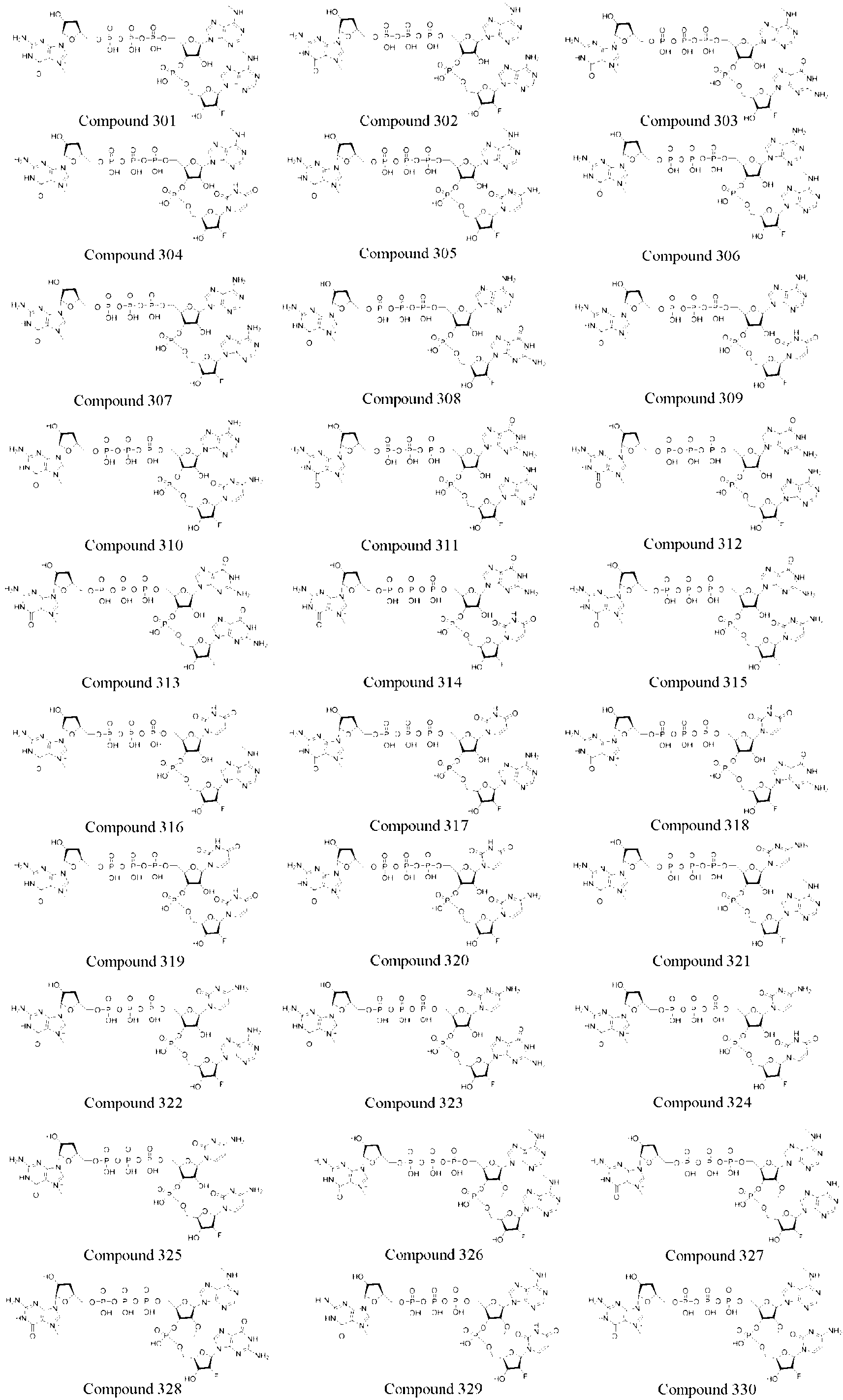

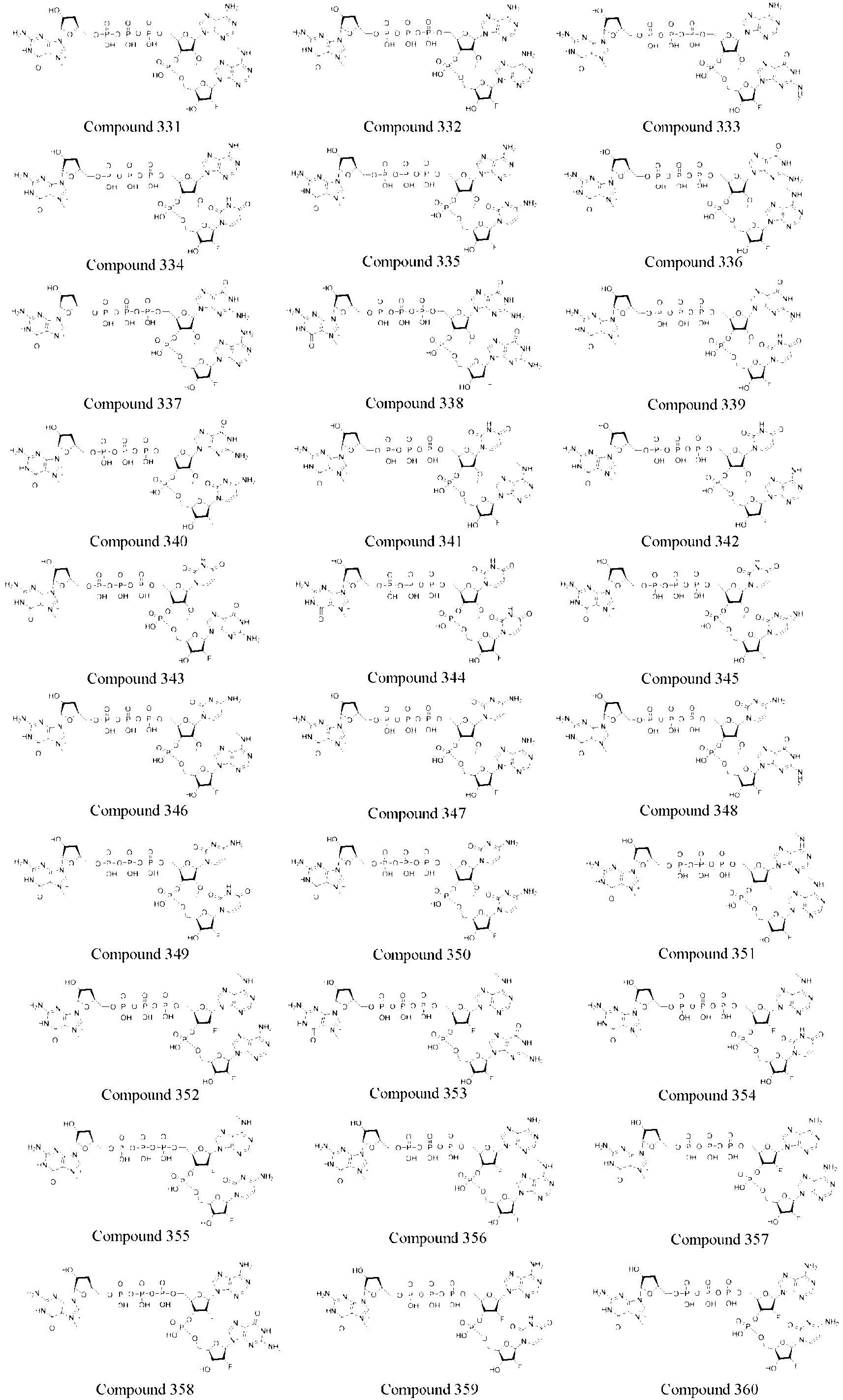

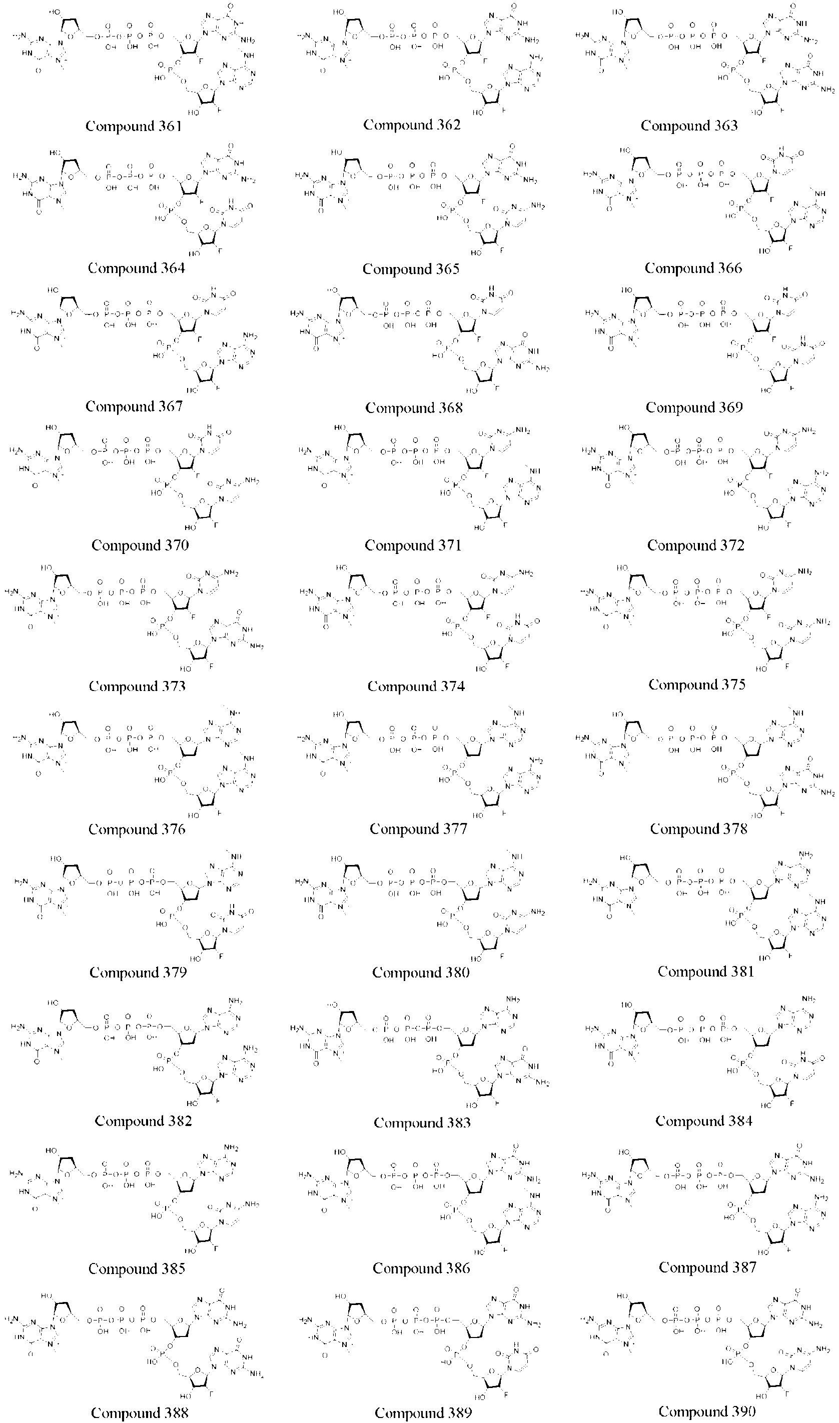

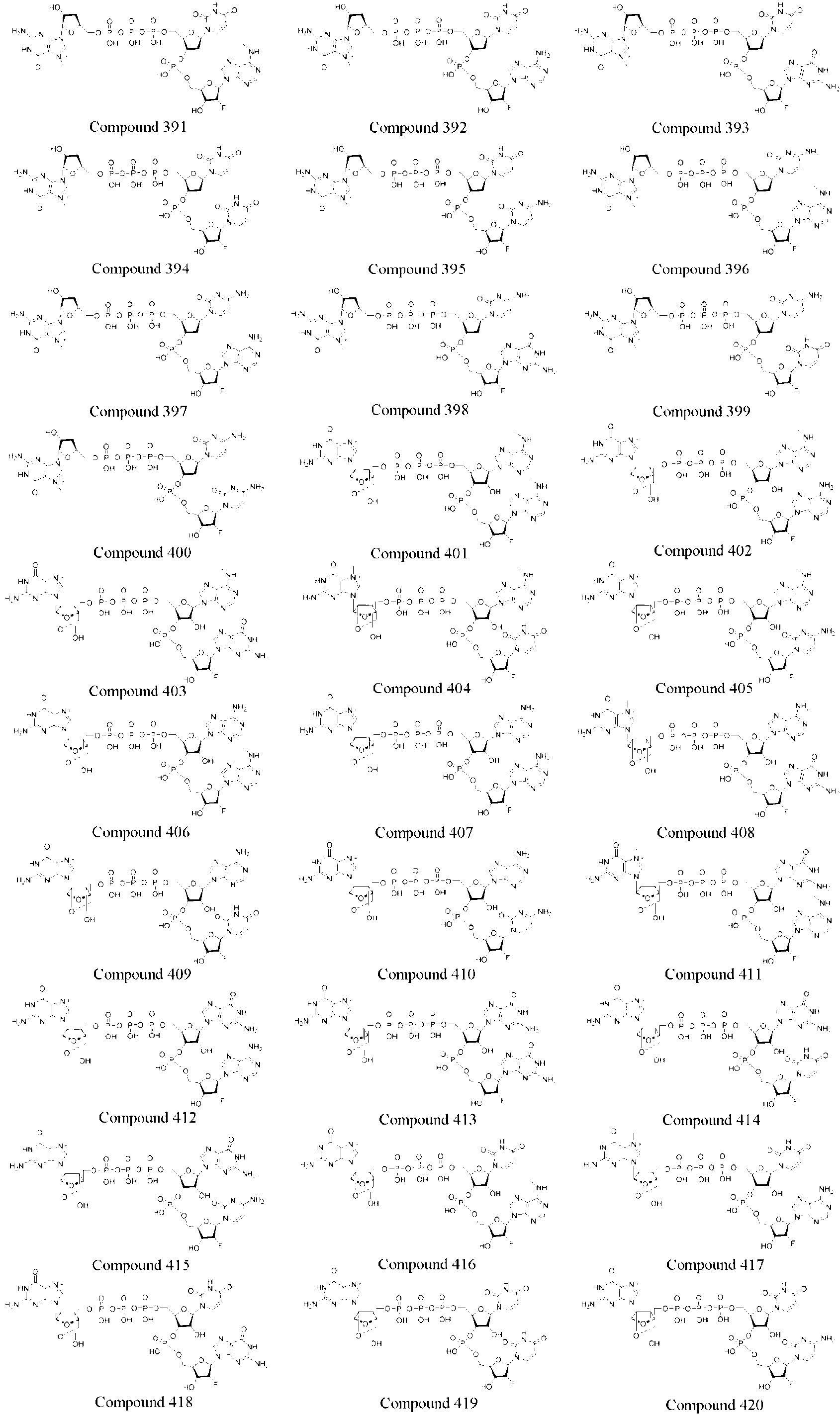

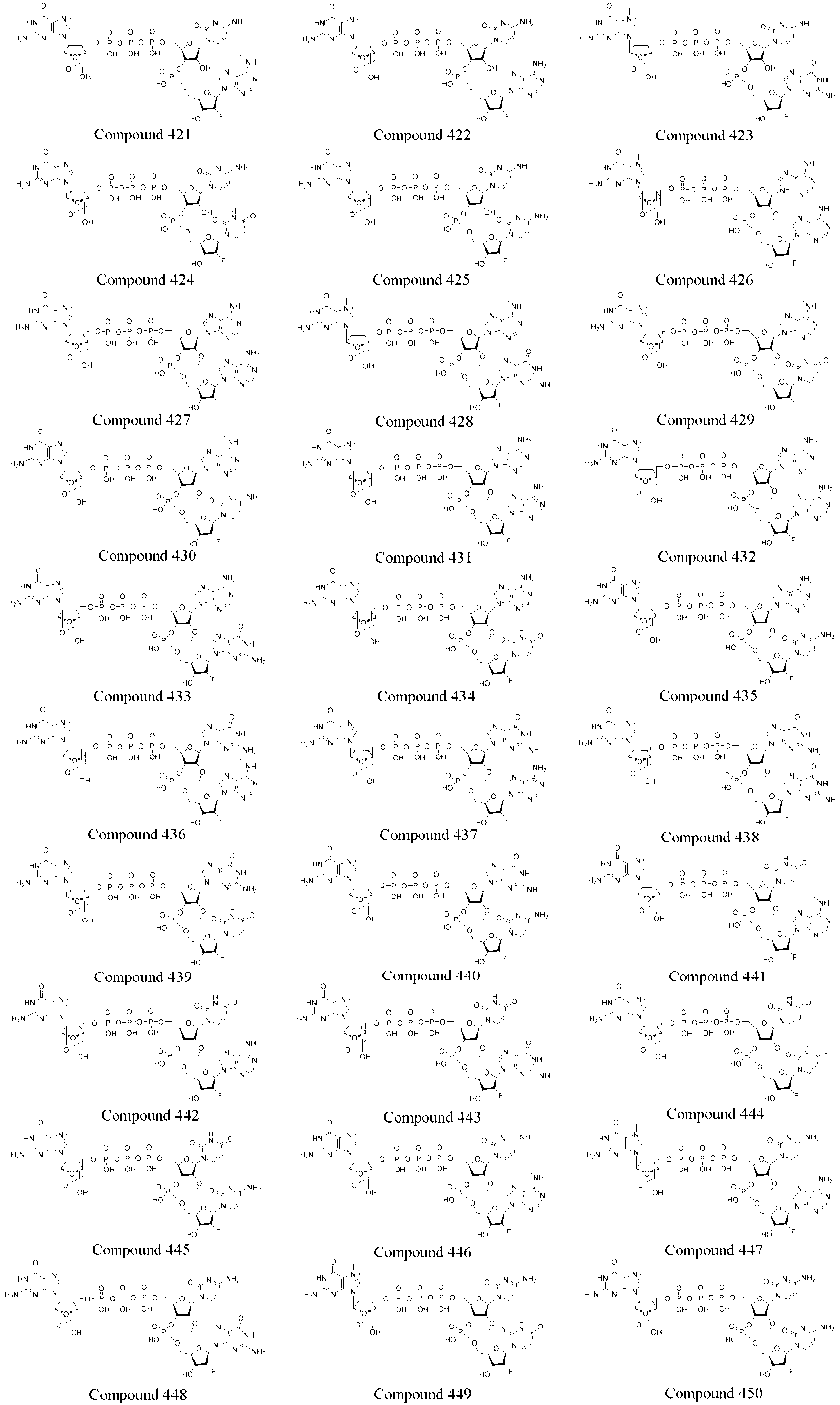

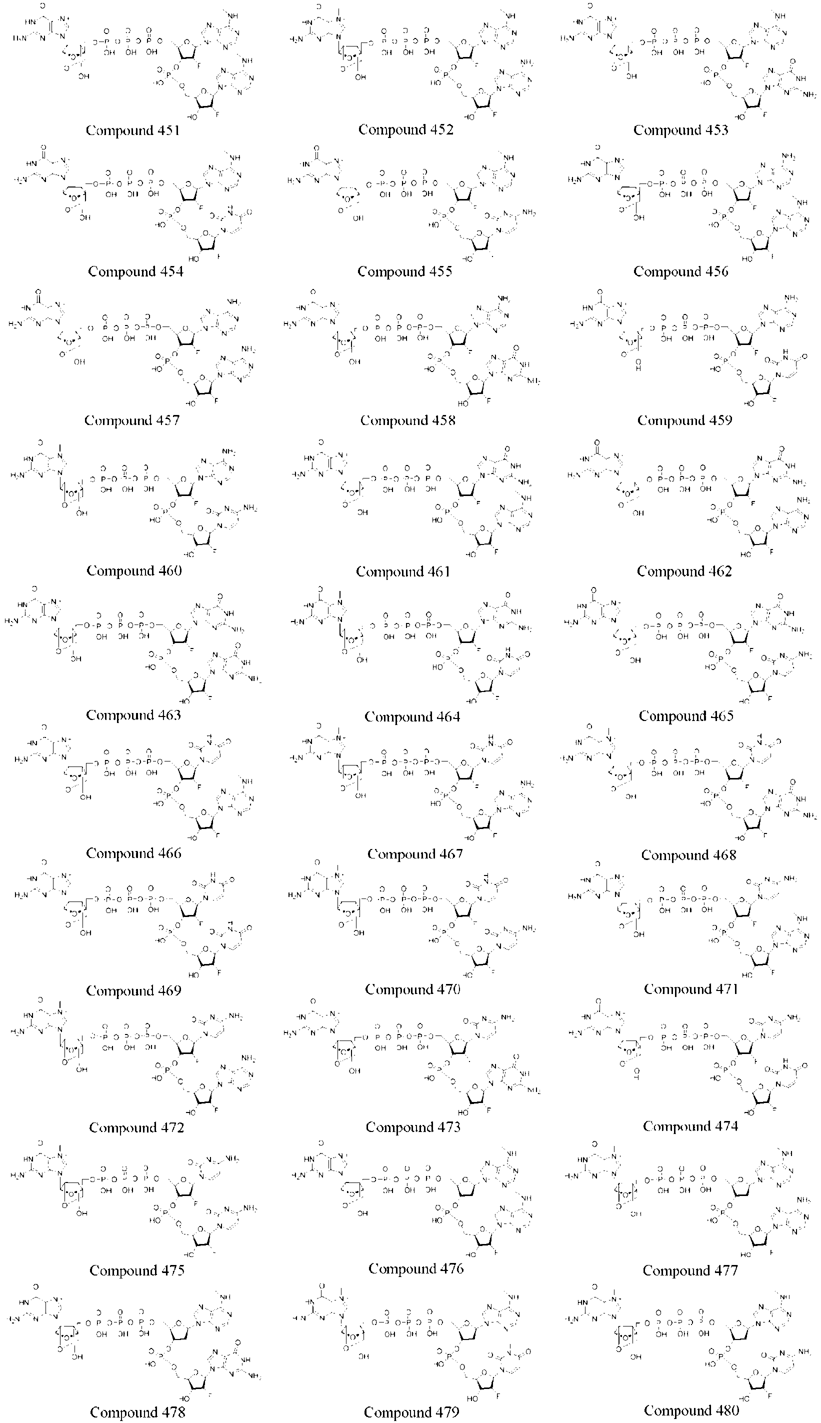

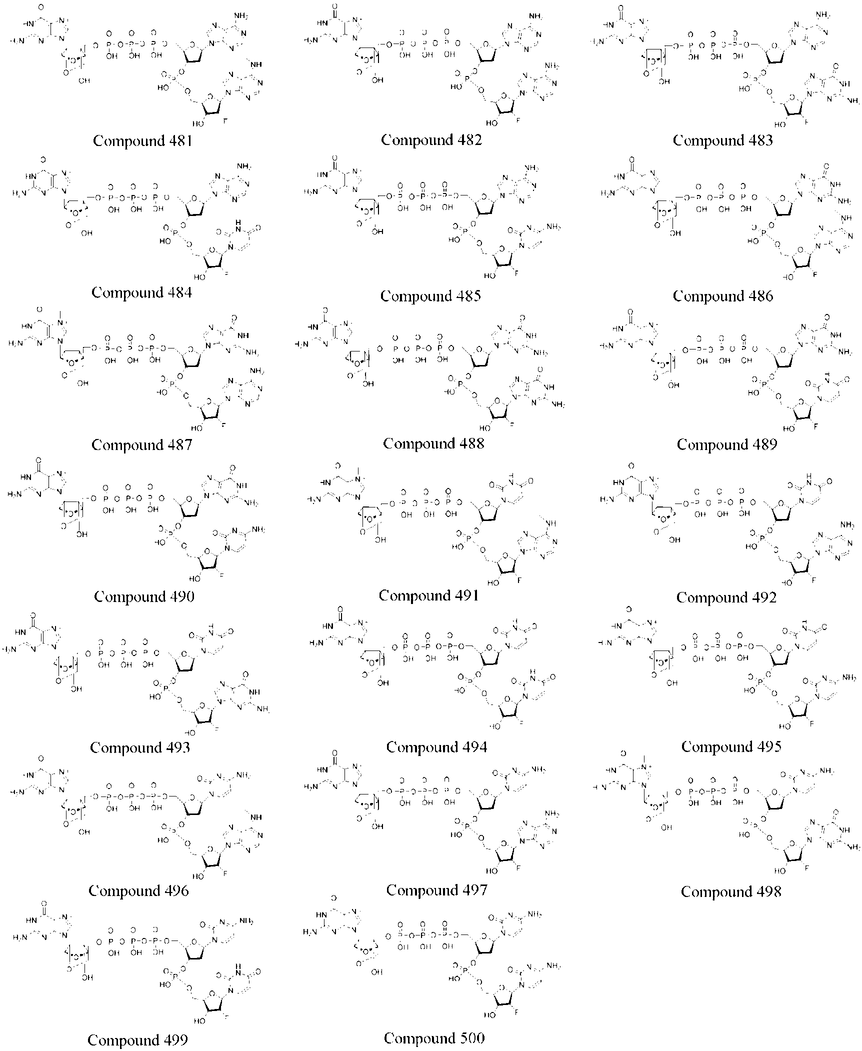

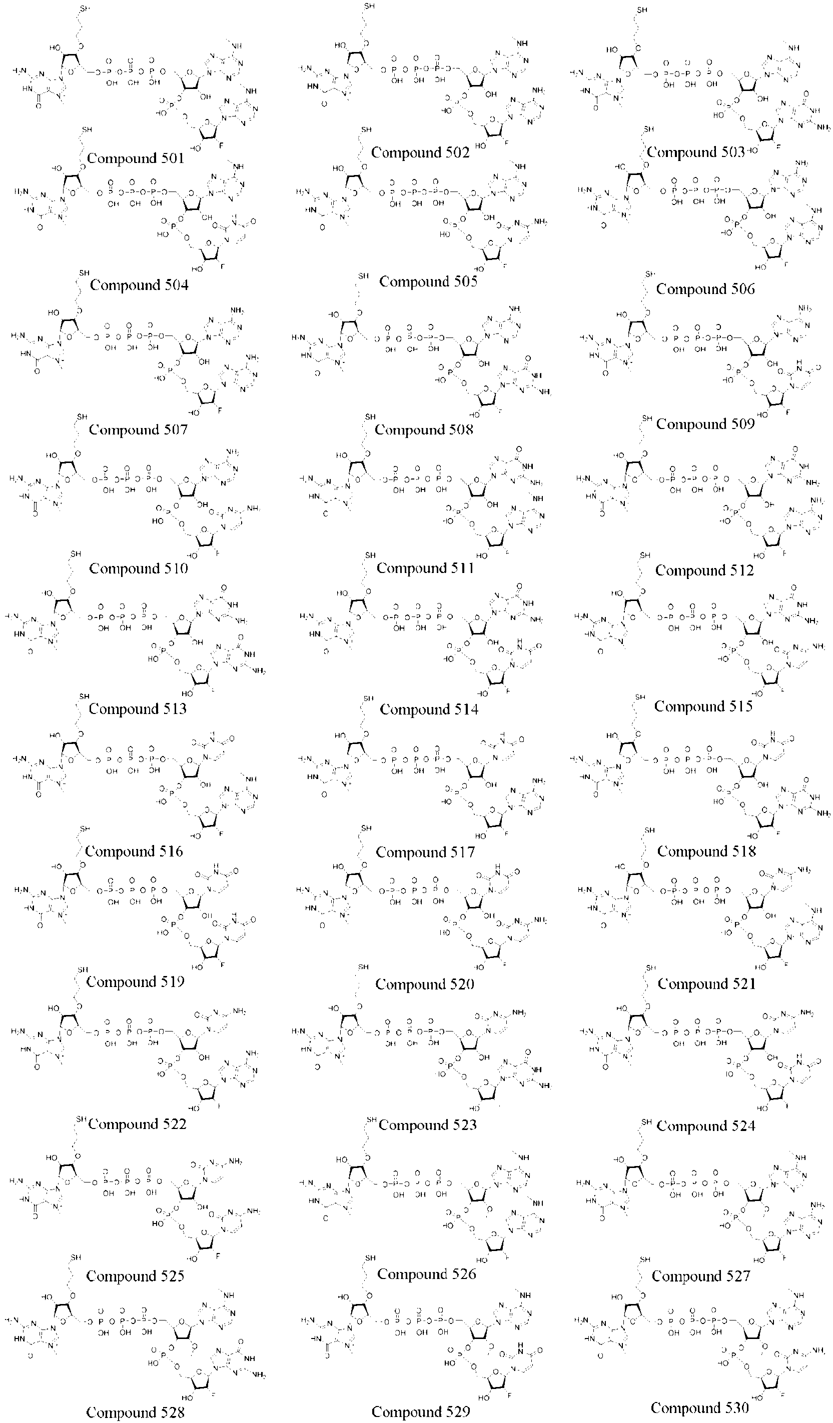

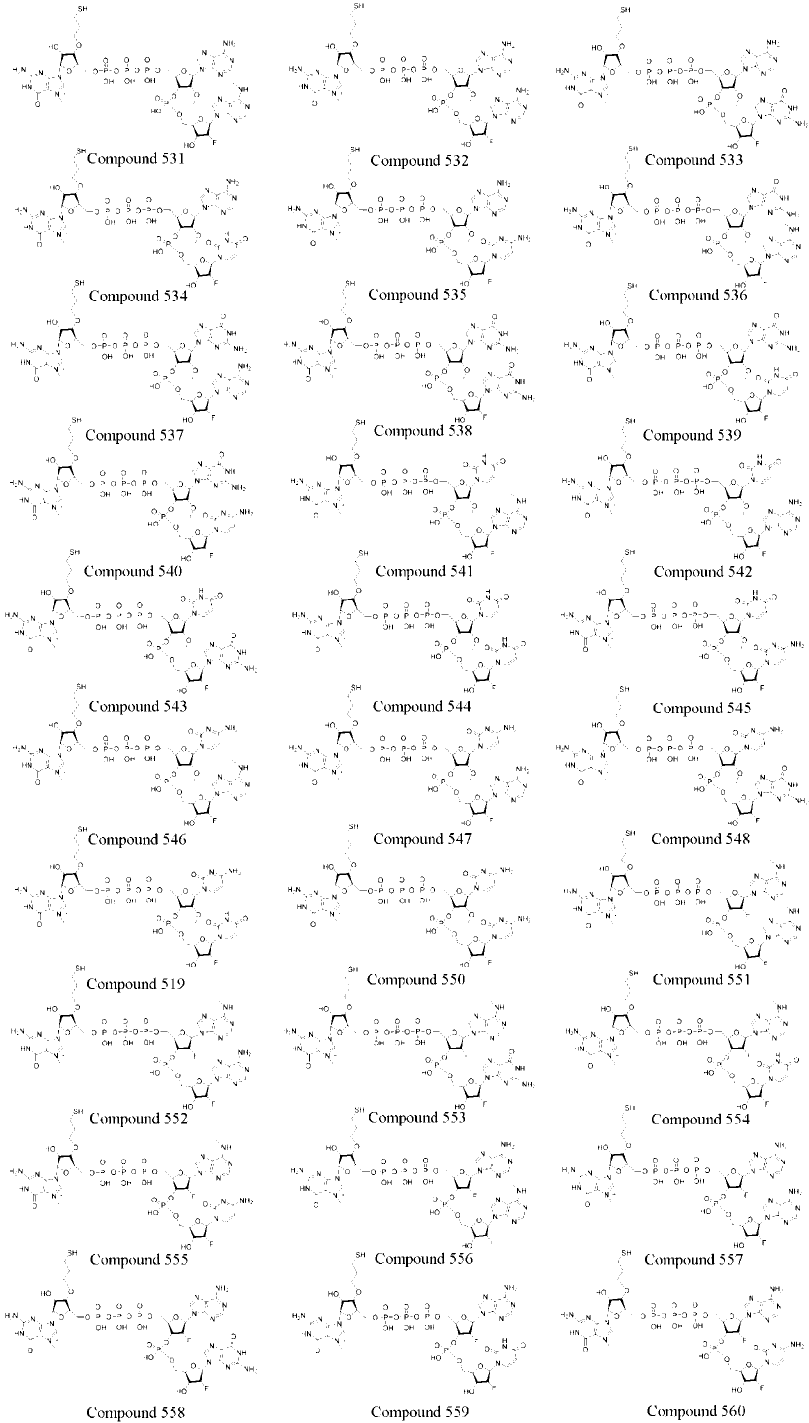

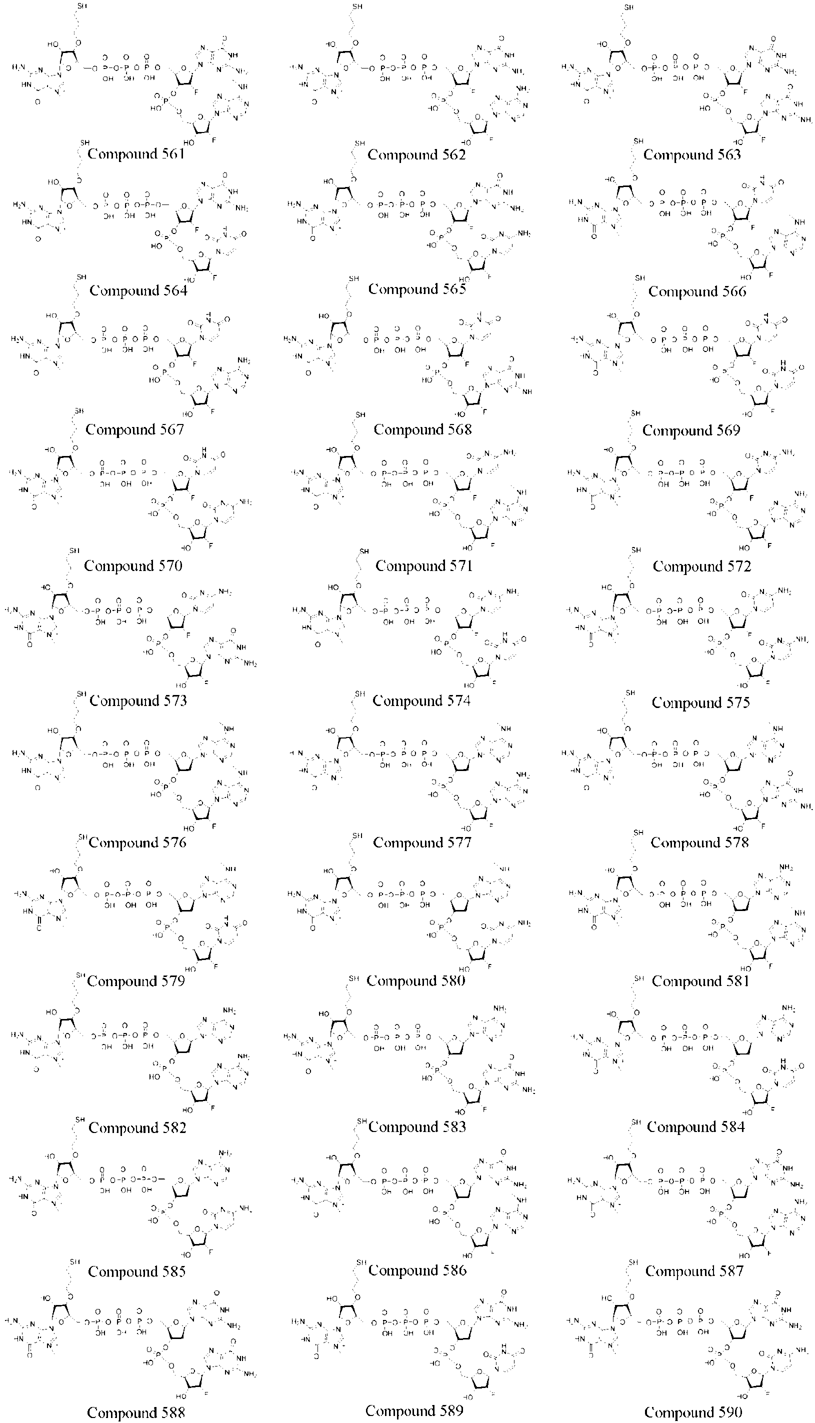

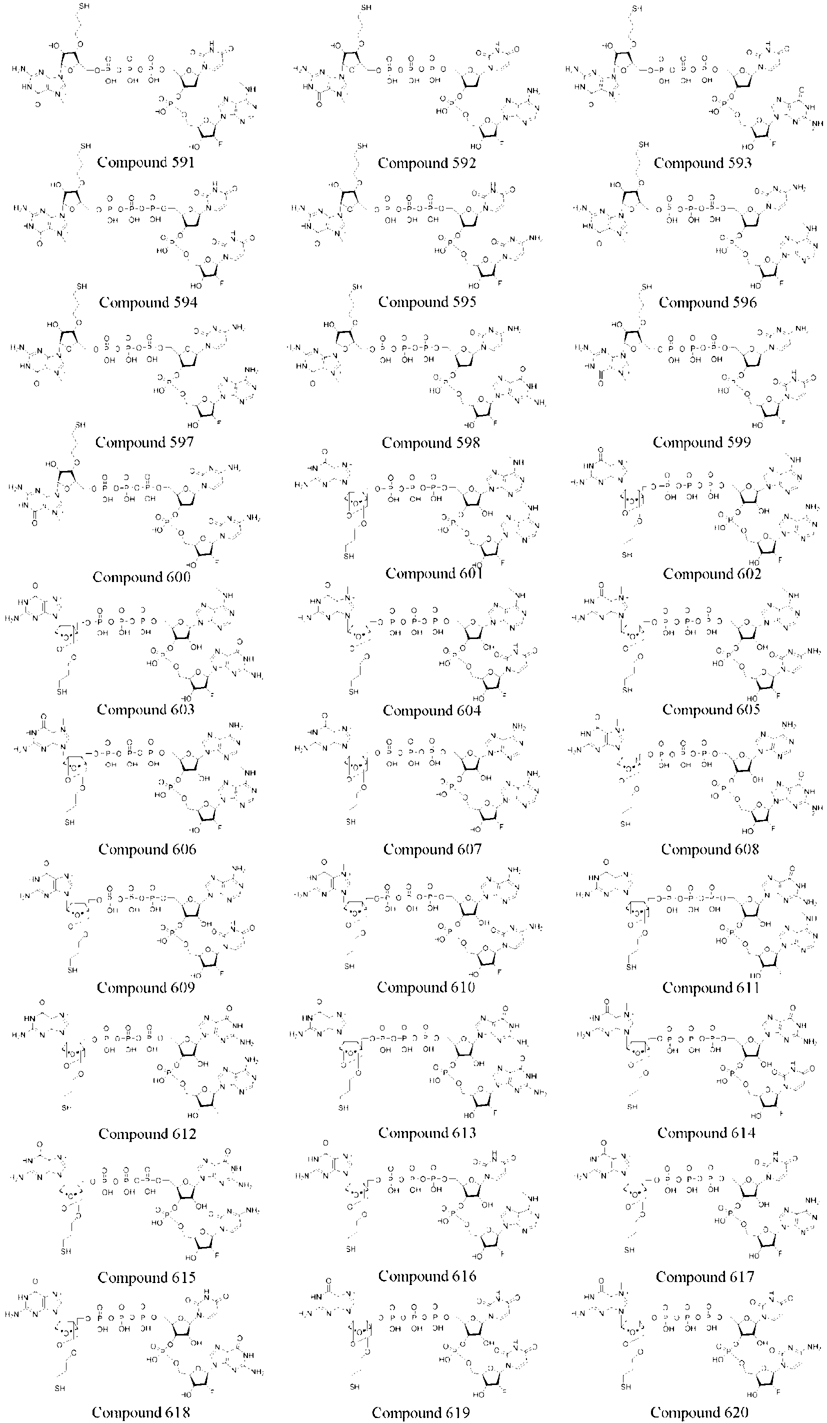

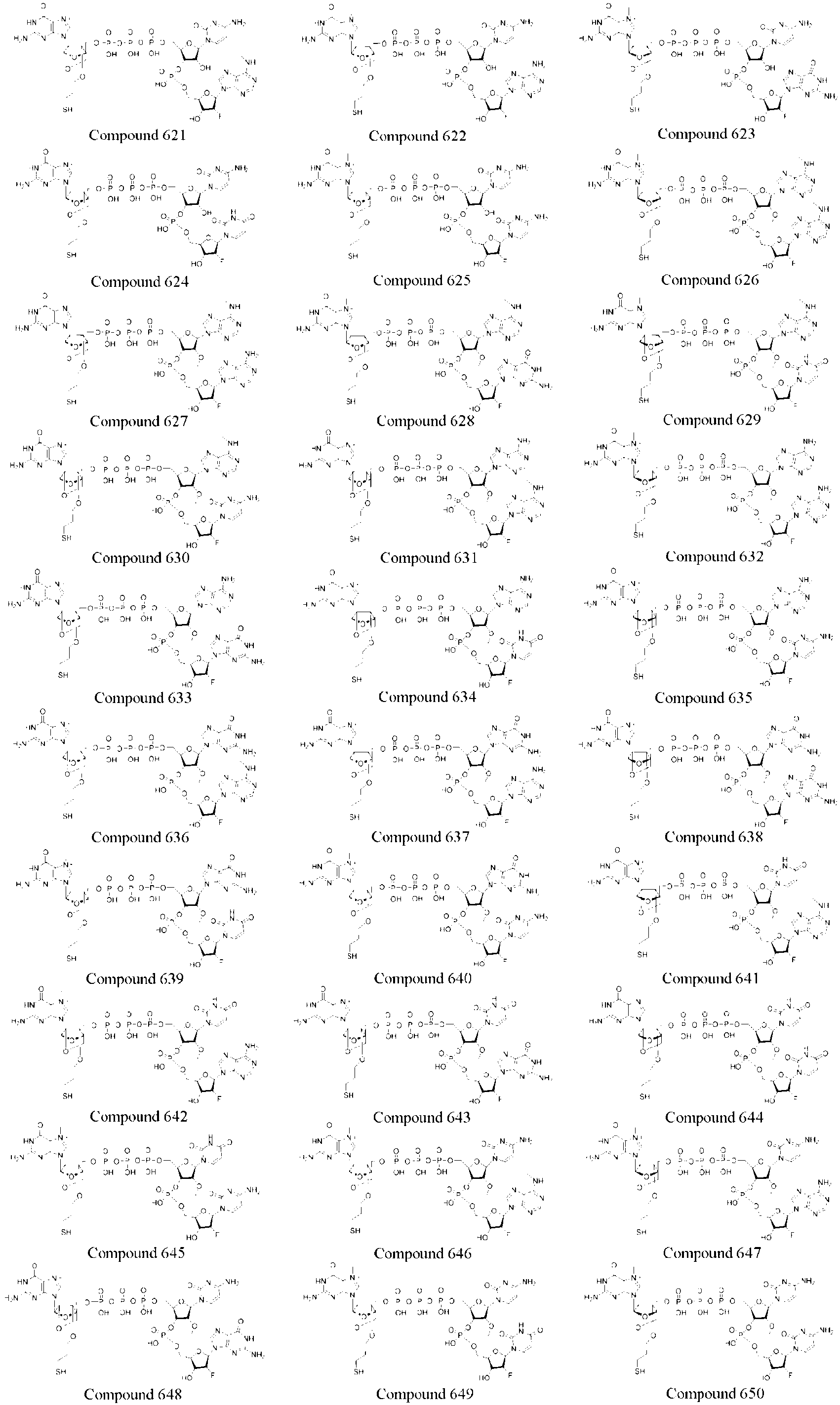

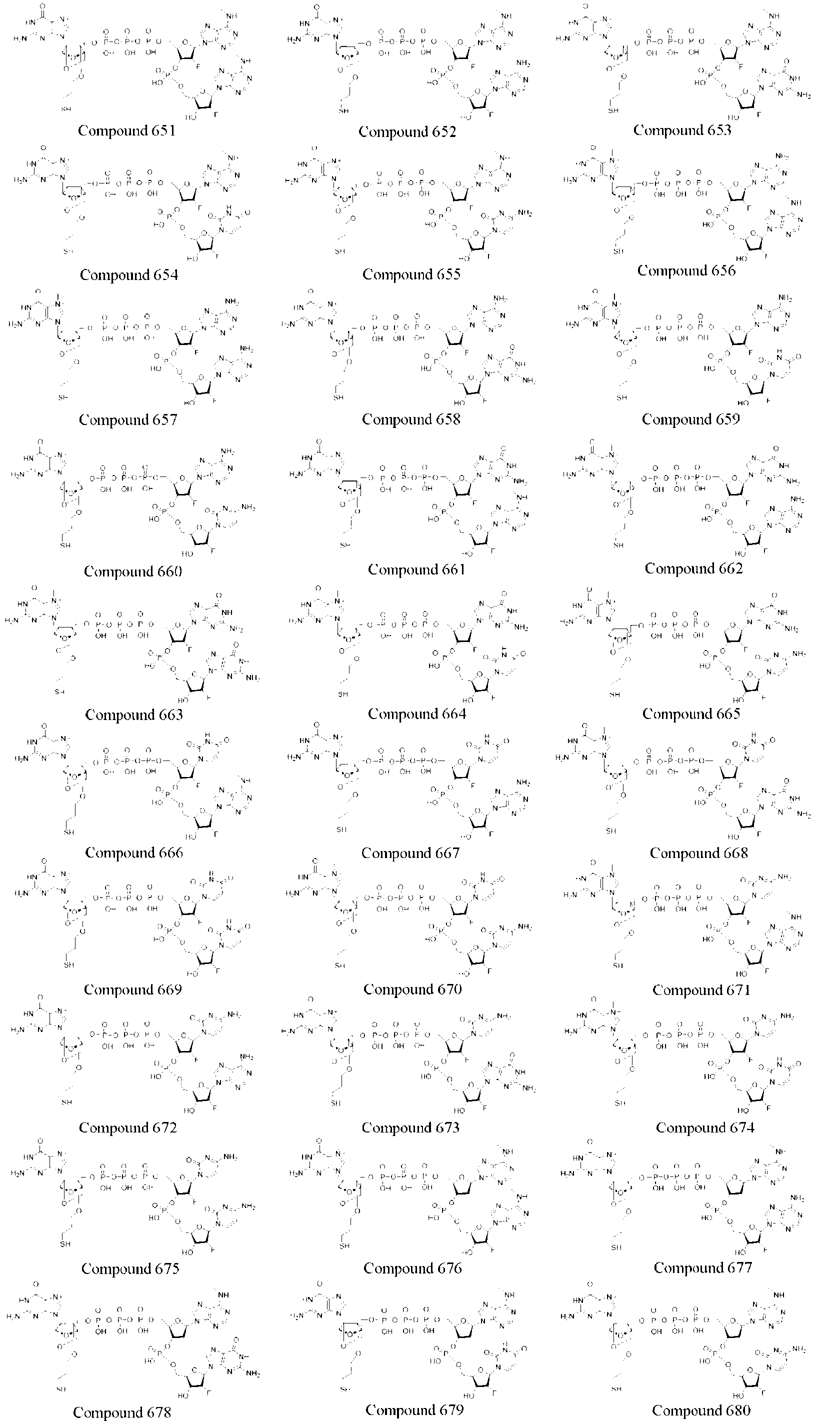

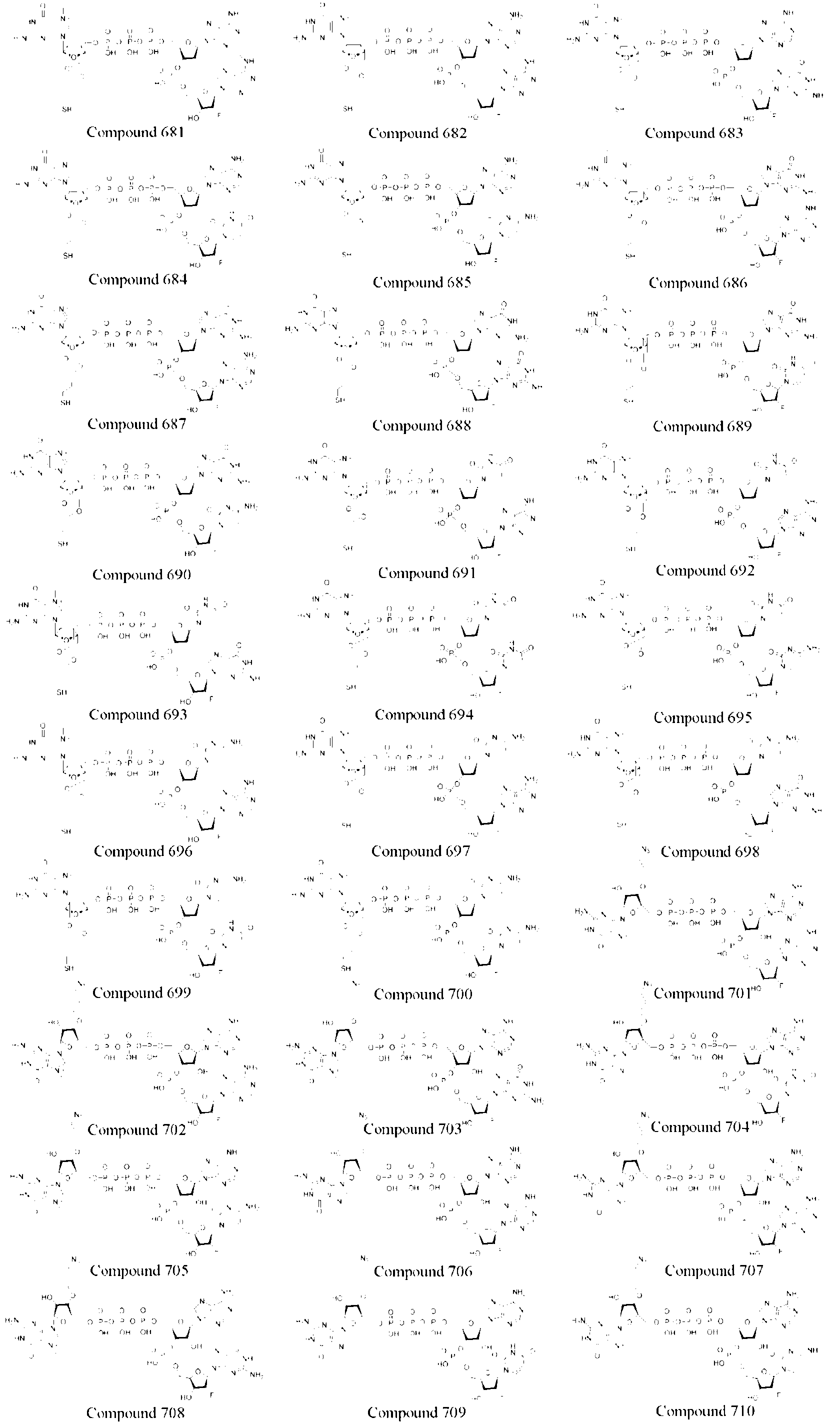

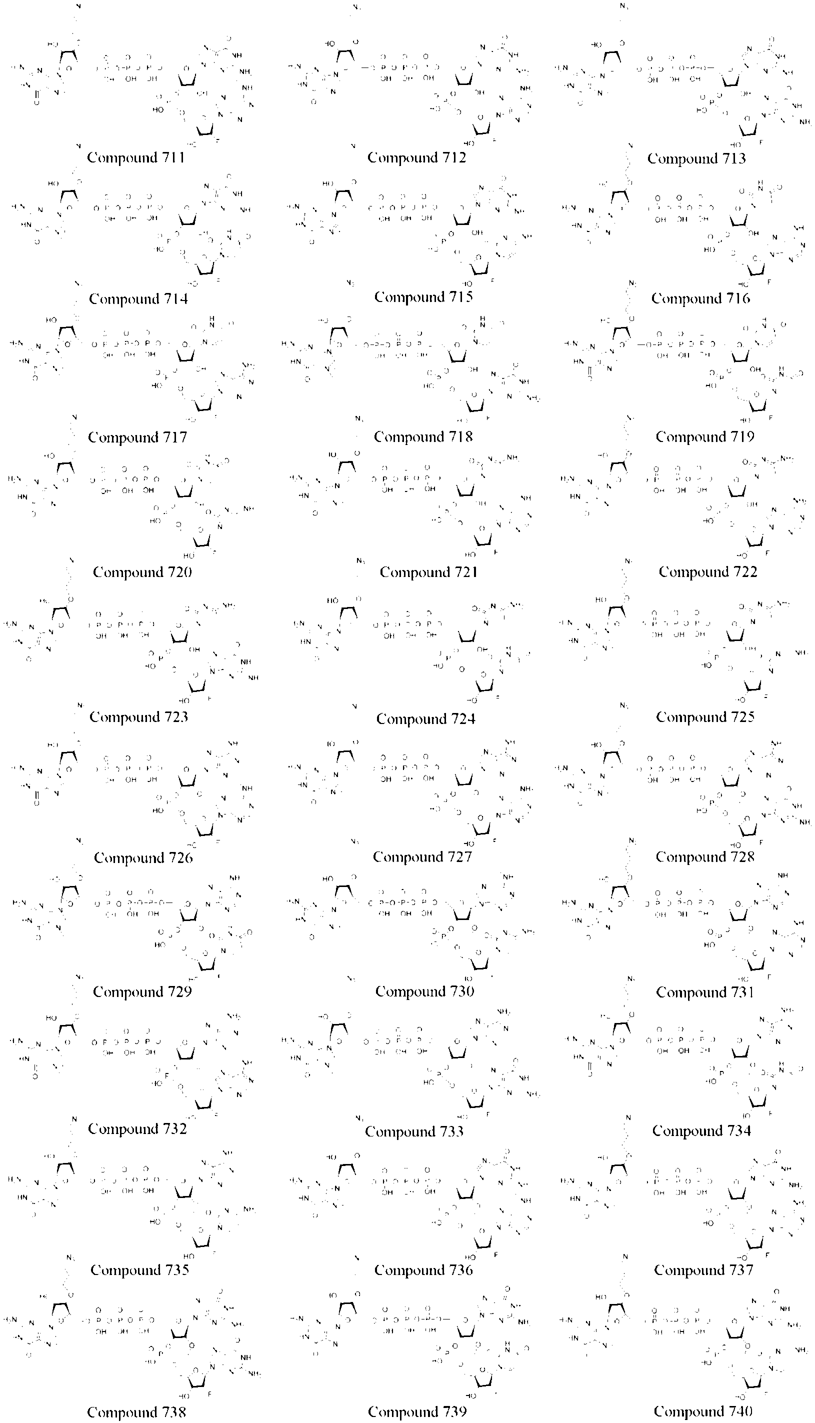

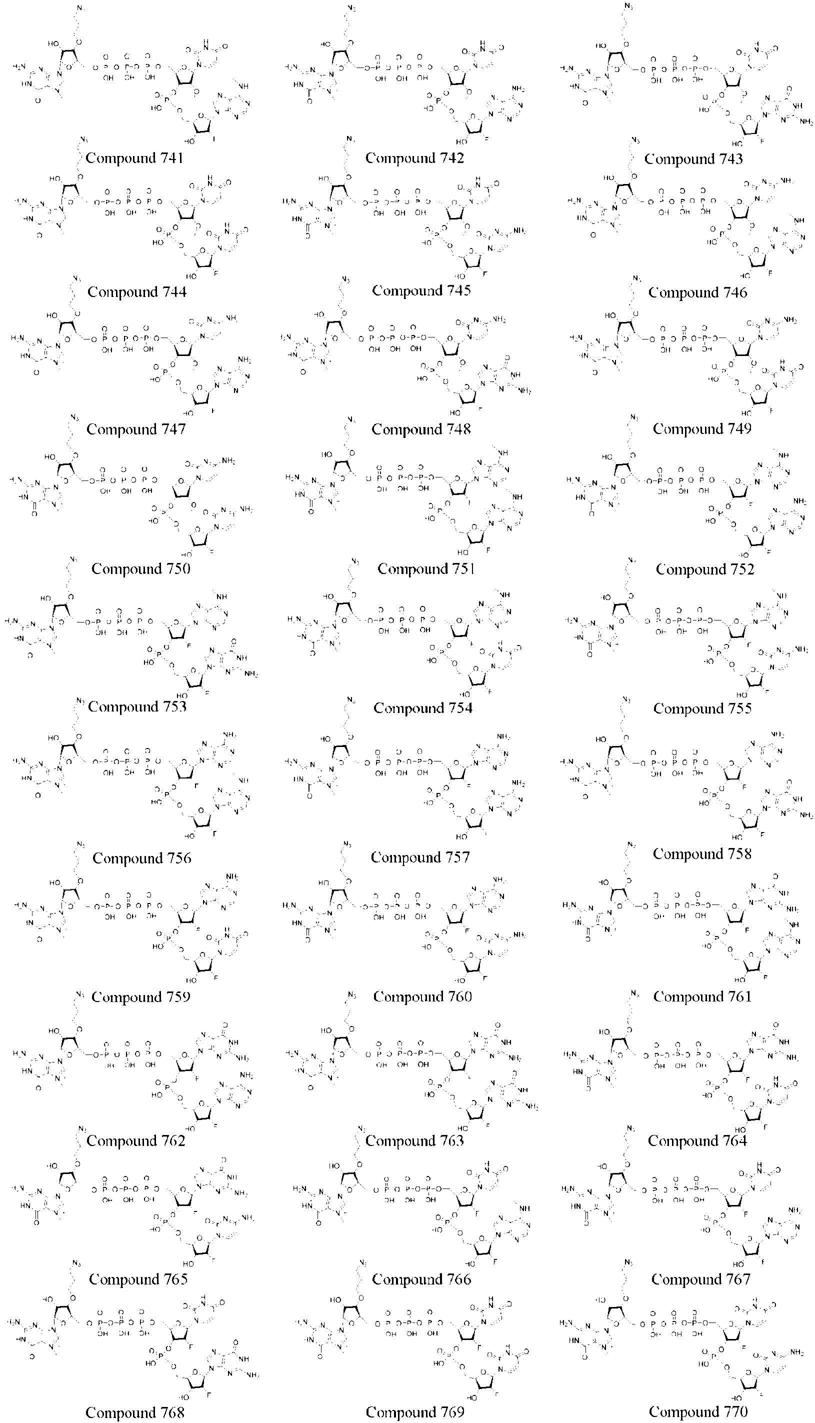

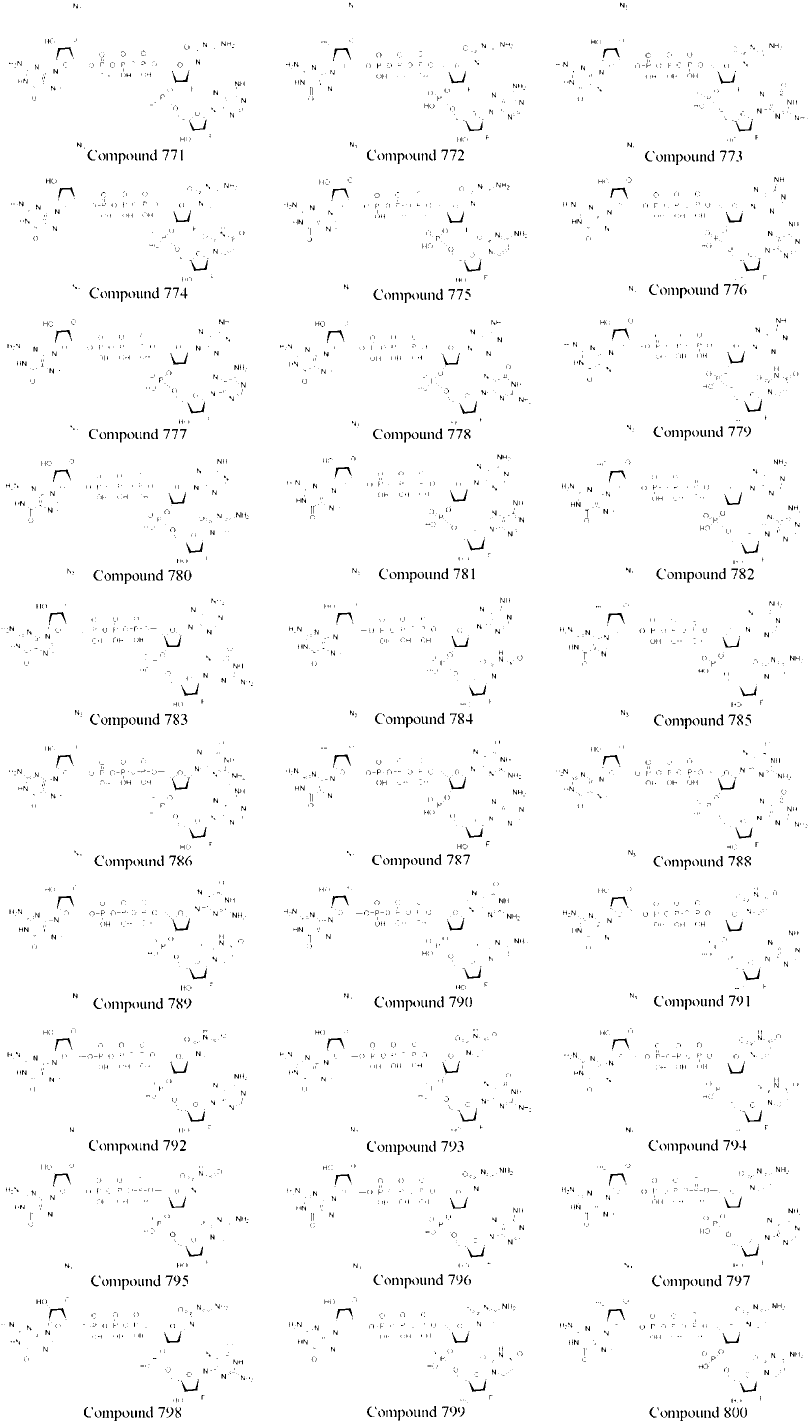

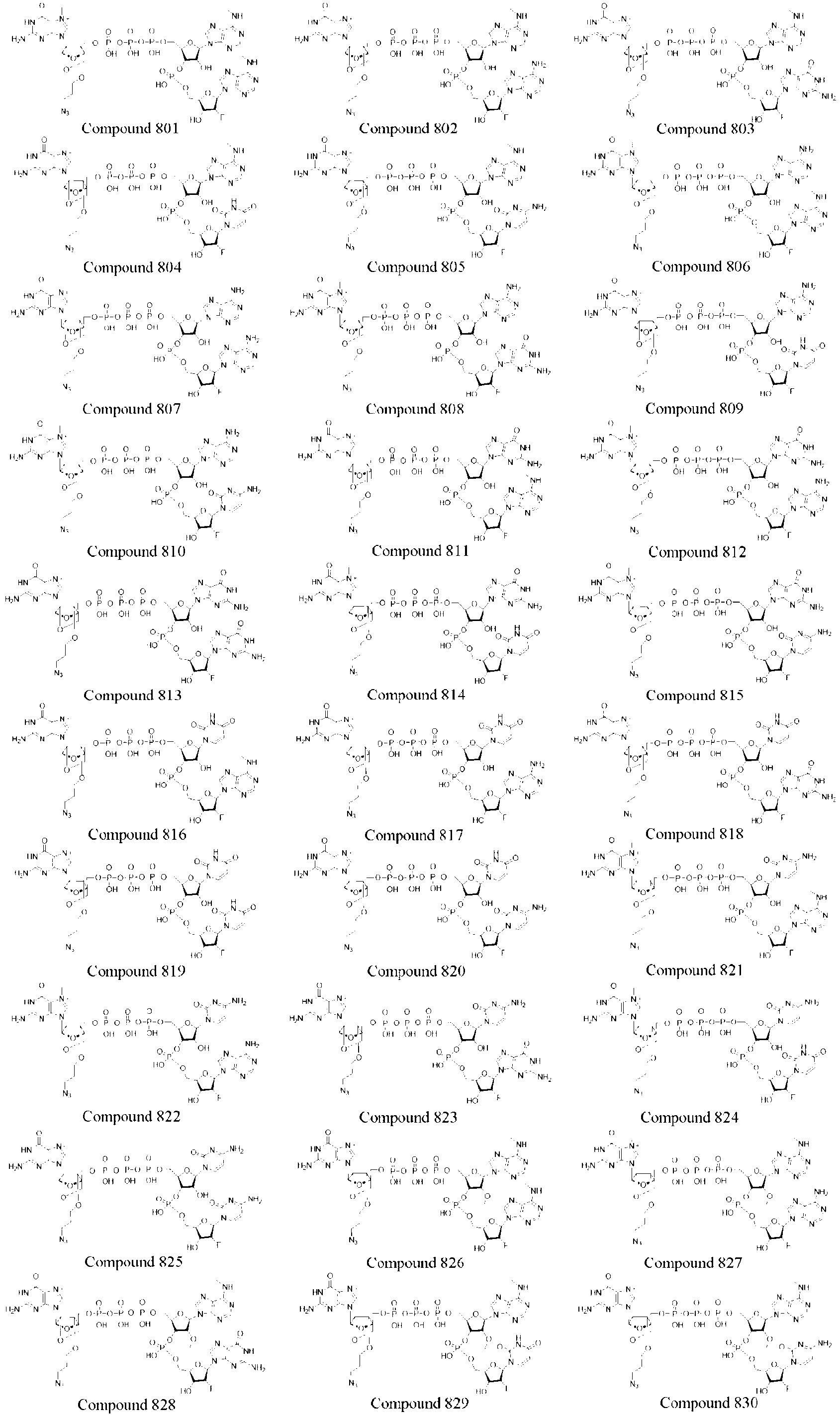

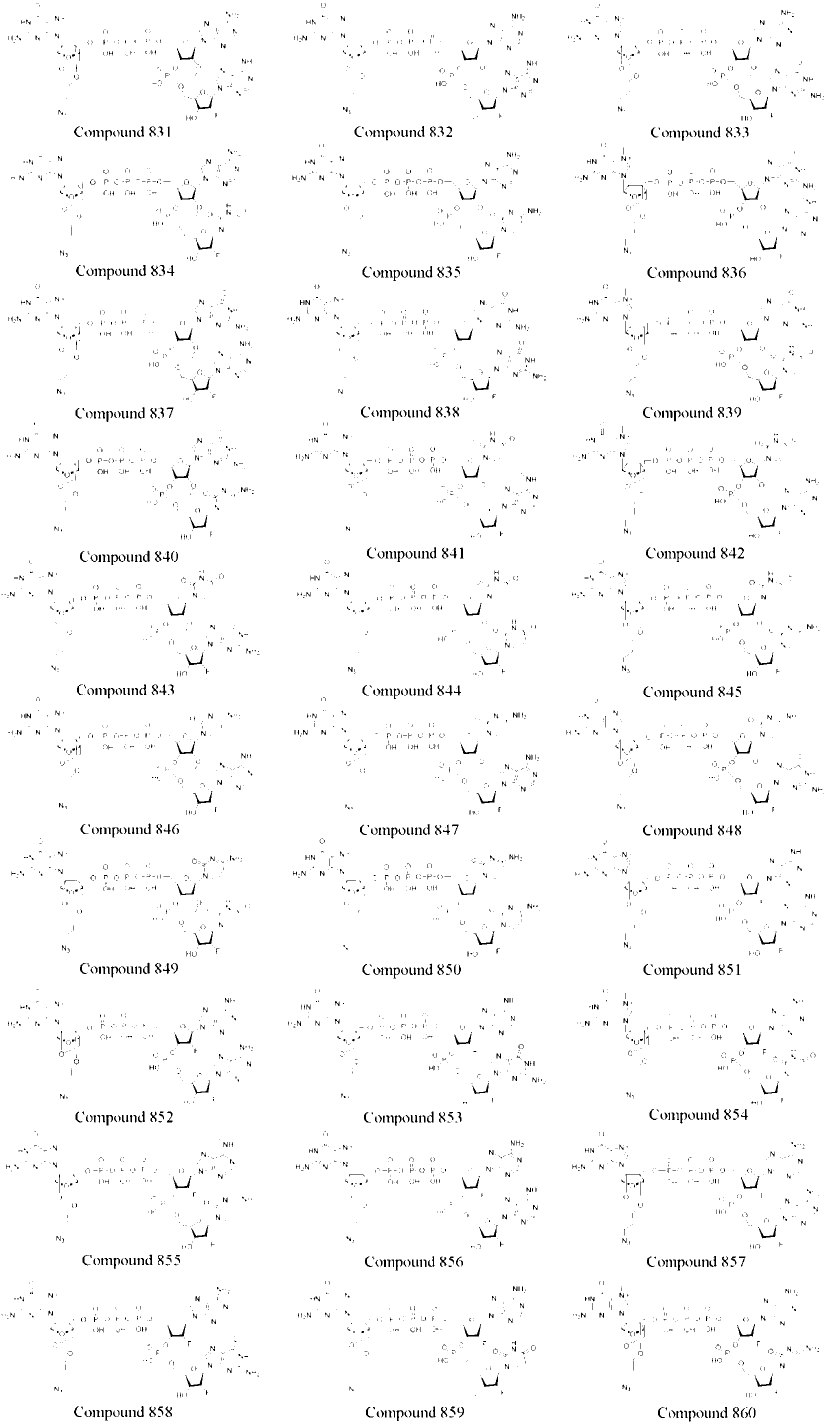

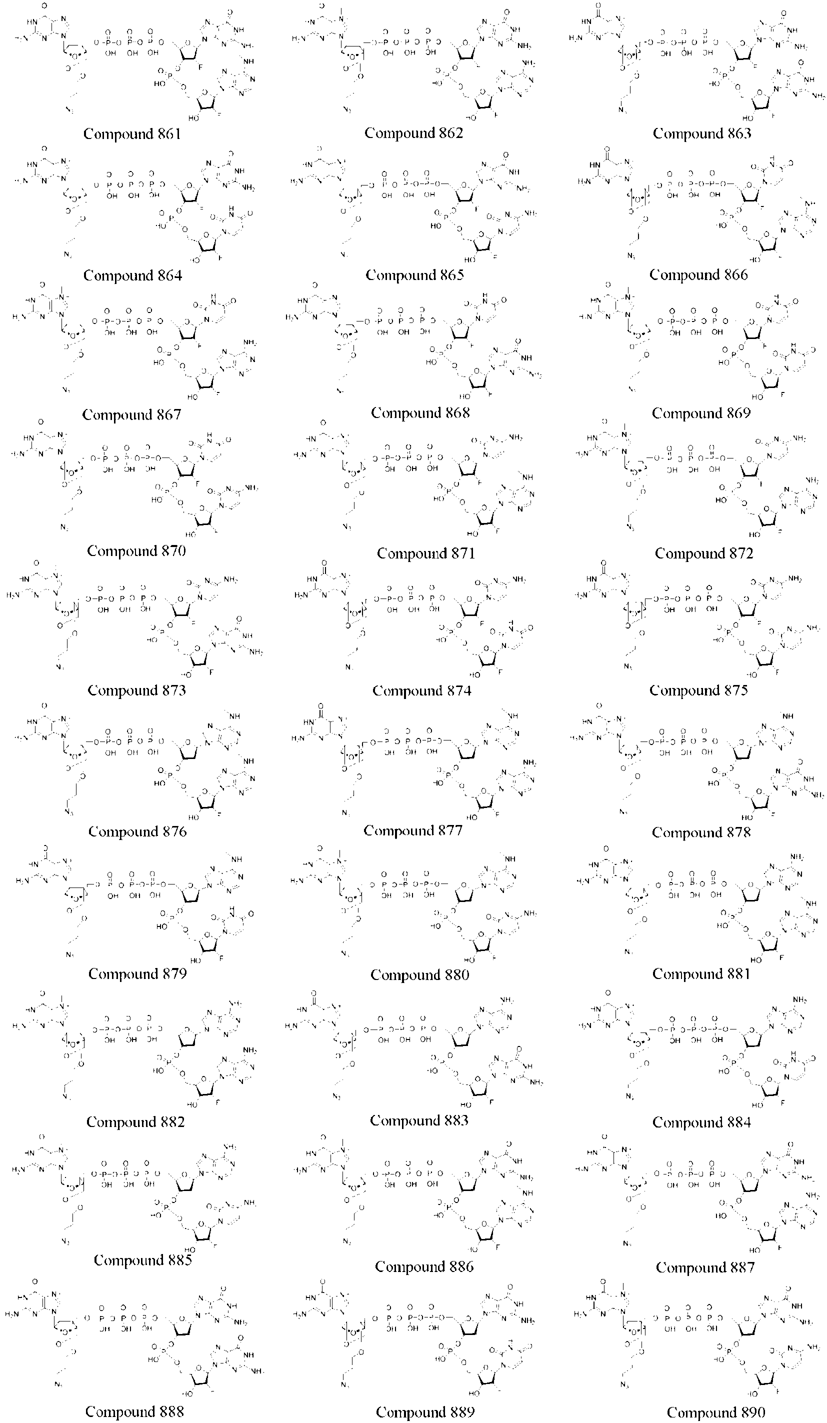

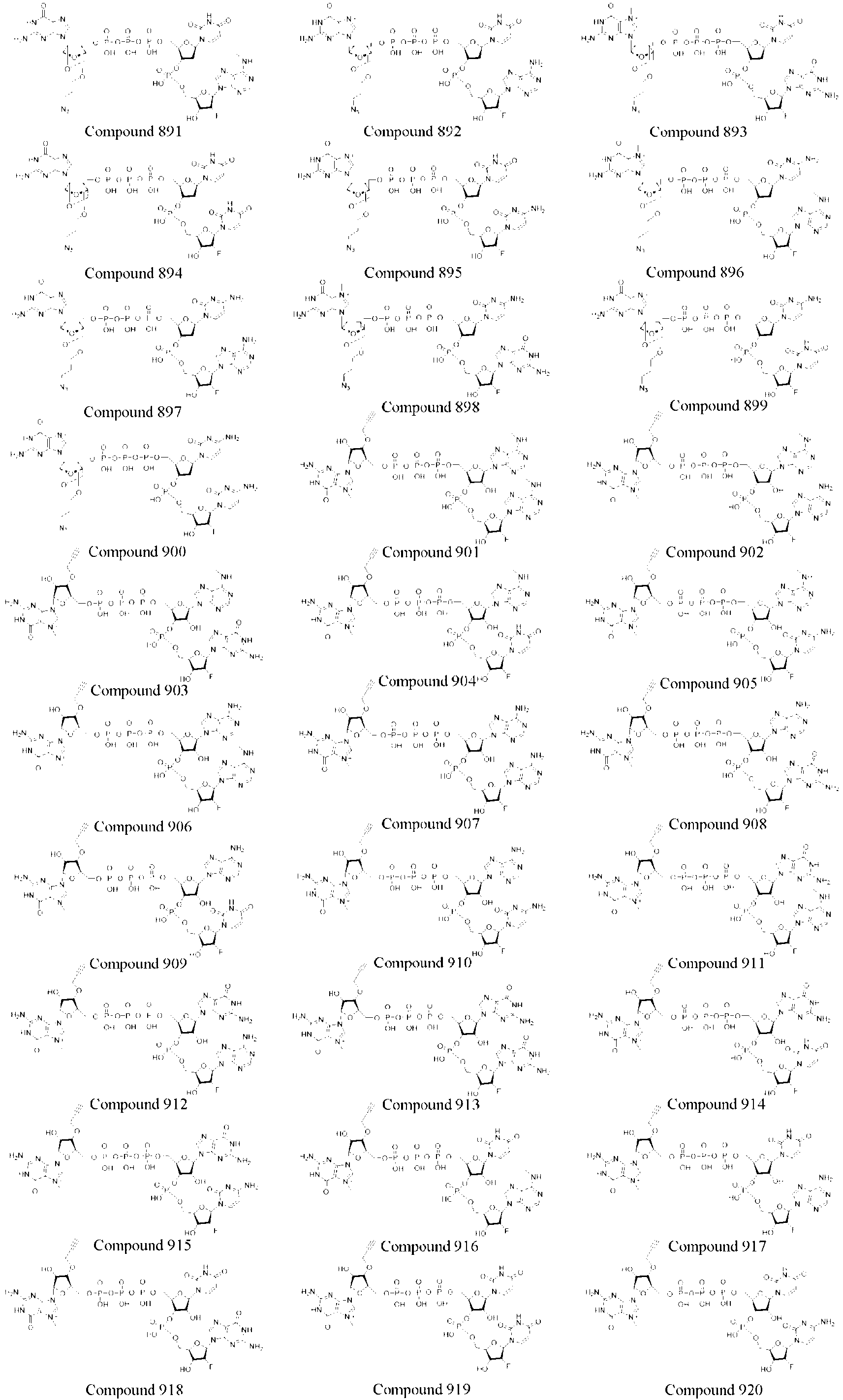

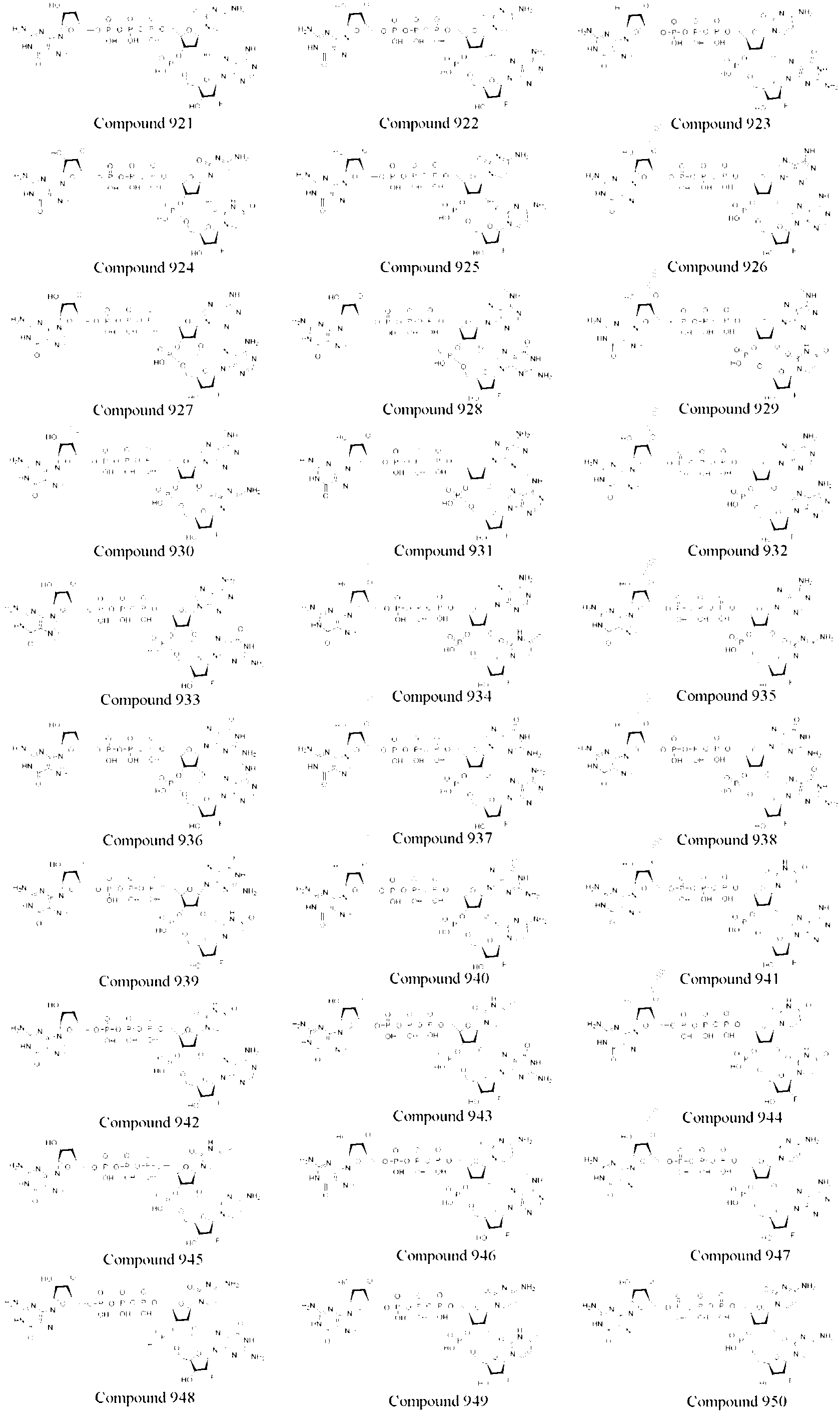

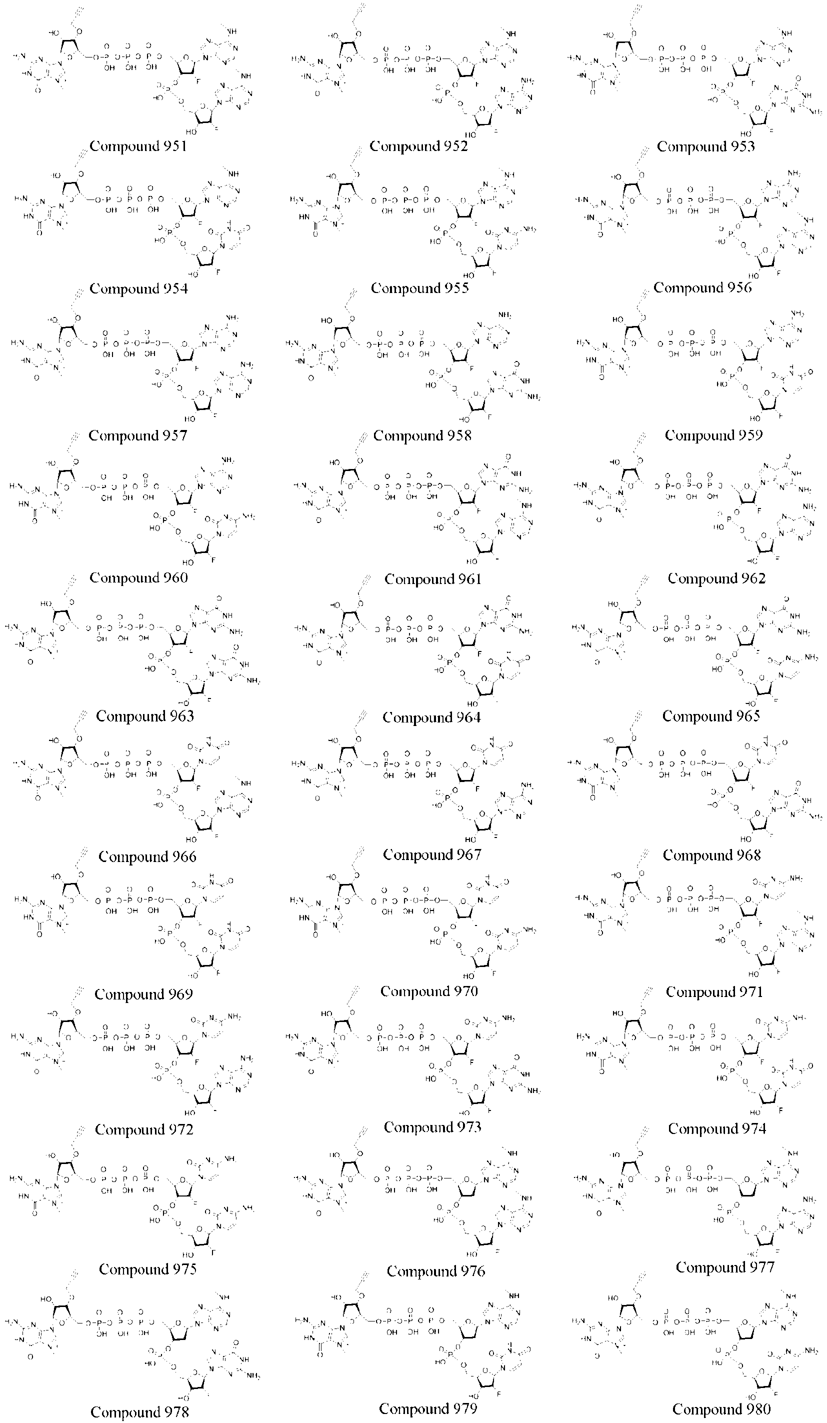

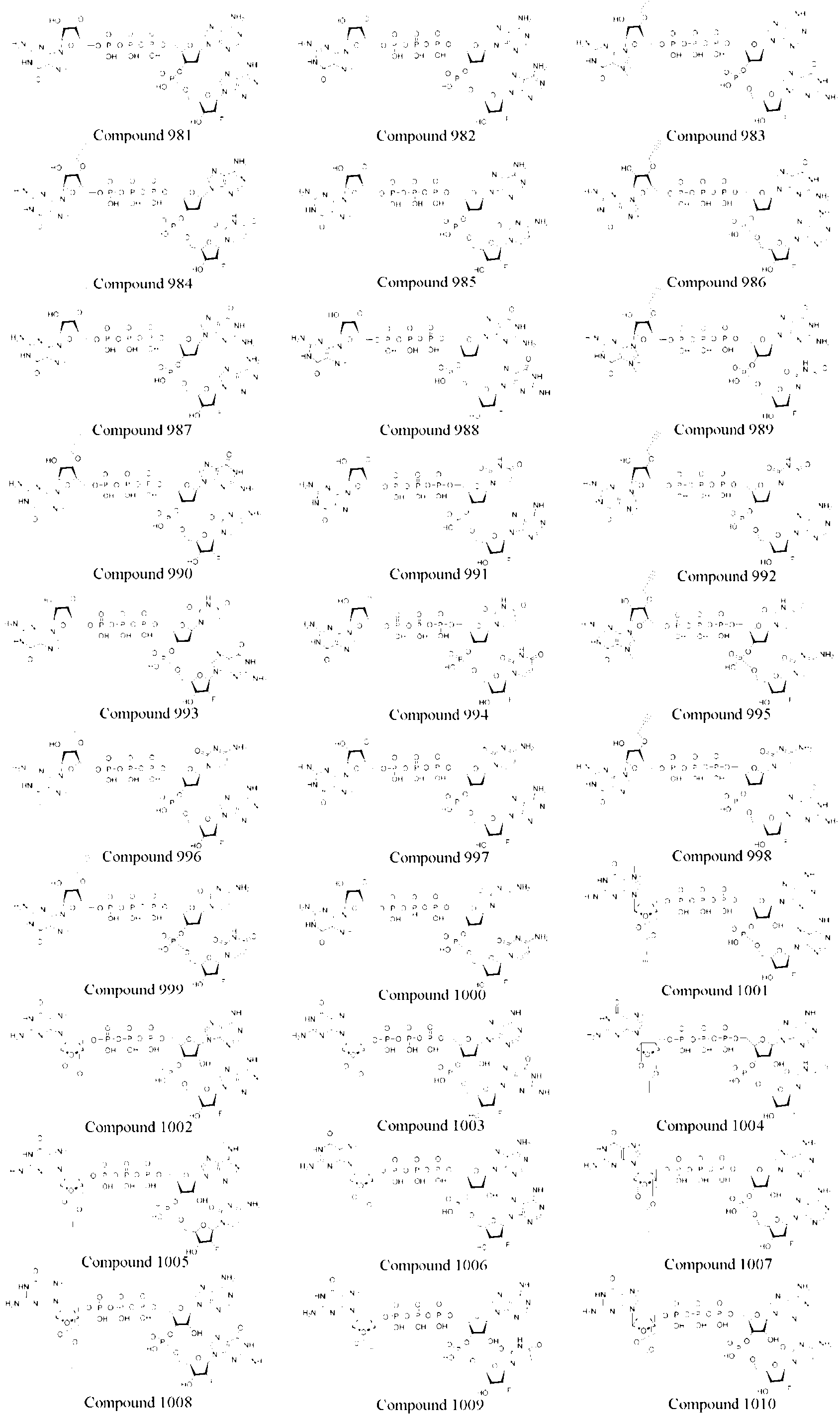

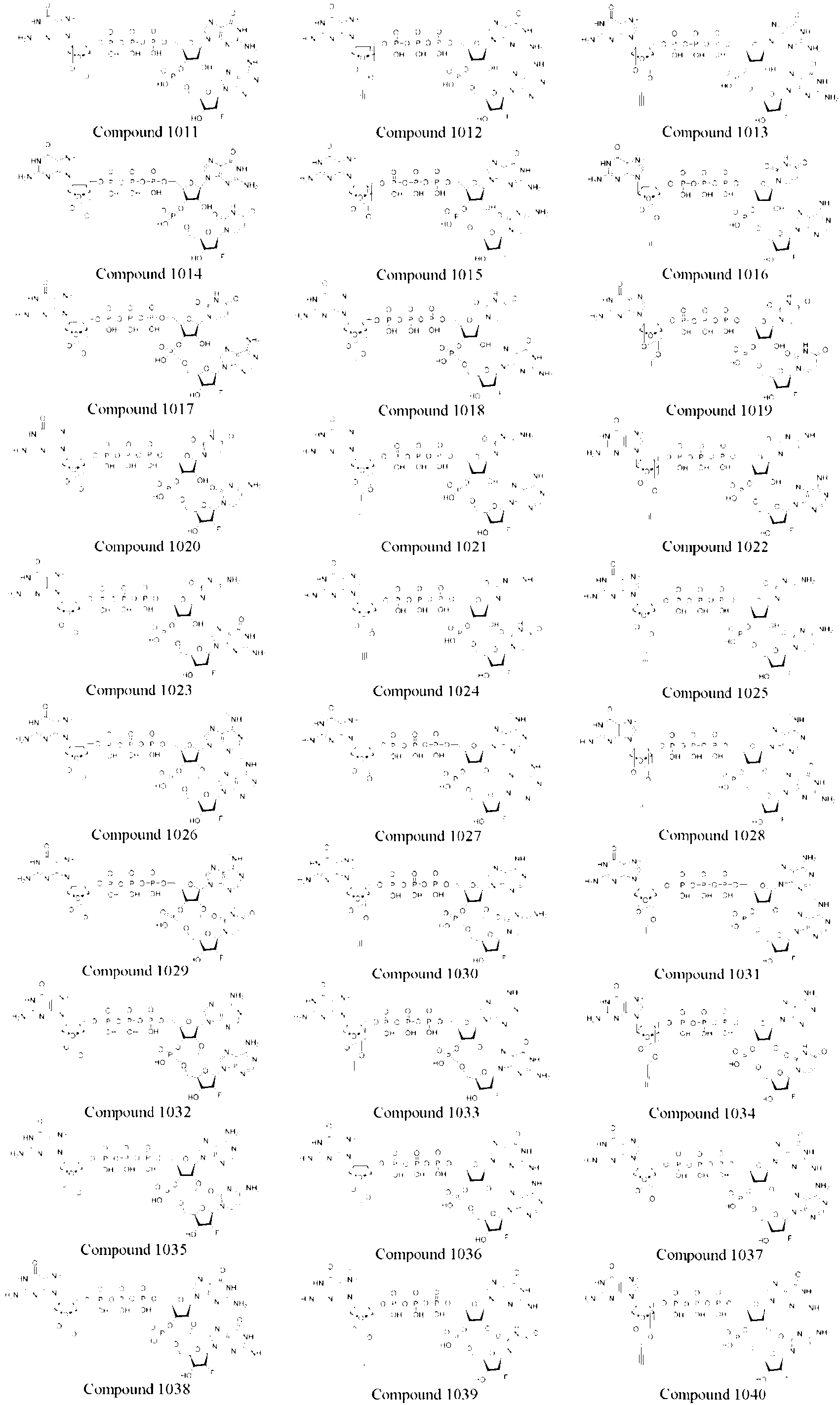

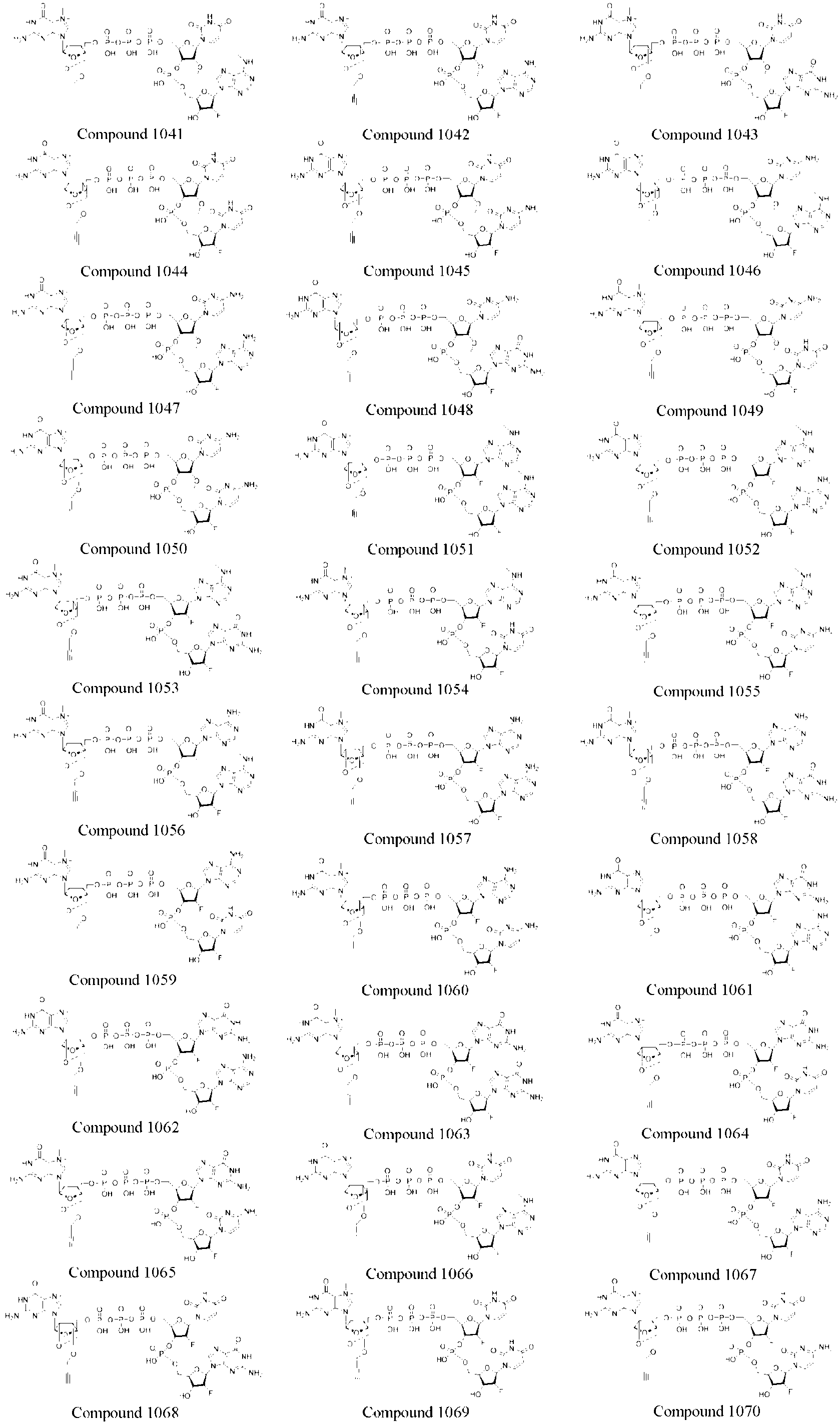

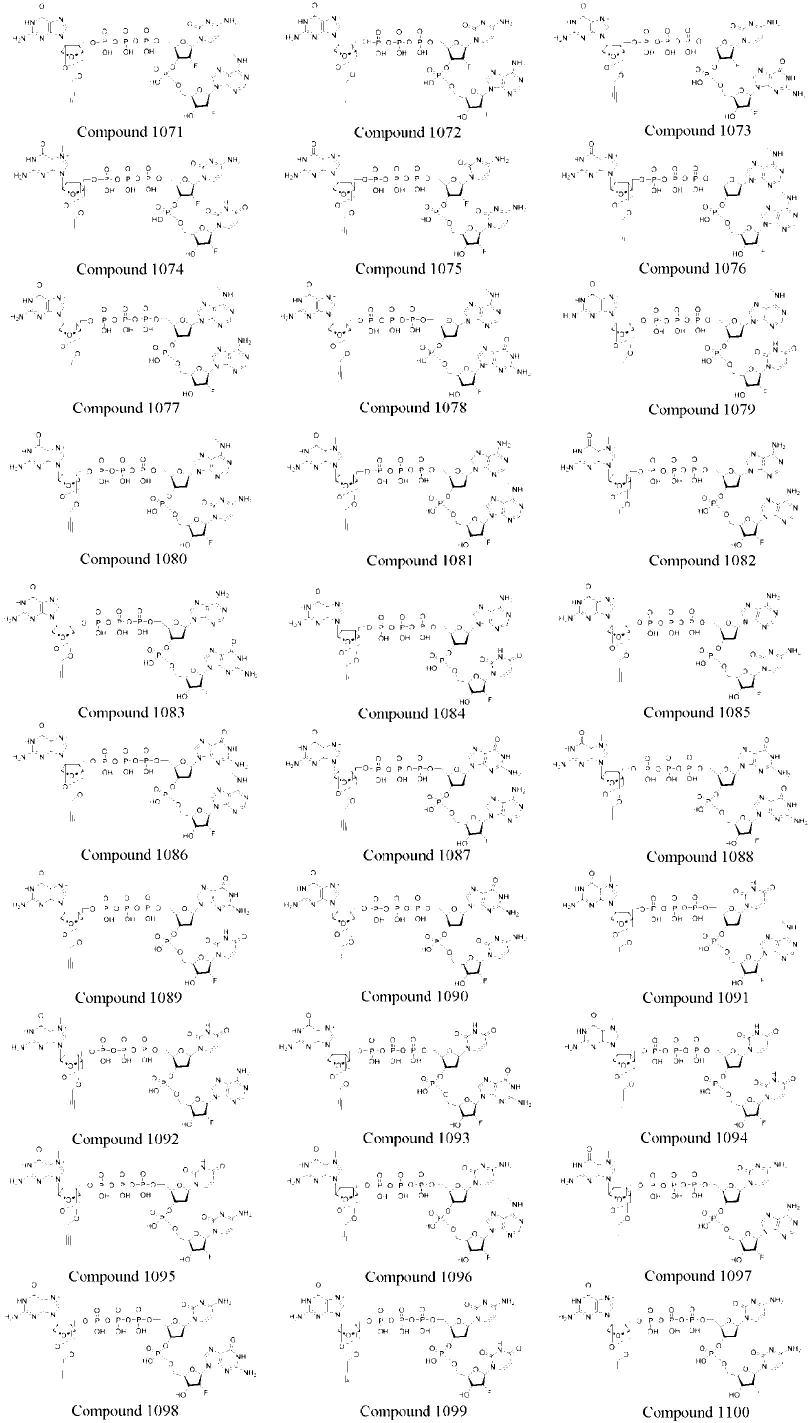

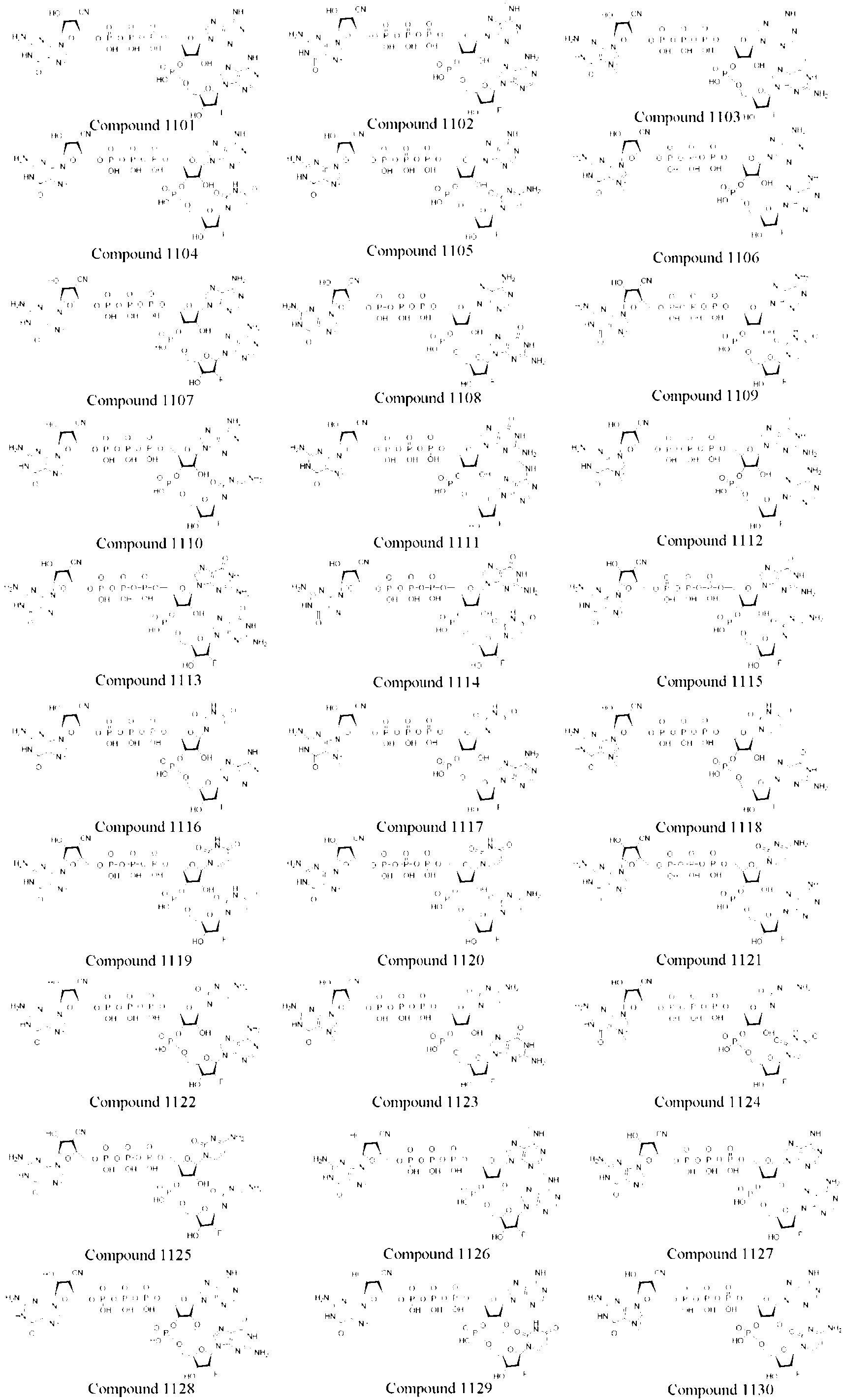

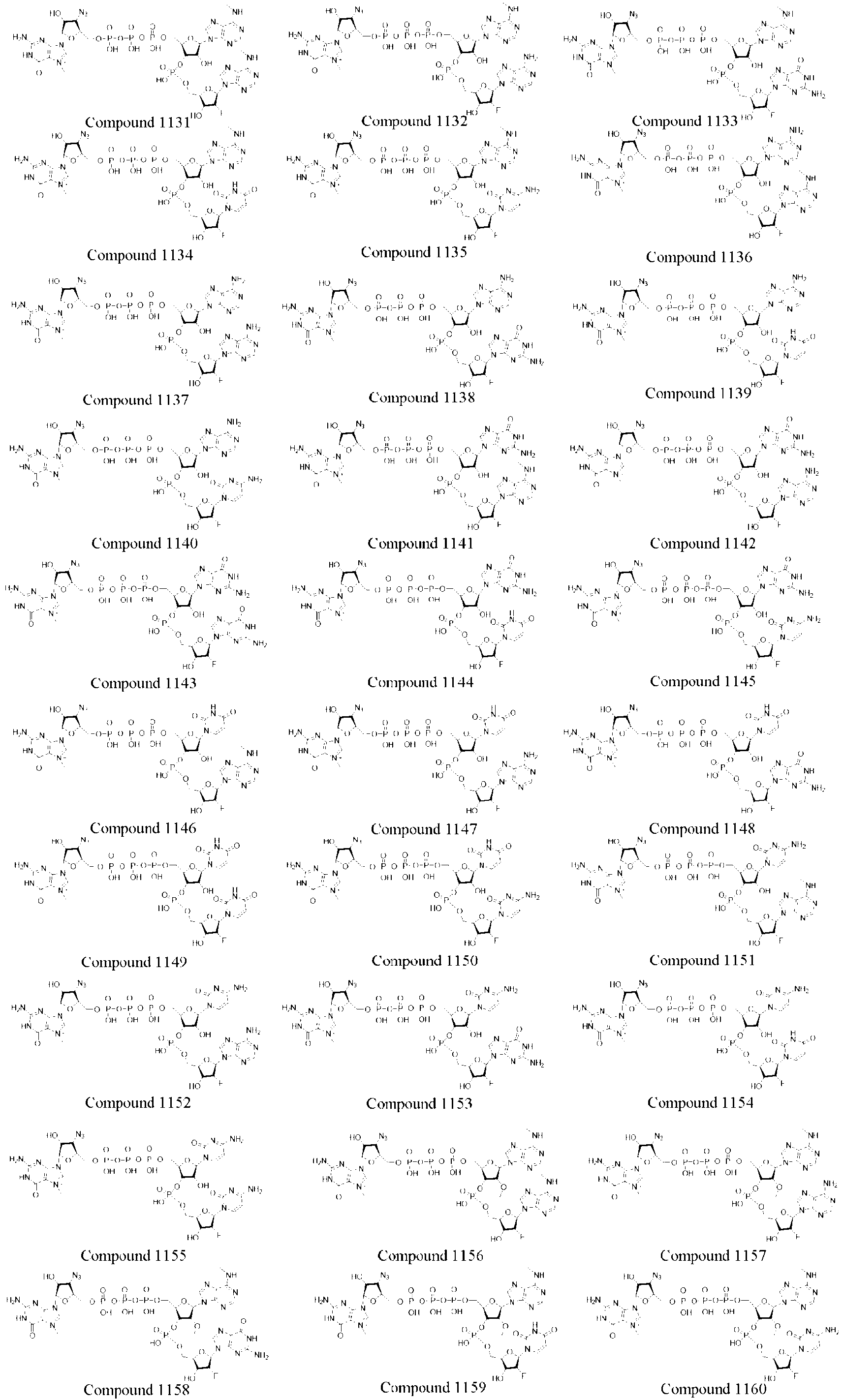

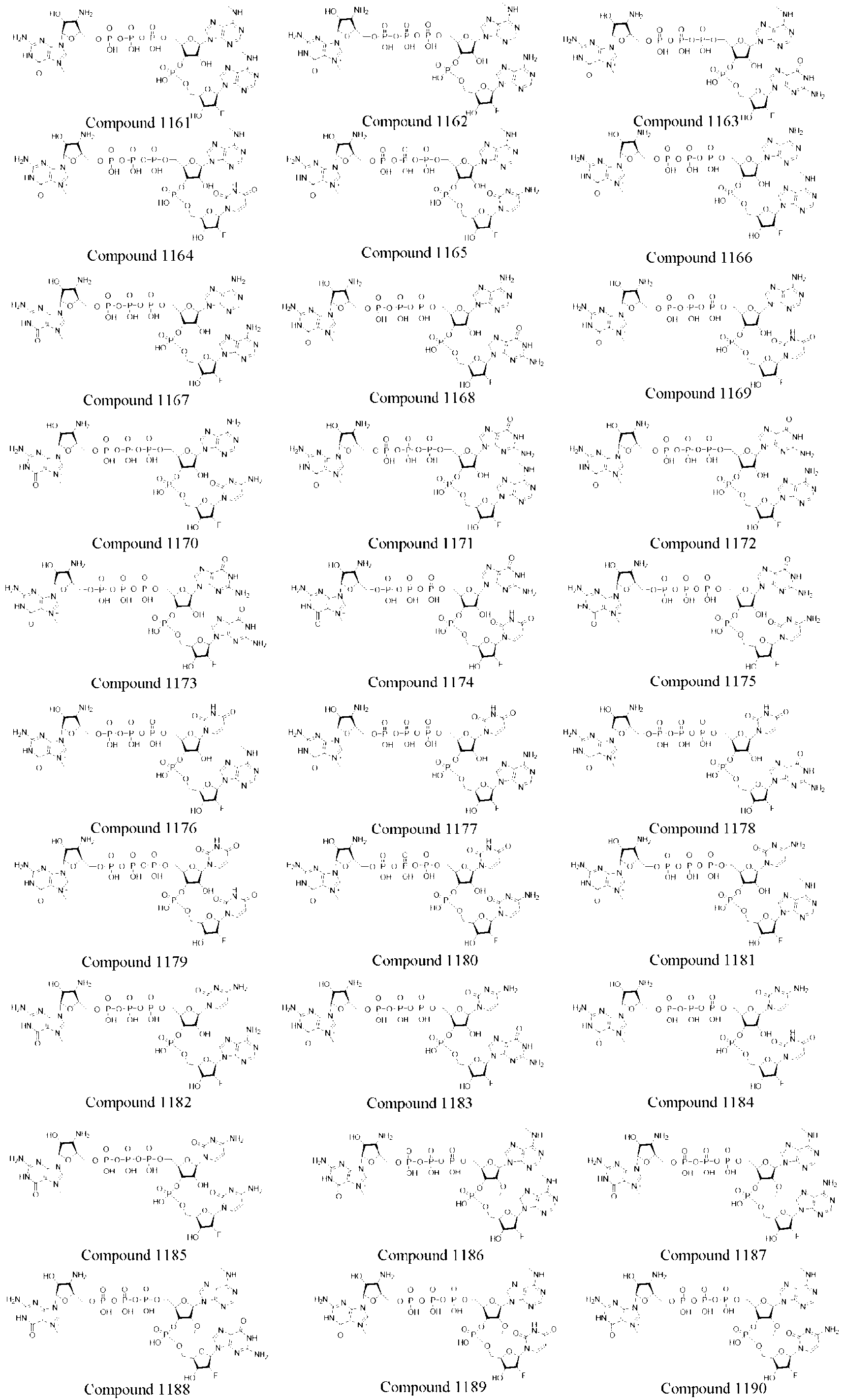

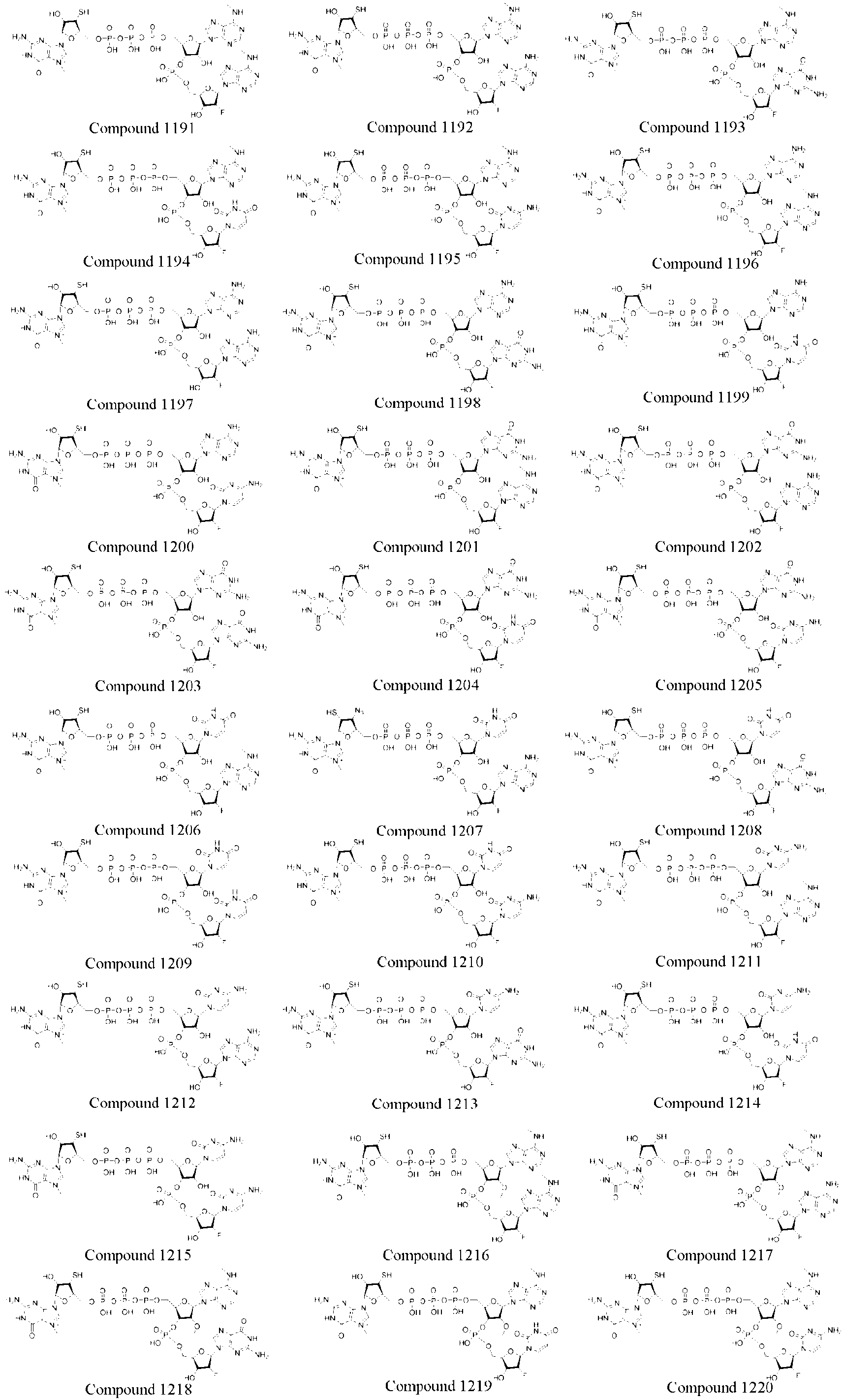

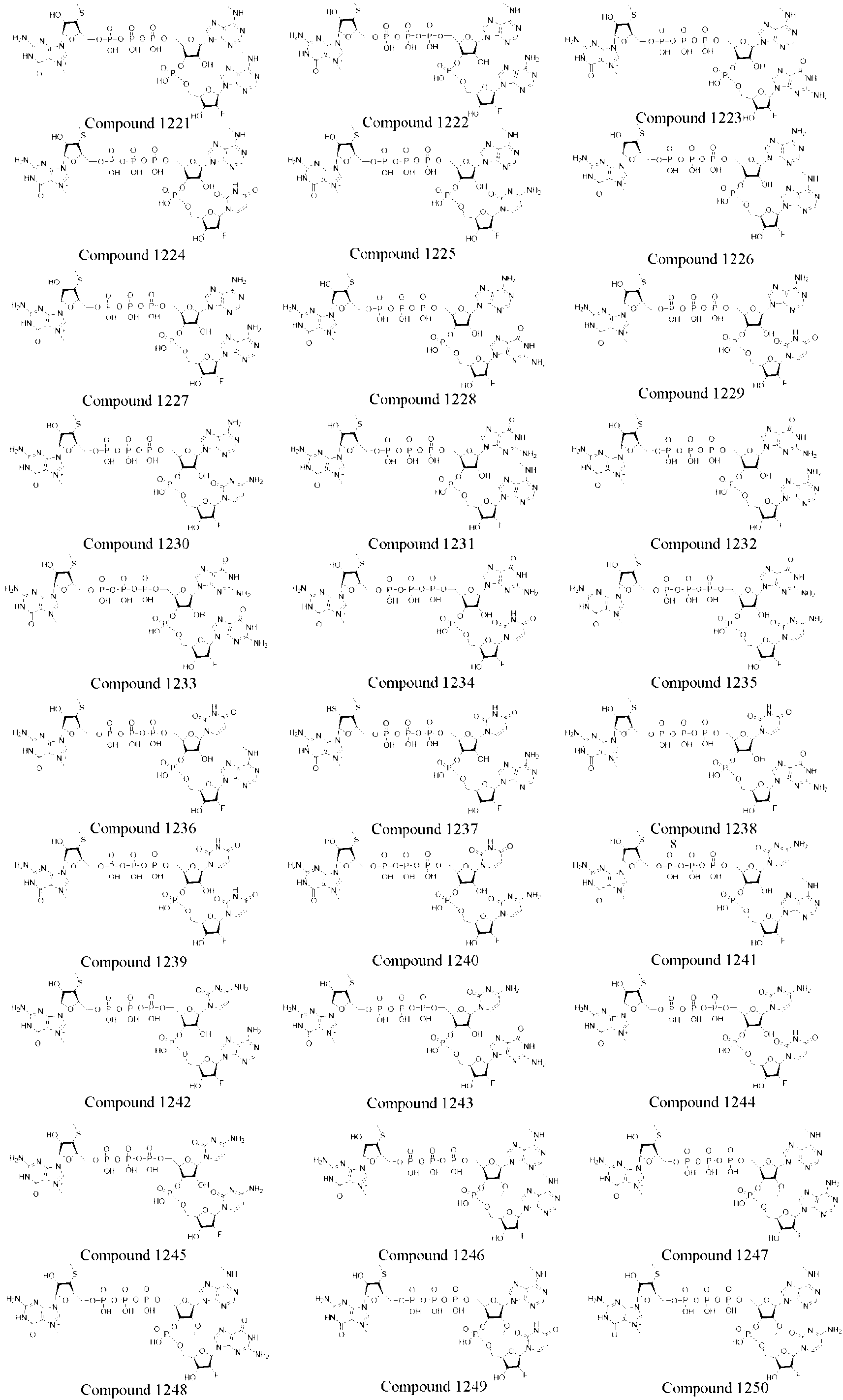

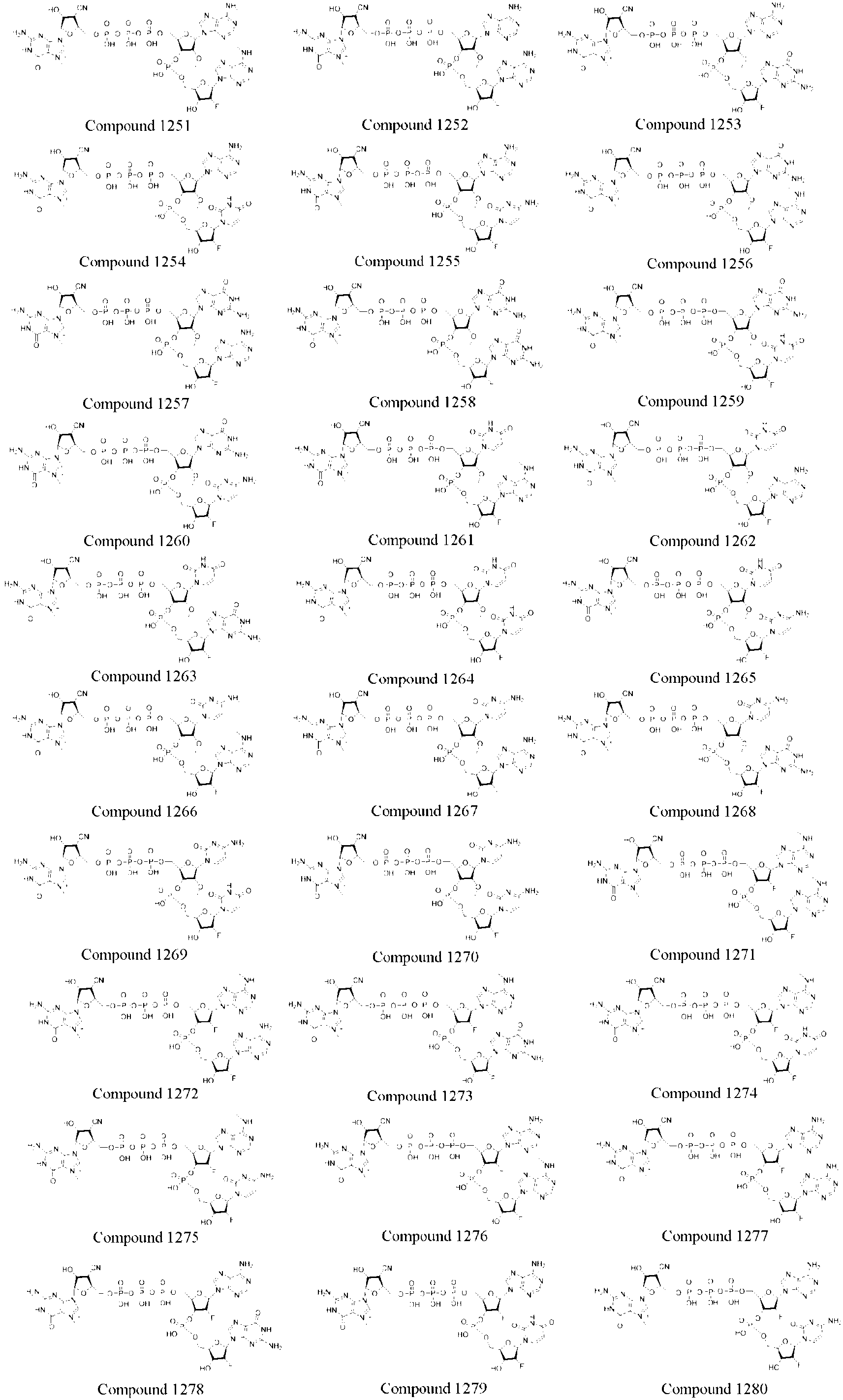

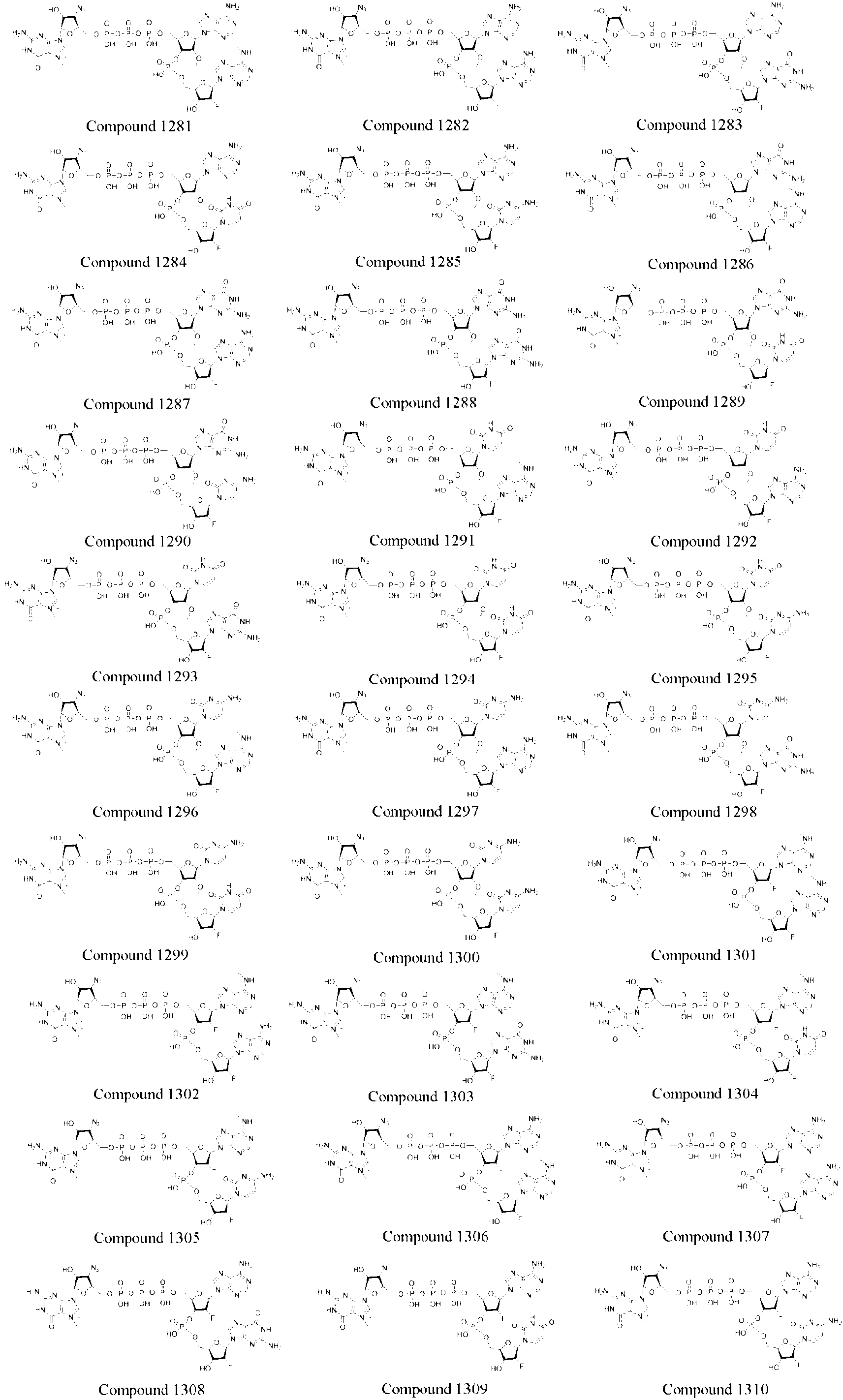

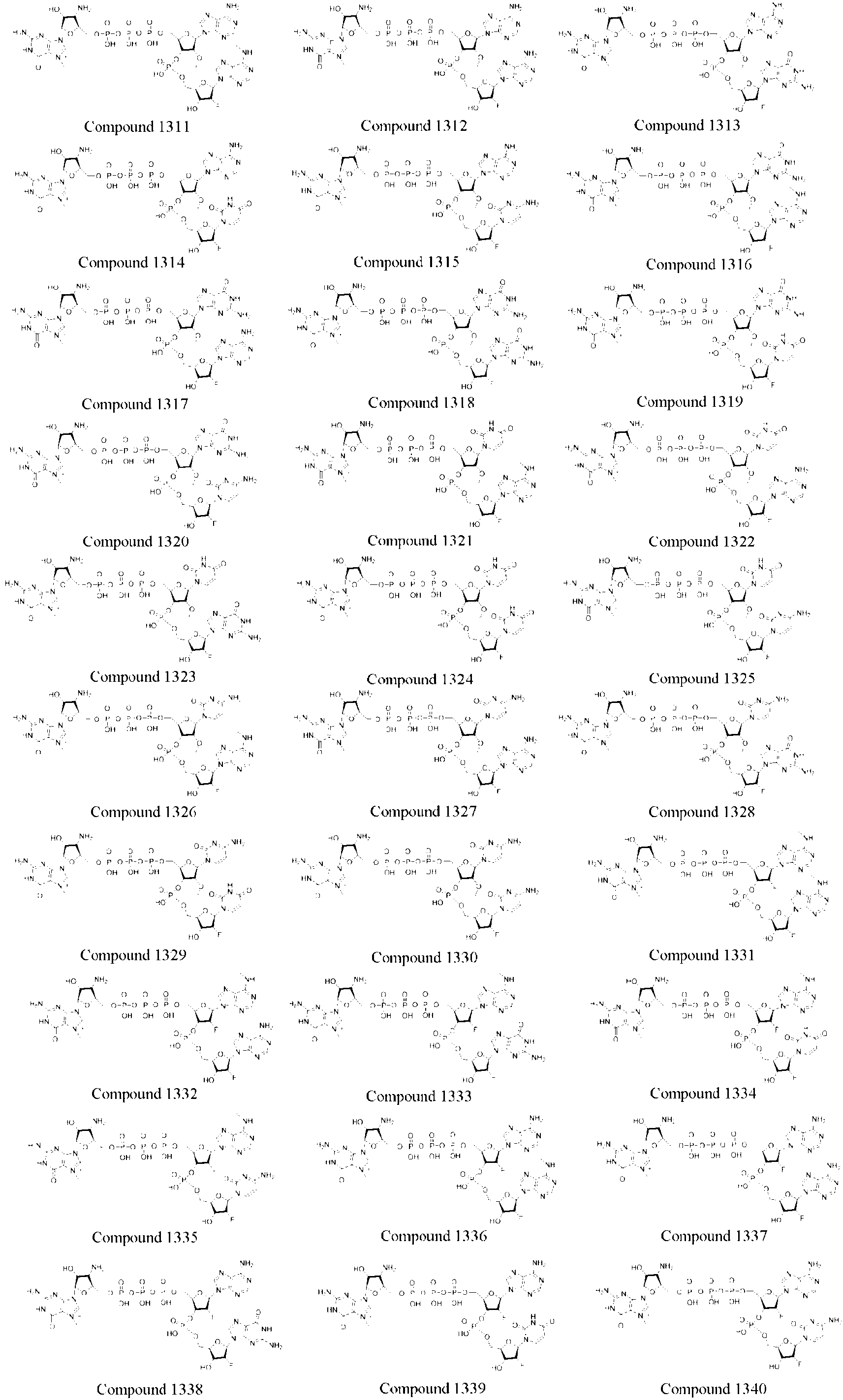

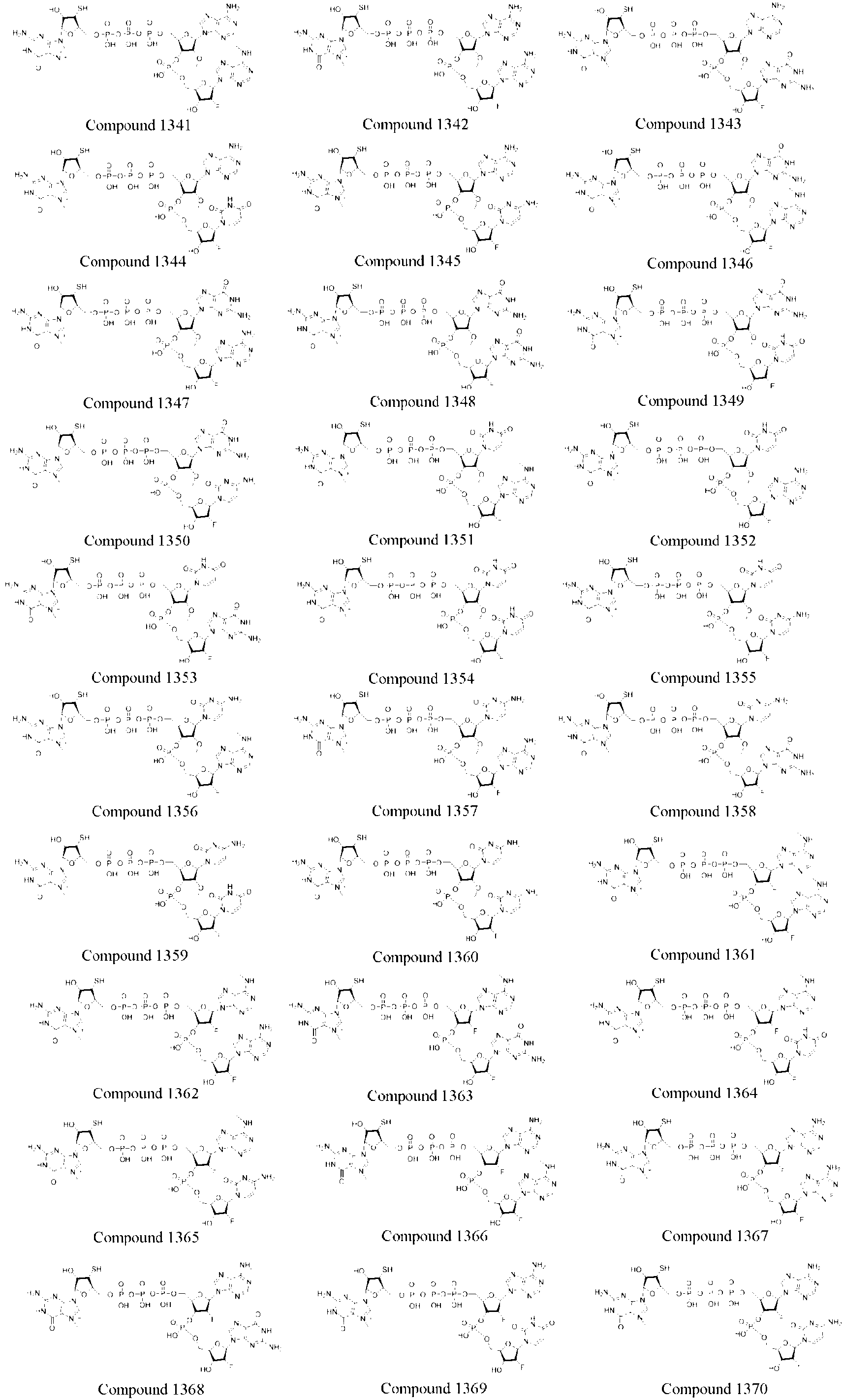

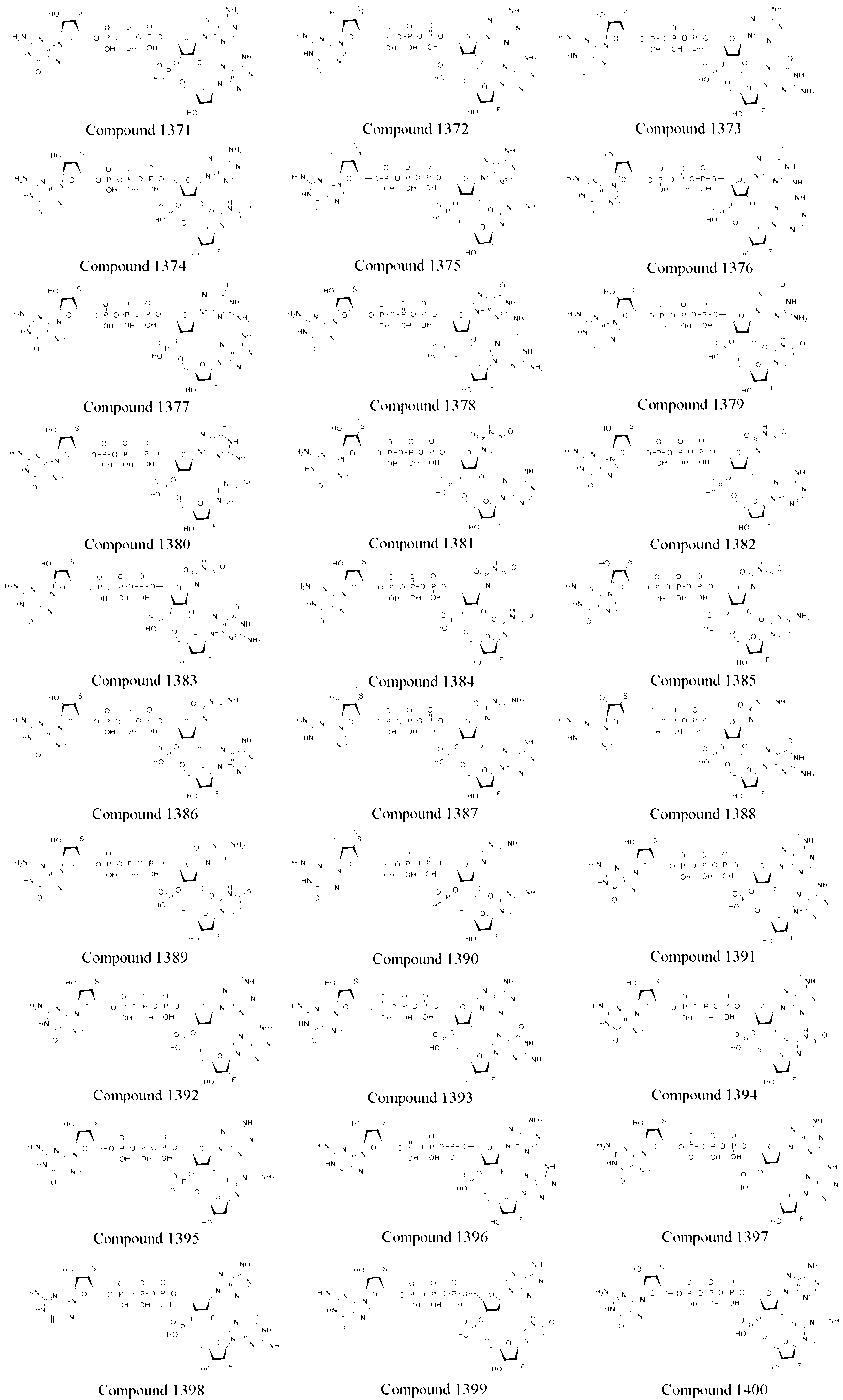

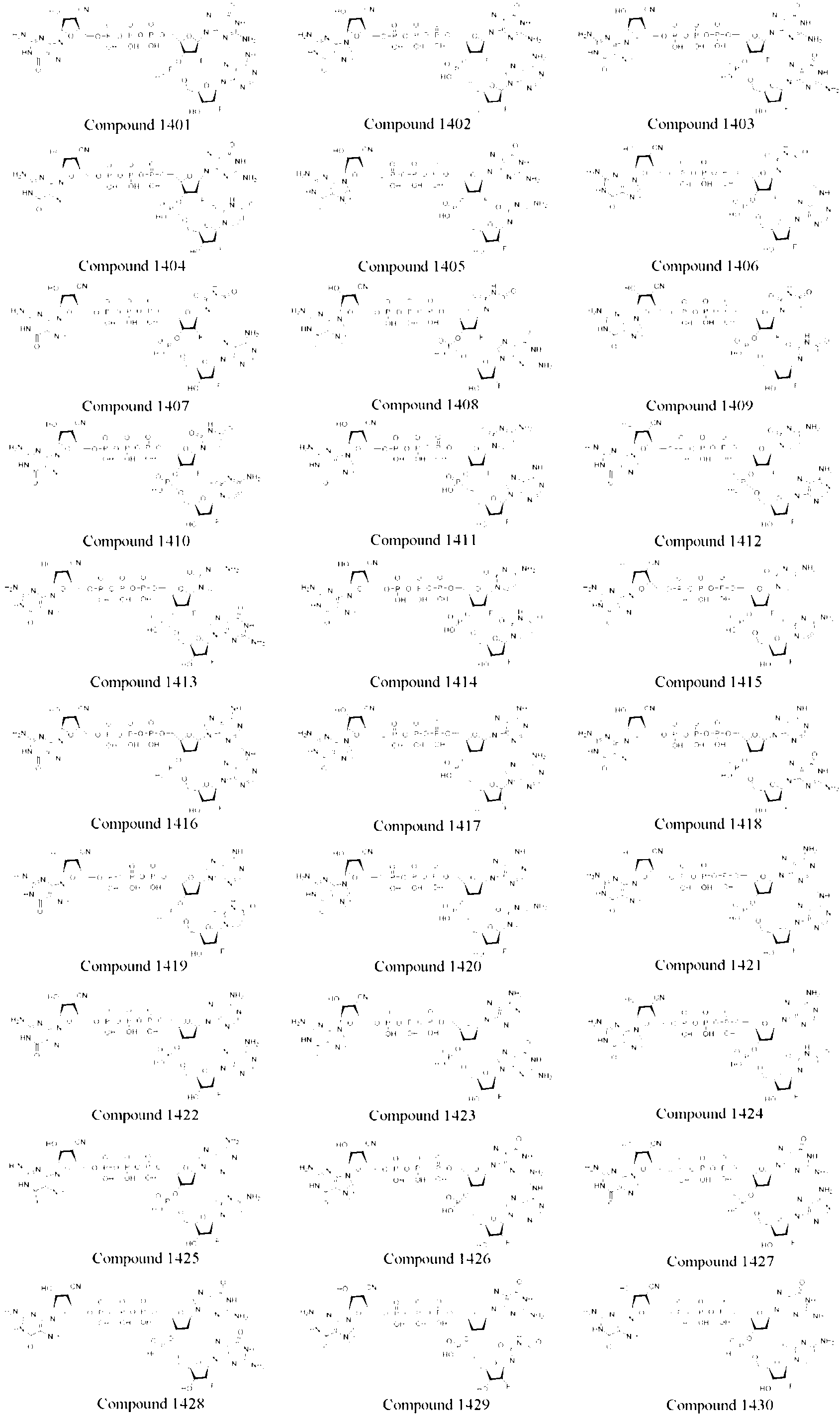

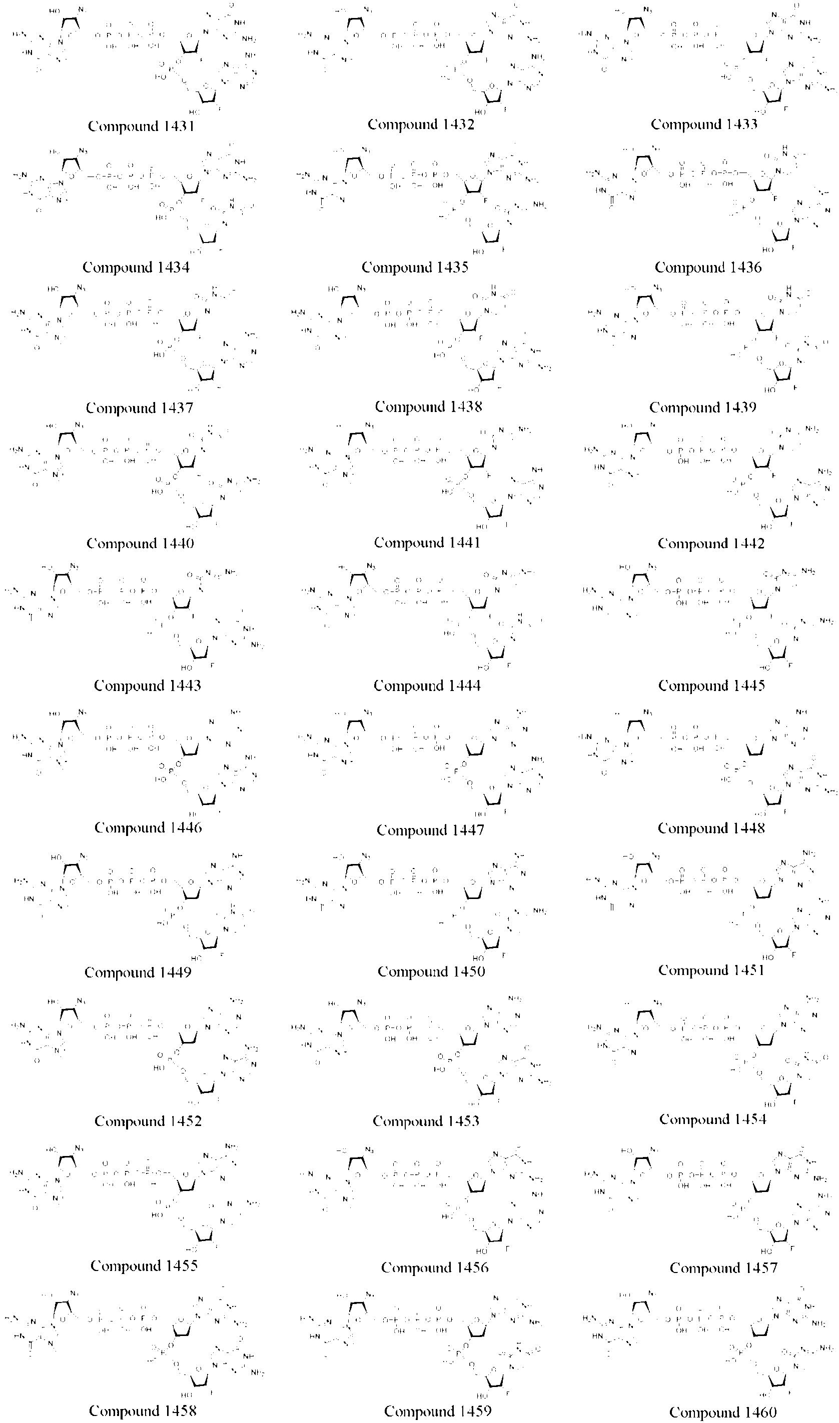

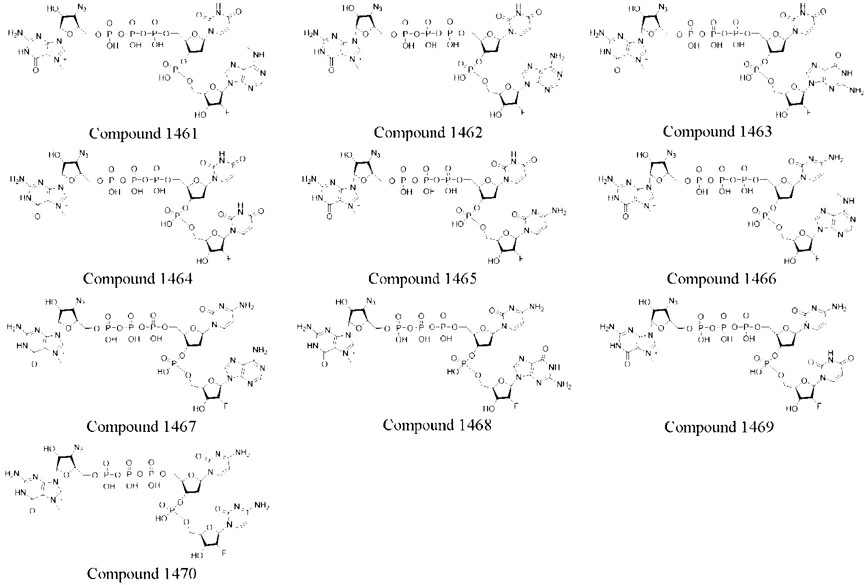

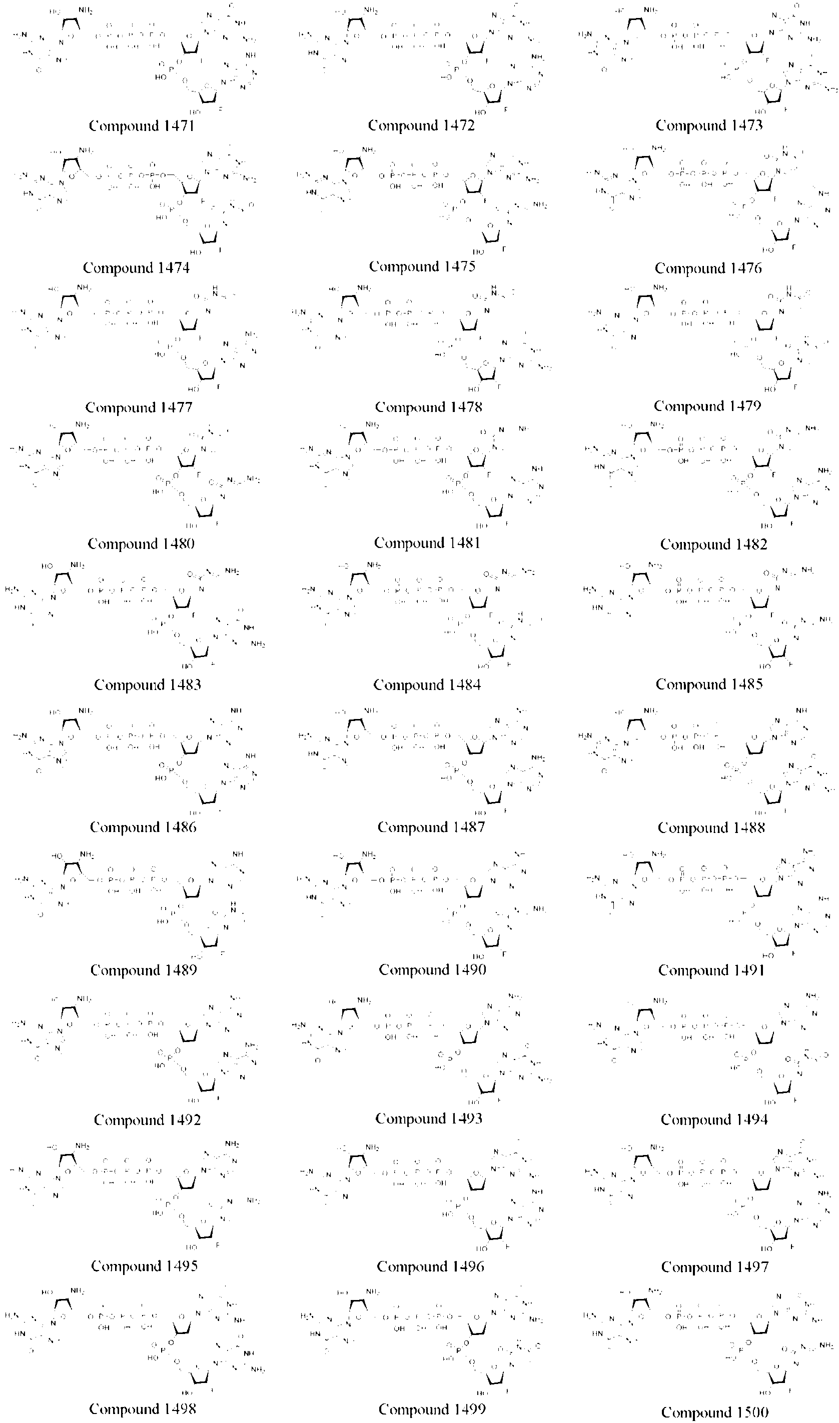

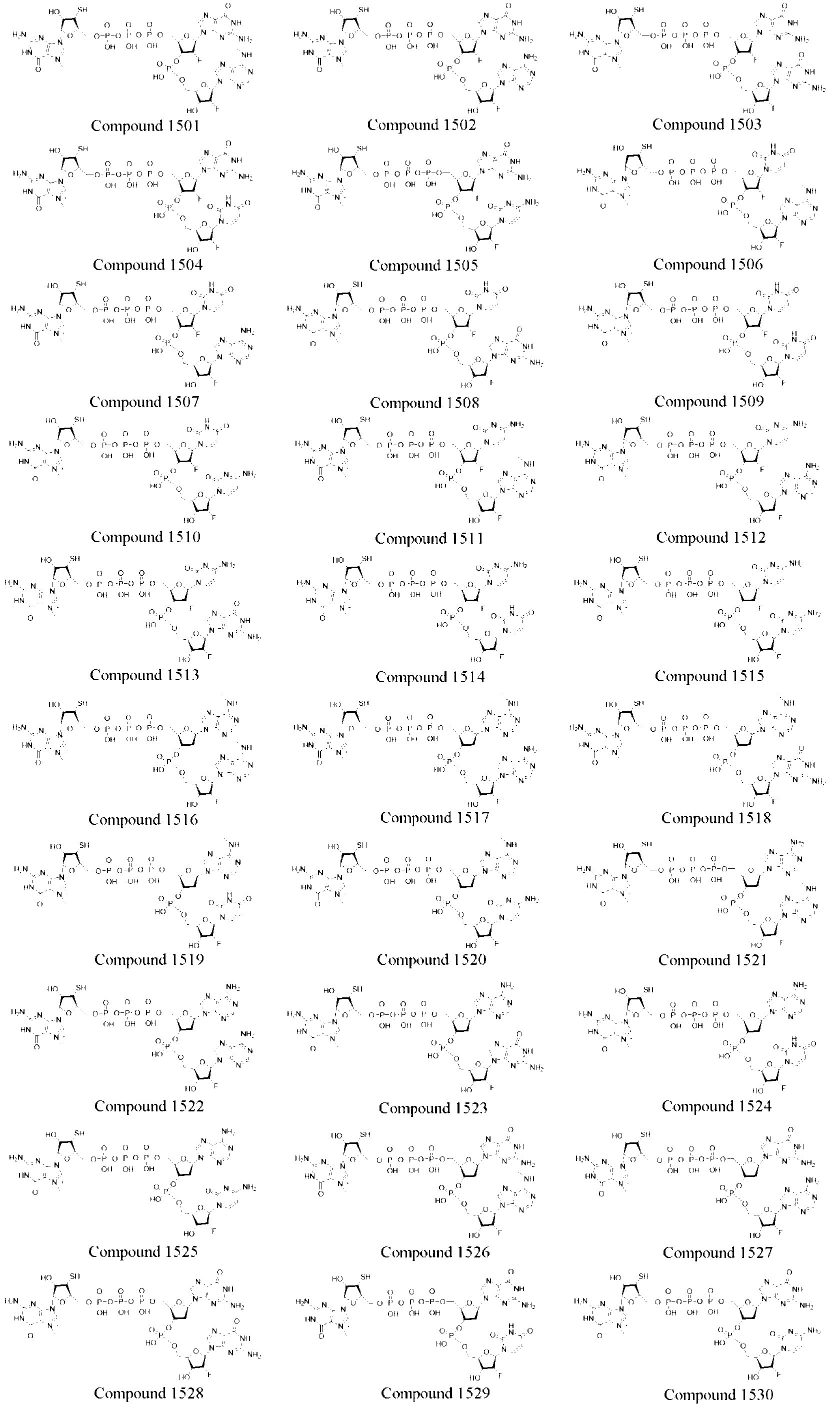

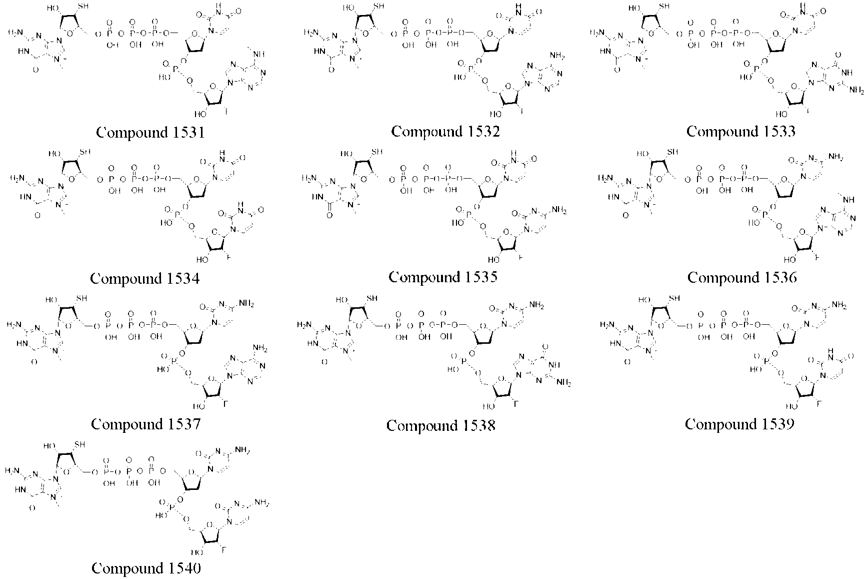

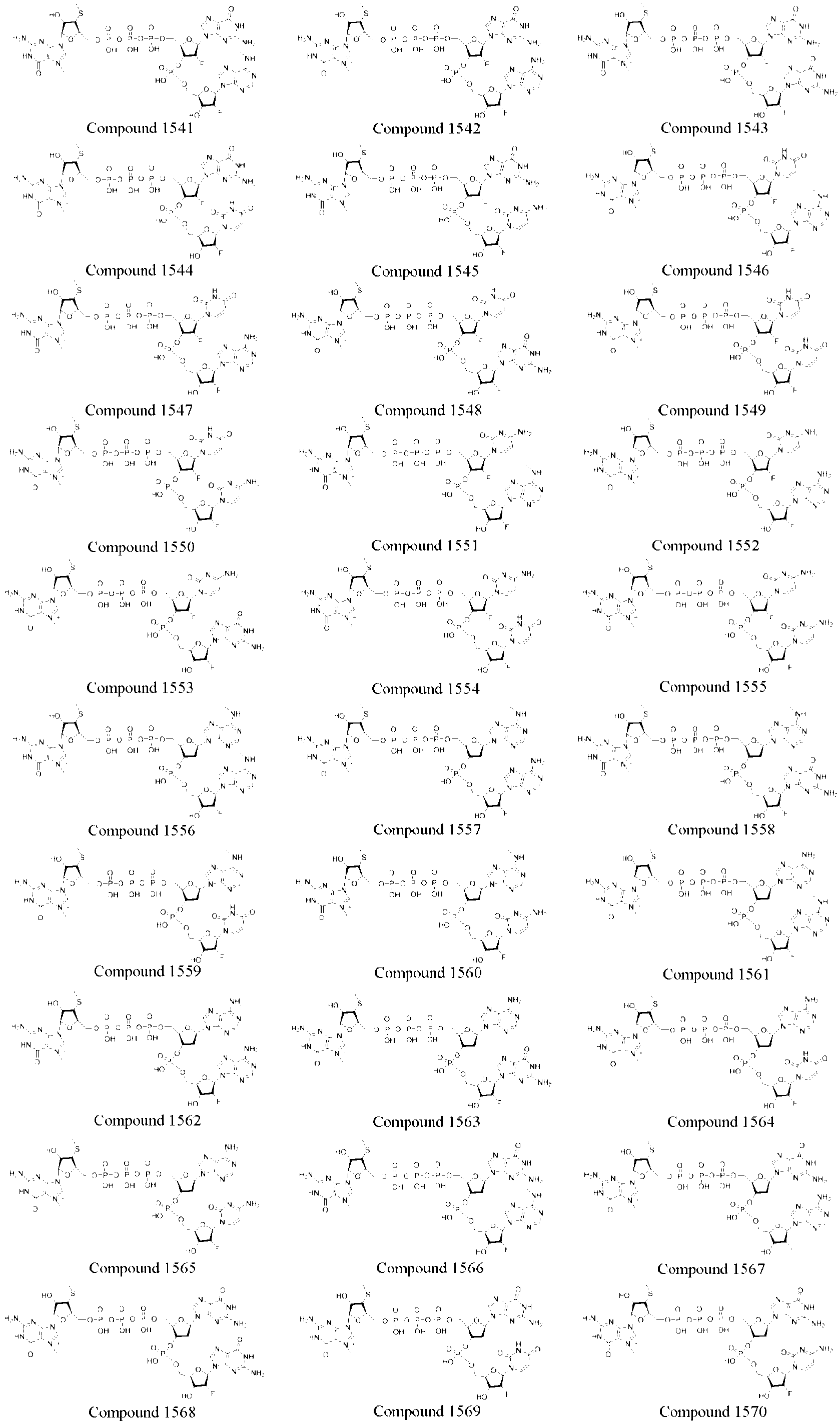

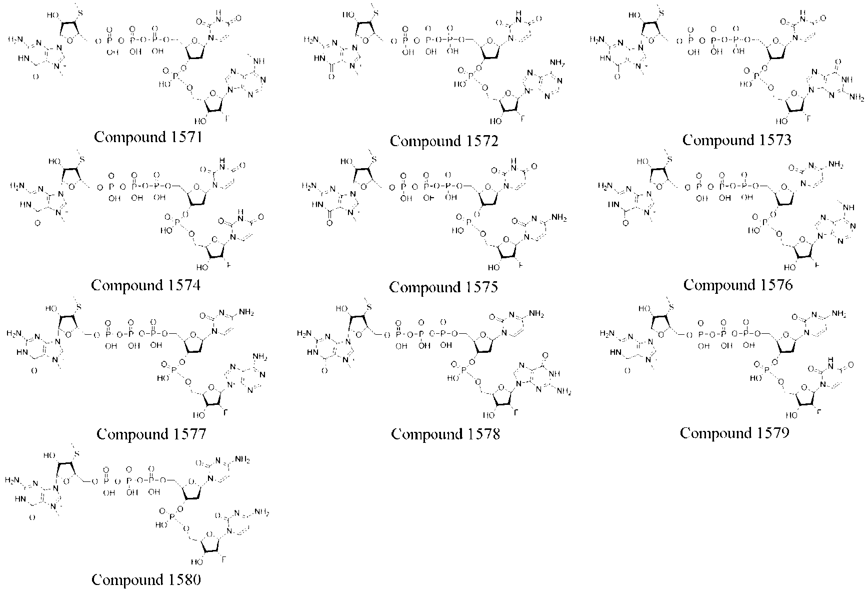

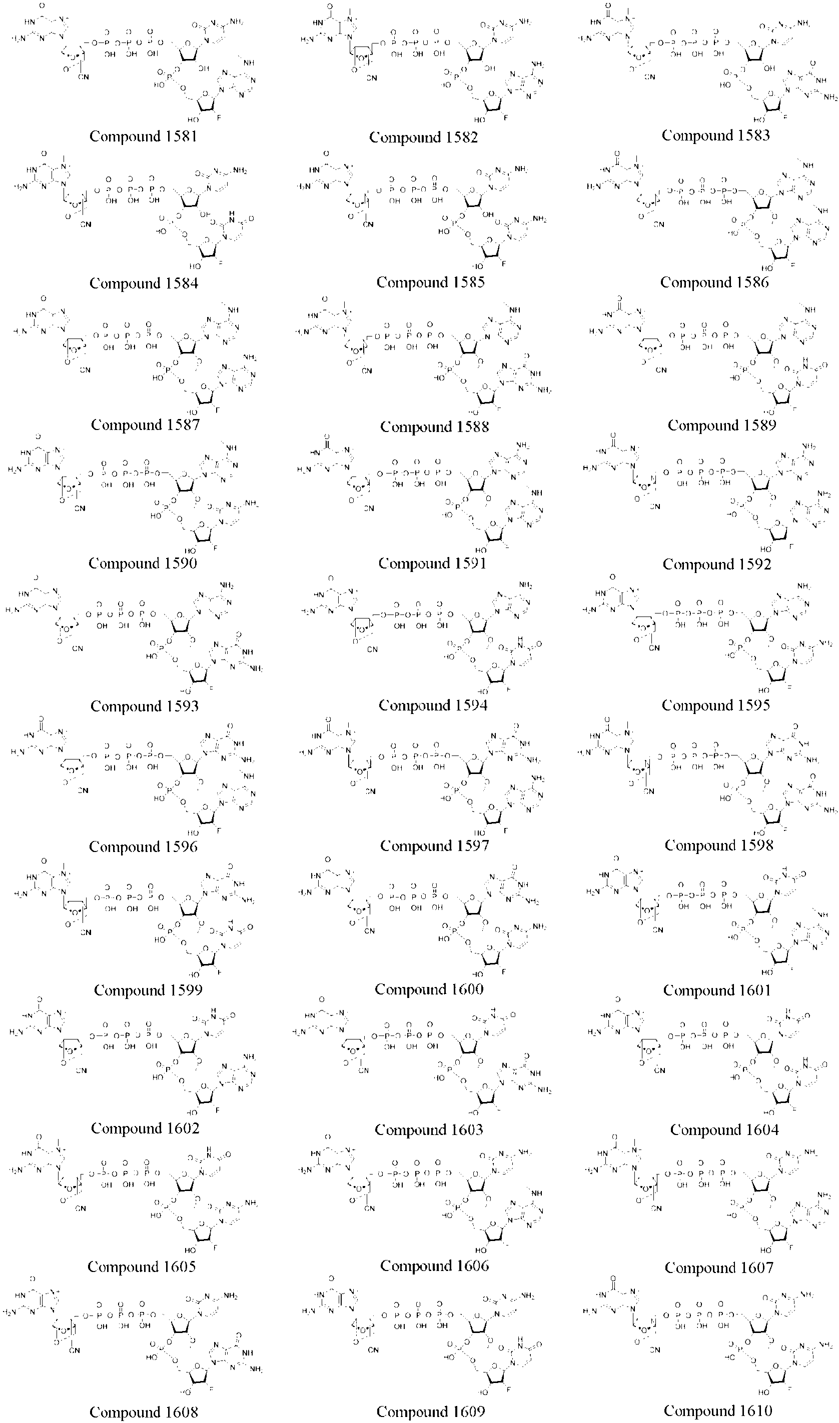

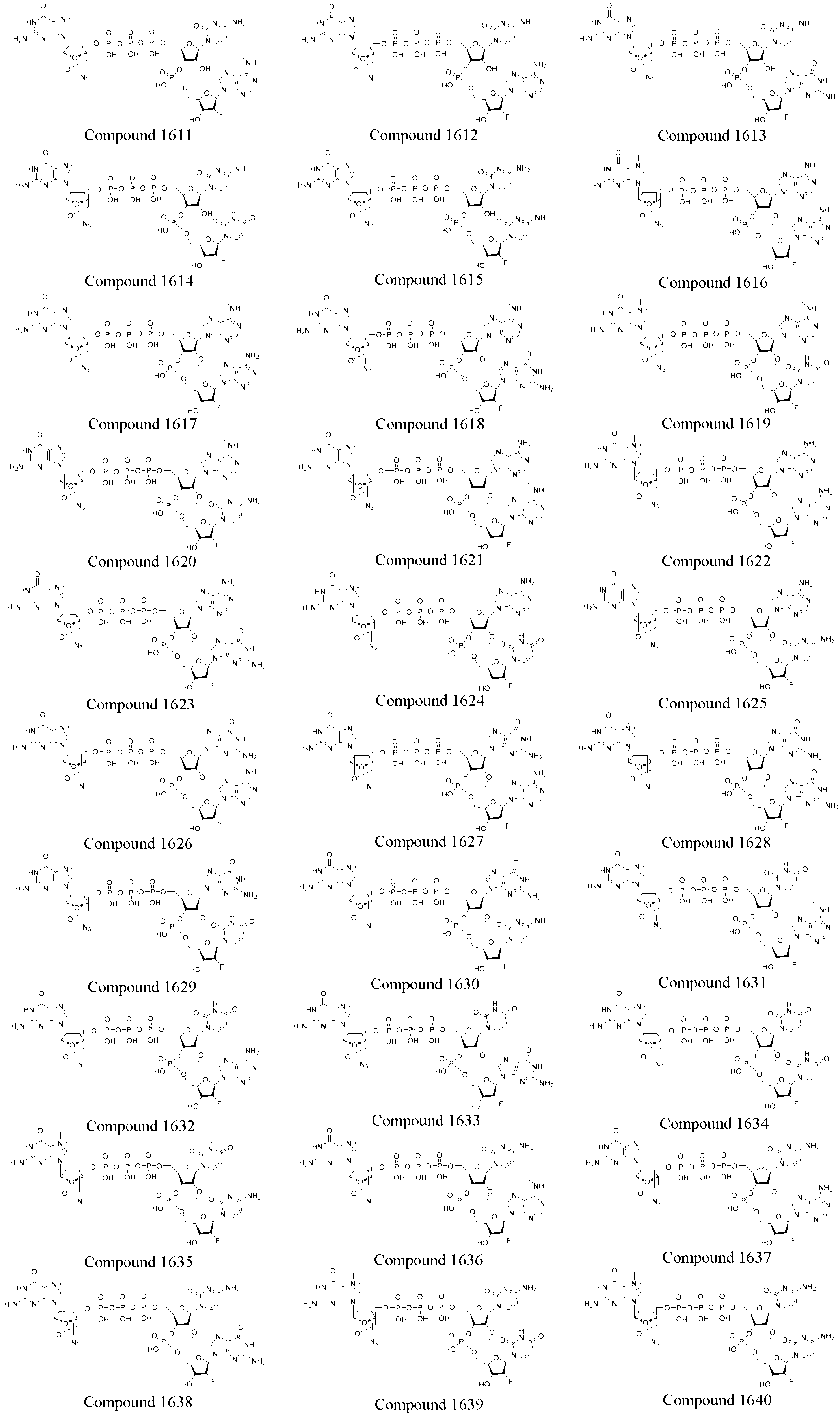

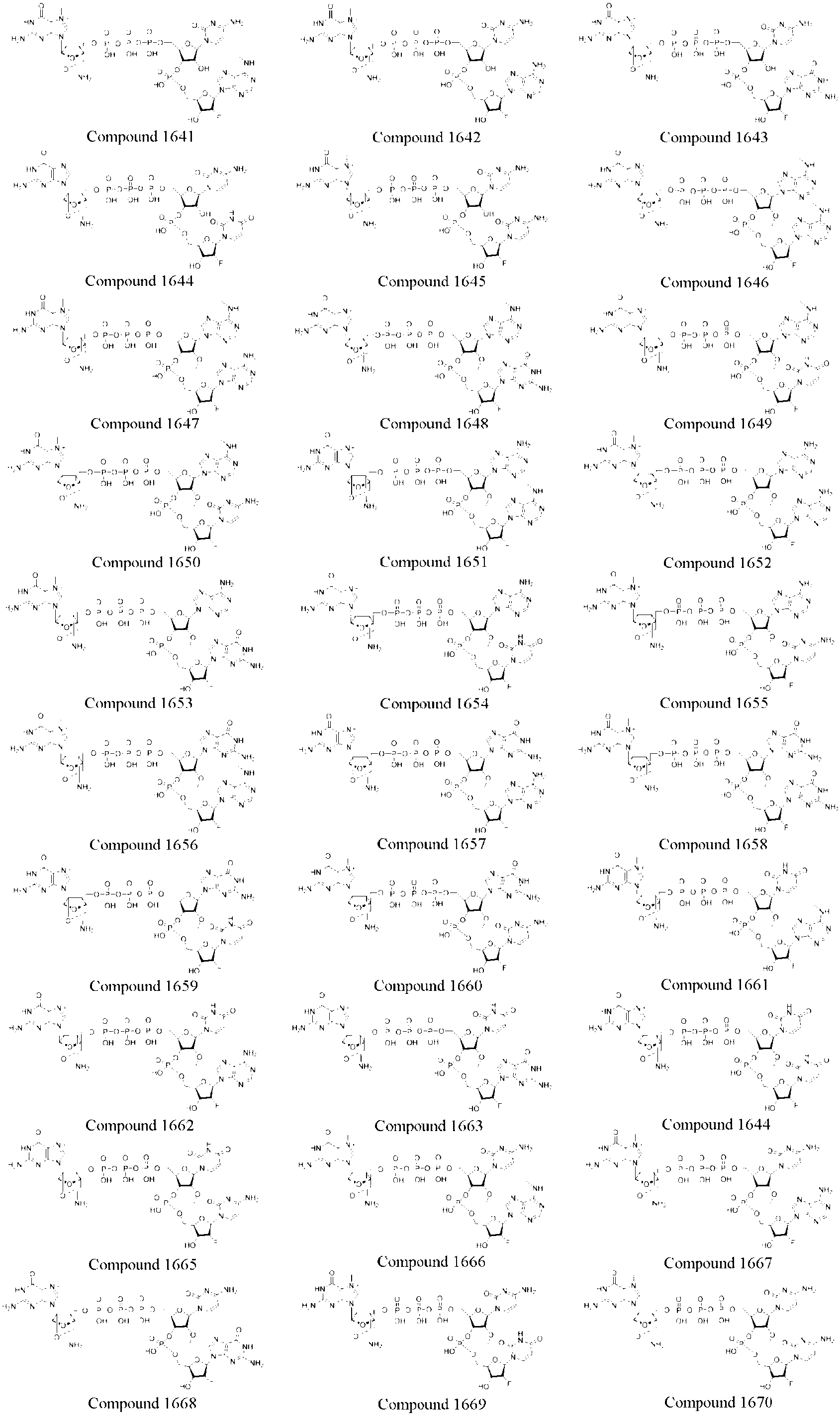

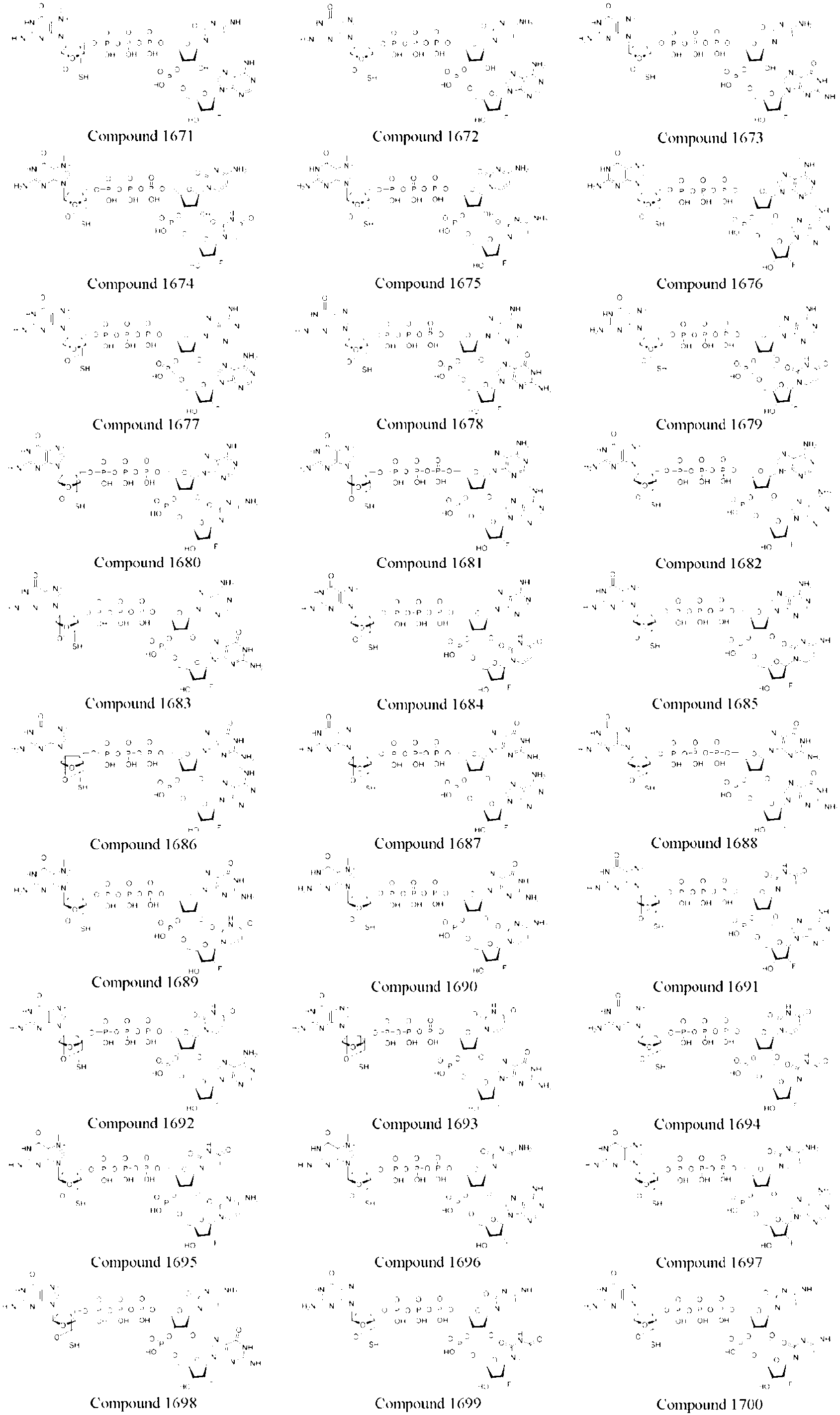

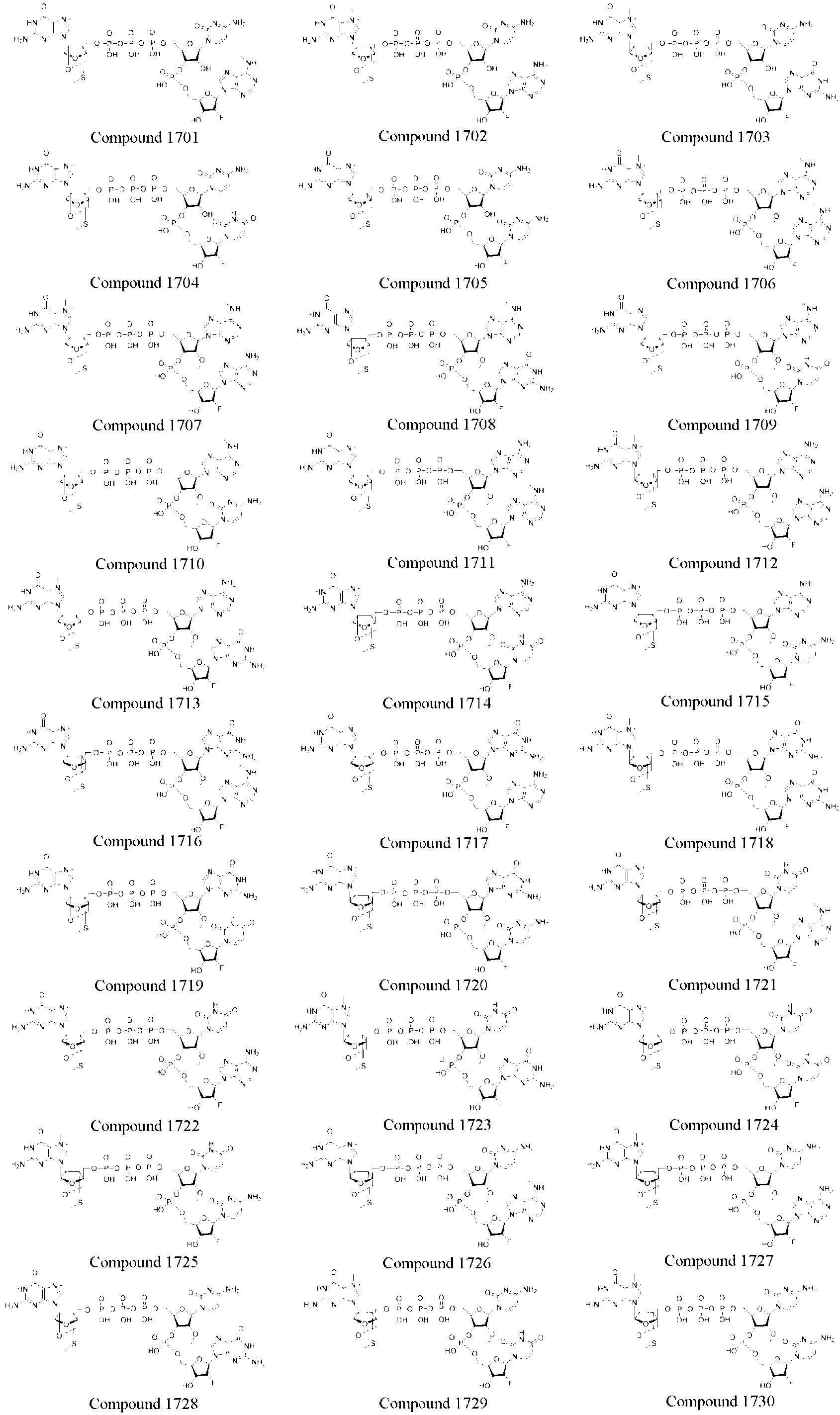

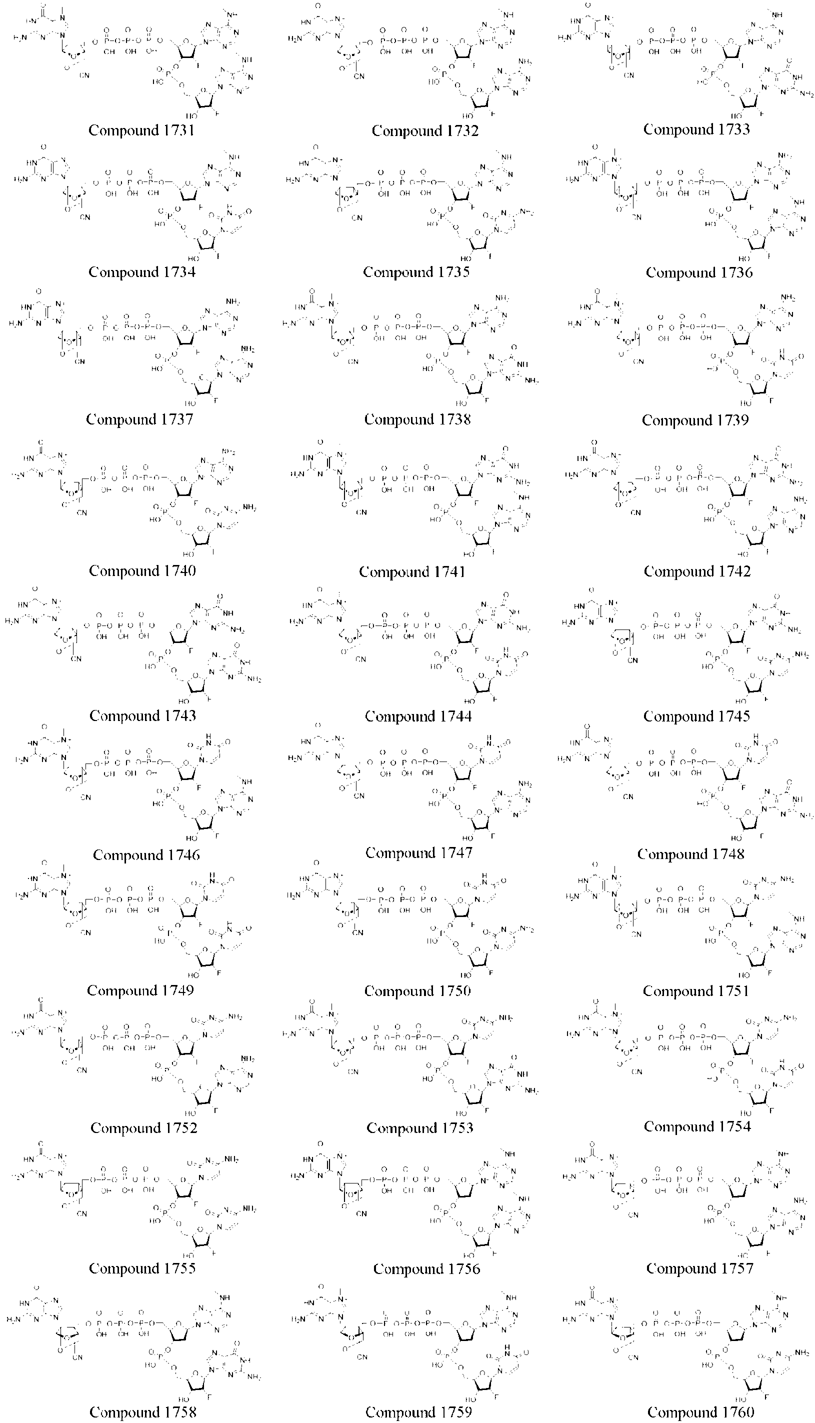

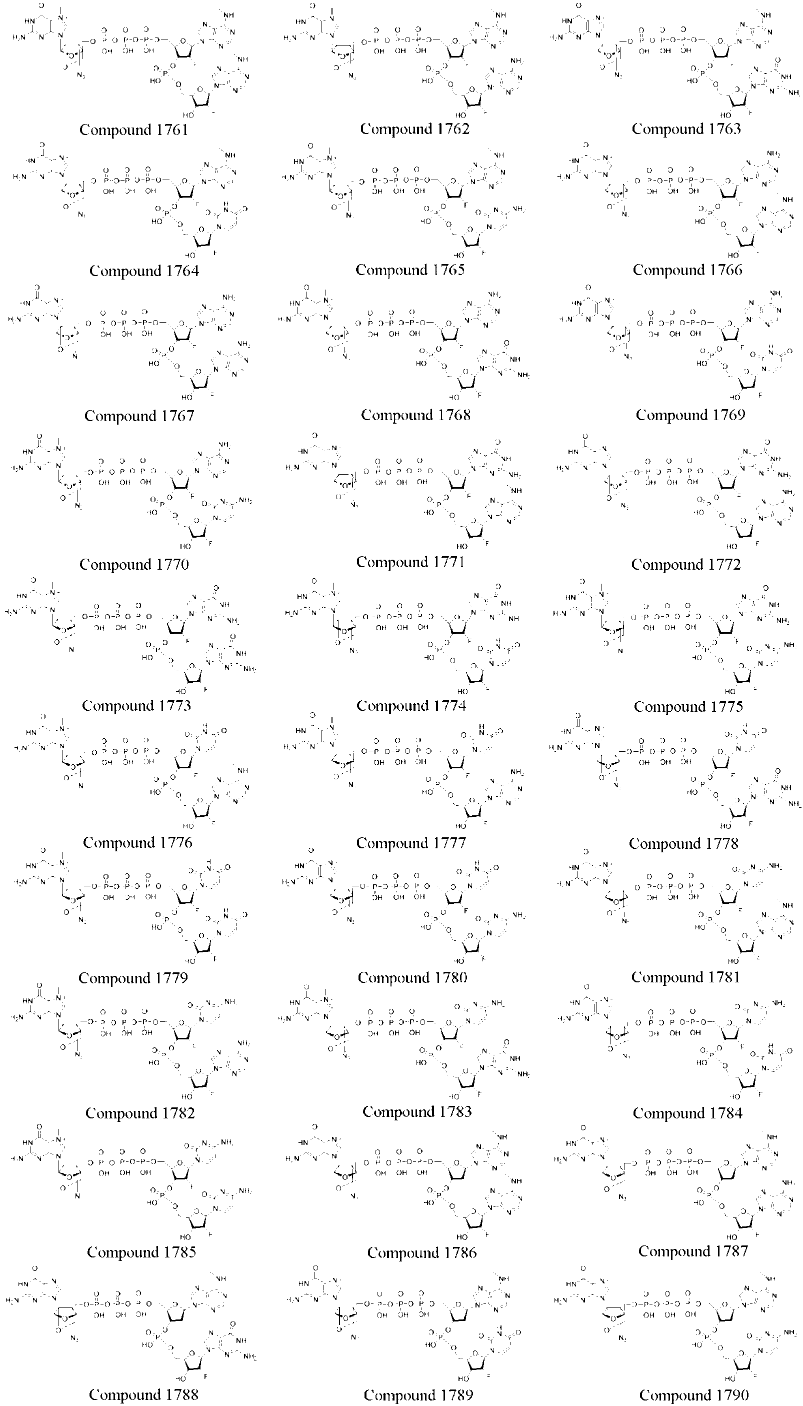

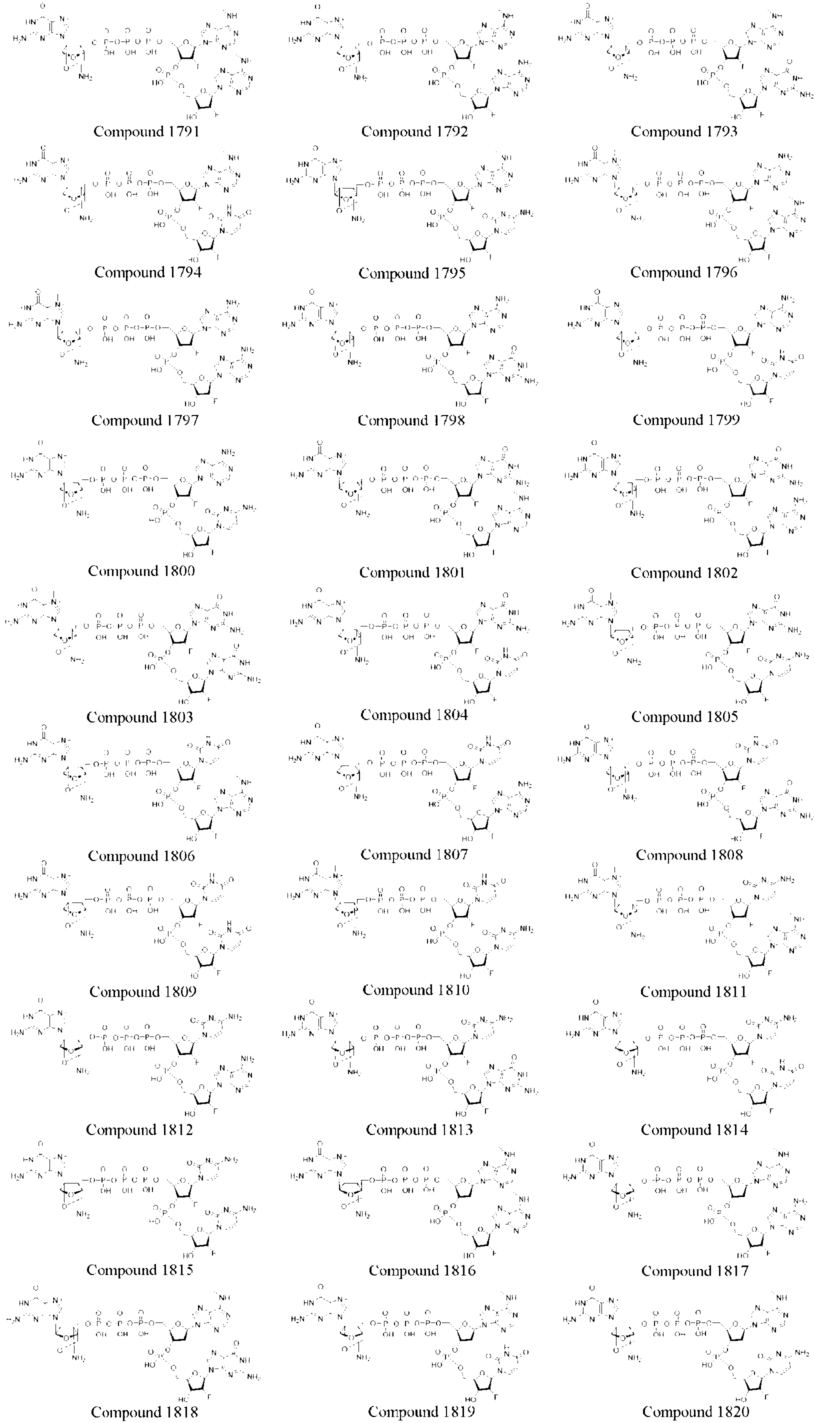

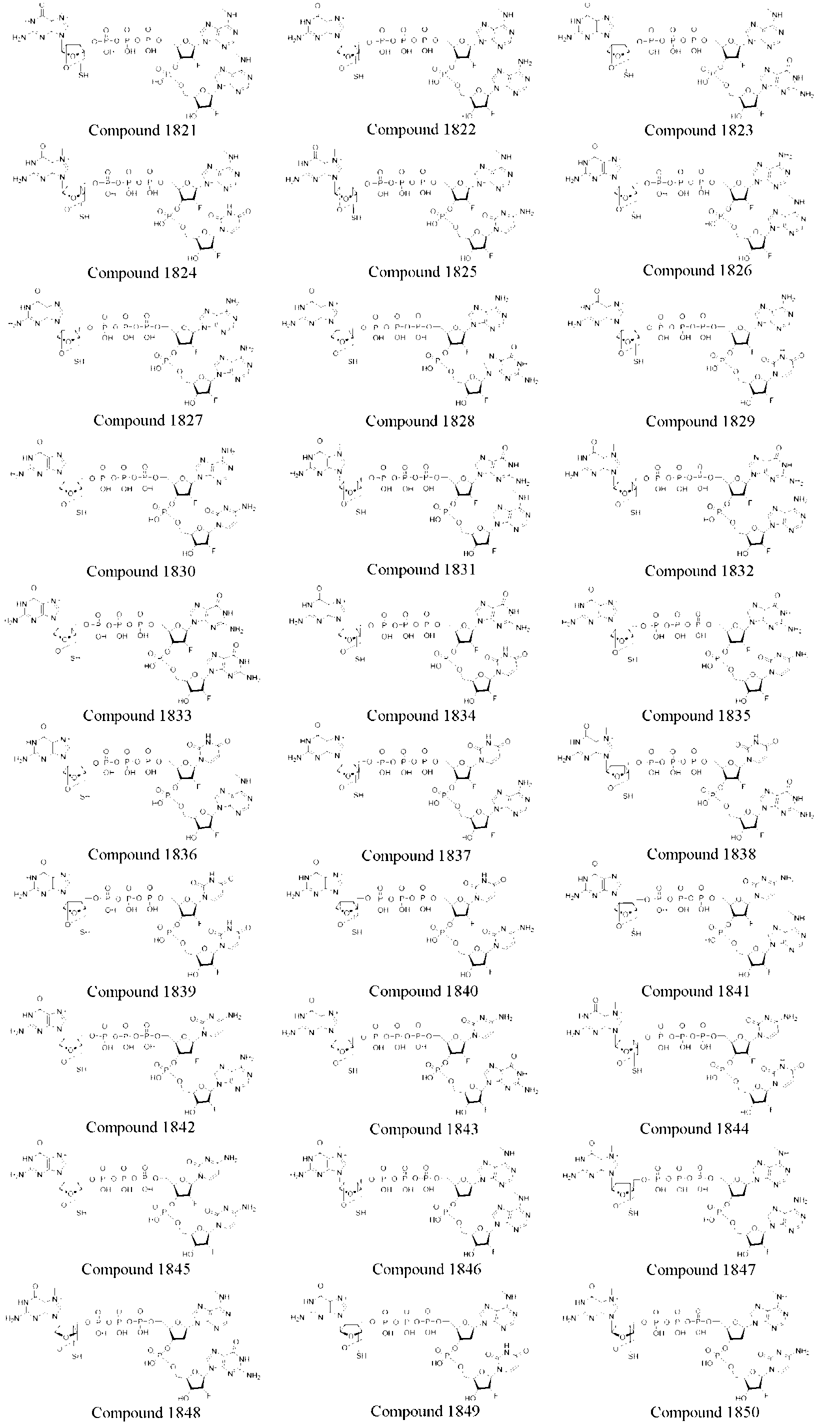

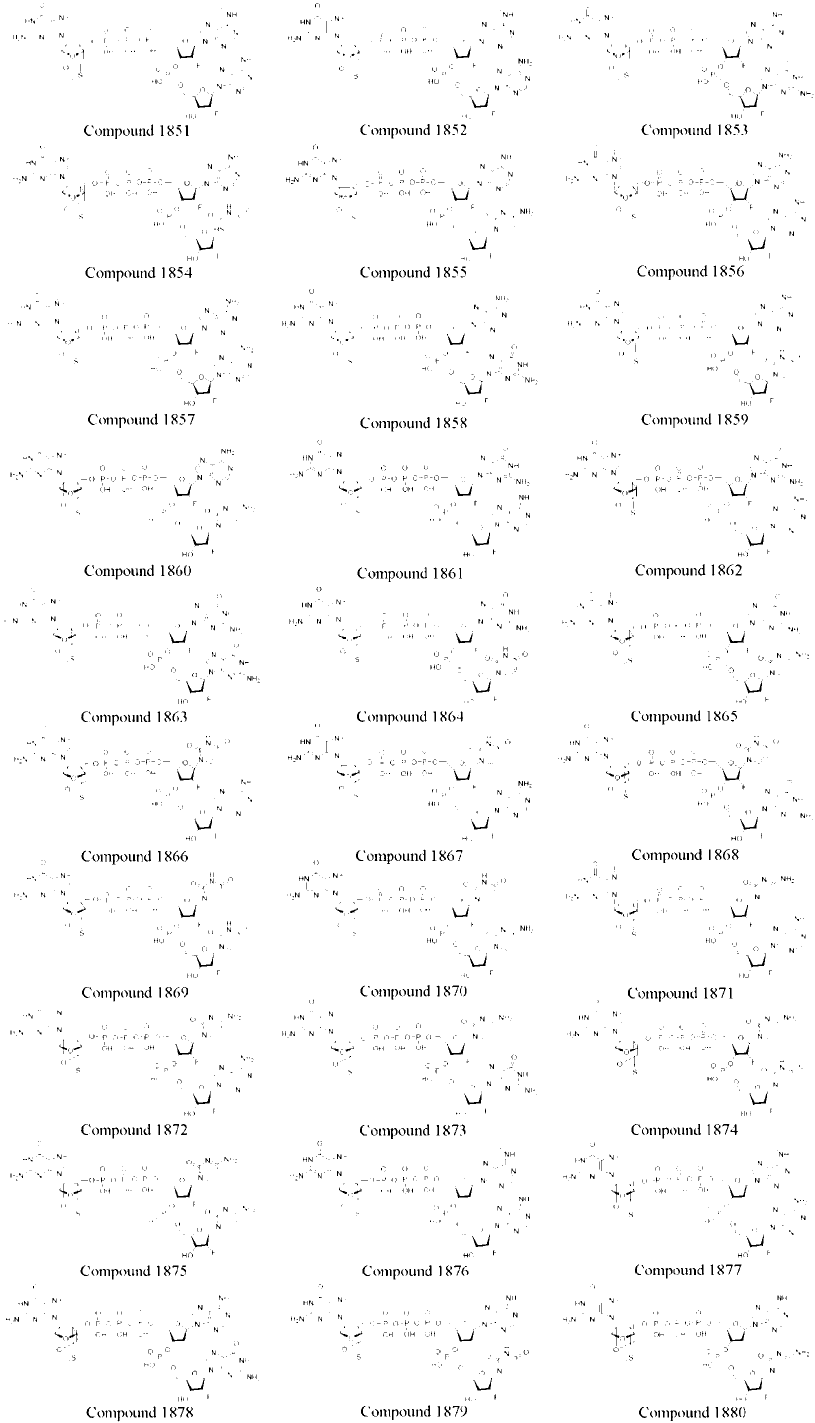

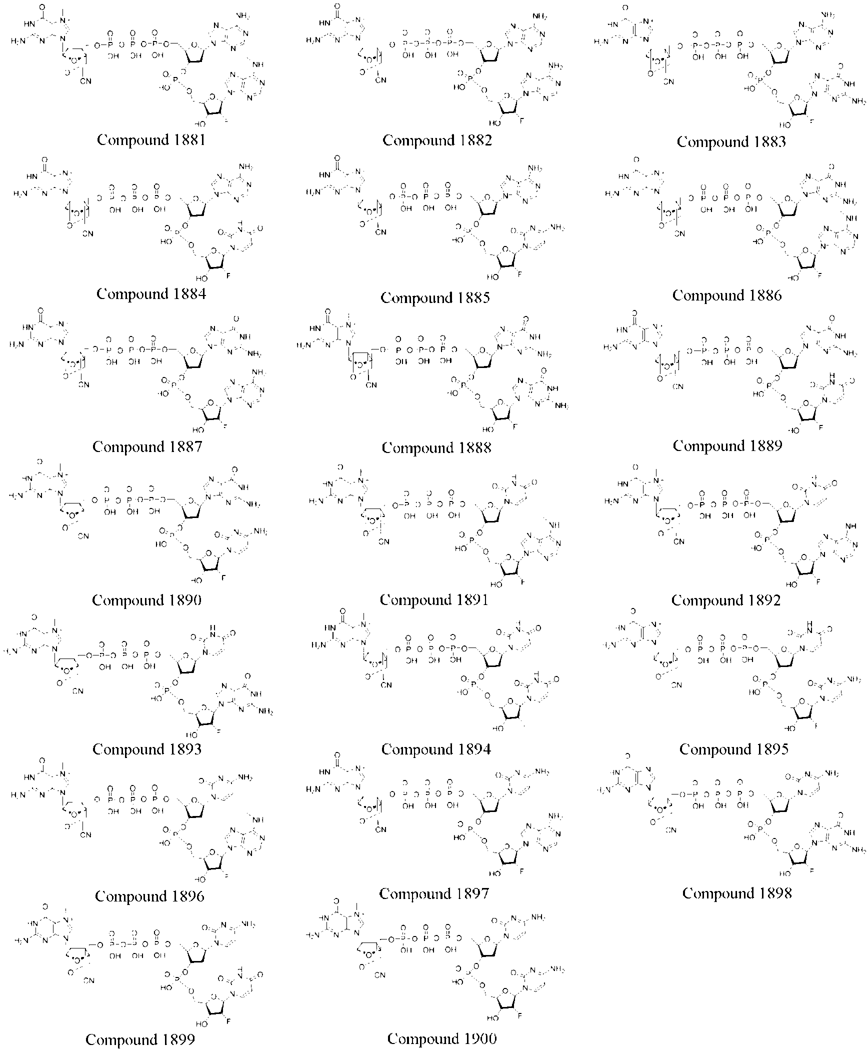

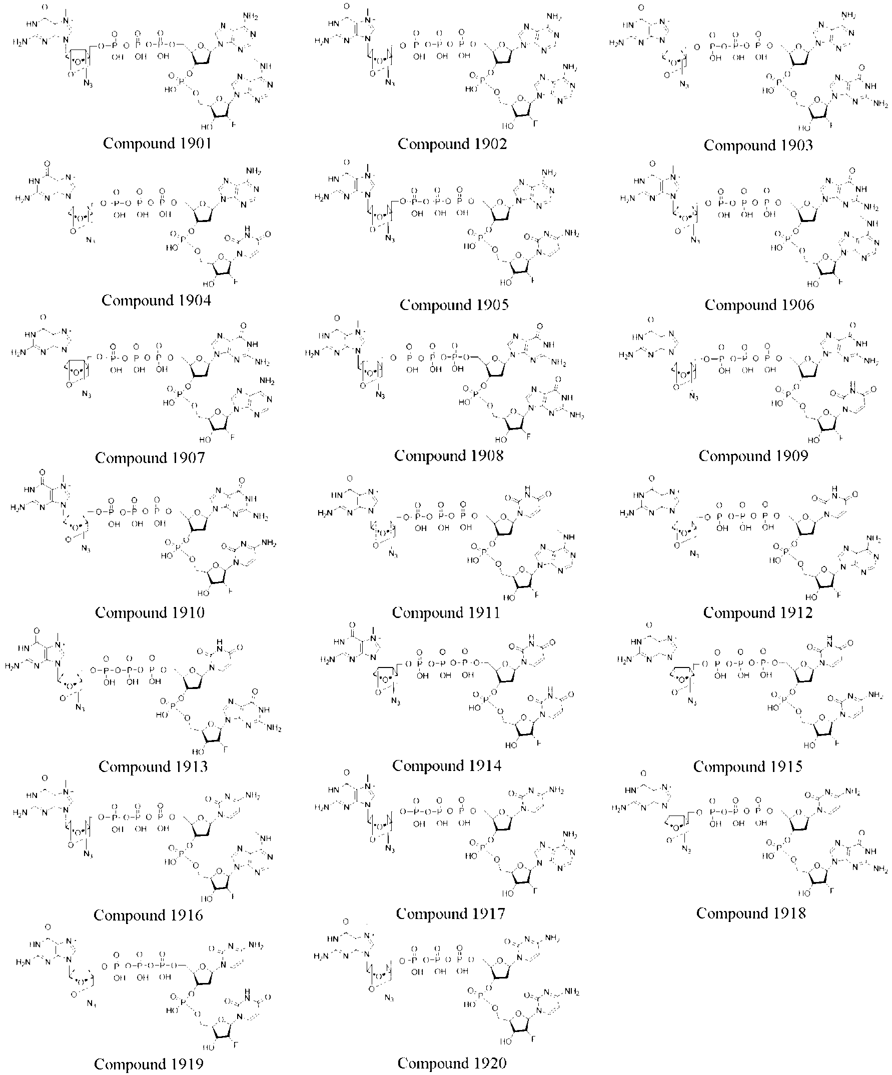

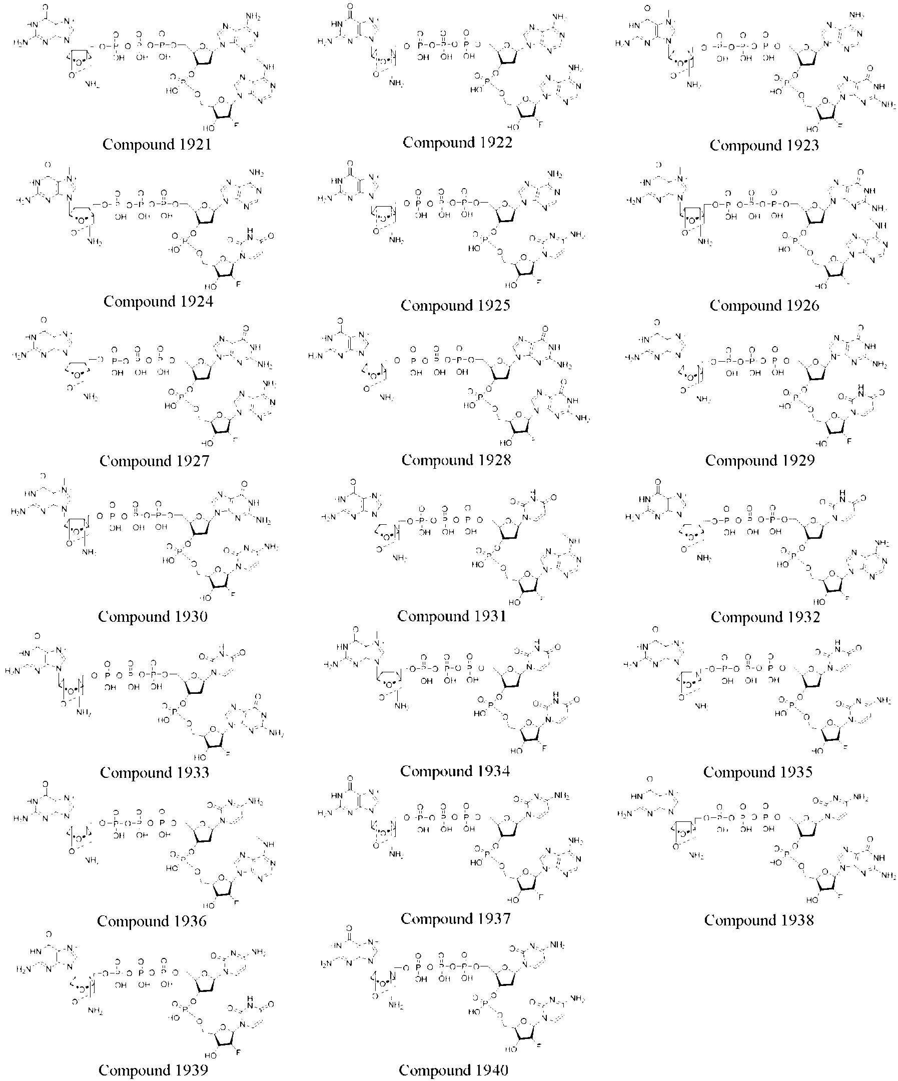

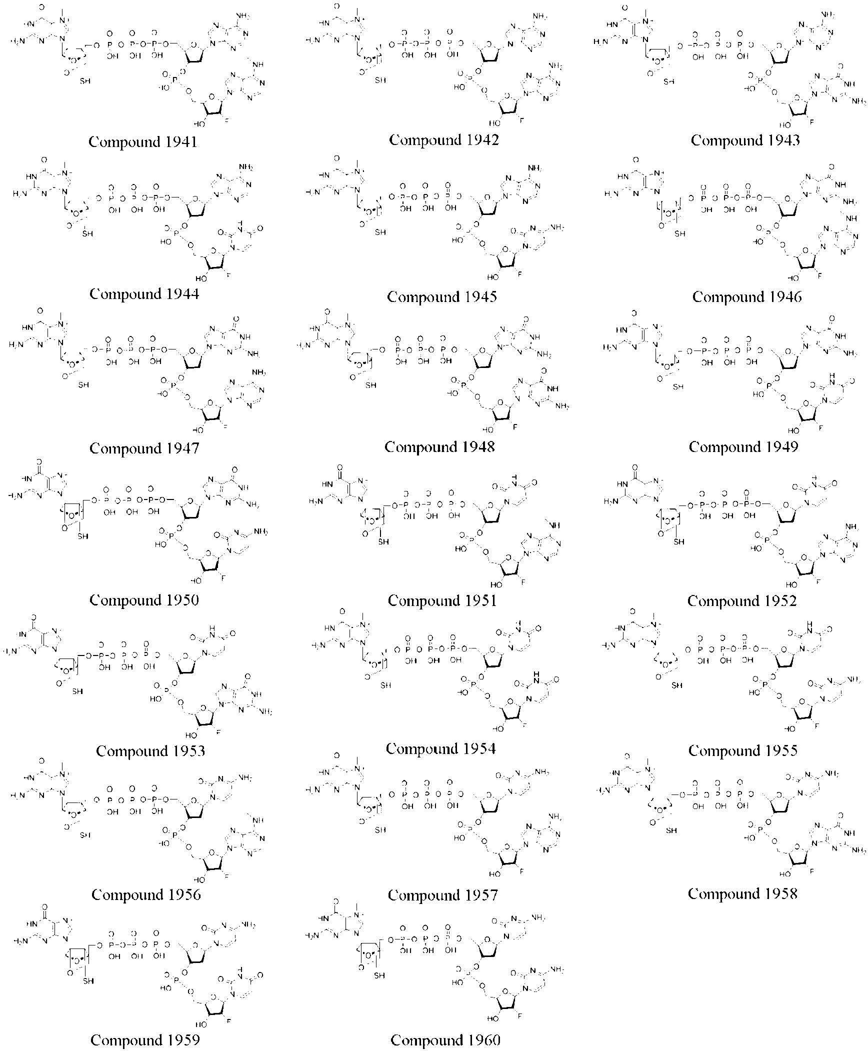

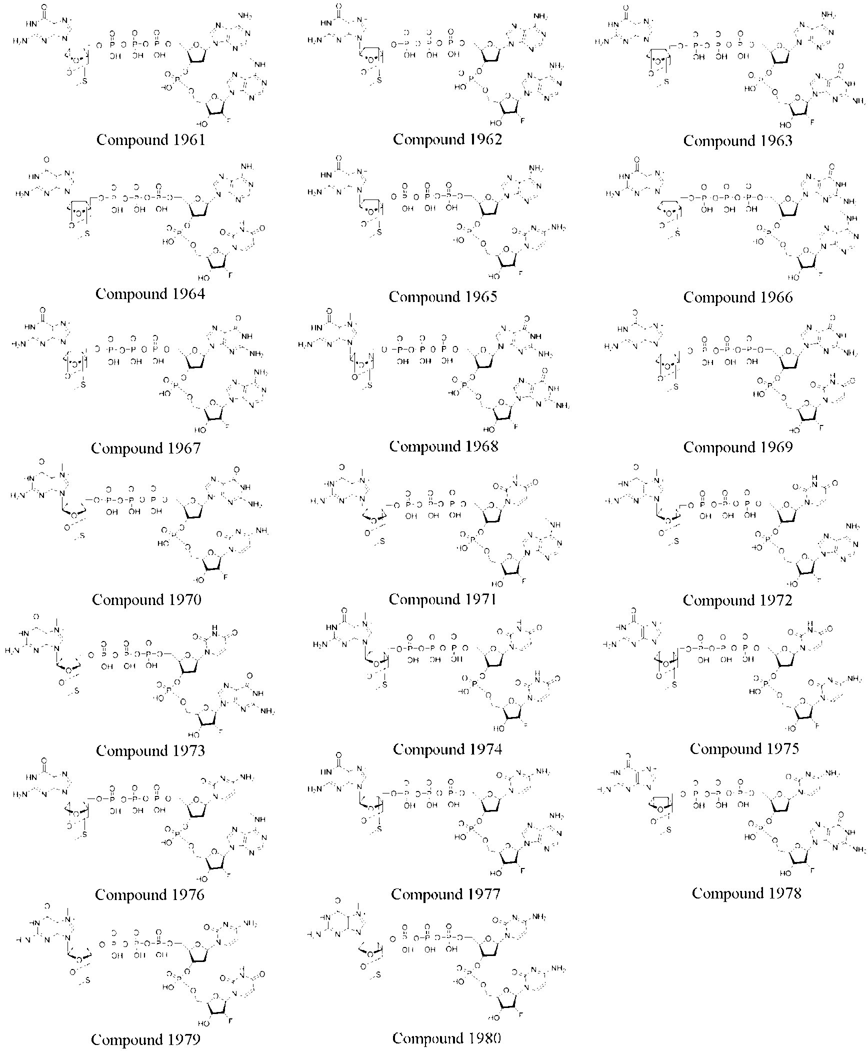

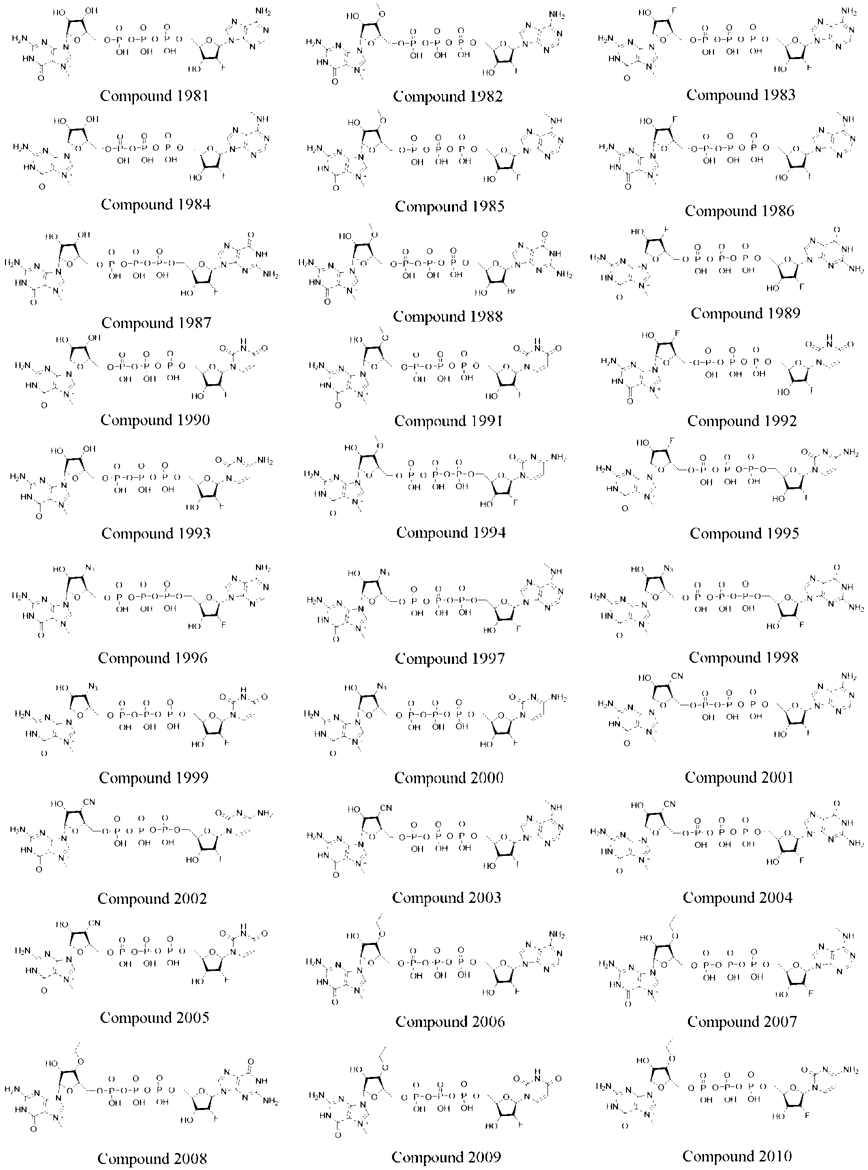

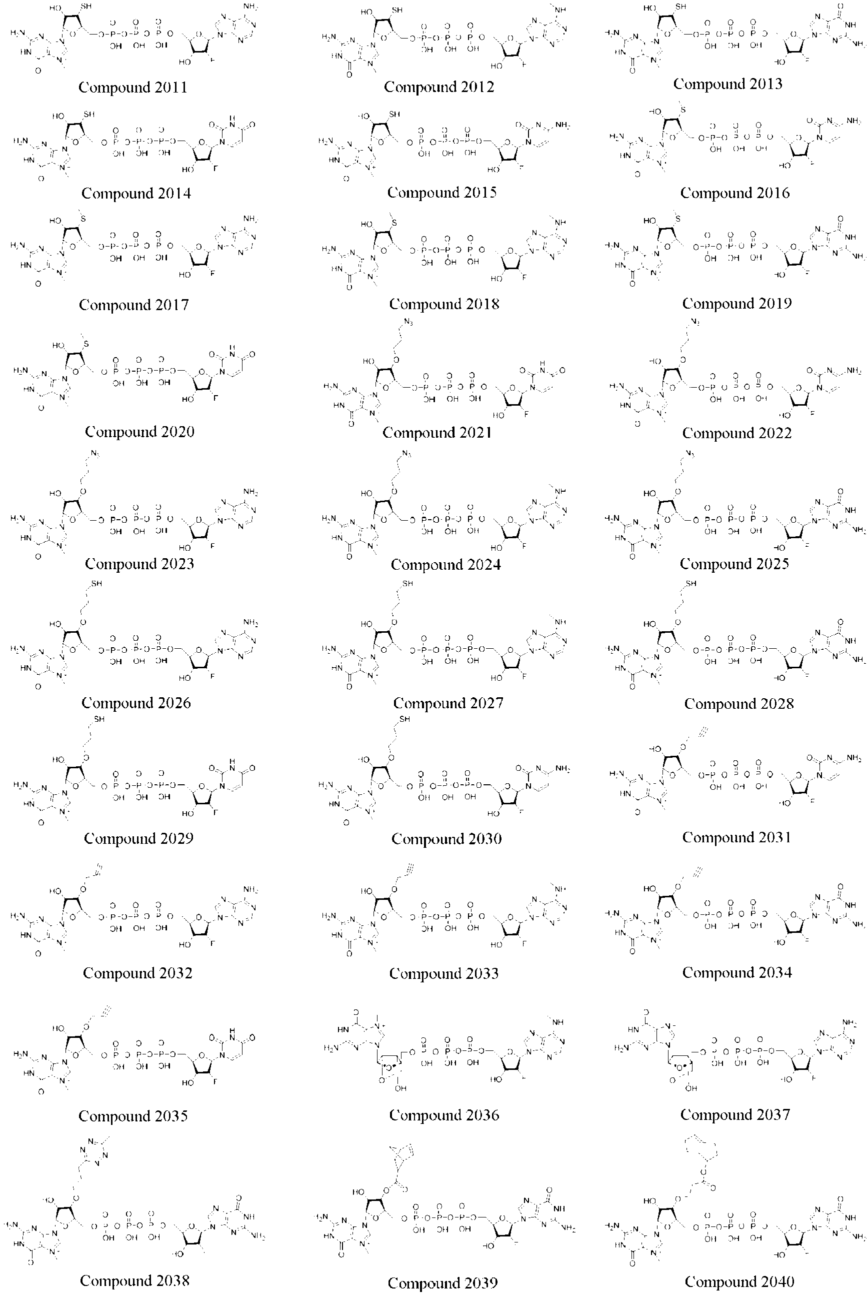

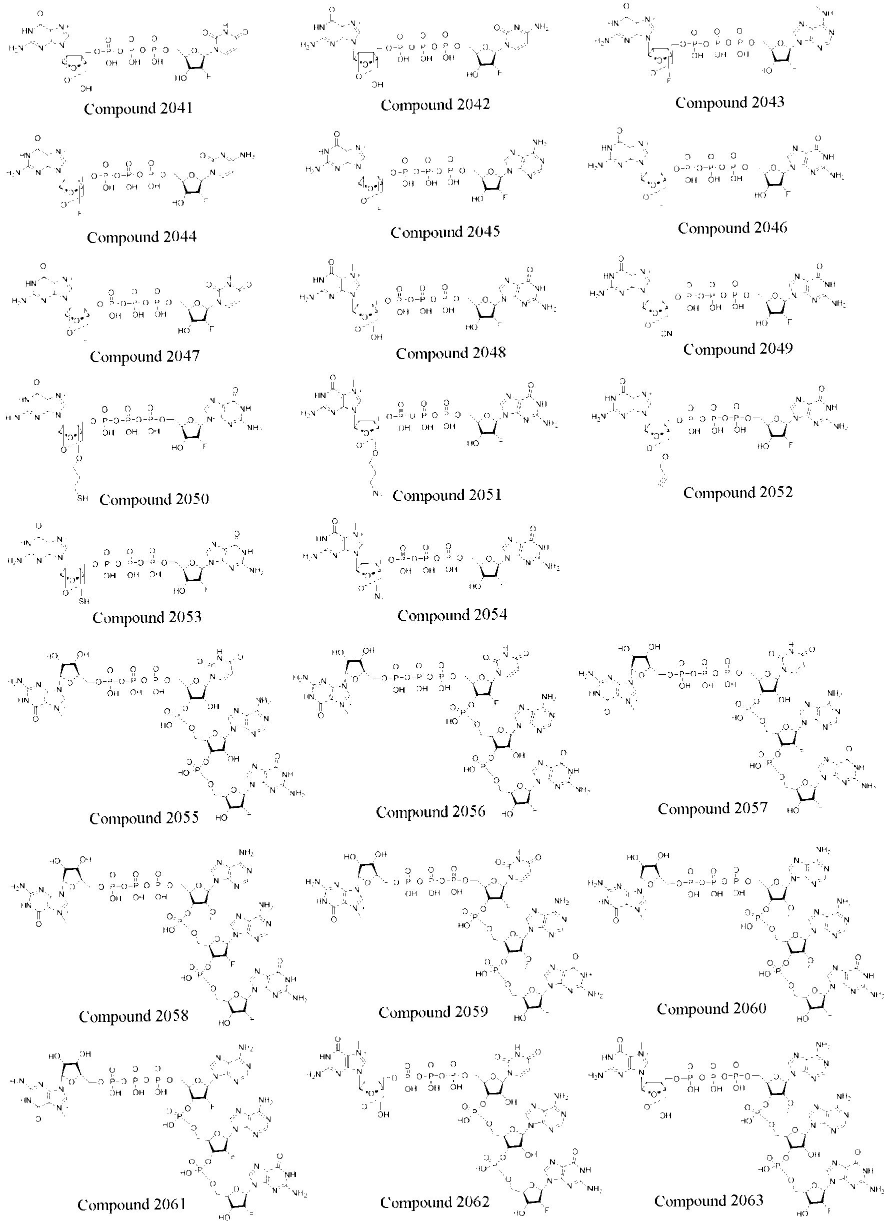

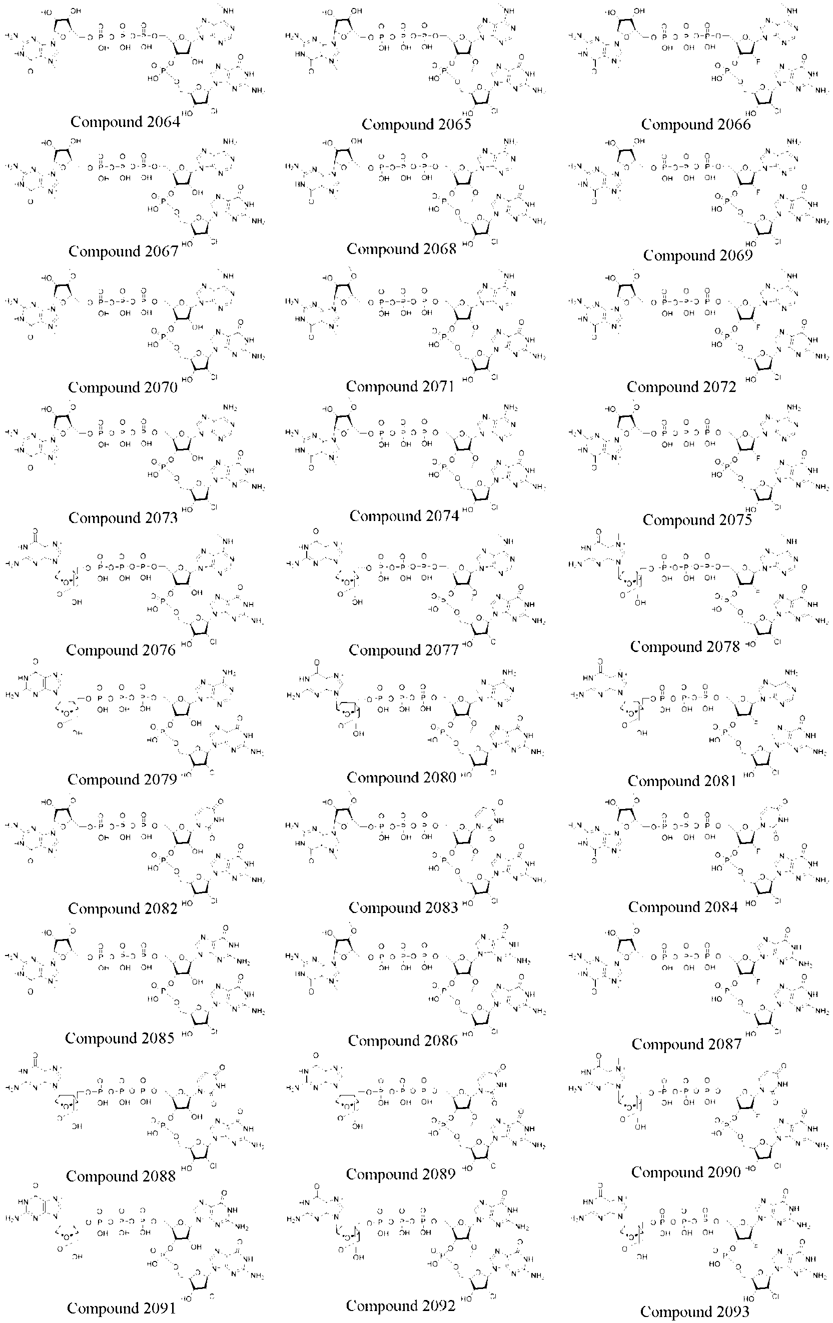

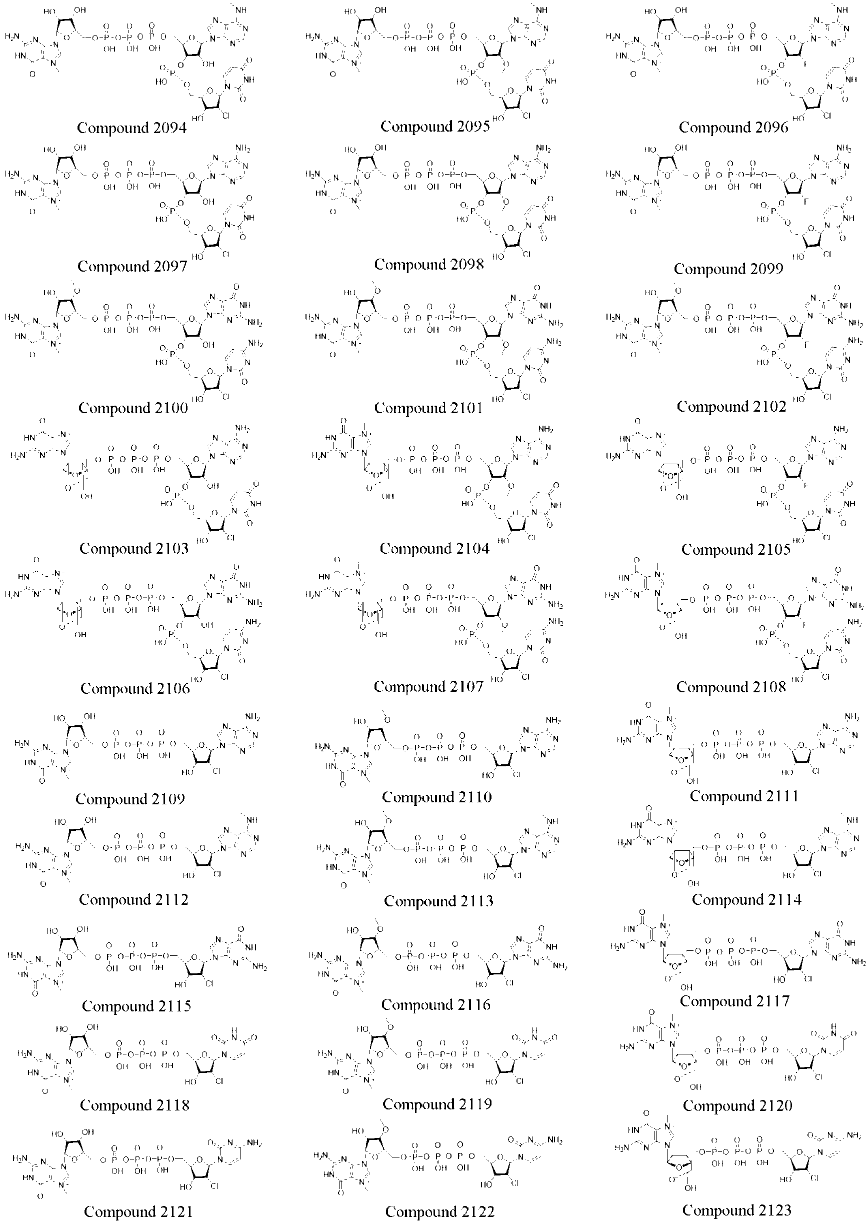

### Brief Description of Drawings

The present invention will be further described below with reference to the accompanying drawings.
FIG. 1 illustrates the fluorescence imaging of mRNA with different cap analogs in HEK293T cells.
FIG. 2 illustrates the fluorescence intensity of mRNA with different cap analogs in HEK293T cells.
FIG. 3 illustrates the fluorescence intensity of mRNA with different cap analogs in HepG2 cells.
FIG. 4 illustrates the expression efficiency of mRNA with different cap analogs in different organs.

### Detailed Description

The present invention provides a halogen-containing compound ("cap analog" or "capping analog") for capping 5' end of RNA, a pharmaceutically acceptable salt, solvate, and stereoisomer thereof, and a use of the halogen-containing compound. The use of this compound to cap 5' end of mRNA has a high capping rate and good *in vitro* transcription efficiency. At the same time, it has a high translation expression efficiency at the cellular level.

Before further describing the present invention, several terms used in the specification, examples, and appended claims are collected in the following sections. The definitions listed herein should be read and understood by those skilled in the art in light of the rest of the present invention. Unless otherwise defined, all technical and scientific terms used herein have the same meaning as commonly understood by those skilled in the art to which the present invention belongs.

### Definition

Unless otherwise stated, when disclosing or claiming any type of range, it is intended to disclose or claim individually each possible value that the range may reasonably cover, including any sub-ranges contained therein. For example, if the number of groups is 1 to 6, it indicates an integer within the range, and 1 to 6 is understood to include 1, 2, 3, 4, 5, and 6, and should also be understood to include sub-ranges of 1 to 5, 1 to 4, and 1 to 3.

The specification of the present disclosure should be interpreted in consistent with the laws and principles of chemical bonding. In some cases, a hydrogen atom may be removed to accommodate a substituent at a given position.

The words "include", "contain", "comprise", or the like used in the present disclosure means that the elements preceding the word include the elements listed after the word and equivalents thereof, without excluding elements that are not described. The terms "contain" or "include (comprise)" used herein may be open, semi-closed or closed. In other words, the terms also include "essentially consisting of" or "consisting of".

The term "pharmaceutically acceptable" as used herein means that a compound or composition is chemically and/or toxicologically compatible with the other ingredients constituting a formulation and/or with humans or mammals for the prevention or treatment of a disease or condition for which the compound or composition is used.

Natural or modified pyrimidine nucleotide bases include, but are not limited to, uracil, thymine, cytosine, 5-methylcytosine, 5-fluorouracil, 5-fluorocytosine, and the like.

Natural or modified purine nucleotide bases include, but are not limited to, adenine, guanine, 6-N-methyladenine, 6-N,N-dimethylaminopurine, 2-N-methylguanine, 2-N,N-dimethylguanine, 7-methylguanine, and the like. The structure of 6-N-methyladenine is 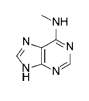 .

Nucleotide bases as used herein can be modified or substituted to provide oligonucleotides. For example, modification can be performed by these bases or synthetic and natural nucleotide bases (for example, inoside, thymine, xanthine, hypoxanthine, nubularine, isoguanine or tuberculin) and optionally. Alternatively, substituted or modified analogs of any natural or synthetic base may be used. Examples include 2-(halogenated)adenine, 2-(alkyl)adenine, 2-(propyl)adenine, 2-(amino)adenine, 2-(aminoalkyl)adenine, 2-(aminopropyl)adenine, 2-(methylthio)-N6-(isopentenyl)adenine, 6-(alkyl)adenine, 6-(methyl)adenine, 7-(deaza)adenine, 8-(alkenyl)adenine, 8-(alkyl)adenine, 8-(alkynyl)adenine, 8-(amino)adenine, 8-(halogenated)adenine, 8-(hydroxy)adenine, 8-(thioalkyl)adenine, 8-(thiol)adenine, N6-(isopentyl)adenine, N6-(methyl)adenine, N6,N6-(dimethyl)adenine, 2-(alkyl)guanine, 2-(propyl)guanine, 6-(alkyl)guanine, 6-(methyl)guanine, 7-(alkyl)guanine, 7-(methyl)guanine, 7-(deaza)guanine, 8-(alkyl)guanine, 8-(alkenyl)guanine, 8-(alkynyl)guanine, 8-(amino)guanine, 8-(halogenated)guanine, 8-(hydroxy)guanine, 8-(thioalkyl)guanine, 8-(thiol)guanine, N-(methyl)guanine, 2-(thio)cytosine, 3-(deaza)-5-(aza)cytosine, 3-(alkyl)cytosine, 3-(methyl)cytosine, 5-(alkyl)cytosine, 5-(alkynyl)cytosine, 5-(halogenated)cytosine, 5-(methyl)cytosine, 5-(propynyl)cytosine, 5-(propynyl)cytosine, 5-(trifluoromethyl)cytosine, 6-(azo)cytosine, N-4-(acetyl)cytosine, 3-(3-amino-3-carboxypropyl)uracil, 2-(thio)uracil, 5-(methyl)-2-(thio)uracil, 5-(methylaminomethyl)-2-(thio)uracil, 4-(thio)uracil, 5-(methyl)-4-(thio)uracil, 5-(methylaminomethyl)-4-(thio)uracil, 5-(methyl)-2,4-(dithio)uracil, 5-(methylaminomethyl)-2,4-(dithio)uracil, 5-(2-aminopropyl)uracil, 5-(alkyl)uracil, 5-(alkynyl)uracil, 5-(allylamino)uracil, 5-(aminoallyl)uracil, 5-(aminoalkyl)uracil, 5-(guanidinyl)uracil, 5-(1,3-diazole-1-alkyl)uracil, 5-(cyanoalkyl)uracil, 5-(dialkylaminoalkyl)uracil, 5-(dimethylaminoalkyl)uracil, 5-(halogenated)uracil, 5-(methoxy)uracil, uracil-5-oxoacetic acid, 5-(methoxycarbonylmethyl)-2-(thio)uracil, 5-(methoxycarbonylmethyl)uracil, 5-(propynyl)uracil, 5-(propynyl)uracil, 5-(trifluoromethyl)uracil, 6-(azo)uracil, dihydrouracil, 3-(methyl)uracil, 5-uracil (that is, pseudouracil), 2-(thio)pseudouracil, 4-(thio)pseudouracil, 2,4-(dithio)purine pyrimidine, 5-(alkyl)pseudouracil, 5-(methyl)pseudouracil, 5-(alkyl)-2-(thio)pseudouracil, 5-(methyl)-2-(thio)pseudouracil, 5-(alkyl)-4-(thio)pseudouracil, 5-(methyl)-4-(thio)pseudouracil, 5-(alkyl)-2,4-(dithio)pseudouracil, 5-(methyl)-2,4-(dithio)pseudouracil, 1-substituted pseudouracil, 1-substituted 2(thio)-pseudouracil, 1-substituted 4-(thio)pseudouracil, 1-substituted 2,4-(dithio)pseudouracil, 1-(aminocarbonylvinyl)-pseudouracil, 1-(aminocarbonylvinyl)-2(thio)-pseudouracil, 1-(aminocarbonylvinyl)-4-(thio)pseudouracil, 1-(aminocarbonylvinyl)-2,4-(dithio)pseudouracil, 1-(aminoalkylaminocarbonylvinyl)-pseudouracil, 1-(aminoalkylamino-carbonylvinyl)-2(thio)-pseudouracil, 1-(aminoalkylaminocarbonylvinyl)-4-(thio)pseudouracil, 1-(aminoalkylaminocarbonylvinyl)-2,4-(dithio)pseudouracil, 1,3-(diaza)-2-(oxo)-phenoxazine-1-yl, 1-(aza)-2-(thio)-3-(aza)-phenoxazine-1-yl, 1,3-(diaza)-2-(oxo)-phenothiazine-1-yl, 1-(aza)-2-(thio)-3-(aza)-phenothiazine-1-yl, 7-substituted 1,3-(diaza)-2-(oxo)-phenoxazine-1-yl, 7-substituted 1-(aza)-2-(thioxo)-3-(aza)-phenoxazine-1-yl, 7-substituted 1,3-(diaza)-2-(oxo)-phenothiazine-1-yl, 7-substituted 1-(aza)-2-(thioxo)-3-(aza)-phenothiazine-1-yl, 7-(aminoalkylhydroxy)-1,3-(diaza)-2-(oxo)-phenoxazine-1-yl, 7-(aminoalkylhydroxy)-1-(aza)-2-(thioxo)-3-(aza)-phenoxazine-1-yl, 7-(aminoalkylhydroxy)-1,3-(diaza)-2-(oxo)-phenothiazine-1-yl, 7-(aminoalkylhydroxy)-1-(aza)-2-(thio)-3-(aza)-phenothiazine-1-yl, 7-(guanidinoalkylhydroxy)-1,3-(diaza)-2-(oxo)-phenoxazine-1-yl, 7-(guanidinoalkylhydroxy)-1-(aza)-2-(thio)-3-(aza)-phenoxazine-1-yl, 7-(guanidinium alkylhydroxy)-1,3-(diaza)-2-(oxo)-phenothiazine-1-yl, 7-(guanidinoalkylhydroxy)-1-(aza)-2-(thio)-3-(aza)-phenothiazine-1-yl, 1,3,5-(triaza)-2,6-(dioxa)naphthalene, inosine, xanthine, hypoxanthine, Zebularine, tuberculin, isoguanosine, inosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, 3-(methyl)isocarbostyryl, 5-(methyl)isocarbostyryl, 3-(methyl)-7-(propynyl)isocarbostyryl, 7-(aza)indolyl, 6-(methyl)-7-(aza)indolyl, iminopyridinyl, 9-(methyl)-iminopyridinyl, pyrrolopyrazinyl, isocarbenyryl, 7-(propynyl)isocarbenyryl, propynyl-7-(aza)indolyl, 2,4,5-(trimethyl)phenyl, 4-(methyl)indolyl, 4,6-(dimethyl)indolyl, phenyl, naphthyl, anthracenyl, phenanthrenyl, pyrenyl, stilbenyl, tetraphenyl, pentaphenyl, difluorotolyl, 4-(fluoro)-6-(methyl)benzimidazole, 4-(methyl)benzimidazole, 6-(azo)thymine, 2-pyridone, 5-nitroindole, 3-nitropyrrole, 6-(aza)pyrimidine, 2-(amino)purine, 2,6-(diamino)purine, 5-substituted pyrimidine, 2-substituted purine, N6-substituted purine, O6-substituted purine, substituted 1,2,4-triazole or any O-alkylated or N-alkylated derivative thereof.

"Stereoisomers" refer to compounds that have the same chemical constitution but differ in the arrangement of the atoms or groups in space. Stereoisomers include enantiomers, diastereomers, conformational isomers (rotamers), geometric isomers (cis/trans isomers), atropisomers, and the like.

The expression "connected to form a ring by a chemical bond" means connecting two groups by a carbon-carbon bond, a carbon-oxygen bond, a carbon-nitrogen bond, a carbon-sulfur bond, or the like to form a ring structure. If necessary, hydrogen atoms can be reduced by 1 or 2 from corresponding group.

The expression "optionally substituted" means that one, two, three or more than three hydrogen atoms in a group may be replaced independently by respective substituents. The substituents may be selected from the group consisting of alkyl, alkenyl, alkoxy, halogen, cyano, amino, nitro, and -OH.

The term "alkyl" refers to a saturated straight or branched carbon chain. Preferably, the chain contains 1 to 10 carbon atoms, that is, 1, 2, 3, 4, 5, 6, 7, 8, 9 or 10 carbon atoms, preferably 1 to 6 carbon atoms, most preferably 1 to 3 carbon atoms. The alkyl is, for example, methyl, ethyl, n-propyl, isopropyl, butyl, isobutyl, tert-butyl, pentyl, hexyl, pentyl or octyl. The alkyl is optionally substituted.

The term "alkoxy" includes -O- alkyl groups and alkyl groups in which the O atom is in the alkyl chain, such as -CH₂-O-CH₃. The alkoxy contains 1 to 10 carbon atoms, preferably 1 to 6 carbon atoms, and most preferably 1 to 3 carbon atoms. The alkoxy is optionally substituted.

The term "alkenyl" includes both straight and branched alkyl groups containing at least two carbon atoms and at least one carbon-carbon double bond. The alkenyl contains 2 to 10 carbon atoms, preferably 2 to 6 carbon atoms, and most preferably 2 to 3 carbon atoms. The alkenyl is optionally substituted.

The term "alkynyl" includes both straight and branched alkyl groups containing at least two carbon atoms and at least one carbon-carbon triple bond. The alkynyl contains 2 to 10 carbon atoms, preferably 2 to 6 carbon atoms, and most preferably 2 to 3 carbon atoms. The alkynyl group is optionally substituted.

The terms "cycloalkyl", "cycloalkenyl" and "cycloalkynyl" are used by themselves or in combination with other terms to represent cyclic forms of "alkyl", "alkenyl" and "alkynyl", respectively. Preferably, the cyclic forms are formed by 3, 4, 5, 6, 7, 8, 9 or 10 atoms in the ring, such as cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, cyclooctenyl, cyclopropynyl, cyclobutynyl, cyclohexynyl, cyclopentynyl, and the like. The connection position of the cycloalkenyl or cycloalkynyl to the other group can be at any suitable position. The terms "cycloalkyl", "cycloalkenyl" and "cycloalkynyl" are also intended to include bicyclic, tricyclic and polycyclic forms thereof, which may be spirocyclic or bridged rings. The "cycloalkyl", "cycloalkenyl" and "cycloalkynyl" are optionally substituted. Examples of cycloalkyl and cycloalkenyl include cyclopropyl, cyclobutyl, cyclopentyl, cyclohexyl, 1-cyclohexenyl, 3-cyclohexenyl, cycloheptyl, spiro [3,3] heptyl, spiro [3,4] octyl, spiro [4,3] octyl, spiro [3,5] nonyl, spiro [5,3] nonyl, spiro [3,6] decyl, spiro [6,3] decyl, spiro [4,5] decyl, spiro [5,4] decyl, bicyclo [2.2.1] heptyl, bicyclo [2.2.2] octyl, adamantyl, norbornenyl, and the like.

The term "aryl" preferably refers to an aromatic monocyclic ring containing 6 carbon atoms, an aromatic bicyclic ring system containing 10 carbon atoms or an aromatic tricyclic ring system containing 14 carbon atoms. Examples include phenyl, naphthyl, or anthracenyl. The aryl is optionally substituted.

The term "heteroaryl" preferably refers to: a five-membered aromatic monocyclic ring or a six-membered aromatic monocyclic ring in which at least one carbon atom is replaced by 1, 2, 3 or 4 (for a five-membered ring) or 1, 2, 3, 4 or 5 (for a six-membered ring) identical or different heteroatoms, the heteroatoms being preferably selected from the group consisting of O, N and S; an aromatic bicyclic ring system in which 1, 2, 3, 4, 5 or 6 carbon atoms of 8, 9, 10, 11 or 12 carbon atoms are replaced by identical or different heteroatoms, the heteroatoms being preferably selected from the group consisting of O, N and S; or an aromatic tricyclic ring system in which 1, 2, 3, 4, 5 or 6 carbon atoms of 13, 14, 15, 16 carbon atoms are replaced by identical or different heteroatoms, the heteroatoms being preferably selected from the group consisting of O, N and S. Examples include oxazolyl, isoxazolyl, 1,2,5-oxadiazolyl, 1,2,3-oxadiazolyl, pyrrolyl, imidazolyl, pyrazolyl, 1,2,3-triazolyl, thiazolyl, isothiazolyl, 1,2,3-thiadiazolyl, 1,2,5-thiadiazolyl, pyridyl, pyrimidinyl, pyrazinyl, 1,2,3-triazinyl, 1,2,4-triazinyl, 1,3,5-triazinyl, 1,2,4,5-tetrazinyl, 1-benzofuranyl, 2-benzofuranyl, indolyl, isoindolyl, benzothienyl, 2-benzothienyl, 1H-indazolyl, benzimidazolyl, benzoxazolyl, indoloxazinyl, 2,1-benzoxazolyl, benzothiazolyl, 1,2-benzisothiazolyl, 2,1-benzisothiazolyl, benzotriazolyl, quinolyl, isoquinolyl, quinoxalinyl, quinazolinyl, quinolyl, 1,2,3-benzotriazinyl or 1,2,4-benzotriazinyl.

The term "PEG group" refers to a group in which one or more -CH₂-CH₂-O- or CH₃-CH₂-O-units are connected, for example, CH₃-CH₂-O-(CH₂-CH₂-O)ₓ-, wherein x is selected from an integer from 0 to 6, preferably an integer from 0 to 4. The PEG group may be optionally substituted.

The term "pharmaceutically acceptable salt" refers to relatively non-toxic addition salts of the compound of the present disclosure. See, for example, S. M. Berge et al. "Pharmaceutical Salts"", J. Pharm. Sci. 1977, 66, 1-19.

Suitable pharmaceutically acceptable salts of the compound of the present disclosure may be acid addition salts of the compound of the present disclosure having a nitrogen atom in a chain or ring and having sufficient basicity, such as acid addition salts formed with inorganic acids such as hydrochloric acid, hydrobromic acid, hydroiodic acid, sulfuric acid, phosphoric acid or nitric acid, or acid addition salts formed with organic acids such as formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecyl sulfuric acid, ethanesulfonic acid, benzenesulfonic acid, p-toluenesulfonic acid, methanesulfonic acid, 2-naphthalenesulfonic acid, naphthalene disulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid or thiocyanic acid.

In addition, another suitable pharmaceutically acceptable salt of the compound of the present invention having sufficient acidity is an alkali metal salt such as a sodium salt or a potassium salt, an alkaline earth metal salt such as a calcium salt or a magnesium salt, an ammonium salt, a triethylamine salt, or a salt formed with an organic base providing a physiologically acceptable cation, such as a salt formed with the following substances: N-methylglucamine, dimethylglucamine, ethylglucosamine, lysine, dicyclohexylamine, 1,6-hexanediamine, ethanolamine, glucosamine, sarcosine, serinol, trihydroxymethylaminomethane, aminopropanediol, 1-amino-2,3,4-butanetriol. In addition, basic nitrogen-containing groups can be quaternized using the following reagents: lower alkyl halides such as methyl, ethyl, propyl and butyl chlorides, bromides, and iodides; dialkyl sulfates such as dimethyl sulfate, diethyl sulfate, dibutyl sulfate, and dipentyl sulfate; long chain halides such as decyl, lauryl, myristyl and stearyl chlorides, bromides, and iodides; aralkyl halides such as benzyl bromides and phenethyl bromides, and the like.

Those skilled in the art could also recognize that the acid addition salts of the claimed compound can be prepared by reacting the compound with a suitable inorganic or organic acid by any of a number of known methods. Alternatively, alkali metal salts and alkaline earth metal salts of the acidic compound of the present disclosure can be prepared by reacting the compound with a suitable base by various known methods.

The present invention includes all possible salts of the compound of the present disclosure, which may be a single salt or any mixture of any proportion of the salts.

The term "solvate" is a substance formed by combining, physically binding and/or solvating the compound of the present invention with a solvent molecule, such as a disolvate, a monosolvate or a hemisolvate, in which the ratio of the solvent molecule to the compound of the present invention is about 2:1, about 1:1 or about 1:2, respectively. This physical binding involves ionization and covalent bonding (including hydrogen bonding) to varying degrees. In some cases (for example, when one or more solvent molecules are incorporated into the lattice of a crystalline solid), the solvate can be separated. Therefore, the solvate includes solution phases and separable solvates. The compound of the present invention, together with a pharmaceutically acceptable solvent (such as water, methanol and ethanol), can be present in solvated form, and the present application is intended to cover solvated and non-solvated forms of the compound of the present invention. One of the solvates is a hydrate.

The term "pharmaceutical composition" as used in the present application refers to a substance and/or a combination of substances used to identify, prevent or treat a tissue condition or disease. The pharmaceutical composition is formulated to be suitable for administration to a patient to diagnose, prevent and/or treat a disease. In addition, the pharmaceutical composition refers to a combination of an active agent and an inert or active carrier that makes the composition suitable for therapeutic use.

As used herein, the term "carrier" refers to a diluent, adjuvant, excipient or vehicle used together with a therapeutic agent. This pharmaceutical carrier can be a sterile liquid, such as a saline solution in water and oil, including those of petroleum, animal, plant or synthetic origin, such as peanut oil, soybean oil, mineral oil, sesame oil, and the like. When the pharmaceutical composition is administered intravenously, a saline solution is a preferred carrier. A saline solution, an aqueous glucose solution and a glycerol solution can also be used as liquid carriers, particularly as injectable solutions. Suitable pharmaceutical excipients include starch, glucose, lactose, sucrose, gelatin, malt, rice, flour, chalk, silica gel, sodium stearate, glyceryl monostearate, talc, sodium chloride, skim milk powder, glycerol, propylene, ethylene glycol, water, ethanol, and the like. If necessary, the composition may also contain a small amount of a wetting agent, emulsifier, or pH buffer. Examples of suitable pharmaceutical carriers are described in "Remington's Pharmaceutical Sciences" by E. W. Martin.

The term "halogen" refers to fluorine, chlorine, bromine and iodine.

The term "optionally" means that this may occur, or may not occur.

### Examples

### Reagents and Models Used

The starting materials in the examples are commercially available and/or can be prepared by a number of methods well known to those skilled in the art of organic synthesis. Those skilled in the art of organic synthesis will appropriately select reaction conditions (including solvent, reaction atmosphere, reaction temperature, duration of experiment and post-treatment) in the following synthetic methods. Those skilled in the art of organic synthesis will understand that functional groups present on each part of a molecule should be compatible with reagents and reactions provided.

All reagents and compounds synthesized can be purchased through general commercial channels in China. The suppliers include Sigma-Aldrich (USA), Shanghai Hongene Biotech Corporation. (Trinlink Cleancap), jetMESSENGER (Polyplus-transfection^{®}), Shanghai Titan Technology Co., Ltd., and the like.

Cell model: HEK293T cells were purchased from the Shanghai Cell Bank of the Chinese Academy of Sciences, and HepG2 cells were purchased from Wuhan Pricella Biotechnology Co., Ltd.

Main instruments used: Multi-mode microplate reader (Molecular Devices), and flow cytometer (CytoFLEX S series).

Compound preparation and identification: Nuclear magnetic resonance spectrometer (Bruker 300MHz), liquid chromatography-mass spectrometry (Agilent 6150/1290), and high-performance liquid chromatography (Agilent 1260).

Cell experiment: Inverted fluorescence microscope (Guangzhou Mingmei Optoelectronic Technology Co., Ltd), and cell culture incubator (Thermo Fisher Scientific).

The implementation process and beneficial effects of the present invention will be described in detail below through specific examples, which are intended to help readers better understand the essence and characteristics of the present invention, and are not intended to limit the implementable scope of the present invention.

### Final Product Synthesis:

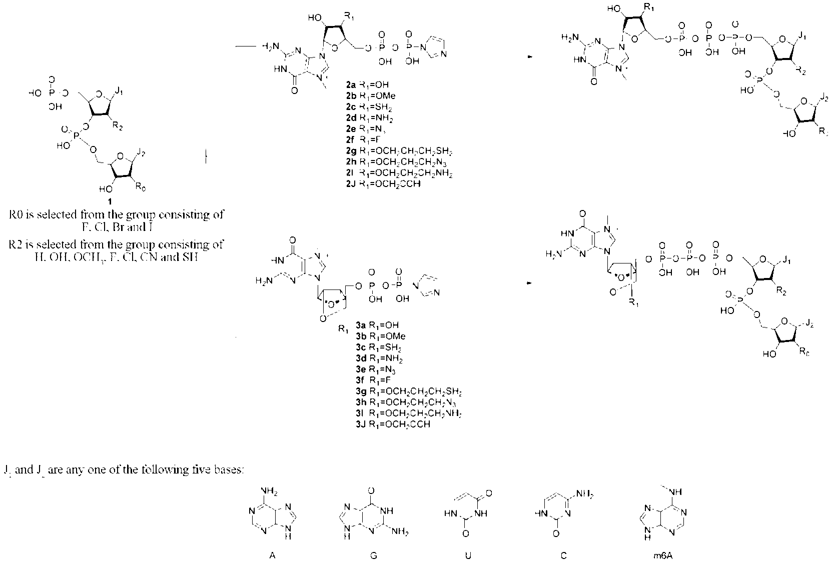

### Final Product Synthesis Method 1

To 8 mL of anhydrous DMSO, 0.4 g of a compound ***1*** was dissolved, and 2 eq of a compound ***2*** and 20 eq of anhydrous zinc chloride were added under the protection of argon. The reaction solution was stirred at room temperature 25°C for 24 hours under the protection of argon. After the reaction was completed by TLC monitoring, 150 mL of 0.25 M EDTA solution was used to terminate the reaction, and the mixture was loaded onto a DEAE Sephadex column. The product was eluted by linear gradient elution using an ammonium bicarbonate eluent of 0 M to 1.0 M. The product-containing eluent was collected and freeze-dried to obtain a product.

### Final Product Synthesis Method 2

To 16 mL of an aqueous solution with a pH of 7.0, containing 0.2 mol/L N-methylmorpholine and 0.2 mol/L manganous chloride, 0.2 g of the compound ***2*** was dissolved, and then 0.2 g of the compound ***1*** was added to the solution. The reaction solution was stirred at room temperature of 25°C for 16 hours. After the reaction was completed by TLC monitoring, 150 mL of 0.25 M EDTA solution was used to terminate the reaction, and the mixture was loaded onto a DEAE Sephadex column. The product was eluted by linear gradient elution using an ammonium bicarbonate eluent of 0 M to 1.0 M. The product-containing eluent was collected and freeze-dried to obtain a product.

### Synthesis of Representative Structural Compounds

### Example 1

### Synthesis of Compound 3

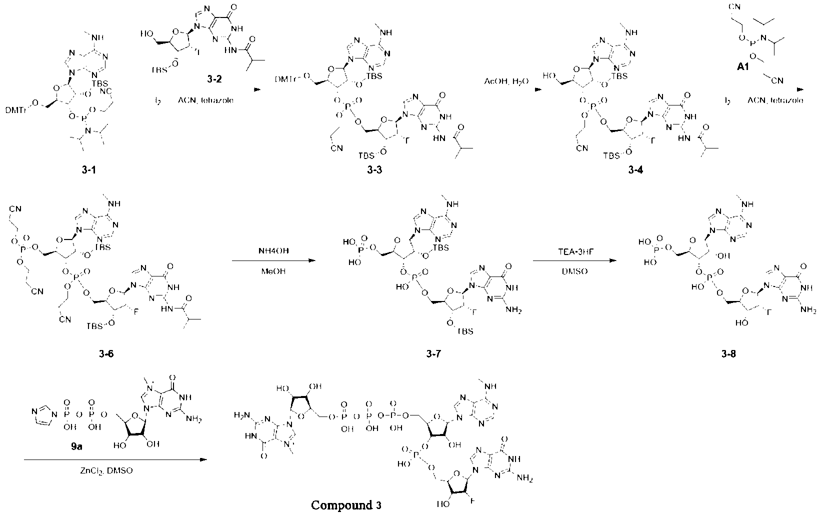

### Step 1:

A compound ***3-2*** (2.38 g) was added to a solution of tetrazole (1.76 g) in acetonitrile (63 mL) in a three-necked flask, and the atmosphere was replaced with argon three times. Next, at room temperature of 25°C, a compound ***3-1*** (5 g) was dissolved in 10 mL of acetonitrile and added to the above solution. The resulting solution was stirred at room temperature of 25°C for 1 hour. No obvious heat release was found, and TLC monitoring showed that the compound 3-1 disappeared. Then, into the solution, a solution of iodine in pyridine/tetrahydrofuran/water (0.5 mmol/mL, pyridine: tetrahydrofuran: water = 1:8:1) was added dropwise until the solution no longer faded. After that, the reaction solution was stirred for another 0.5 hours, and TLC monitoring showed that the oxidation was completed. After adding an aqueous saturated solution (10 mL) of sodium sulfite to the reaction solution for quenching, 50 mL of water was further added for dilution, and the mixture was extracted with dichloromethane (50 mL x 2). The organic phases were combined and washed once with water (50 mL), and concentrated to obtain a light yellow oily product ***3-3*** (8 g, crude product).

### Step 2:

The compound ***3-3*** (8 g, crude product) was dissolved in 40 mL of acetic acid and 10 mL of water, and the reaction solution was stirred at 25°C for 16 hours. TLC monitoring showed that the compound ***3-3*** disappeared and a spot with large polarity was generated. The reaction solution was directly concentrated in vacuo. After concentration, appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white solid product ***3-4*** (2.8 g, 51% overall yield over two-step) was obtained.

### Step 3:

28 mL of a tetrazole solution in acetonitrile (0.4 mmol/mL) was prepared. The compound ***3-4*** (2.8 g) was added to the above solution, and then a compound ***A1*** (3 g) was added to the solution at room temperature of 25°C. The atmosphere was replaced with nitrogen three times, and the reaction solution was stirred at room temperature of 25°C for 1 hour. TLC monitoring showed that the reaction was completed. The reaction solution was cooled to below 10°C in an ice-water bath, and a solution of iodine in pyridine/tetrahydrofuran/water (0.5 mmol/mL, pyridine:tetrahydrofuran:water = 1:8:1) was added dropwise until the reaction solution no longer faded. TLC monitoring showed that the oxidation reaction was completed. The reaction solution was quenched by adding 10 mL of an aqueous saturated solution of sodium sulfite, diluted with water, and extracted three times with ethyl acetate. The organic phases were combined and dried over anhydrous sodium sulfate and filtered. Appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white foamy solid compound ***3-6*** (2.6 g, 78.2% yield) was obtained.

### Step 4:

The compound ***3-6*** (2.6 g) was dissolved in methanol (30 mL) and concentrated aqueous ammonia (30 mL) was added. The resulting solution was stirred at room temperature of 25°C for 60 hours. TLC monitoring showed that the compound ***3-6*** was completely reacted. The reaction solution was concentrated in vacuo and concentrated again with methanol to obtain a light yellow oily liquid compound ***3-7*** (2.4 g, crude product), which was directly used for the next step.

### Step 5:

The compound ***3-7*** (2.4 g, crude product) was dissolved in DMSO (3 mL) and triethylamine trihydrofluoride (3.5 mL) was added. The reaction solution was stirred at 50°C for 1 hour. TLC monitoring showed that the compound ***3-7*** was completely reacted. The reaction solution was diluted to 50 mL with water, and the pH was adjusted to 5.5 with 1 N NaOH aqueous solution. The mixture was loaded onto a DEAE Sephadex column. The product was eluted by linear gradient elution using an eluent of 0 M to 1.0 M ammonium bicarbonate aqueous solution. Most of the water in the obtained fraction was concentrated in vacuo, and the remaining liquid was freeze-dried to obtain a target compound, amine salt ***3-8*** (0.8 g, 33.7% yield), which was a white solid.

### Step 6:

A compound ***9a*** (200 mg) was added to 16 mL of an aqueous solution with a pH of 7.0, containing 0.2 mol/L N-methylmorpholine and 0.2 mol/L manganous chloride, and the compound ***3-8*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***5,*** which was an ammonium salt in the form of white powder (65 mg).

¹H NMR (400 MHz, D₂O) δ 8.08 (s, 1H), 7.89 (s, 1H), 7.65 (s, 1H), 5.93 (d, J = 17.9 Hz, 1H), 5.70 (d, J = 6.4 Hz, 2H), 5.28 - 5.02 (m, 1H), 4.58 - 3.99 (m, 14H), 3.84 (s, 3H), 3.00 - 2.70 (m, 3H).

³¹P NMR (162 MHz, D₂O) δ -0.80, -11.55, -23.03.

### Example 2

### Synthesis of Compound 35

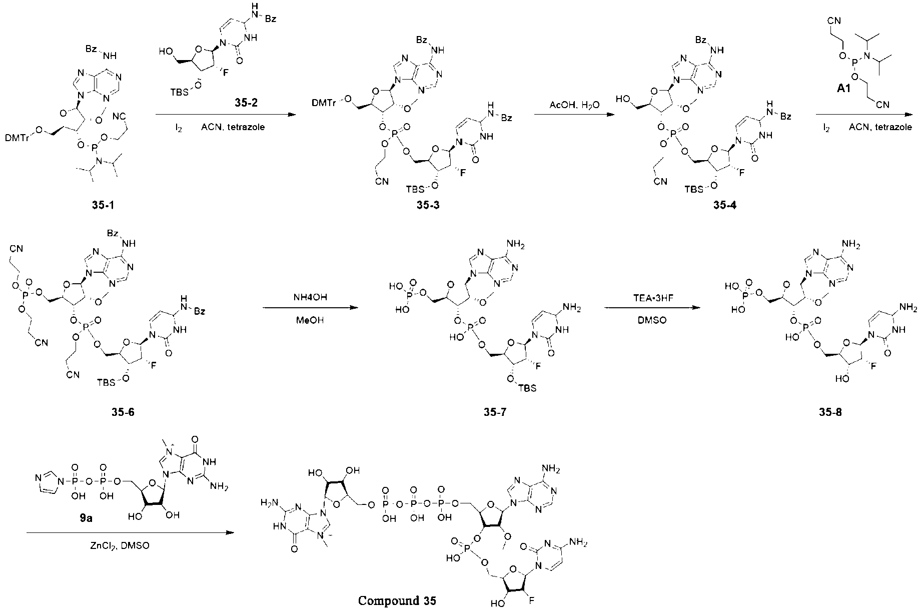

### Step 1:

A compound ***35-2*** (2.07 g) was added to a solution of tetrazole (1.6 g) in acetonitrile (56 mL) in a three-necked flask, and the atmosphere was replaced with argon three times. Next, at room temperature of 25°C, a compound ***35-1*** (5 g) was dissolved in 10 mL of acetonitrile and added to the above solution. The resulting solution was stirred at room temperature of 25°C for 1 hour. No obvious heat release was found, and TLC monitoring showed that the compound ***2-1*** disappeared. Then, into the solution, iodine solution (a 0.5 mmol/mL solution was prepared by dissolving 5 g iodine in 40 mL of the mixed solution: THF:H₂O:pyridine = 8:1:1) was added dropwise until the solution no longer faded. After that, the reaction solution was stirred for another 0.5 hours, and TLC monitoring showed that the oxidation was completed. After adding Na₂SO₃ aqueous solution (10 mL) to the reaction solution for quenching, 50 mL of water was further added for dilution, and the mixture was extracted with dichloromethane (50 mL x 2). The organic phases were combined and washed once with water (50 mL), and concentrated to obtain a light yellow oily product ***35-3*** (7.5 g, crude product).

### Step 2:

The compound ***35-3*** (7.2 g, crude product) was dissolved in 40 mL of acetic acid and 10 mL of water, and the reaction solution was stirred at 25°C for 16 hours. The TLC monitoring showed that the compound ***35-3*** disappeared and a spot with large polarity was generated. The reaction solution was directly concentrated in vacuo. After concentration, appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white solid product ***35-4*** (2.8 g, 54.8% overall yield over two-step) was obtained.

### Step 3:

28 mL of a tetrazole solution in acetonitrile (0.4 mmol/mL) was prepared. The compound ***35-4*** (2.8 g) was added to the above solution, and then a compound ***A1*** (2.26 g) was added to the solution at room temperature of 25°C. The atmosphere was replaced with nitrogen three times, and the reaction solution was stirred at room temperature of 25°C for 1 hour. TLC monitoring showed that the reaction was completed. The reaction solution was cooled to below 10°C in an ice-water bath, and a solution of iodine in pyridine/tetrahydrofuran/water (0.5 mmol/mL, pyridine:tetrahydrofuran:water = 1:8:1) was added dropwise until the reaction solution no longer faded. TLC monitoring showed that the oxidation reaction was completed. The reaction solution was quenched by adding 10 mL of an aqueous saturated solution of sodium sulfite, diluted with water, and extracted three times with ethyl acetate. The organic phases were combined and dried over anhydrous sodium sulfate and filtered. Appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white foamy solid compound ***35-6*** (3.1 g, 93.5% yield) was obtained.

### Step 4:

The compound ***35-6*** (3.1 g) was dissolved in methanol (30 mL) and concentrated aqueous ammonia (30 mL) was added. The resulting solution was stirred at room temperature of 25°C for 60 hours. TLC monitoring showed that the compound ***35-6*** was completely reacted. The reaction solution was concentrated in vacuo and concentrated again with methanol to obtain a light yellow oily liquid compound ***35-7*** (2.4 g, crude product).

### Step 5:

The compound ***35-7*** (2.4 g, crude product) was dissolved in DMSO (3 mL) and triethylamine trihydrofluoride (3.5 mL) was added. The reaction solution was stirred at 50°C for 1 hour. TLC monitoring showed that the compound ***35-7*** was completely reacted. The reaction solution was diluted to 50 mL with water, and the pH was adjusted to 5.5 with 1 N NaOH aqueous solution. The mixture was loaded onto a DEAE Sephadex column. The product was eluted by linear gradient elution using 0 M to 1.0 M TEAB eluent. Most of the water in the obtained fraction was concentrated in vacuo, and the remaining liquid was freeze-dried to obtain a target compound triethylamine salt ***35-8*** (1.5 g, 51% yield), which was a white solid.

### Step 6:

At room temperature (25°C), under the protection of argon, the compound ***35-8*** (500 mg), the compound ***9a*** (500 mg) and anhydrous zinc chloride (1.2 g) were added, and anhydrous DMSO (8 mL) was added with a syringe to react for 24 hours. TLC monitoring showed that most of the compounds were reacted. The reaction solution was added to a disodium EDTA solution (1.6 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***35,*** which was an ammonium salt in the form of white powder (120 mg).

¹H NMR (400 MHz, D₂O) δ 8.25 (s, 1H), 7.98 (s, 1H), 7.59 (d, J = 7.3 Hz, 1H), 5.99 - 5.53 (m, 4H), 4.96 - 4.80 (m, 1H), 4.46 - 3.99 (m, 14H), 3.90 (s, 3H), 3.41 (s, 3H).

³¹P NMR (162 MHz, D₂O) δ -1.25, -11.58, -22.98.

### Example 3

### Synthesis of Compound 68

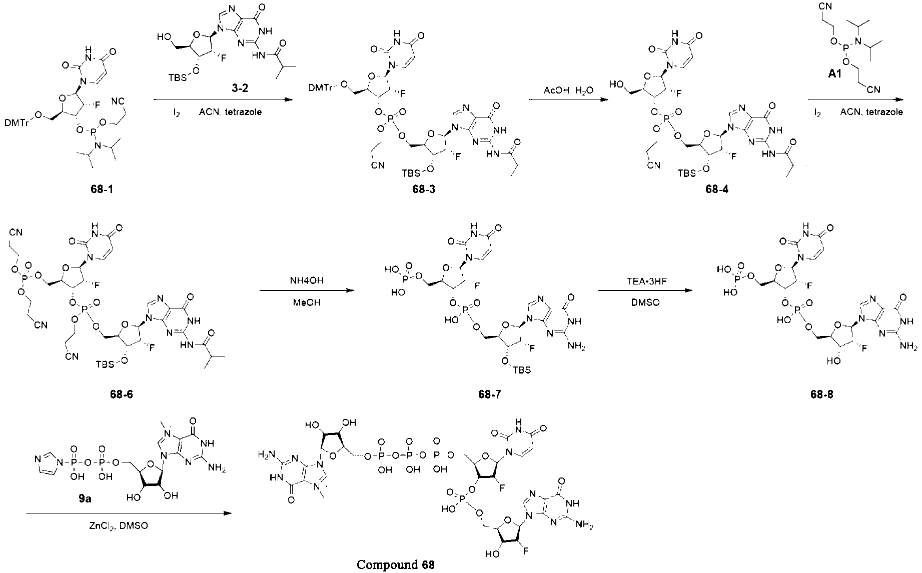

### Step 1:

The compound ***3-2*** (2.07 g) was added to a solution of tetrazole (1.6 g) in acetonitrile (56 mL) in a three-necked flask, and the atmosphere was replaced with argon three times. Next, at room temperature of 25°C, a compound ***68-1*** (5 g) was dissolved in 10 mL of acetonitrile and added to the above solution. The resulting solution was stirred at room temperature of 25°C for 1 hour. No obvious heat release was found, and TLC monitoring showed that the compound ***68-1*** disappeared. Then, into the solution, iodine solution (a 0.5 mmol/mL solution was prepared by dissolving 5 g iodine in 40 mL of the mixed solution: THF:H₂O:pyridine = 8:1:1) was added dropwise until the solution no longer faded. After that, the reaction solution was stirred for another 0.5 hours, and TLC monitoring showed that the oxidation was completed. After adding Na₂SO₃ aqueous solution (10 mL) to the reaction solution for quenching, 50 mL of water was further added for dilution, and the mixture was extracted with dichloromethane (50 mL x 2). The organic phases were combined and washed once with water (50 mL), and concentrated to obtain a light yellow oily product ***68-3*** (7.5 g, crude product).

### Step 2:

The compound ***68-3*** (7.2 g, crude product) was dissolved in 40 mL of acetic acid and 10 mL of water, and the reaction solution was stirred at 25°C for 16 hours. TLC monitoring showed that the compound ***68-3*** disappeared and a spot with large polarity was generated. The reaction solution was directly concentrated in vacuo. After concentration, appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white solid product ***68-4*** (2.8 g, 54.8% overall yield over two-step) was obtained.

### Step 3:

28 mL of a tetrazole solution in acetonitrile (0.4 mmol/mL) was prepared. The compound ***68-4*** (2.8 g) was added to the above solution, and then a compound ***A1*** (2.26 g) was added to the solution at room temperature of 25°C. The atmosphere was replaced with nitrogen three times, and the reaction solution was stirred at room temperature of 25°C for 1 hour. TLC monitoring showed that the reaction was completed. The reaction solution was cooled to below 10°C in an ice-water bath, and a solution of iodine in pyridine/tetrahydrofuran/water (0.5 mmol/mL, pyridine:tetrahydrofuran:water = 1:8:1) was added dropwise until the reaction solution no longer faded. TLC monitoring showed that the oxidation reaction was completed. The reaction solution was quenched by adding 10 mL of an aqueous saturated solution of sodium sulfite, diluted with water, and extracted three times with ethyl acetate. The organic phases were combined and dried over anhydrous sodium sulfate and filtered. Appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white foamy solid compound ***68-6*** (3.1 g, 93.5% yield) was obtained.

### Step 4:

The compound ***68-6*** (3.1 g) was dissolved in methanol (30 mL) and concentrated aqueous ammonia (30 mL) was added. The resulting solution was stirred at room temperature of 25°C for 60 hours. TLC monitoring showed that the compound ***68-6*** was completely reacted. The reaction solution was concentrated in vacuo and concentrated again with methanol to obtain a light yellow oily liquid compound ***68-7*** (2.4 g, crude product).

### Step 5:

The compound ***68-7*** (2.4 g, crude product) was dissolved in DMSO (3 mL) and triethylamine trihydrofluoride (3.5 mL) was added. The reaction solution was stirred at 50°C for 1 hour. TLC monitoring showed that the compound ***68-7*** was completely reacted. The reaction solution was diluted to 50 mL with water, and the pH was adjusted to 5.5 with 1 N NaOH aqueous solution. The mixture was loaded onto a DEAE Sephadex column. The product was eluted by linear gradient elution using 0 M to 1.0 M TEAB eluent. Most of the water in the obtained fraction was concentrated in vacuo, and the remaining liquid was freeze-dried to obtain a target compound triethylamine salt ***68-8*** (1.5 g, 51% yield), which was a white solid.

### Step 6:

At room temperature (25°C), under the protection of argon, the compound ***68-8*** (500 mg), the compound ***9a*** (500 mg) and anhydrous zinc chloride (1.2 g) were added, and anhydrous DMSO (8 mL) was added with a syringe to react for 24 hours. TLC monitoring showed that most of the materials were reacted. The reaction solution was added to a disodium EDTA solution (1.6 g, 80 mL of water was added) cooled to 0°C in advance, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***68,*** which was an ammonium salt in the form of white powder (120 mg).

¹H NMR (400 MHz, D₂O) δ 7.75 (s, 1H), 7.61 (d, J = 8.1 Hz, 1H), 5.97 (dd, J = 17.8, 2.3 Hz, 1H), 5.85 - 5.65 (m, 3H), 5.28 (ddd, J = 52.2, 4.7, 2.3 Hz, 1H), 5.01 (dt, J = 51.5, 3.4 Hz, 1H), 4.57 - 4.51 (m, 2H), 4.48 (t, J = 4.2 Hz, 1H), 4.34 (t, J = 5.1 Hz, 1H), 4.24 - 4.07 (m, 7H), 4.02 (t, J = 4.3 Hz, 2H), 3.93 (s, 3H).

³¹P NMR (162 MHz, D₂O) δ -1.30, -11.57 (dd, J = 25.8, 18.4 Hz), -22.98 (t, J = 18.3 Hz).

### Example 4

### Synthesis of Compound 83

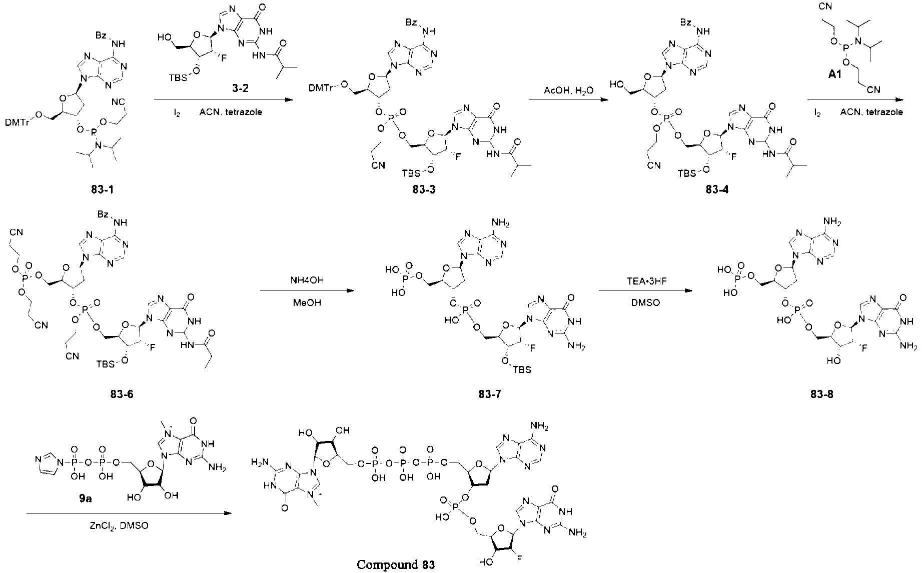

### Step 1:

The compound ***3-2*** (2.1 g) was added to a solution of tetrazole (1.6 g) in acetonitrile (56 mL) in a three-necked flask, and the atmosphere was replaced with argon three times. Next, at room temperature of 25°C, a compound ***83-1*** (5 g) was dissolved in 10 mL of acetonitrile and added to the above solution. The resulting solution was stirred at room temperature of 25°C for 1 hour. No obvious heat release was found, and TLC monitoring showed that the compound ***83-1*** disappeared. Then, into the solution, iodine solution (a 0.5 mmol/mL solution was prepared by dissolving 5 g iodine in 40 mL of the mixed solution: THF:H₂O:pyridine = 8:1:1) was added dropwise until the solution no longer faded. After that, the reaction solution was stirred for another 0.5 hours, and TLC monitoring showed that the oxidation was completed. After adding Na₂SO₃ aqueous solution (10 mL) to the reaction solution for quenching, 50 mL of water was further added for dilution, and the mixture was extracted with dichloromethane (50 mL x 2). The organic phases were combined and washed once with water (50 mL), and concentrated to obtain a light yellow oily product ***83-3*** (7.4 g, crude product).

### Step 2:

The compound ***83-3*** (7.4 g, crude product) was dissolved in 40 mL of acetic acid and 10 mL of water, and the reaction solution was stirred at 25°C for 16 hours. TLC monitoring showed that the compound ***83-3*** disappeared and a spot with large polarity was generated. The reaction solution was directly concentrated in vacuo. After concentration, appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white solid product ***83-4*** (2.8 g, 54.8% overall yield over two-step) was obtained.

### Step 3:

28 mL of a tetrazole solution in acetonitrile (0.4 mmol/mL) was prepared. The compound ***83-4*** (2.8 g) was added to the above solution, and then a compound ***A1*** (2.26 g) was added to the solution at room temperature of 25°C. The atmosphere was replaced with nitrogen three times, and the reaction solution was stirred at room temperature of 25°C for 1 hour. TLC monitoring showed that the reaction was completed. The reaction solution was cooled to below 10°C in an ice-water bath, and a solution of iodine in pyridine/tetrahydrofuran/water (0.5 mmol/mL, pyridine:tetrahydrofuran:water = 1:8:1) was added dropwise until the reaction solution no longer faded. TLC monitoring showed that the oxidation reaction was completed. The reaction solution was quenched by adding 10 mL of an aqueous saturated solution of sodium sulfite, diluted with water, and extracted three times with ethyl acetate. The organic phases were combined and dried over anhydrous sodium sulfate and filtered. Appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white foamy solid compound ***83-6*** (3.1 g, 93.5% yield) was obtained.

### Step 4:

The compound ***83-6*** (3.1 g) was dissolved in methanol (30 mL) and concentrated aqueous ammonia (30 mL) was added. The resulting solution was stirred at room temperature of 25°C for 60 hours. TLC monitoring showed that the compound ***83-6*** was completely reacted. The reaction solution was concentrated in vacuo and concentrated again with methanol to obtain a light yellow oily liquid compound ***83-7*** (2.4 g, crude product).

### Step 5:

The compound ***83-7*** (2.4 g, crude product) was dissolved in DMSO (3 mL) and triethylamine trihydrofluoride (3.5 mL) was added. The reaction solution was stirred at 50°C for 1 hour. TLC monitoring showed that the compound ***83-7*** was completely reacted. The reaction solution was diluted to 50 mL with water, and the pH was adjusted to 5.5 with 1 N NaOH aqueous solution. The mixture was loaded onto a DEAE Sephadex column. The product was eluted by linear gradient elution using 0 M to 1.0 M TEAB eluent. Most of the water in the obtained fraction was concentrated in vacuo, and the remaining liquid was freeze-dried to obtain a target compound triethylamine salt ***83-8*** (1.5 g, 51% yield), which was a white solid.

### Step 6:

At room temperature (25°C), under the protection of argon, the compound ***83-8*** (500 mg), the compound ***9a*** (500 mg) and anhydrous zinc chloride (1.2 g) were added, and anhydrous DMSO (8 mL) was added with a syringe to react for 24 hours. TLC monitoring showed that most of the materials were reacted. The reaction solution was added to a disodium EDTA solution (1.6 g, 80 mL of water was added) cooled to 0°C in advance, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***83,*** which was an ammonium salt in the form of white powder (120 mg).

¹H NMR (400 MHz, D₂O) δ 8.30 (d, J = 0.6 Hz, 1H), 8.20 (s, 1H), 8.02 (d, J = 0.7 Hz, 1H), 6.59 - 6.30 (m, 2H), 6.23 - 6.08 (m, 1H), 5.22 (dddd, J = 46.4, 7.0, 1.7, 0.7 Hz, 1H), 5.08 - 4.90 (m, 1H), 4.68 - 4.03 (m, 12H), 3.98 (d, J = 0.7 Hz, 3H), 2.76 - 2.48 (m, 2H).

³¹P NMR (162 MHz, D₂O) δ -1.30, -11.57 (dd, J = 25.8, 18.4 Hz), -22.98 (t, J = 18.3 Hz).

### Example 5

### Synthesis of Compound 90

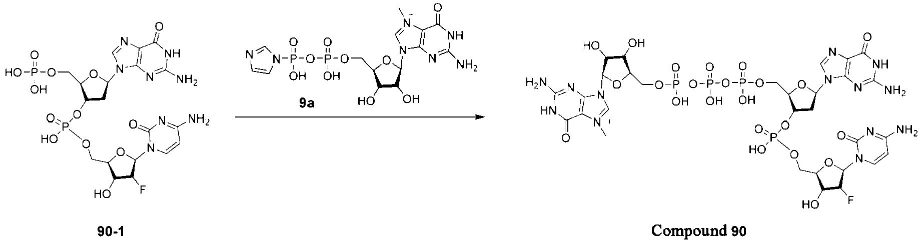

The compound ***9a*** (200 mg) was added to 16 mL of an aqueous solution with a pH of 7.0, containing 0.2 mol/L N-methylmorpholine and 0.2 mol/L manganous chloride, and a compound ***90-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***90,*** which was an ammonium salt in the form of white powder (50 mg).

¹H NMR (400 MHz, D₂O) δ 7.83 (d, J = 0.6 Hz, 1H), 7.70 (dd, J = 7.4, 1.8 Hz, 1H), 6.28 (ddq, J = 3.1, 1.5, 0.7 Hz, 1H), 6.20 - 6.13 (m, 1H), 6.04 (d, J = 7.3 Hz, 1H), 5.40 (dddd, J = 25.2, 3.5, 1.8, 0.9 Hz, 1H), 5.09 - 4.93 (m, 1H), 4.81 (dddd, J = 46.4, 7.1, 3.7, 0.7 Hz, 1H), 4.61 (ddd, J = 5.2, 2.8, 0.7 Hz, 1H), 4.41 - 4.05 (m, 11H), 3.98 (d, J = 0.7 Hz, 3H), 2.81 - 2.38 (m, 2H).

³¹P NMR (162 MHz, D₂O) δ -1.17, -11.50, -22.81.

### Example 6

### Synthesis of Compound 103

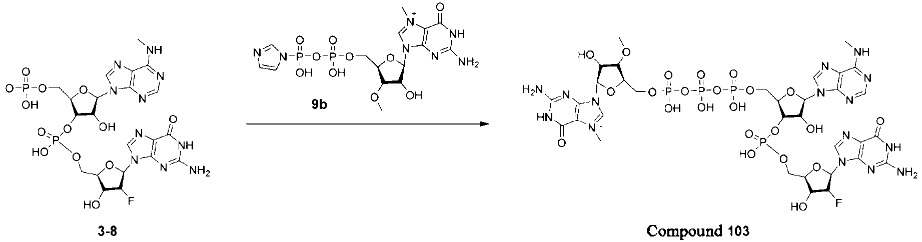

A compound ***9b*** (200 mg) was added to 16 mL of an aqueous solution with a pH of 7.0, containing 0.2 mol/L N-methylmorpholine and 0.2 mol/L manganous chloride, and the compound 3-8 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound *103,* which was an ammonium salt in the form of white powder (60 mg).

¹H NMR (400 MHz, D₂O) δ 8.33 (d, J = 0.5 Hz, 1H), 8.29 (s, 1H), 8.02 (d, J = 0.7 Hz, 1H), 6.45 (ddq, J = 25.2, 1.4, 0.7 Hz, 1H), 6.17 (dq, J = 2.3, 0.8 Hz, 1H), 6.11 (dq, J = 3.1, 0.8 Hz, 1H), 5.22 (dddd, J = 46.4, 7.0, 1.7, 0.7 Hz, 1H), 4.88 - 4.62 (m, 3H), 4.45 (dddd, J = 25.2, 6.8, 5.0, 0.7 Hz, 1H), 4.33 - 4.09 (m, 9H), 4.05 (ttd, J = 2.9, 1.5, 0.7 Hz, 1H), 3.98 (d, J = 0.7 Hz, 3H), 3.44 (d, J = 1.4 Hz, 3H), 3.08 (s, 3H).

³¹P NMR (162 MHz, D₂O) δ 0.60, -10.29, -21.23.

### Example 7

### Synthesis of Compound 119

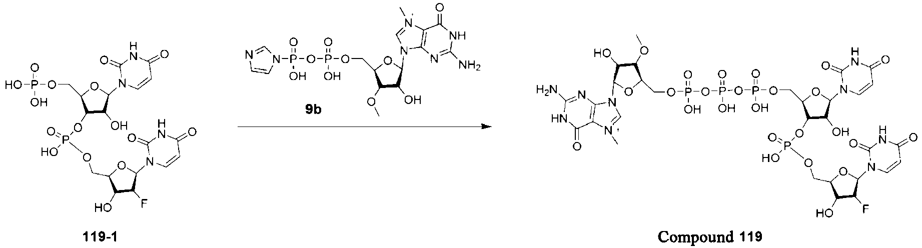

The compound ***9b*** (200 mg) was added to 16 mL of an aqueous solution with a pH of 7.0, containing 0.2 mol/L N-methylmorpholine and 0.2 mol/L manganous chloride, and a compound ***119-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***119,*** which was an ammonium salt in the form of white powder (65 mg).

¹H NMR (400 MHz, D₂O) δ 7.81 (dd, J = 7.8, 1.8 Hz, 1H), 7.76 (dd, J = 7.8, 1.8 Hz, 1H), 6.17 (dt, J = 2.5, 0.7 Hz, 1H), 5.90 (d, J = 7.8 Hz, 1H), 5.85 - 5.73 (m, 2H), 5.53 (dddd, J = 25.2, 3.9, 1.7, 0.9 Hz, 1H), 4.89 - 4.60 (m, 3H), 4.46 - 4.02 (m, 12H), 3.98 (d, J = 0.7 Hz, 3H), 3.44 (d, J = 1.4 Hz, 3H).

³¹P NMR (162 MHz, D₂O) δ 0.62, -10.29, -21.22.

### Example 8

### Synthesis of Compound 127

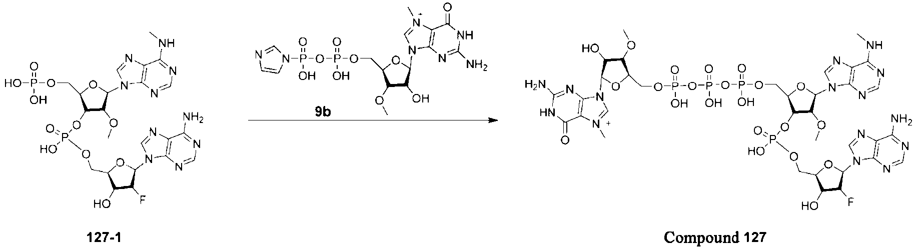

The compound ***9b*** (200 mg) was added to 16 mL of an aqueous solution with a pH of 7.0, containing 0.2 mol/L N-methylmorpholine and 0.2 mol/L manganous chloride, and a compound 127-1 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***127,*** which was an ammonium salt in the form of white powder (82 mg).

¹H NMR (400 MHz, D₂O) δ 8.29 (s, 1H), 8.24 (d, J = 0.7 Hz, 1H), 8.21 - 8.18 (m, 2H), 6.54 - 6.00 (m, 3H), 5.27 (dddd, J = 46.5, 7.0, 4.0, 0.6 Hz, 1H), 4.97 (dddd, J = 8.0, 4.4, 2.9, 0.7 Hz, 1H), 4.77 (tqd, J = 4.4, 1.5, 0.7 Hz, 1H), 4.71 (ddd, J = 3.9, 2.6, 0.7 Hz, 1H), 4.45 (dddd, J = 25.2, 6.8, 5.1, 0.7 Hz, 1H), 4.35 (qt, J = 3.0, 0.7 Hz, 1H), 4.29 - 4.08 (m, 8H), 4.05 (ttd, J = 2.9, 1.5, 0.7 Hz, 1H), 3.98 (d, J = 0.7 Hz, 3H), 3.42 (dd, J = 18.0, 1.5 Hz, 6H), 3.08 (s, 3H).

³¹P NMR (162 MHz, D₂O) δ 0.60, -10.29, -21.22.

### Example 9

### Synthesis of Compound 167

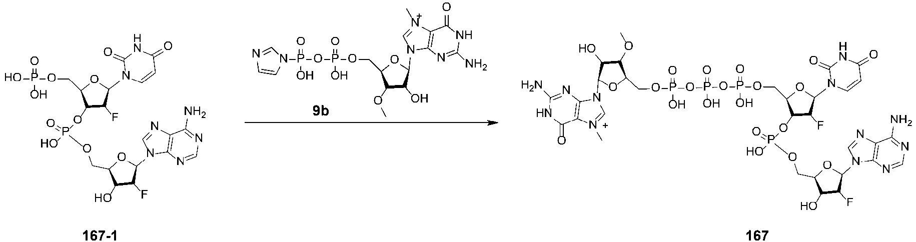

The compound ***9b*** (200 mg) was added to 16 mL of an aqueous solution with a pH of 7.0, containing 0.2 mol/L N-methylmorpholine and 0.2 mol/L manganous chloride, and a compound 24-***1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***167,*** which was an ammonium salt in the form of white powder (62 mg).

¹H NMR (400 MHz, D₂O) δ 8.24 (d, J = 0.7 Hz, 1H), 8.20 (s, 1H), 7.81 (dd, J = 7.9, 1.8 Hz, 1H), 6.39 (ddt, J = 25.2, 4.0, 0.7 Hz, 1H), 6.17 (dq, J = 2.3, 0.7 Hz, 1H), 5.90 (d, J = 7.8 Hz, 1H), 5.56 (dddt, J = 25.3, 3.4, 1.8, 0.9 Hz, 1H), 5.43 - 4.99 (m, 3H), 4.79 - 4.65 (m, 1H), 4.56 - 4.38 (m, 2H), 4.35 - 4.06 (m, 8H), 4.05 (ddq, J = 4.6, 2.2, 1.1 Hz, 1H), 3.98 (d, J = 0.7 Hz, 3H), 3.44 (d, J = 1.4 Hz, 3H).

³¹P NMR (162 MHz, D₂O) δ 0.60, -10.29, -21.22.

### Example 10

### Synthesis of Compound 180

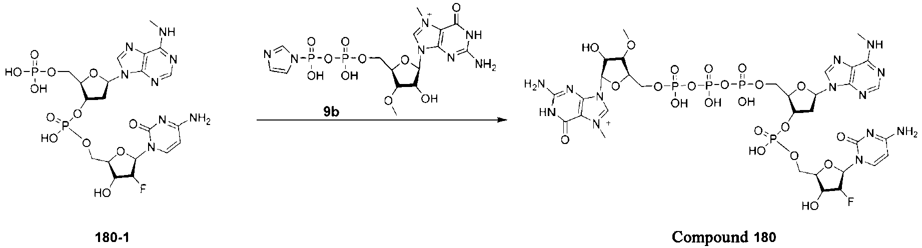

The compound ***9b*** (200 mg) was added to 16 mL of an aqueous solution with a pH of 7.0, containing 0.2 mol/L N-methylmorpholine and 0.2 mol/L manganous chloride, and a compound ***180-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***180***, which was an ammonium salt in the form of white powder (95 mg).

¹H NMR (400 MHz, D₂O) δ 8.35 (d, J = 0.6 Hz, 1H), 8.29 (s, 1H), 7.70 (dd, J = 7.4, 1.8 Hz, 1H), 6.47 - 6.32 (m, 1H), 6.17 (dq, J = 2.3, 0.8 Hz, 1H), 6.04 (d, J = 7.3 Hz, 1H), 5.54 - 5.33 (m, 1H), 5.10 - 4.94 (m, 1H), 4.91 - 4.64 (m, 2H), 4.48 - 4.03 (m, 11H), 3.98 (d, J = 0.7 Hz, 3H), 3.44 (d, J = 1.4 Hz, 3H), 3.08 (s, 3H), 2.83 - 2.35 (m, 2H).

³¹P NMR (162 MHz, D₂O) δ 0.60, -10.29, -21.22.

### Example 11

### Synthesis of Compound 203

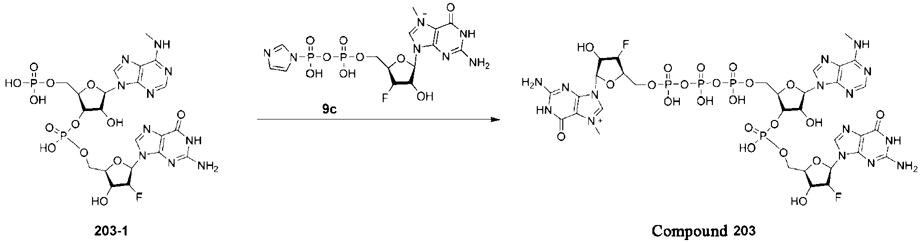

A compound ***9c*** (200 mg) was added to 16 mL of an aqueous solution with a pH of 7.0, containing 0.2 mol/L N-methylmorpholine and 0.2 mol/L manganous chloride, and a compound ***203-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound 203, which was an ammonium salt in the form of white powder (95 mg).

¹H NMR (400 MHz, D₂O) δ 8.33 (d, J = 0.6 Hz, 1H), 8.29 (s, 1H), 8.02 (d, J = 0.7 Hz, 1H), 6.45 (ddq, J = 25.2, 1.4, 0.7 Hz, 1H), 6.23 (dt, J = 2.3, 0.8 Hz, 1H), 6.11 (dq, J = 3.1, 0.8 Hz, 1H), 5.22 (dddd, J = 46.4, 7.0, 1.7, 0.7 Hz, 1H), 5.00 - 4.68 (m, 4H), 4.65 - 4.39 (m, 2H), 4.38 - 4.05 (m, 8H), 3.98 (d, J = 0.7 Hz, 3H), 3.08 (s, 3H).

³¹P NMR (162 MHz, D₂O) δ 0.60, -10.29, -21.22.

### Example 12

### Synthesis of Compound 239

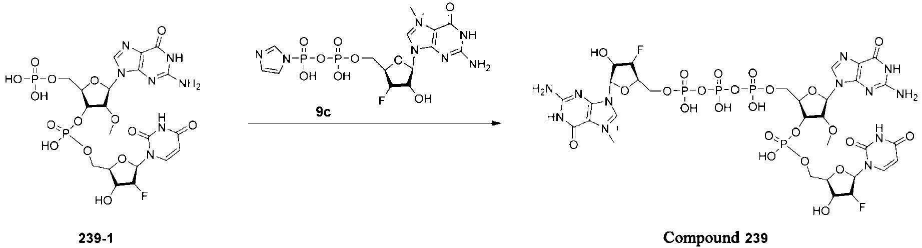

The compound ***9c*** (200 mg) was added to 16 mL of an aqueous solution with a pH of 7.0, containing 0.2 mol/L N-methylmorpholine and 0.2 mol/L manganous chloride, and a compound 239-1 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***239,*** which was an ammonium salt in the form of white powder (95 mg).

¹H NMR (400 MHz, D₂O) δ 7.88 (d, J = 0.7 Hz, 1H), 7.81 (dd, J = 7.8, 1.8 Hz, 1H), 6.33 - 6.06 (m, 2H), 5.90 (d, J = 7.8 Hz, 1H), 5.53 (dddd, J = 25.2, 3.9, 1.7, 0.9 Hz, 1H), 5.03 - 4.50 (m, 6H), 4.43 - 4.07 (m, 9H), 3.98 (d, J = 0.7 Hz, 3H), 3.40 (d, J = 1.6 Hz, 3H).

³¹P NMR (162 MHz, D₂O) δ 0.60, -10.29, -21.22.

### Example 13

### Synthesis of Compound 260

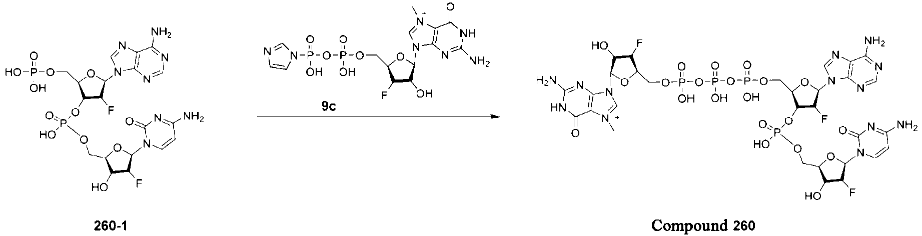

The compound ***9c*** (200 mg) was added to 16 mL of an aqueous solution with a pH of 7.0, containing 0.2 mol/L N-methylmorpholine and 0.2 mol/L manganous chloride, and a compound ***260-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***260,*** which was an ammonium salt in the form of white powder (75 mg).

¹H NMR (400 MHz, D₂O) δ 8.24 (d, J = 0.7 Hz, 1H), 8.20 (s, 1H), 7.70 (dd, J = 7.4, 1.8 Hz, 1H), 6.43 (ddq, J = 25.2, 1.6, 0.8 Hz, 1H), 6.27 - 6.20 (m, 1H), 6.04 (d, J = 7.3 Hz, 1H), 5.51 - 5.12 (m, 3H), 5.05 - 4.70 (m, 3H), 4.66 - 4.10 (m, 10H), 3.98 (d, J = 0.7 Hz, 3H).

³¹P NMR (162 MHz, D₂O) δ -0.90, -10.29, -21.22.

### Example 14

### Synthesis of Compound 287

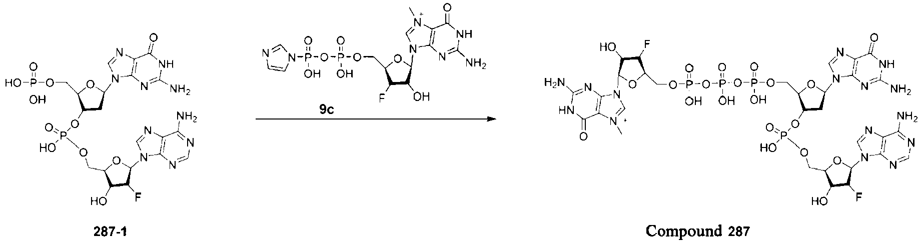

The compound ***9c*** (200 mg) was added to 16 mL of an aqueous solution with a pH of 7.0, containing 0.2 mol/L N-methylmorpholine and 0.2 mol/L manganous chloride, and a compound ***287-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***287,*** which was an ammonium salt in the form of white powder (75 mg).

¹H NMR (400 MHz, D₂O) δ 8.24 (d, J = 0.7 Hz, 1H), 8.20 (s, 1H), 7.83 (d, J = 0.6 Hz, 1H), 6.39 (ddt, J = 25.2, 4.0, 0.7 Hz, 1H), 6.28 (ddt, J = 3.1, 1.5, 0.7 Hz, 1H), 6.26 - 6.15 (m, 1H), 5.27 (dddd, J = 46.5, 7.0, 4.0, 0.6 Hz, 1H), 5.11 - 4.79 (m, 3H), 4.65 - 4.03 (m, 10H), 3.98 (d, J = 0.7 Hz, 3H), 2.88 - 2.51 (m, 2H).

³¹P NMR (162 MHz, D₂O) δ -1.11, -10.31, -21.25.

### Example 15

### Synthesis of Compound 126

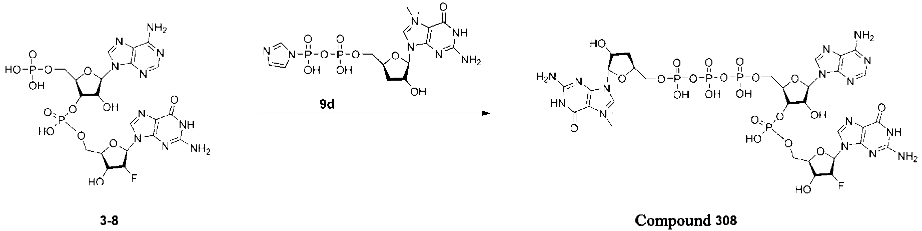

A compound ***9d*** (200 mg) was added to 16 mL of an aqueous solution with a pH of 7.0, containing 0.2 mol/L N-methylmorpholine and 0.2 mol/L manganous chloride, and the compound ***3-8*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***308***, which was an ammonium salt in the form of white powder (60 mg).

¹H NMR (400 MHz, D₂O) δ 8.28 (d, J = 0.6 Hz, 1H), 8.20 (s, 1H), 8.02 (d, J = 0.7 Hz, 1H), 6.45 (ddq, J = 25.3, 1.6, 0.9 Hz, 1H), 6.21 - 6.07 (m, 2H), 5.22 (dddd, J = 46.4, 7.0, 1.7, 0.7 Hz, 1H), 4.98 - 4.68 (m, 2H), 4.57 - 4.03 (m, 11H), 3.98 (d, J = 0.7 Hz, 3H), 2.45 - 1.75 (m, 2H).

³¹P NMR (162 MHz, D₂O) δ -0.9, -10.29, -21.27.

### Example 16

### Synthesis of Compound 326

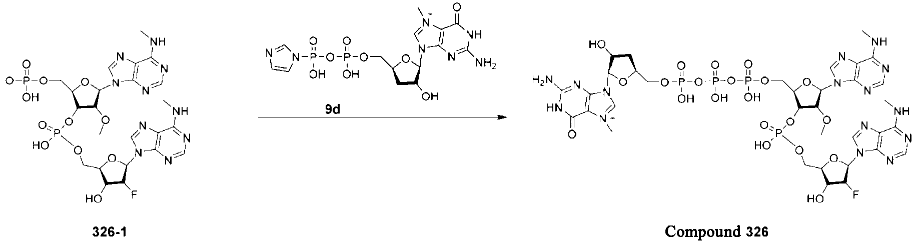

The compound ***9d*** (200 mg) was added to 16 mL of an aqueous solution with a pH of 7.0, containing 0.2 mol/L N-methylmorpholine and 0.2 mol/L manganous chloride, and a compound 326-1 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound 326, which was an ammonium salt in the form of white powder (60 mg).

¹H NMR (400 MHz, D₂O) δ 8.29 (d, J = 1.7 Hz, 3H), 8.20 (d, J = 0.7 Hz, 1H), 6.39 (ddt, J = 25.1, 3.9, 0.7 Hz, 1H), 6.29 - 6.04 (m, 2H), 5.27 (dddd, J = 46.5, 7.0, 4.0, 0.6 Hz, 1H), 4.97 (dddd, J = 8.0, 4.4, 2.9, 0.7 Hz, 1H), 4.77 (tqd, J = 4.4, 1.5, 0.7 Hz, 1H), 4.61 - 4.06 (m, 11H), 3.98 (d, J = 0.7 Hz, 3H), 3.40 (d, J = 1.6 Hz, 3H), 3.08 (s, 6H), 2.56 - 1.44 (m, 2H).

³¹P NMR (162 MHz, D₂O) δ -0.78, -10.29, -21.22.

### Example 17

### Synthesis of Compound 352

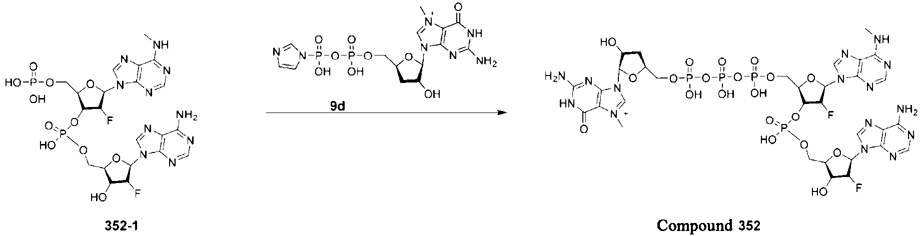

The compound ***9d*** (200 mg) was added to 16 mL of an aqueous solution with a pH of 7.0, containing 0.2 mol/L N-methylmorpholine and 0.2 mol/L manganous chloride, and a compound ***352-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***352,*** which was an ammonium salt in the form of white powder (74 mg).

¹H NMR (400 MHz, D₂O) δ 8.33 - 8.27 (m, 2H), 8.24 (d, J = 0.6 Hz, 1H), 8.20 (s, 1H), 6.63 - 6.28 (m, 2H), 6.17 (dt, J = 1.3, 0.7 Hz, 1H), 5.57 - 5.04 (m, 3H), 4.63 - 4.03 (m, 11H), 3.98 (d, J = 0.7 Hz, 3H), 3.08 (s, 3H), 2.63 - 1.60 (m, 2H).

³¹P NMR (162 MHz, D₂O) -0.55, -10.32, -21.28.

### Example 18

### Synthesis of Compound 386

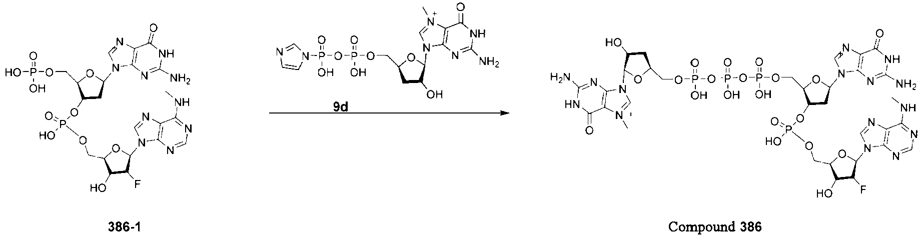

The compound ***9d*** (200 mg) was added to 16 mL of an aqueous solution with a pH of 7.0, containing 0.2 mol/L N-methylmorpholine and 0.2 mol/L manganous chloride, and a compound ***386-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***386,*** which was an ammonium salt in the form of white powder (60 mg).

¹H NMR (400 MHz, D₂O) δ 8.38 - 8.22 (m, 2H), 8.18 (d, J = 0.6 Hz, 1H), 6.58 - 6.30 (m, 2H), 6.17 (t, J = 1.0 Hz, 1H), 5.27 (dddd, J = 46.5, 7.0, 4.0, 0.6 Hz, 1H), 5.10 - 4.83 (m, 1H), 4.60 - 4.07 (m, 11H), 3.98 (d, J = 0.7 Hz, 3H), 3.08 (s, 3H), 2.69 - 2.46 (m, 2H), 2.34 - 1.69 (m, 2H).

³¹P NMR (162 MHz, D₂O) δ -0.85, -10.09, -21.23.

### Example 19

### Synthesis of Compound 403

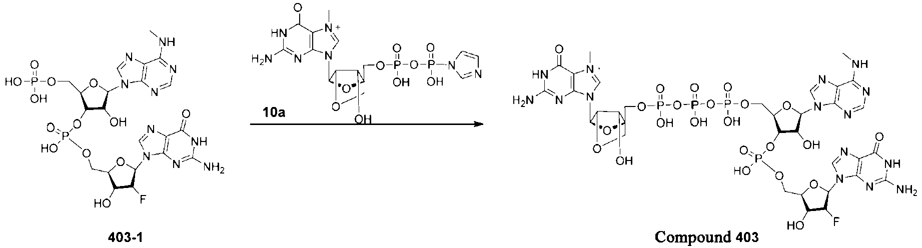

A compound ***10a*** (200 mg) was added to 16 mL of an aqueous solution with a pH of 7.0, containing 0.2 mol/L N-methylmorpholine and 0.2 mol/L manganous chloride, and a compound ***403-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***403,*** which was an ammonium salt in the form of white powder (55 mg).

¹H NMR (400 MHz, D₂O) δ 8.40 - 8.19 (m, 2H), 8.02 (d, J = 0.7 Hz, 1H), 6.45 (ddq, J = 25.2, 1.4, 0.7 Hz, 1H), 6.33 (dd, J = 2.7, 0.6 Hz, 1H), 6.11 (dq, J = 3.0, 0.7 Hz, 1H), 5.22 (dddd, J = 46.4, 7.0, 1.7, 0.7 Hz, 1H), 4.80 (ddd, J = 3.9, 3.1, 0.7 Hz, 1H), 4.74 (dddd, J = 7.9, 3.9, 2.9, 0.7 Hz, 1H), 4.64 (t, J = 2.8 Hz, 1H), 4.51 - 4.08 (m, 10H), 4.02 (d, J = 0.7 Hz, 2H), 3.98 (s, 3H), 3.08 (s, 3H).

³¹P NMR (162 MHz, D₂O) δ -0.90, -11.41, -22.93.

### Example 20

### Synthesis of Compound 443

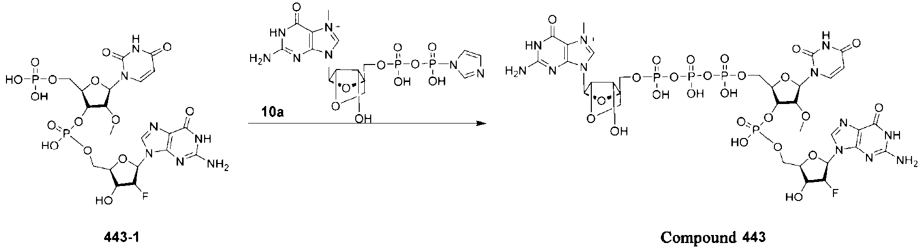

The compound ***10a*** (200 mg) was added to 16 mL of an aqueous solution with a pH of 7.0, containing 0.2 mol/L N-methylmorpholine and 0.2 mol/L manganous chloride, and a compound ***443-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***443,*** which was an ammonium salt in the form of white powder (70 mg).

¹H NMR (400 MHz, D₂O) δ 8.02 (d, J = 0.7 Hz, 1H), 7.75 (dd, J = 7.8, 1.8 Hz, 1H), 6.45 (ddq, J = 25.2, 1.6, 0.9 Hz, 1H), 6.33 (dd, J = 2.7, 0.6 Hz, 1H), 5.93 - 5.65 (m, 2H), 5.22 (dddd, J = 46.4, 7.0, 1.7, 0.7 Hz, 1H), 4.89 (dddd, J = 8.0, 5.4, 4.6, 0.6 Hz, 1H), 4.64 (t, J = 2.8 Hz, 1H), 4.51 - 4.07 (m, 11H), 4.02 (d, J = 0.7 Hz, 2H), 3.98 (s, 3H), 3.47 (s, 3H).

³¹P NMR (162 MHz, D₂O) δ 0.50, -9.18, -10.26, -21.22.

### Example 21

### Synthesis of Compound 467

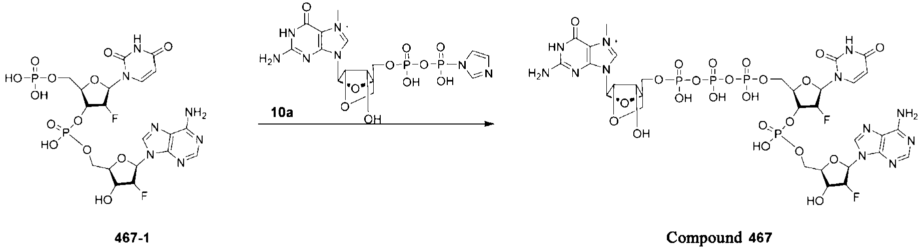

The compound ***10a*** (200 mg) was added to 16 mL of an aqueous solution with a pH of 7.0, containing 0.2 mol/L N-methylmorpholine and 0.2 mol/L manganous chloride, and a compound ***467-7*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***467,*** which was an ammonium salt in the form of white powder (75 mg).

¹H NMR (400 MHz, D₂O) δ 8.32 - 8.10 (m, 2H), 7.81 (dd, J = 7.9, 1.8 Hz, 1H), 6.54 - 6.21 (m, 2H), 5.90 (d, J = 7.8 Hz, 1H), 5.71 - 4.98 (m, 4H), 4.73 - 4.09 (m, 11H), 4.02 (d, J = 0.7 Hz, 2H), 3.98 (s, 3H).

³¹P NMR (162 MHz, D₂O) δ -0.99, -11.40, -23.06.

### Example 22

### Synthesis of Compound 480

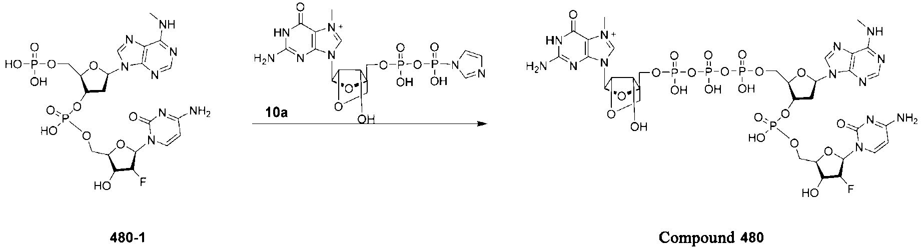

The compound ***10a*** (200 mg) was added to 16 mL of an aqueous solution with a pH of 7.0, containing 0.2 mol/L N-methylmorpholine and 0.2 mol/L manganous chloride, and a compound ***480-***1 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***480**,* which was an ammonium salt in the form of white powder (85 mg).

¹H NMR (400 MHz, D₂O) δ 8.41 - 8.23 (m, 2H), 7.70 (dd, J = 7.4, 1.8 Hz, 1H), 6.43 - 6.28 (m, 2H), 6.04 (d, J = 7.3 Hz, 1H), 5.56 - 5.21 (m, 1H), 5.09 - 4.93 (m, 1H), 4.81 (dddd, J = 46.5, 7.1, 3.7, 0.7 Hz, 1H), 4.64 (t, J = 2.8 Hz, 1H), 4.48 - 4.03 (m, 10H), 4.02 (d, J = 0.7 Hz, 2H), 3.98 (d, J = 0.7 Hz, 3H), 3.08 (s, 3H), 2.79 - 2.46 (m, 2H).

³¹P NMR (162 MHz, D₂O) δ 0.60, -9.01, -10.23, -21.17.

### Example 23

### Synthesis of Compound 501

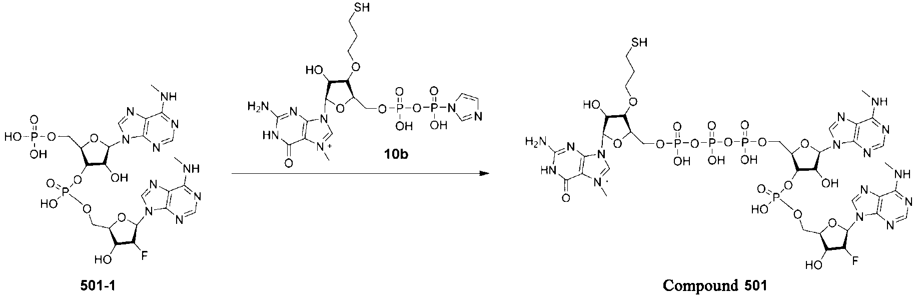

A compound ***10b*** (200 mg) was added to 16 mL of an aqueous solution with a pH of 7.0, containing 0.2 mol/L N-methylmorpholine and 0.2 mol/L manganous chloride, and a compound ***501-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***501**,* which was an ammonium salt in the form of white powder (92 mg).

¹H NMR (400 MHz, D₂O) δ 8.41 - 8.14 (m, 4H), 6.39 (ddt, J = 25.3, 4.1, 0.8 Hz, 1H), 6.25 - 5.98 (m, 2H), 5.27 (dddd, J = 46.5, 7.0, 4.0, 0.6 Hz, 1H), 4.83 - 4.61 (m, 3H), 4.45 (dddd, J = 25.2, 6.8, 5.0, 0.7 Hz, 1H), 4.36 - 4.07 (m, 10H), 3.98 (d, J = 0.7 Hz, 3H), 3.69 - 3.48 (m, 2H), 3.08 (s, 6H), 2.90 - 2.61 (m, 2H), 1.84 (p, J = 6.5 Hz, 2H).

³¹P NMR (162 MHz, D₂O) δ 0.60, -9.10, -10.31, -21.17.

### Example 24

### Synthesis of Compound 541

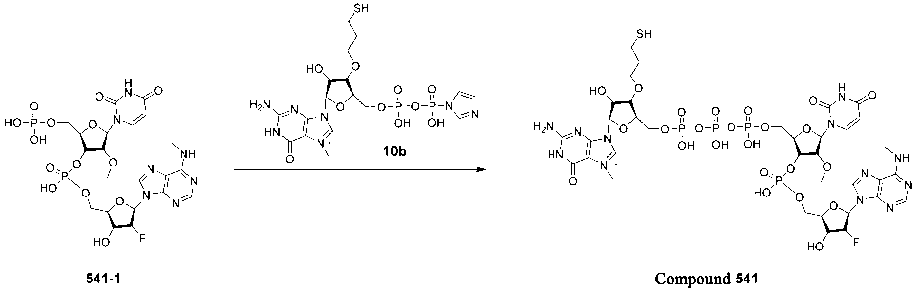

The compound ***10b*** (200 mg) was added to 16 mL of an aqueous solution with a pH of 7.0, containing 0.2 mol/L N-methylmorpholine and 0.2 mol/L manganous chloride, and a compound ***541-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***541,*** which was an ammonium salt in the form of white powder (85 mg).

¹H NMR (400 MHz, D₂O) δ 8.38 - 8.21 (m, 2H), 7.75 (dd, J = 7.8, 1.8 Hz, 1H), 6.50 - 6.30 (m, 1H), 6.27 - 6.10 (m, 1H), 5.95 - 5.71 (m, 2H), 5.27 (dddd, J = 46.5, 7.0, 4.0, 0.6 Hz, 1H), 4.89 (dddd, J = 8.0, 5.4, 4.7, 0.6 Hz, 1H), 4.71 (ddd, J = 3.3, 2.5, 0.7 Hz, 1H), 4.52 - 4.04 (m, 12H), 3.98 (d, J = 0.7 Hz, 3H), 3.75 - 3.54 (m, 2H), 3.47 (d, J = 1.6 Hz, 3H), 3.08 (s, 3H), 2.90 - 2.51 (m, 2H), 1.84 (p, J = 6.5 Hz, 2H).

³¹P NMR (162 MHz, D₂O) δ 0.60, -9.10, -10.22, -21.17.

### Example 25

### Synthesis of Compound 574

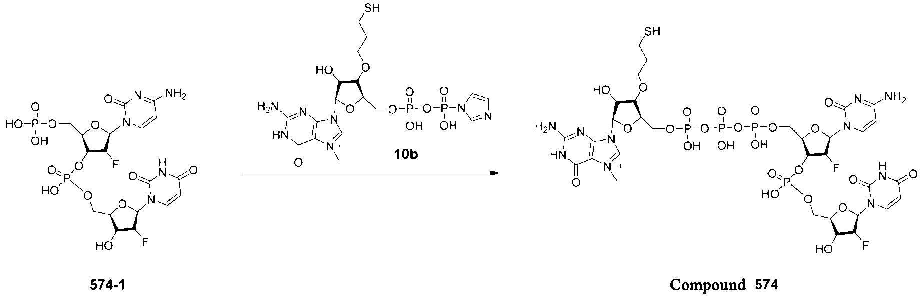

The compound ***10b*** (200 mg) was added to 16 mL of an aqueous solution with a pH of 7.0, containing 0.2 mol/L N-methylmorpholine and 0.2 mol/L manganous chloride, and a compound 574-1 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***574,*** which was an ammonium salt in the form of white powder (80 mg).

¹H NMR (400 MHz, D₂O) δ 7.81 (dd, J = 7.8, 1.8 Hz, 1H), 7.70 (dd, J = 7.4, 1.8 Hz, 1H), 6.29 - 6.11 (m, 1H), 6.04 (d, J = 7.3 Hz, 1H), 5.90 (d, J = 7.8 Hz, 1H), 5.63 - 5.42 (m, 2H), 5.33 - 5.00 (m, 2H), 4.89 - 4.64 (m, 2H), 4.55 - 4.05 (m, 11H), 3.98 (d, J = 0.7 Hz, 3H), 3.75 - 3.50 (m, 2H), 2.89 - 2.50 (m, 2H), 1.84 (p, J = 6.5 Hz, 2H).

³¹P NMR (162 MHz, D₂O) δ -0.22, -9.07, -10.25, -21.27.

### Example 26

### Synthesis of Compound 589

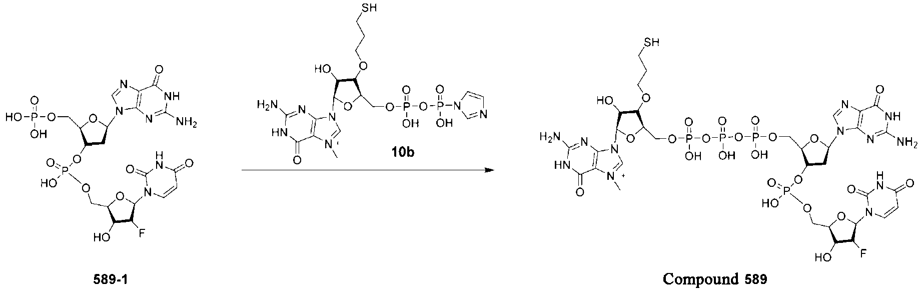

The compound ***10b*** (200 mg) was added to 16 mL of an aqueous solution with a pH of 7.0, containing 0.2 mol/L N-methylmorpholine and 0.2 mol/L manganous chloride, and a compound ***589-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***589,*** which was an ammonium salt in the form of white powder (58 mg).

¹H NMR (400 MHz, D₂O) δ 7.93 - 7.46 (m, 2H), 6.31 - 6.11 (m, 2H), 5.90 (d, J = 7.8 Hz, 1H), 5.53 (dddd, J = 25.2, 3.9, 1.7, 0.9 Hz, 1H), 5.13 - 4.93 (m, 1H), 4.88 - 4.61 (m, 2H), 4.43 - 4.04 (m, 11H), 3.98 (d, J = 0.7 Hz, 3H), 3.70 - 3.28 (m, 2H), 2.79 - 2.71 (m, 2H), 2.70 - 2.46 (m, 2H), 1.84 (p, J = 6.5 Hz, 2H).

³¹P NMR (162 MHz, D₂O) δ 0.66, -9.13, -10.26, -21.25.

### Example 27

### Synthesis of Compound 589

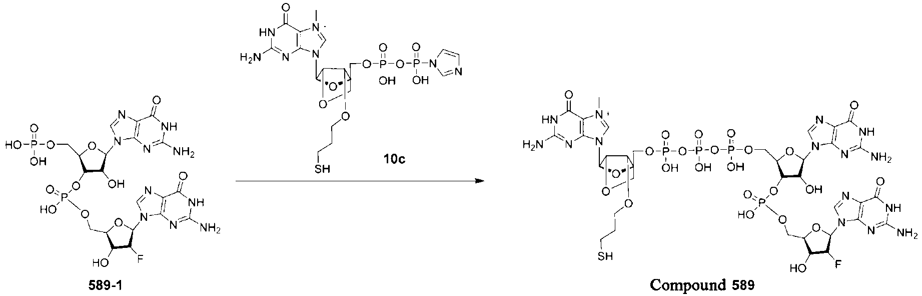

A compound ***10c*** (200 mg) was added to 16 mL of an aqueous solution with a pH of 7.0, containing 0.2 mol/L N-methylmorpholine and 0.2 mol/L manganous chloride, and the compound ***589-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***589,*** which was an ammonium salt in the form of white powder (88 mg).

¹H NMR (400 MHz, D₂O) δ 7.97 (dd, J = 37.6, 0.6 Hz, 2H), 6.71 - 6.22 (m, 2H), 6.10 (dq, J = 1.7, 0.7 Hz, 1H), 5.22 (dddd, J = 46.4, 7.0, 1.7, 0.7 Hz, 1H), 4.87 (t, J = 3.1 Hz, 1H), 4.74 (qt, J = 4.1, 2.2 Hz, 2H), 4.55 - 4.10 (m, 10H), 4.02 (s, 2H), 3.98 (s, 3H), 3.66 (td, J = 5.3, 2.9 Hz, 2H), 3.00 - 2.60 (m, 2H), 1.85 (tt, J = 6.4, 5.3 Hz, 2H).

³¹P NMR (162 MHz, D₂O) δ 0.85, -9.12, -10.24, -21.26.

### Example 28

### Synthesis of Compound 664

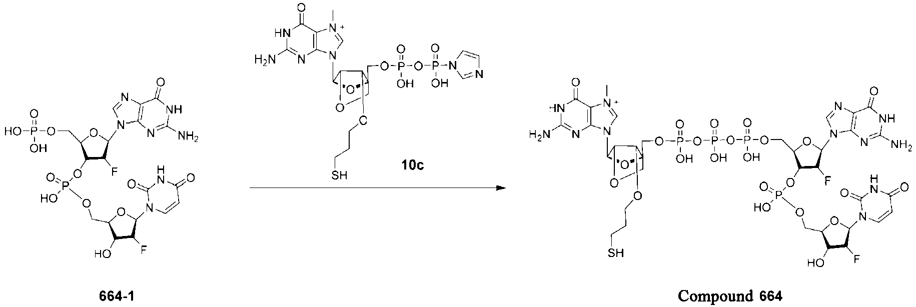

The compound ***10c*** (200 mg) was added to 16 mL of an aqueous solution with a pH of 7.0, containing 0.2 mol/L N-methylmorpholine and 0.2 mol/L manganous chloride, and a compound ***664-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***664,*** which was an ammonium salt in the form of white powder (60 mg).

¹H NMR (400 MHz, D₂O) δ 8.02 (d, J = 0.7 Hz, 1H), 7.81 (dd, J = 7.8, 1.8 Hz, 1H), 6.57 - 6.23 (m, 2H), 5.90 (d, J = 7.8 Hz, 1H), 5.71 - 5.42 (m, 1H), 5.39 - 5.16 (m, 2H), 4.94 - 4.67 (m, 2H), 4.56 - 4.08 (m, 10H), 4.02 (s, 2H), 3.98 (s, 3H), 3.66 (td, J = 5.3, 2.9 Hz, 2H), 2.98 - 2.65 (m, 2H), 1.85 (tt, J = 6.4, 5.3 Hz, 2H).

³¹P NMR (162 MHz, D₂O) δ -0.85, -10.09, -21.23.

### Example 29

### Synthesis of Compound 706

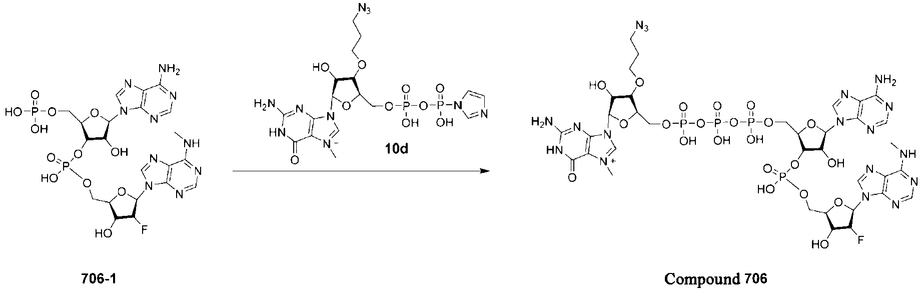

A compound ***10d*** (200 mg) was added to 16 mL of an aqueous solution with a pH of 7.0, containing 0.2 mol/L N-methylmorpholine and 0.2 mol/L manganous chloride, and a compound ***706-***1 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***706**,* which was an ammonium salt in the form of white powder (55 mg).

¹H NMR (400 MHz, D₂O) δ 8.40 - 7.81 (m, 4H), 6.84 - 5.90 (m, 3H), 5.31 (dddd, J = 46.5, 7.0, 4.0, 0.6 Hz, 1H), 4.76 (dddd, J = 46.0, 3.3, 2.8, 0.7 Hz, 2H), 4.54 - 4.18 (m, 9H), 4.09 - 3.95 (m, 3H), 3.92 (d, J = 0.6 Hz, 3H), 3.56 (td, J = 6.4, 1.5 Hz, 2H), 3.23 (td, J = 6.3, 0.8 Hz, 2H), 3.10 (s, 3H), 1.94 (pd, J = 6.3, 1.0 Hz, 2H).

³¹P NMR (162 MHz, D₂O) δ -0.90, -11.41, -22.93.

### Example 30

### Synthesis of Compound 760

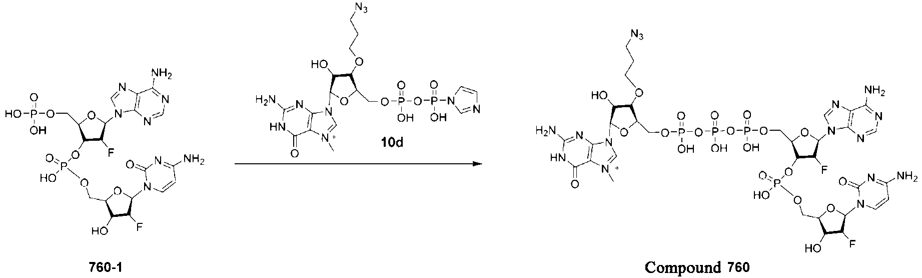

The compound ***10d*** (200 mg) was added to 16 mL of an aqueous solution with a pH of 7.0, containing 0.2 mol/L N-methylmorpholine and 0.2 mol/L manganous chloride, and a compound ***760-***1 (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***760**,* which was an ammonium salt in the form of white powder (70 mg).

¹H NMR (400 MHz, D₂O) δ 8.28 - 8.06 (m, 2H), 7.69 (dd, J = 7.4, 1.8 Hz, 1H), 6.47 (ddt, J = 25.2, 1.5, 0.8 Hz, 1H), 6.28 (dq, J = 2.3, 0.8 Hz, 1H), 6.15 (d, J = 7.3 Hz, 1H), 5.60 (dddt, J = 25.3, 3.6, 1.8, 0.8 Hz, 1H), 5.42 (dddd, J = 46.5, 2.8, 1.9, 0.7 Hz, 1H), 4.70 (ddd, J = 3.3, 2.5, 0.7 Hz, 1H), 4.50 - 3.96 (m, 13H), 3.92 (d, J = 0.6 Hz, 3H), 3.56 (td, J = 6.5, 1.5 Hz, 2H), 3.36 (t, J = 6.2 Hz, 2H), 1.94 (pd, J = 6.3, 1.0 Hz, 2H).

³¹P NMR (162 MHz, D₂O) δ 0.50, -9.18, -10.26, -21.22.

### Example 31

### Synthesis of Compound 787

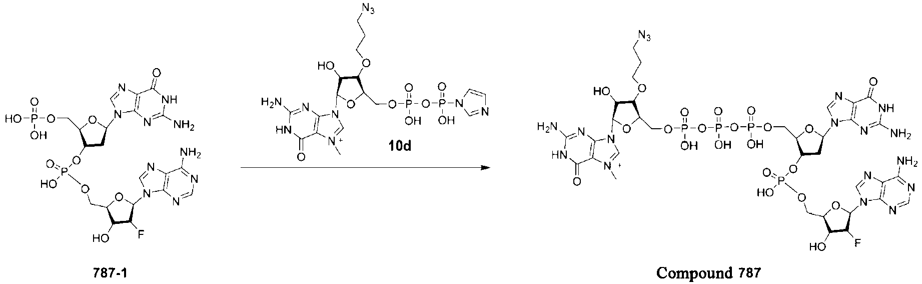

The compound ***10d*** (200 mg) was added to 16 mL of an aqueous solution with a pH of 7.0, containing 0.2 mol/L N-methylmorpholine and 0.2 mol/L manganous chloride, and a compound ***787-7*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***7*87,** which was an ammonium salt in the form of white powder (75 mg).

¹H NMR (400 MHz, D₂O) δ 8.49 - 7.98 (m, 2H), 7.75 (d, J = 0.5 Hz, 1H), 6.50 - 6.32 (m, 1H), 6.28 (dtd, J = 3.2, 1.6, 0.8 Hz, 2H), 5.31 (dddd, J = 46.5, 7.0, 4.0, 0.6 Hz, 1H), 4.70 (ddd, J = 3.3, 2.5, 0.7 Hz, 1H), 4.52 - 4.19 (m, 9H), 4.14 - 3.95 (m, 2H), 3.94 - 3.84 (m, 4H), 3.56 (td, J = 6.4, 1.5 Hz, 2H), 3.23 (td, J = 6.3, 0.8 Hz, 2H), 2.83 - 2.48 (m, 2H), 1.94 (pd, J = 6.3, 1.0 Hz, 2H).

³¹P NMR (162 MHz, D₂O) δ -0.99, -11.40, -23.06.

### Example 32

### Synthesis of Compound 828

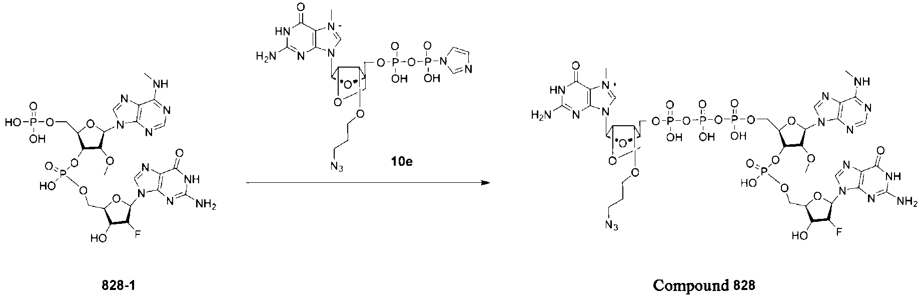

A compound ***10e*** (200 mg) was added to 16 mL of an aqueous solution with a pH of 7.0, containing 0.2 mol/L N-methylmorpholine and 0.2 mol/L manganous chloride, and a compound ***8*28-*1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***8*28,** which was an ammonium salt in the form of white powder (85 mg).

¹H NMR (400 MHz, D₂O) δ 8.27 (s, 1H), 8.19 (d, J = 0.6 Hz, 1H), 7.95 (d, J = 0.7 Hz, 1H), 6.63 - 6.29 (m, 3H), 5.28 (dddd, J = 46.4, 6.9, 1.6, 0.7 Hz, 1H), 4.62 (ddqd, J = 4.5, 3.7, 1.5, 0.6 Hz, 1H), 4.57 - 4.40 (m, 2H), 4.36 - 4.20 (m, 5H), 4.16 - 3.95 (m, 5H), 3.93 - 3.81 (m, 4H), 3.73 - 3.51 (m, 3H), 3.37 (d, J = 1.6 Hz, 3H), 3.23 (td, J = 6.3, 0.8 Hz, 2H), 3.10 (s, 3H), 1.95 (tt, J = 6.3, 5.3 Hz, 2H).

³¹P NMR (162 MHz, D₂O) δ 0.60, -9.01, -10.23, -21.17.

### Example 33

### Synthesis of Compound 880

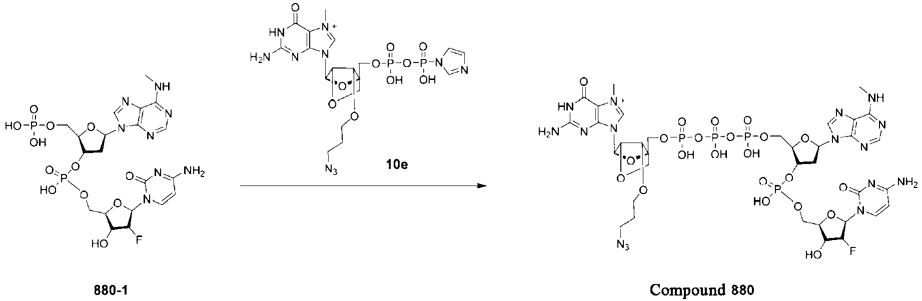

The compound ***10e*** (200 mg) was added to 16 mL of an aqueous solution with a pH of 7.0, containing 0.2 mol/L N-methylmorpholine and 0.2 mol/L manganous chloride, and a compound ***880-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***880**,* which was an ammonium salt in the form of white powder (92 mg).

¹H NMR (400 MHz, D₂O) δ 8.35 (d, J = 0.6 Hz, 1H), 8.27 (s, 1H), 7.69 (dd, J = 7.4, 1.8 Hz, 1H), 6.60 - 6.29 (m, 2H), 6.15 (d, J = 7.3 Hz, 1H), 5.89 - 5.26 (m, 1H), 4.52 (dd, J = 11.6, 8.5 Hz, 1H), 4.44 - 3.84 (m, 16H), 3.75 - 3.48 (m, 3H), 3.23 (td, J = 6.3, 0.8 Hz, 2H), 3.10 (s, 3H), 2.87 - 2.56 (m, 2H), 1.95 (tt, J = 6.3, 5.3 Hz, 2H).

³¹P NMR (162 MHz, D₂O) δ 0.60, -9.10, -10.31, -21.17.

### Example 34

### Synthesis of Compound 903

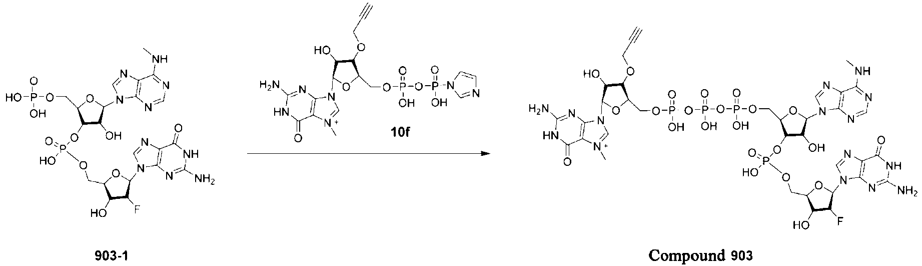

A compound ***10f*** (200 mg) was added to 16 mL of an aqueous solution with a pH of 7.0, containing 0.2 mol/L N-methylmorpholine and 0.2 mol/L manganous chloride, and a compound ***903-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***9*03,** which was an ammonium salt in the form of white powder (85 mg).

¹H NMR (400 MHz, D₂O) δ 8.48 - 8.18 (m, 2H), 8.02 (d, J = 0.7 Hz, 1H), 6.45 (ddq, J = 25.2, 1.6, 0.8 Hz, 1H), 6.25 - 6.00 (m, 2H), 5.22 (dddd, J = 46.4, 7.0, 1.7, 0.7 Hz, 1H), 4.89 - 4.62 (m, 3H), 4.59 - 4.09 (m, 13H), 3.98 (d, J = 0.7 Hz, 3H), 3.08 (s, 3H), 2.42 (t, J = 3.0 Hz, 1H).

³¹P NMR (162 MHz, D₂O) δ 0.60, -9.10, -10.22, -21.17.

### Example 35

### Synthesis of Compound 974

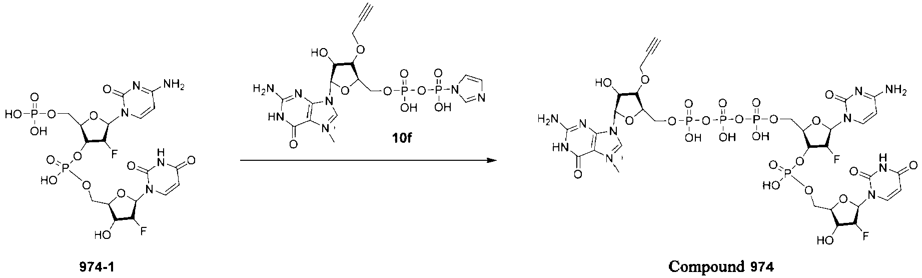

The compound ***10f*** (200 mg) was added to 16 mL of an aqueous solution with a pH of 7.0, containing 0.2 mol/L N-methylmorpholine and 0.2 mol/L manganous chloride, and a compound ***974-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***97*4,** which was an ammonium salt in the form of white powder (80 mg).

¹H NMR (400 MHz, D₂O) δ 7.76 (ddd, J = 44.0, 7.5, 1.8 Hz, 2H), 6.20 (dq, J = 2.3, 0.8 Hz, 1H), 5.97 (dd, J = 56.9, 7.5 Hz, 2H), 5.64 - 5.41 (m, 2H), 5.34 - 4.98 (m, 2H), 4.89 - 4.63 (m, 2H), 4.55 - 4.08 (m, 13H), 3.98 (d, J = 0.7 Hz, 3H), 2.42 (t, J = 3.0 Hz, 1H).

³¹P NMR (162 MHz, D₂O) δ -0.22, -9.07, -10.25, -21.27.

### Example 36

### Synthesis of Compound 1040

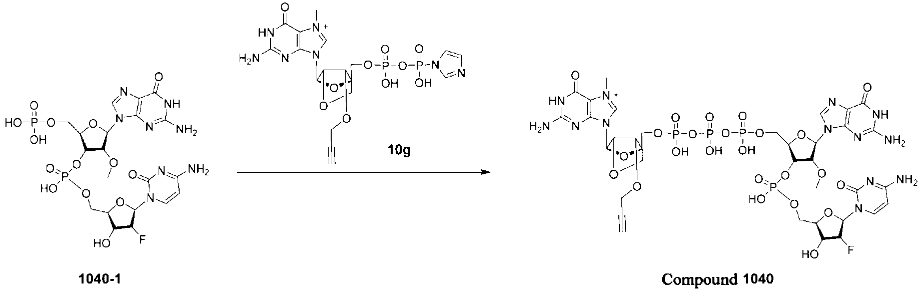

A compound ***10g*** (200 mg) was added to 16 mL of an aqueous solution with a pH of 7.0, containing 0.2 mol/L N-methylmorpholine and 0.2 mol/L manganous chloride, and a compound ***1040-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1040,*** which was an ammonium salt in the form of white powder (58 mg).

¹H NMR (400 MHz, D₂O) δ 7.88 (d, J = 0.7 Hz, 1H), 7.70 (dd, J = 7.4, 1.8 Hz, 1H), 6.47 - 6.14 (m, 2H), 6.04 (d, J = 7.3 Hz, 1H), 5.40 (dddd, J = 25.2, 3.5, 1.8, 0.9 Hz, 1H), 5.10 - 4.64 (m, 4H), 4.43 - 4.08 (m, 12H), 4.02 (s, 2H), 3.98 (s, 3H), 3.40 (s, 3H), 2.42 (t, J = 3.0 Hz, 1H).

³¹P NMR (162 MHz, D₂O) δ 0.66, -9.13, -10.26, -21.25.

### Example 37

### Synthesis of Compound 1087

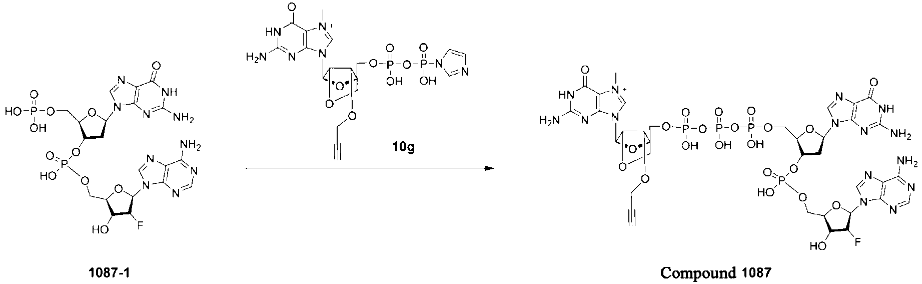

The compound ***10g*** (200 mg) was added to 16 mL of an aqueous solution with a pH of 7.0, containing 0.2 mol/L N-methylmorpholine and 0.2 mol/L manganous chloride, and a compound ***1087-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1087**,* which was an ammonium salt in the form of white powder (88 mg).

¹H NMR (400 MHz, D₂O) δ 8.31 - 8.11 (m, 2H), 7.83 (d, J = 0.6 Hz, 1H), 6.88 - 6.07 (m, 3H), 5.27 (dddd, J = 46.5, 7.0, 4.0, 0.6 Hz, 1H), 5.05 - 4.88 (m, 2H), 4.54 - 4.04 (m, 12H), 4.02 (s, 2H), 3.98 (s, 3H), 2.77 - 2.52 (m, 2H), 2.42 (t, J = 3.0 Hz, 1H).

³¹P NMR (162 MHz, D₂O) δ 0.85, -9.12, -10.24, -21.26.

### Example 38

### Synthesis of Compound 1108

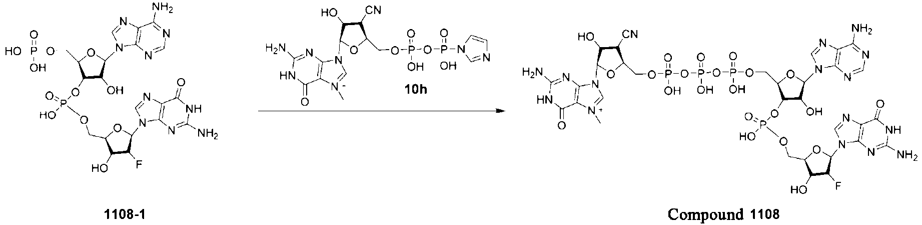

A compound ***10h*** (200 mg) was added to 16 mL of an aqueous solution with a pH of 7.0, containing 0.2 mol/L N-methylmorpholine and 0.2 mol/L manganous chloride, and a compound ***1108-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1108,*** which was an ammonium salt in the form of white powder (88 mg).

¹H NMR (400 MHz, D₂O) δ 8.33 - 8.15 (m, 2H), 8.02 (d, J = 0.7 Hz, 1H), 6.45 (ddq, J = 25.2, 1.5, 0.8 Hz, 1H), 6.32 - 5.94 (m, 2H), 5.22 (dddd, J = 46.4, 7.0, 1.7, 0.7 Hz, 1H), 4.83 - 4.69 (m, 2H), 4.56 (dt, J = 3.8, 0.9 Hz, 1H), 4.51 - 4.06 (m, 10H), 3.98 (d, J = 0.7 Hz, 3H), 3.65 (ddd, J = 3.6, 2.6, 0.6 Hz, 1H).

³¹P NMR (162 MHz, D₂O) δ 0.85, -9.12, -10.24, -21.26.

### Example 39

### Synthesis of Compound 1130

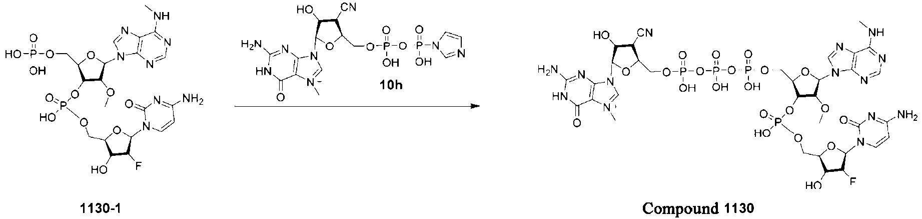

The compound ***10h*** (200 mg) was added to 16 mL of an aqueous solution with a pH of 7.0, containing 0.2 mol/L N-methylmorpholine and 0.2 mol/L manganous chloride, and a compound ***1130-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1130**,* which was an ammonium salt in the form of white powder (88 mg).

¹H NMR (400 MHz, D₂O) δ 8.51 - 8.01 (m, 2H), 7.70 (dd, J = 7.4, 1.8 Hz, 1H), 6.31 - 6.15 (m, 2H), 6.04 (d, J = 7.3 Hz, 1H), 5.56 - 5.22 (m, 1H), 4.97 (dddd, J = 8.0, 4.4, 2.9, 0.7 Hz, 1H), 4.90 - 4.69 (m, 2H), 4.56 (dt, J = 3.8, 0.9 Hz, 1H), 4.48 - 4.10 (m, 10H), 3.98 (s, 3H), 3.65 (ddd, J = 3.6, 2.6, 0.6 Hz, 1H), 3.40 (s, 3H), 3.08 (s, 3H).

³¹P NMR (162 MHz, D₂O) δ 0.85, -9.12, -10.24, -21.26.

### Example 40

### Synthesis of Compound 1130

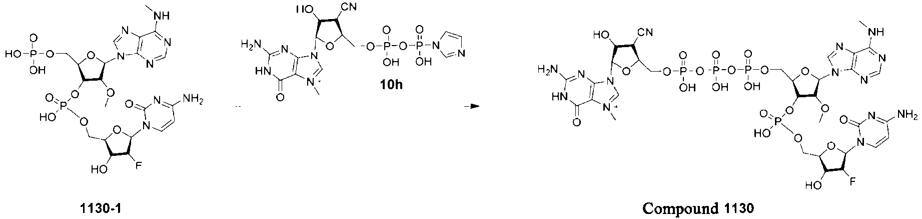

The compound ***10h*** (200 mg) was added to 16 mL of an aqueous solution with a pH of 7.0, containing 0.2 mol/L N-methylmorpholine and 0.2 mol/L manganous chloride, and a compound ***1130-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1130**,* which was an ammonium salt in the form of white powder (88 mg).

¹H NMR (400 MHz, D₂O) δ 8.51 - 8.01 (m, 2H), 7.70 (dd, J = 7.4, 1.8 Hz, 1H), 6.31 - 6.15 (m, 2H), 6.04 (d, J = 7.3 Hz, 1H), 5.56 - 5.22 (m, 1H), 4.97 (dddd, J = 8.0, 4.4, 2.9, 0.7 Hz, 1H), 4.90 - 4.69 (m, 2H), 4.56 (dt, J = 3.8, 0.9 Hz, 1H), 4.48 - 4.10 (m, 10H), 3.98 (s, 3H), 3.65 (ddd, J = 3.6, 2.6, 0.6 Hz, 1H), 3.40 (s, 3H), 3.08 (s, 3H).

³¹P NMR (162 MHz, D₂O) δ 0.85, -9.12, -10.24, -21.26.

### Example 41

### Synthesis of Compound 1140

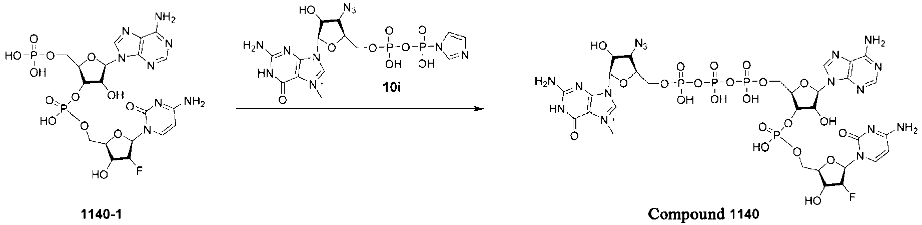

A compound ***10i*** (200 mg) was added to 16 mL of an aqueous solution with a pH of 7.0, containing 0.2 mol/L N-methylmorpholine and 0.2 mol/L manganous chloride, and a compound ***1140-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1140**,* which was an ammonium salt in the form of white powder (88 mg).

¹H NMR (400 MHz, D₂O) δ 8.18 (d, J = 1.3 Hz, 2H), 7.69 (dd, J = 7.4, 1.8 Hz, 1H), 6.36 - 6.26 (m, 1H), 6.20 - 6.09 (m, 2H), 5.80 - 5.38 (m, 1H), 4.82 (ddd, J = 3.9, 3.1, 0.7 Hz, 1H), 4.62 (ddd, J = 5.5, 2.8, 0.7 Hz, 1H), 4.42 - 3.95 (m, 13H), 3.92 (s, 3H).

³¹P NMR (162 MHz, D₂O) δ 0.85, -9.12, -10.24, -21.26.

### Example 42

### Synthesis of Compound 1198

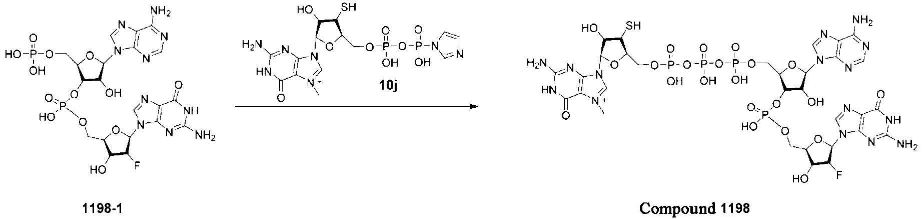

A compound ***10j*** (200 mg) was added to 16 mL of an aqueous solution with a pH of 7.0, containing 0.2 mol/L N-methylmorpholine and 0.2 mol/L manganous chloride, and a compound ***1198-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1198*,** which was an ammonium salt in the form of white powder (88 mg).

¹H NMR (400 MHz, D₂O) δ 8.28 (d, J = 0.5 Hz, 1H), 8.20 (s, 1H), 8.02 (d, J = 0.7 Hz, 1H), 6.45 (ddq, J = 25.2, 1.5, 0.8 Hz, 1H), 6.22 (dd, J = 3.1, 0.7 Hz, 1H), 6.11 (dd, J = 3.1, 0.7 Hz, 1H), 5.22 (dddd, J = 46.4, 7.0, 1.7, 0.7 Hz, 1H), 4.86 - 4.66 (m, 2H), 4.59 - 4.39 (m, 2H), 4.36 - 4.06 (m, 9H), 3.98 (s, 3H), 2.80 (ddd, J = 5.1, 4.0, 0.7 Hz, 1H).

³¹P NMR (162 MHz, D₂O) δ 0.85, -9.12, -10.24, -21.26.

### Example 43

### Synthesis of Compound 1220

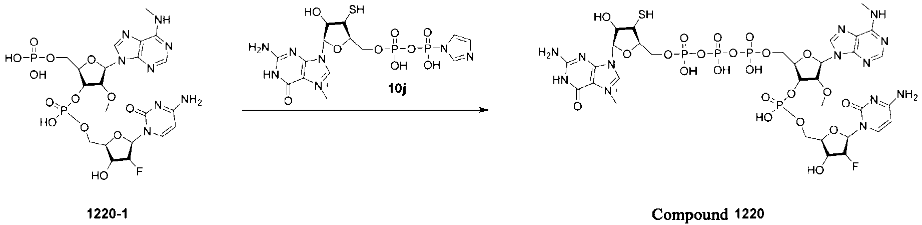

The compound ***10j*** (200 mg) was added to 16 mL of an aqueous solution with a pH of 7.0, containing 0.2 mol/L N-methylmorpholine and 0.2 mol/L manganous chloride, and a compound ***1220-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1220*,** which was an ammonium salt in the form of white powder (88 mg).

¹H NMR (400 MHz, D₂O) δ 8.29 (s, 1H), 8.20 (d, J = 0.7 Hz, 1H), 7.70 (dd, J = 7.4, 1.8 Hz, 1H), 6.32 - 6.16 (m, 2H), 6.04 (d, J = 7.3 Hz, 1H), 5.61 - 5.20 (m, 1H), 5.07 - 4.65 (m, 3H), 4.50 - 4.06 (m, 11H), 3.98 (s, 3H), 3.40 (s, 3H), 3.08 (s, 3H), 2.86 (ddd, J = 3.9, 3.3, 0.7 Hz, 1H).

³¹P NMR (162 MHz, D₂O) δ 0.85, -9.12, -10.24, -21.26.

### Example 44

### Synthesis of Compound 1280

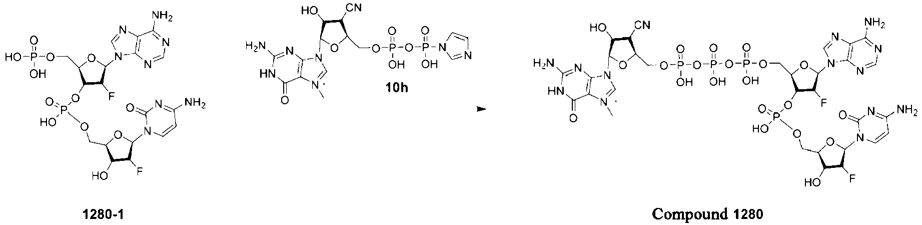

A compound ***10h*** (200 mg) was added to 16 mL of an aqueous solution with a pH of 7.0, containing 0.2 mol/L N-methylmorpholine and 0.2 mol/L manganous chloride, and a compound ***1280-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1280**,* which was an ammonium salt in the form of white powder (88 mg).

¹H NMR (400 MHz, D₂O) δ 8.24 (d, J = 0.7 Hz, 1H), 8.20 (s, 1H), 7.70 (dd, J = 7.4, 1.8 Hz, 1H), 6.43 (ddq, J = 25.2, 1.5, 0.8 Hz, 1H), 6.24 (p, J = 0.8 Hz, 1H), 6.04 (d, J = 7.3 Hz, 1H), 5.50 - 5.10 (m, 3H), 4.81 (dddd, J = 46.5, 7.1, 3.7, 0.7 Hz, 1H), 4.56 (dt, J = 3.8, 0.9 Hz, 1H), 4.48 (tdd, J = 3.5, 2.4, 0.8 Hz, 1H), 4.44 - 4.09 (m, 9H), 3.98 (s, 3H), 3.65 (ddd, J = 3.6, 2.6, 0.6 Hz, 1H).

³¹P NMR (162 MHz, D₂O) δ 0.85, -9.12, -10.24, -21.26.

### Example 45

### Synthesis of Compound 1307

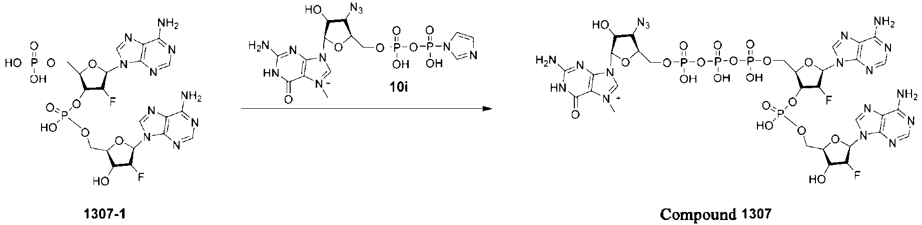

A compound ***10i*** (200 mg) was added to 16 mL of an aqueous solution with a pH of 7.0, containing 0.2 mol/L N-methylmorpholine and 0.2 mol/L manganous chloride, and a compound ***1307-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1307**,* which was an ammonium salt in the form of white powder (88 mg).

¹H NMR (400 MHz, D₂O) δ 8.22 - 8.03 (m, 4H), 6.60 - 6.35 (m, 2H), 6.31 - 6.22 (m, 1H), 5.54 - 5.15 (m, 2H), 4.62 (ddd, J = 5.5, 2.8, 0.7 Hz, 1H), 4.51 - 4.01 (m, 12H), 3.92 (s, 3H).

³¹P NMR (162 MHz, D₂O) δ 0.85, -9.12, -10.24, -21.26.

### Example 46

### Synthesis of Compound 1373

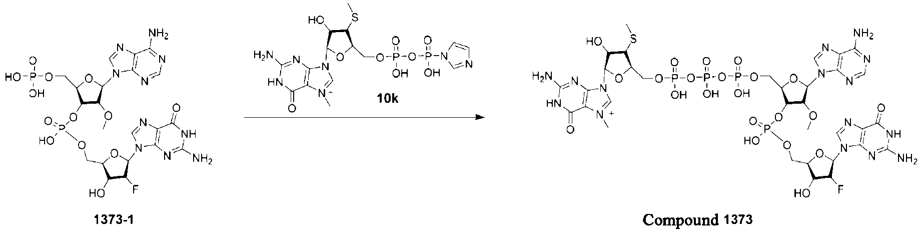

A compound ***10k*** (200 mg) was added to 16 mL of an aqueous solution with a pH of 7.0, containing 0.2 mol/L N-methylmorpholine and 0.2 mol/L manganous chloride, and a compound ***1373-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1373**,* which was an ammonium salt in the form of white powder (88 mg).

¹H NMR (400 MHz, D₂O) δ 8.40 (d, J = 0.5 Hz, 1H), 8.20 (s, 1H), 8.02 (d, J = 0.7 Hz, 1H), 6.45 (ddq, J = 25.2, 1.6, 0.8 Hz, 1H), 6.35 - 5.85 (m, 2H), 5.22 (dddd, J = 46.4, 7.0, 1.7, 0.7 Hz, 1H), 4.97 (dddd, J = 8.0, 4.4, 2.9, 0.7 Hz, 1H), 4.84 - 4.69 (m, 1H), 4.58 (ddd, J = 3.8, 1.6, 0.7 Hz, 1H), 4.45 (dddd, J = 25.2, 6.8, 5.0, 0.7 Hz, 1H), 4.36 - 4.30 (m, 2H), 4.30 - 4.05 (m, 7H), 3.98 (s, 3H), 3.61 (dddd, J = 3.8, 2.6, 1.8, 1.1 Hz, 1H), 3.40 (s, 3H), 2.09 (s, 3H).

³¹P NMR (162 MHz, D₂O) δ 0.85, -9.12, -10.24, -21.26.

### Example 47

### Synthesis of Compound 1400

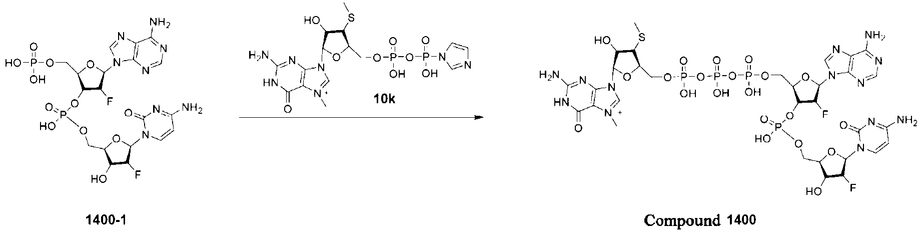

The compound ***10k*** (200 mg) was added to 16 mL of an aqueous solution with a pH of 7.0, containing 0.2 mol/L N-methylmorpholine and 0.2 mol/L manganous chloride, and a compound ***1400-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1400,*** which was an ammonium salt in the form of white powder (88 mg).

¹H NMR (400 MHz, D₂O) δ 8.43 - 8.11 (m, 2H), 7.70 (dd, J = 7.4, 1.8 Hz, 1H), 6.43 (ddq, J = 25.2, 1.6, 0.8 Hz, 1H), 6.20 (dt, J = 1.6, 0.8 Hz, 1H), 6.04 (d, J = 7.3 Hz, 1H), 5.49 - 5.13 (m, 3H), 4.81 (dddd, J = 46.5, 7.1, 3.7, 0.7 Hz, 1H), 4.58 (ddd, J = 3.8, 1.6, 0.7 Hz, 1H), 4.48 (tdd, J = 3.5, 2.4, 0.8 Hz, 1H), 4.42 - 4.03 (m, 9H), 3.98 (s, 3H), 3.61 (dddd, J = 3.8, 2.5, 1.8, 1.1 Hz, 1H), 2.09 (s, 3H).

³¹P NMR (162 MHz, D₂O) δ 0.85, -9.12, -10.24, -21.26.

### Example 48

### Synthesis of Compound 1421

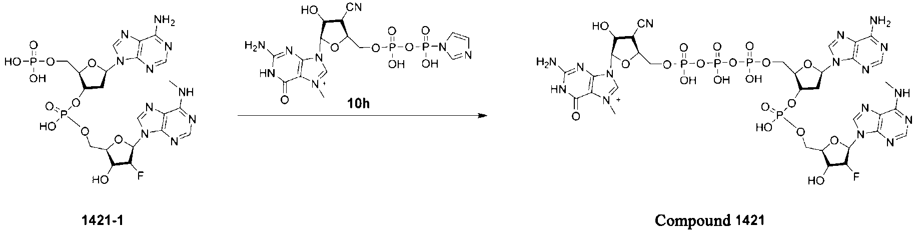

The compound ***10h*** (200 mg) was added to 16 mL of an aqueous solution with a pH of 7.0, containing 0.2 mol/L N-methylmorpholine and 0.2 mol/L manganous chloride, and a compound ***1421-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1421*,** which was an ammonium salt in the form of white powder (88 mg).

¹H NMR (400 MHz, D₂O) δ 8.51 - 7.89 (m, 4H), 6.46 - 6.31 (m, 2H), 6.24 (p, J = 0.8 Hz, 1H), 5.27 (dddd, J = 46.5, 7.0, 4.0, 0.6 Hz, 1H), 5.08 - 4.88 (m, 1H), 4.56 (dt, J = 3.8, 0.9 Hz, 1H), 4.52 - 4.38 (m, 2H), 4.36 - 4.04 (m, 8H), 3.98 (s, 3H), 3.65 (ddd, J = 3.6, 2.6, 0.6 Hz, 1H), 3.08 (s, 3H), 2.80 - 2.52 (m, 2H).

³¹P NMR (162 MHz, D₂O) δ 0.85, -9.12, -10.24, -21.26.

### Example 49

### Synthesis of Compound 1449

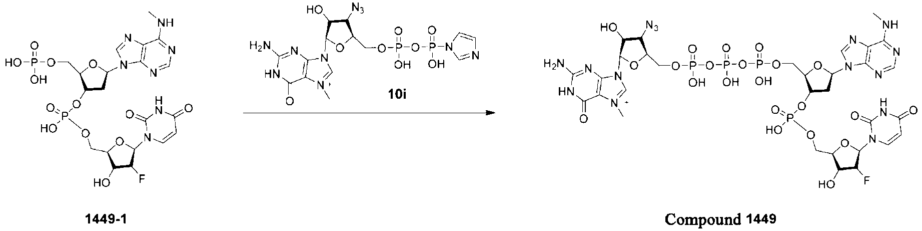

The compound ***10i*** (200 mg) was added to 16 mL of an aqueous solution with a pH of 7.0, containing 0.2 mol/L N-methylmorpholine and 0.2 mol/L manganous chloride, and a compound ***1449-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1449*,** which was an ammonium salt in the form of white powder (88 mg).

¹H NMR (400 MHz, D₂O) δ 8.40 - 8.08 (m, 2H), 7.59 - 7.18 (m, 2H), 6.48 - 6.34 (m, 1H), 6.24 (dq, J = 4.7, 0.8 Hz, 1H), 5.98 (dddt, J = 25.2, 4.0, 1.7, 0.8 Hz, 1H), 4.72 (ddd, J = 7.4, 4.7, 0.7 Hz, 1H), 4.49 - 3.97 (m, 12H), 3.95 - 3.80 (m, 4H), 3.10 (s, 3H), 2.93 - 2.53 (m, 2H).

³¹P NMR (162 MHz, D₂O) δ 0.85, -9.12, -10.24, -21.26.

### Example 50

### Synthesis of Compound 1449

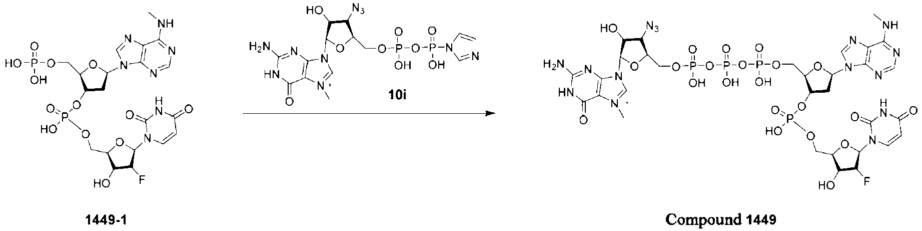

The compound ***10i*** (200 mg) was added to 16 mL of an aqueous solution with a pH of 7.0, containing 0.2 mol/L N-methylmorpholine and 0.2 mol/L manganous chloride, and a compound ***1449-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1449*,** which was an ammonium salt in the form of white powder (88 mg).

¹H NMR (400 MHz, D₂O) δ 8.40 - 8.08 (m, 2H), 7.59 - 7.18 (m, 2H), 6.48 - 6.34 (m, 1H), 6.24 (dq, J = 4.7, 0.8 Hz, 1H), 5.98 (dddt, J = 25.2, 4.0, 1.7, 0.8 Hz, 1H), 4.72 (ddd, J = 7.4, 4.7, 0.7 Hz, 1H), 4.49 - 3.97 (m, 12H), 3.95 - 3.80 (m, 4H), 3.10 (s, 3H), 2.93 - 2.53 (m, 2H).

³¹P NMR (162 MHz, D₂O) δ 0.85, -9.12, -10.24, -21.26.

### Example 51

### Synthesis of Compound 1495

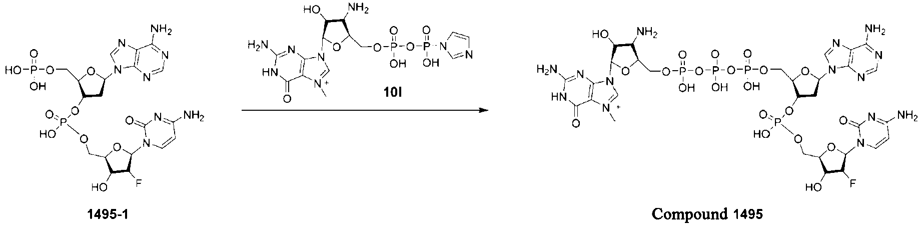

A compound ***10l*** (200 mg) was added to 16 mL of an aqueous solution with a pH of 7.0, containing 0.2 mol/L N-methylmorpholine and 0.2 mol/L manganous chloride, and a compound ***1495-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1495*,** which was an ammonium salt in the form of white powder (88 mg).

¹H NMR (400 MHz, D₂O) δ 8.30 (d, J = 0.6 Hz, 1H), 8.20 (s, 1H), 7.70 (dd, J = 7.4, 1.8 Hz, 1H), 6.55 - 6.34 (m, 1H), 6.16 (dq, J = 2.2, 0.8 Hz, 1H), 6.04 (d, J = 7.3 Hz, 1H), 5.40 (dddd, J = 25.2, 3.4, 1.8, 0.9 Hz, 1H), 5.12 - 4.93 (m, 1H), 4.81 (dddd, J = 46.5, 7.1, 3.7, 0.7 Hz, 1H), 4.41 - 4.04 (m, 11H), 3.98 (s, 3H), 3.63 (ddd, J = 4.6, 3.1, 0.7 Hz, 1H), 2.81 - 2.48 (m, 2H).

³¹P NMR (162 MHz, D₂O) δ 0.85, -10.30, -21.26.

### Example 52

### Synthesis of Compound 1580

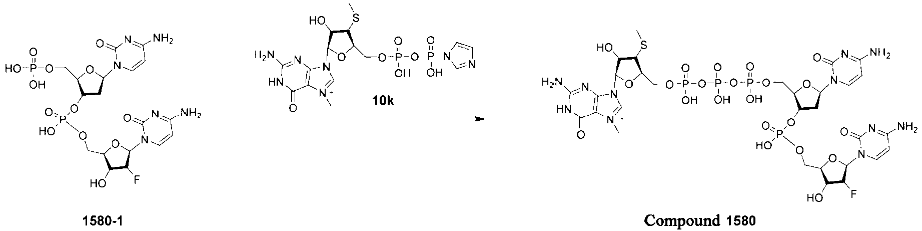

The compound ***10k*** (200 mg) was added to 16 mL of an aqueous solution with a pH of 7.0, containing 0.2 mol/L N-methylmorpholine and 0.2 mol/L manganous chloride, and a compound ***1580-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1580,*** which was an ammonium salt in the form of white powder (88 mg).

¹H NMR (400 MHz, D₂O) δ 7.73 (ddd, J = 24.9, 7.2, 1.8 Hz, 2H), 6.35 - 6.15 (m, 2H), 6.03 (dd, J = 12.2, 7.2 Hz, 2H), 5.40 (dddd, J = 25.2, 3.5, 1.8, 0.9 Hz, 1H), 5.10 (ddddd, J = 8.3, 6.4, 4.5, 3.7, 0.7 Hz, 1H), 4.81 (dddd, J = 46.5, 7.1, 3.7, 0.7 Hz, 1H), 4.58 (ddd, J = 3.8, 1.6, 0.7 Hz, 1H), 4.39 - 4.00 (m, 10H), 3.98 (s, 3H), 3.74 - 3.47 (m, 1H), 2.61 - 2.28 (m, 2H), 2.09 (s, 3H).

³¹P NMR (162 MHz, D₂O) δ 0.85, -10.24, -21.26.

### Example 53

### Synthesis of Compound 1581

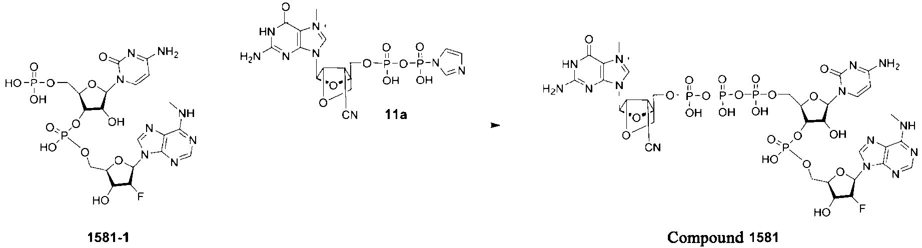

A compound ***11a*** (200 mg) was added to 16 mL of an aqueous solution with a pH of 7.0, containing 0.2 mol/L N-methylmorpholine and 0.2 mol/L manganous chloride, and a compound ***1581-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1581*,** which was an ammonium salt in the form of white powder (88 mg).

¹H NMR (400 MHz, D₂O) δ 8.33 - 7.87 (m, 2H), 7.60 (dd, J = 7.4, 1.8 Hz, 1H), 6.49 - 6.23 (m, 2H), 6.15 - 5.73 (m, 2H), 5.27 (dddd, J = 46.5, 7.0, 4.0, 0.6 Hz, 1H), 5.00 (dd, J = 3.2, 2.5 Hz, 1H), 4.80 - 4.62 (m, 1H), 4.53 - 4.32 (m, 4H), 4.31 - 4.10 (m, 6H), 4.09 (s, 2H), 3.98 (s, 3H), 3.96 (d, J = 3.2 Hz, 1H), 3.08 (s, 3H).

³¹P NMR (162 MHz, D₂O) δ 0.85, -10.24, -21.26.

### Example 54

### Synthesis of Compound 1617

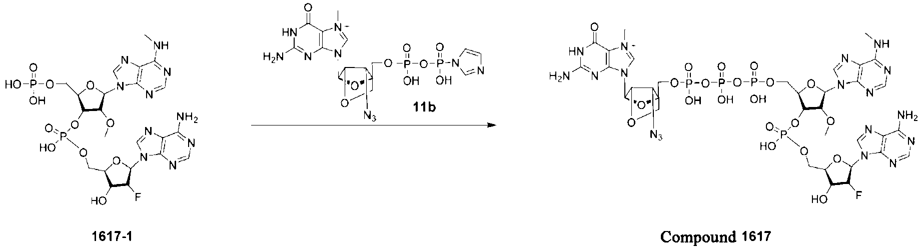

A compound ***11b*** (200 mg) was added to 16 mL of an aqueous solution with a pH of 7.0, containing 0.2 mol/L N-methylmorpholine and 0.2 mol/L manganous chloride, and a compound ***1617-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1617*,** which was an ammonium salt in the form of white powder (88 mg).

¹H NMR (400 MHz, D₂O) δ 8.27 (s, 1H), 8.22 - 8.17 (m, 2H), 8.14 (d, J = 0.6 Hz, 1H), 6.60 - 6.31 (m, 3H), 5.31 (dddd, J = 46.5, 7.0, 4.0, 0.6 Hz, 1H), 4.81 (dd, J = 4.8, 2.0 Hz, 1H), 4.66 - 4.54 (m, 2H), 4.46 (dddd, J = 25.2, 6.8, 5.0, 0.7 Hz, 1H), 4.36 - 4.21 (m, 6H), 4.19 - 3.99 (m, 4H), 3.92 (d, J = 0.6 Hz, 3H), 3.83 (dd, J = 11.3, 8.5 Hz, 1H), 3.37 (s, 3H), 3.10 (s, 3H).

³¹P NMR (162 MHz, D₂O) δ 0.85, -10.24, -21.26.

### Example 55

### Synthesis of Compound 1660

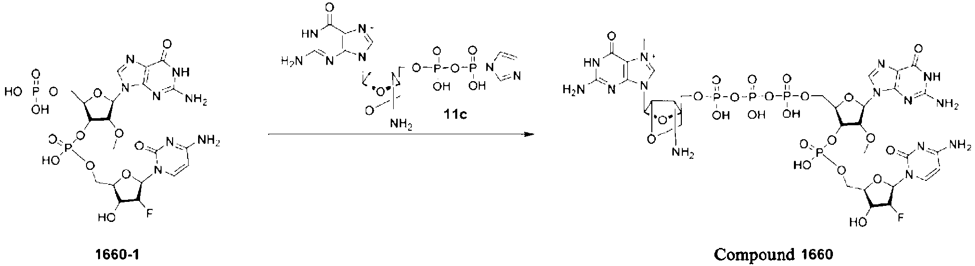

A compound ***11c*** (200 mg) was added to 16 mL of an aqueous solution with a pH of 7.0, containing 0.2 mol/L N-methylmorpholine and 0.2 mol/L manganous chloride, and a compound ***1660-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1660**,* which was an ammonium salt in the form of white powder (88 mg).

¹H NMR (400 MHz, D₂O) δ 7.88 (d, J = 0.7 Hz, 1H), 7.70 (dd, J = 7.4, 1.8 Hz, 1H), 6.51 - 6.18 (m, 2H), 6.04 (d, J = 7.3 Hz, 1H), 5.40 (dddd, J = 25.2, 3.5, 1.8, 0.9 Hz, 1H), 5.05 - 4.59 (m, 4H), 4.48 - 4.09 (m, 9H), 4.04 - 3.82 (m, 6H), 3.40 (s, 3H).

³¹P NMR (162 MHz, D₂O) δ 0.85, -10.24, -21.26.

### Example 56

### Synthesis of Compound 1698

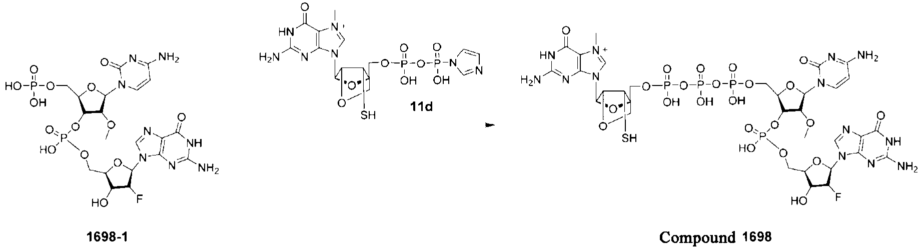

A compound ***11d*** (200 mg) was added to 16 mL of an aqueous solution with a pH of 7.0, containing 0.2 mol/L N-methylmorpholine and 0.2 mol/L manganous chloride, and a compound ***1698-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1698*,** which was an ammonium salt in the form of white powder (88 mg).

¹H NMR (400 MHz, D₂O) δ 8.02 (d, J = 0.7 Hz, 1H), 7.81 (dd, J = 7.3, 1.8 Hz, 1H), 6.63 - 6.39 (m, 2H), 6.18 - 5.82 (m, 2H), 5.22 (dddd, J = 46.4, 7.0, 1.7, 0.7 Hz, 1H), 4.89 (dddd, J = 8.0, 5.4, 4.6, 0.6 Hz, 1H), 4.59 - 4.28 (m, 6H), 4.25 - 4.03 (m, 7H), 3.98 (s, 3H), 3.46 (s, 3H), 3.11 (d, J = 3.1 Hz, 1H).

³¹P NMR (162 MHz, D₂O) δ 0.85, -10.24, -21.26.

### Example 57

### Synthesis of Compound 1728

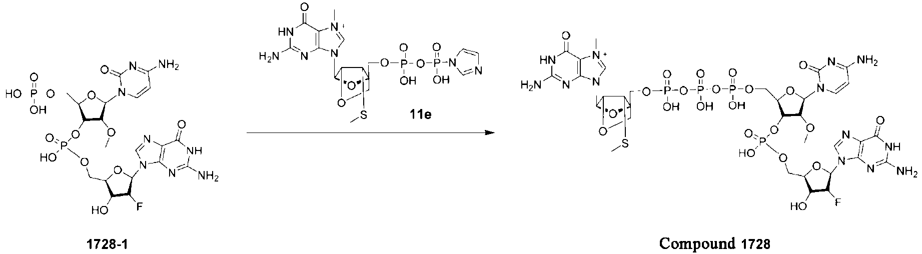

A compound ***11e*** (200 mg) was added to 16 mL of an aqueous solution with a pH of 7.0, containing 0.2 mol/L N-methylmorpholine and 0.2 mol/L manganous chloride, and a compound ***1728-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1728*,** which was an ammonium salt in the form of white powder (88 mg).

¹H NMR (400 MHz, D₂O) δ 8.02 (d, J = 0.7 Hz, 1H), 7.81 (dd, J = 7.3, 1.8 Hz, 1H), 6.73 - 6.18 (m, 2H), 6.18 - 5.82 (m, 2H), 5.22 (dddd, J = 46.4, 7.0, 1.7, 0.7 Hz, 1H), 5.02 - 4.79 (m, 2H), 4.58 - 4.28 (m, 5H), 4.27 - 4.01 (m, 7H), 3.98 (s, 3H), 3.93 (dq, J = 2.9, 1.5 Hz, 1H), 3.46 (s, 3H), 2.10 (d, J = 1.4 Hz, 3H).

³¹P NMR (162 MHz, D₂O) δ 0.85, -10.24, -21.26.

### Example 58

### Synthesis of Compound 1897

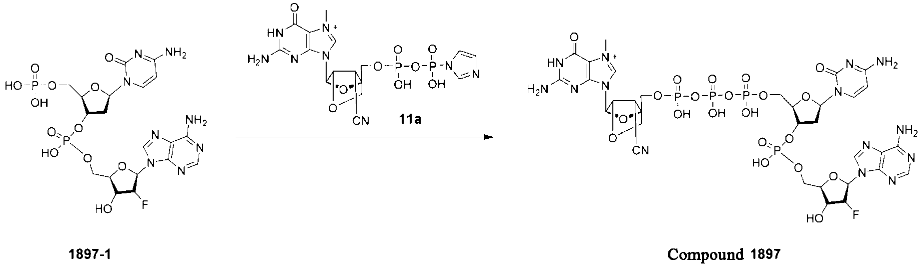

The compound ***11a*** (200 mg) was added to 16 mL of an aqueous solution with a pH of 7.0, containing 0.2 mol/L N-methylmorpholine and 0.2 mol/L manganous chloride, and a compound ***1897-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1897*,** which was an ammonium salt in the form of white powder (88 mg).

¹H NMR (400 MHz, D₂O) δ 8.32 - 8.11 (m, 2H), 7.76 (dd, J = 7.0, 1.8 Hz, 1H), 6.46 - 6.31 (m, 2H), 6.25 (dddd, J = 5.2, 3.3, 1.7, 0.8 Hz, 1H), 6.01 (d, J = 7.2 Hz, 1H), 5.27 (dddd, J = 46.5, 7.0, 4.0, 0.6 Hz, 1H), 5.10 (ddddd, J = 8.3, 6.4, 4.5, 3.7, 0.7 Hz, 1H), 5.00 (dd, J = 3.2, 2.5 Hz, 1H), 4.54 - 4.30 (m, 3H), 4.26 - 4.17 (m, 4H), 4.13 - 4.01 (m, 4H), 3.98 (d, J = 0.7 Hz, 3H), 3.96 (d, J = 3.2 Hz, 1H), 2.60 - 2.26 (m, 2H).

³¹P NMR (162 MHz, D₂O) δ 0.85, -10.24, -21.26.

### Example 59

### Synthesis of Compound 1907

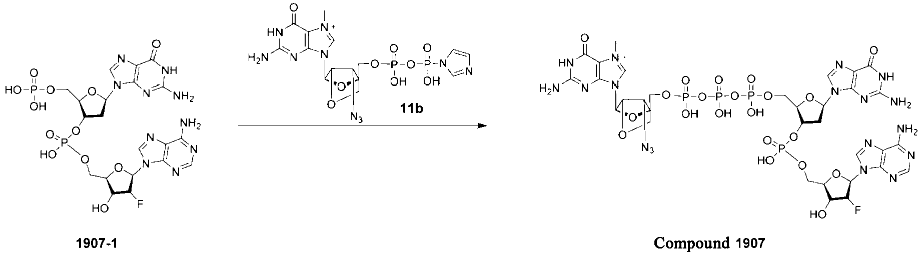

The compound ***11b*** (200 mg) was added to 16 mL of an aqueous solution with a pH of 7.0, containing 0.2 mol/L N-methylmorpholine and 0.2 mol/L manganous chloride, and a compound ***1907-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1907**,* which was an ammonium salt in the form of white powder (88 mg).

¹H NMR (400 MHz, D₂O) δ 8.18 (s, 1H), 8.14 (d, J = 0.6 Hz, 1H), 7.75 (d, J = 0.5 Hz, 1H), 6.49 (dd, J = 2.0, 0.7 Hz, 1H), 6.47 - 6.35 (m, 1H), 6.28 (ddq, J = 3.0, 1.4, 0.7 Hz, 1H), 5.31 (dddd, J = 46.5, 7.0, 4.0, 0.6 Hz, 1H), 4.81 (dd, J = 4.8, 2.0 Hz, 1H), 4.58 (dd, J = 11.3, 8.5 Hz, 1H), 4.50 - 4.39 (m, 1H), 4.37 - 4.19 (m, 6H), 4.14 - 3.98 (m, 3H), 3.96 - 3.76 (m, 5H), 2.92 - 2.35 (m, 2H).

³¹P NMR (162 MHz, D₂O) δ 0.85, -10.24, -21.26.

### Example 60

### Synthesis of Compound 1925

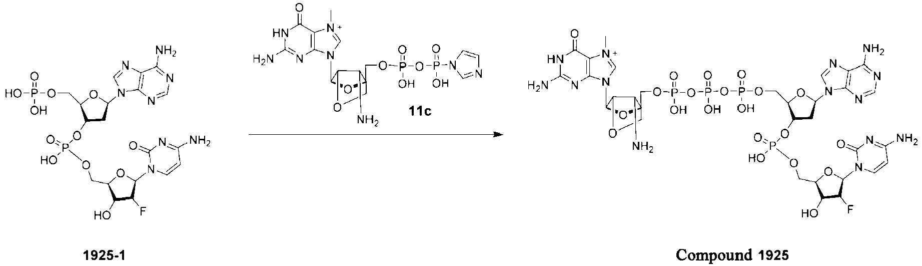

The compound ***11c*** (200 mg) was added to 16 mL of an aqueous solution with a pH of 7.0, containing 0.2 mol/L N-methylmorpholine and 0.2 mol/L manganous chloride, and a compound ***1925-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1925*,** which was an ammonium salt in the form of white powder (88 mg).

¹H NMR (400 MHz, D₂O) δ 8.30 (d, J = 0.5 Hz, 1H), 8.20 (s, 1H), 7.70 (dd, J = 7.4, 1.8 Hz, 1H), 6.42 - 6.36 (m, 1H), 6.31 (dd, J = 2.0, 0.6 Hz, 1H), 6.04 (d, J = 7.3 Hz, 1H), 5.48 - 5.29 (m, 1H), 5.18 - 4.94 (m, 1H), 4.81 (dddd, J = 46.5, 7.1, 3.7, 0.7 Hz, 1H), 4.66 (t, J = 2.2 Hz, 1H), 4.46 - 4.05 (m, 9H), 4.02 - 3.90 (m, 6H), 2.92 - 2.35 (m, 2H).

³¹P NMR (162 MHz, D₂O) δ 0.85, -10.24, -21.26.

### Example 61

### Synthesis of Compound 1978

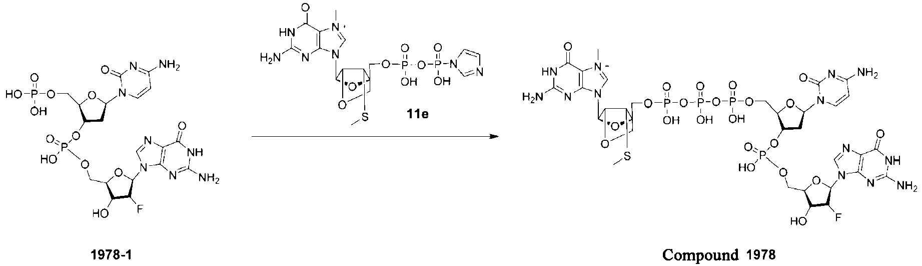

The compound ***11e*** (200 mg) was added to 16 mL of an aqueous solution with a pH of 7.0, containing 0.2 mol/L N-methylmorpholine and 0.2 mol/L manganous chloride, and a compound ***1978-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1978*,** which was an ammonium salt in the form of white powder (88 mg).

¹H NMR (400 MHz, D₂O) δ 8.02 (d, J = 0.7 Hz, 1H), 7.76 (dd, J = 7.0, 1.8 Hz, 1H), 6.54 - 6.35 (m, 2H), 6.25 (dddd, J = 5.2, 3.3, 1.7, 0.8 Hz, 1H), 6.01 (d, J = 7.2 Hz, 1H), 5.37 - 5.03 (m, 2H), 4.88 (dd, J = 3.1, 2.0 Hz, 1H), 4.57 - 4.29 (m, 3H), 4.26 - 4.01 (m, 8H), 3.98 (d, J = 0.7 Hz, 3H), 3.93 (dq, J = 3.0, 1.5 Hz, 1H), 2.71 - 2.26 (m, 2H), 2.10 (s, 3H).

³¹P NMR (162 MHz, D₂O) δ 0.85, -10.24, -21.26.

### Example 62

### Synthesis of Compound 1981

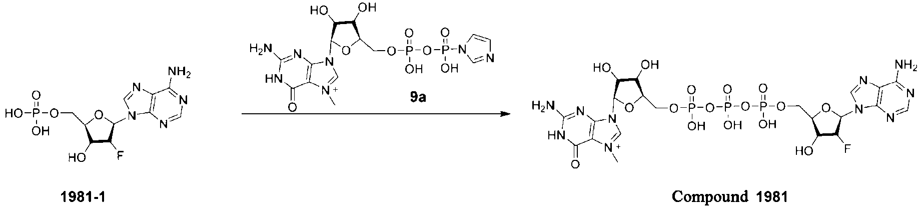

The compound ***9a*** (250 mg) was added to 16 mL of an aqueous solution with a pH of 7.0, containing 0.2 mol/L N-methylmorpholine and 0.2 mol/L manganous chloride, and a compound ***1981-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1981*,** which was an ammonium salt in the form of white powder (88 mg).

¹H NMR (400 MHz, D₂O) δ 8.30 - 8.00 (m, 2H), 6.39 (ddd, J = 25.2, 4.0, 0.7 Hz, 1H), 6.25 - 5.90 (m, 1H), 5.28 (dddd, J = 46.5, 4.9, 4.1, 0.6 Hz, 1H), 4.69 - 4.49 (m, 2H), 4.35 (ddd, J = 5.3, 3.7, 0.7 Hz, 1H), 4.30 - 4.07 (m, 6H), 3.98 (d, J = 0.7 Hz, 3H).

³¹P NMR (162 MHz, D₂O) δ -10.24, -21.26.

### Example 63

### Synthesis of Compound 1988

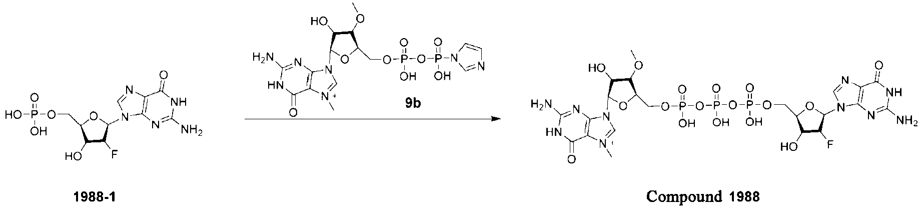

The compound ***9b*** (250 mg) was added to 16 mL of an aqueous solution with a pH of 7.0, containing 0.2 mol/L N-methylmorpholine and 0.2 mol/L manganous chloride, and a compound ***1988-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1988*,** which was an ammonium salt in the form of white powder (88 mg).

¹H NMR (400 MHz, D₂O) δ 8.02 (d, J = 0.7 Hz, 1H), 6.43 (ddd, J = 25.2, 1.5, 0.8 Hz, 1H), 6.17 (dt, J = 2.5, 0.7 Hz, 1H), 5.61 - 5.11 (m, 1H), 4.96 - 4.44 (m, 2H), 4.30 - 4.22 (m, 4H), 4.21 - 4.10 (m, 2H), 4.06 (dtt, J = 4.2, 2.1, 1.1 Hz, 1H), 3.98 (s, 3H), 3.44 (s, 3H).

³¹P NMR (162 MHz, D₂O) δ -10.24, -21.26.

### Example 64

### Synthesis of Compound 1992

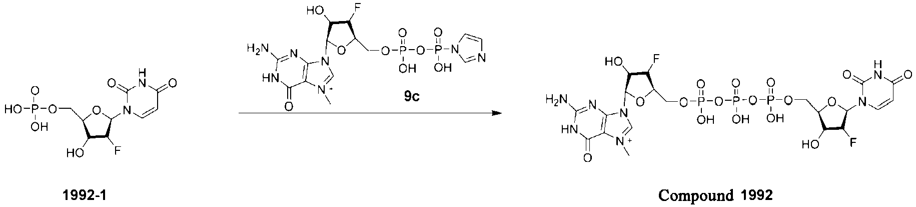

The compound ***9c*** (250 mg) was added to 16 mL of an aqueous solution with a pH of 7.0, containing 0.2 mol/L N-methylmorpholine and 0.2 mol/L manganous chloride, and a compound ***1992-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1992*,** which was an ammonium salt in the form of white powder (88 mg).

¹H NMR (400 MHz, D₂O) δ 7.81 (dd, J = 7.8, 1.8 Hz, 1H), 6.44 - 6.09 (m, 1H), 5.90 (d, J = 7.8 Hz, 1H), 5.53 (dddd, J = 24.4, 3.9, 1.8, 0.9 Hz, 1H), 5.00 - 4.94 (m, 1H), 4.88 - 4.80 (m, 1H), 4.78 - 4.69 (m, 1H), 4.60 (dtd, J = 3.8, 2.7, 0.8 Hz, 1H), 4.57 - 4.51 (m, 1H), 4.33 (ddd, J = 8.5, 3.7, 1.6 Hz, 2H), 4.25 - 4.12 (m, 3H), 3.98 (s, 3H).

³¹P NMR (162 MHz, D₂O) δ -10.24, -21.26.

### Example 65

### Synthesis of Compound 1996

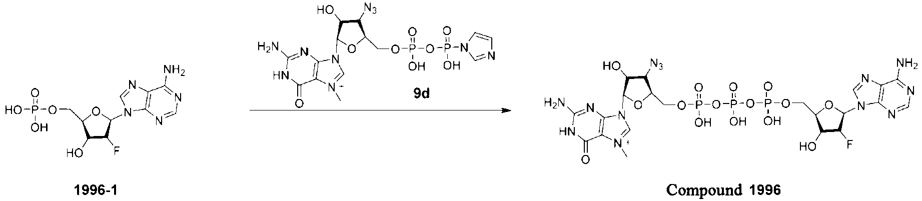

The compound ***9d*** (250 mg) was added to 16 mL of an aqueous solution with a pH of 7.0, containing 0.2 mol/L N-methylmorpholine and 0.2 mol/L manganous chloride, and a compound ***1996-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***1996*,** which was an ammonium salt in the form of white powder (88 mg).

¹H NMR (400 MHz, D₂O) δ 8.18 (s, 1H), 8.14 (d, J = 0.6 Hz, 1H), 6.41 (ddq, J = 25.2, 4.0, 0.8 Hz, 1H), 6.31 - 6.22 (m, 1H), 5.45 - 4.99 (m, 1H), 4.79 - 4.50 (m, 2H), 4.46 - 4.05 (m, 7H), 3.92 (s, 3H).

³¹P NMR (162 MHz, D₂O) δ -10.24, -21.26.

### Example 66

### Synthesis of Compound 2005

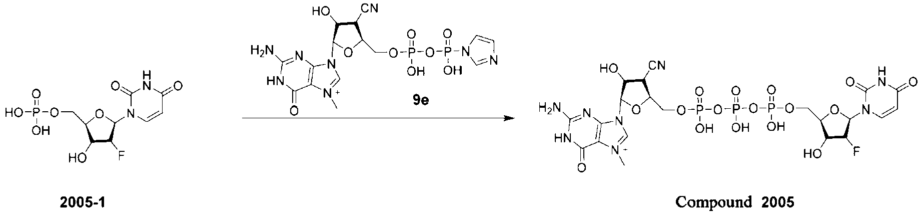

A compound ***9e*** (250 mg) was added to 16 mL of an aqueous solution with a pH of 7.0, containing 0.2 mol/L N-methylmorpholine and 0.2 mol/L manganous chloride, and a compound ***2005-1*** (200 mg) was added to the solution. The reaction solution was stirred at room temperature o**f** 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2005,*** which was an ammonium salt in the form of white powder (88 mg).

¹H NMR (400 MHz, D₂O) δ 7.81 (dd, J = 7.8, 1.8 Hz, 1H), 6.24 (p, J = 0.8 Hz, 1H), 5.90 (d, J = 7.8 Hz, 1H), 5.74 - 5.30 (m, 1H), 4.89 - 4.64 (m, 1H), 4.60 - 4.43 (m, 2H), 4.43 - 4.37 (m, 1H), 4.36 - 4.11 (m, 5H), 3.98 (s, 3H), 3.65 (ddd, J = 3.6, 2.6, 0.6 Hz, 1H).

³¹P NMR (162 MHz, D₂O) δ -10.24, -21.26.

### Example 67

### Synthesis of Compound 2010

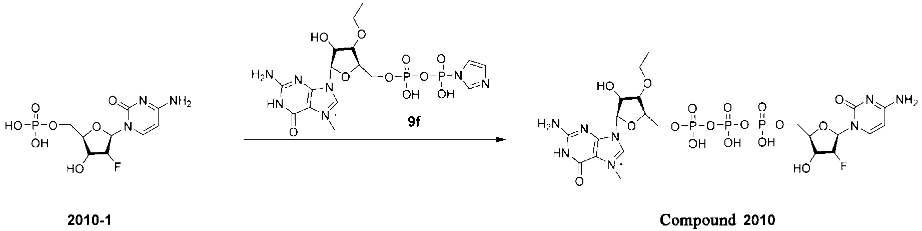

A compound ***9f***(250 mg) was added to 16 mL of an aqueous solution with a pH of 7.0, containing 0.2 mol/L N-methylmorpholine and 0.2 mol/L manganous chloride, and a compound ***2010-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2010,*** which was an ammonium salt in the form of white powder (88 mg).

¹H NMR (400 MHz, D₂O) δ 7.70 (dd, J = 7.3, 1.8 Hz, 1H), 6.18 (dq, J = 2.5, 0.8 Hz, 1H), 6.04 (d, J = 7.3 Hz, 1H), 5.40 (dddt, J = 25.1, 3.4, 1.6, 0.8 Hz, 1H), 4.83 (dddd, J = 46.3, 5.2, 3.7, 0.6 Hz, 1H), 4.68 (ddd, J = 3.2, 2.4, 0.7 Hz, 1H), 4.59 - 4.42 (m, 1H), 4.30 - 4.07 (m, 7H), 3.98 (d, J = 0.7 Hz, 3H), 3.59 (p, J = 5.9 Hz, 2H), 1.24 (t, J = 6.0 Hz, 3H).

³¹P NMR (162 MHz, D₂O) δ -10.24, -21.26.

### Example 68

### Synthesis of Compound 2020

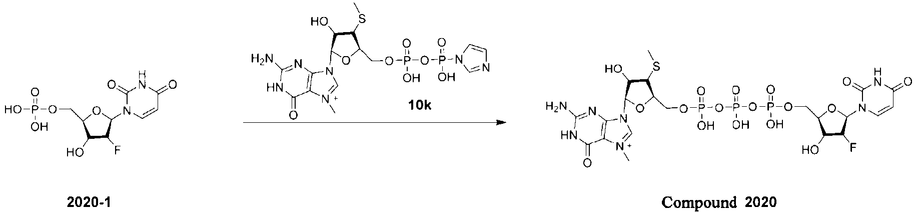

The compound ***10k*** (250 mg) was added to 16 mL of an aqueous solution with a pH of 7.0, containing 0.2 mol/L N-methylmorpholine and 0.2 mol/L manganous chloride, and a compound ***2020-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2020*,** which was an ammonium salt in the form of white powder (88 mg).

¹ H NMR (400 MHz, D₂O) δ 7.81 (dd, J = 7.8, 1.8 Hz, 1H), 6.21 (dq, J = 1.5, 0.8 Hz, 1H), 5.90 (d, J = 7.8 Hz, 1H), 5.62 - 5.35 (m, 1H), 5.00 - 4.69 (m, 1H), 4.62 - 4.41 (m, 2H), 4.36 - 4.11 (m, 6H), 3.98 (d, J = 0.7 Hz, 3H), 3.69 - 3.47 (m, 1H), 2.09 (d, J = 1.4 Hz, 3H).

³¹P NMR (162 MHz, D₂O) δ -10.24, -21.26.

### Example 69

### Synthesis of Compound 2025

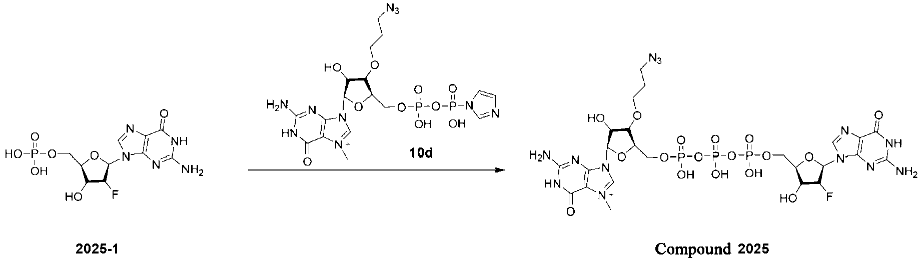

The compound ***10d*** (250 mg) was added to 16 mL of an aqueous solution with a pH of 7.0, containing 0.2 mol/L N-methylmorpholine and 0.2 mol/L manganous chloride, and a compound ***2025-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2025*,** which was an ammonium salt in the form of white powder (88 mg).

¹H NMR (400 MHz, D₂O) δ 7.95 (d, J = 0.7 Hz, 1H), 6.49 (ddt, J = 25.2, 1.5, 0.8 Hz, 1H), 6.28 (dq, J = 2.3, 0.8 Hz, 1H), 5.31 (dddd, J = 46.3, 5.0, 1.6, 0.7 Hz, 1H), 4.70 (ddd, J = 3.3, 2.5, 0.7 Hz, 1H), 4.58 (dddd, J = 25.2, 4.9, 3.1, 0.6 Hz, 1H), 4.44 - 4.21 (m, 5H), 4.13 (qt, J = 3.1, 0.8 Hz, 1H), 3.98 (ddd, J = 3.1, 2.3, 0.6 Hz, 1H), 3.92 (d, J = 0.6 Hz, 3H), 3.56 (td, J = 6.4, 1.5 Hz, 2H), 3.23 (td, J = 6.3, 0.8 Hz, 2H), 1.94 (pd, J = 6.3, 1.0 Hz, 2H).

³¹P NMR (162 MHz, D₂O) δ -10.24, -21.26.

### Example 70

### Synthesis of Compound 2028

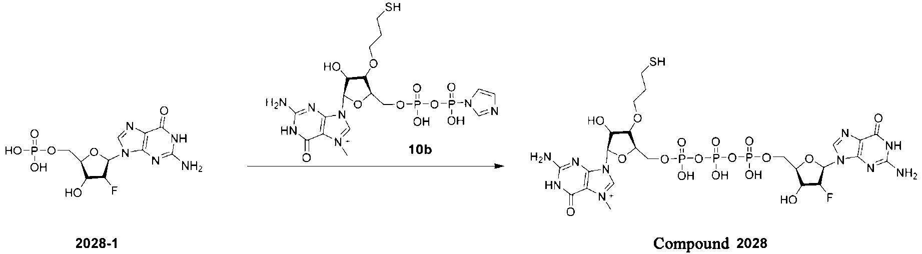

The compound ***10b*** (250 mg) was added to 16 mL of an aqueous solution with a pH of 7.0, containing 0.2 mol/L N-methylmorpholine and 0.2 mol/L manganous chloride, and a compound ***2028-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2028,*** which was an ammonium salt in the form of white powder (88 mg).

¹H NMR (400 MHz, D₂O) δ 8.02 (d, J = 0.7 Hz, 1H), 6.43 (ddd, J = 25.2, 1.5, 0.8 Hz, 1H), 6.27 - 6.03 (m, 1H), 5.54 - 5.05 (m, 1H), 4.71 (ddd, J = 3.3, 2.5, 0.7 Hz, 1H), 4.59 - 4.45 (m, 1H), 4.37 - 4.06 (m, 7H), 3.98 (d, J = 0.7 Hz, 3H), 3.76 - 3.46 (m, 2H), 3.06 - 2.68 (m, 2H), 1.84 (p, J = 6.5 Hz, 2H).

³¹P NMR (162 MHz, D₂O) δ -10.24, -21.26.

### Example 71

### Synthesis of Compound 2031

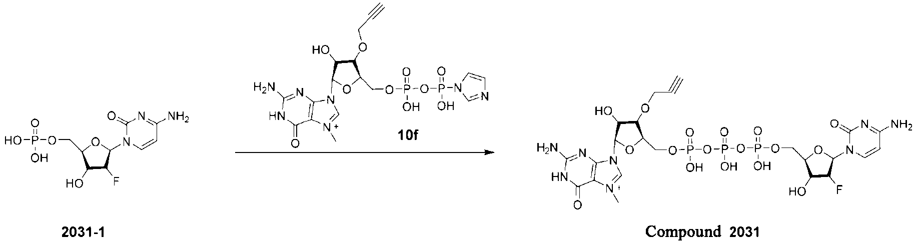

The compound ***10f*** (250 mg) was added to 16 mL of an aqueous solution with a pH of 7.0, containing 0.2 mol/L N-methylmorpholine and 0.2 mol/L manganous chloride, and a compound ***2031-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2031,*** which was an ammonium salt in the form of white powder (88 mg).

¹H NMR (400 MHz, D₂O) δ 7.70 (dd, J = 7.3, 1.8 Hz, 1H), 6.20 (dt, J = 2.3, 0.7 Hz, 1H), 6.04 (d, J = 7.3 Hz, 1H), 5.40 (dddd, J = 25.2, 3.5, 1.7, 0.8 Hz, 1H), 4.97 - 4.69 (m, 2H), 4.59 - 4.41 (m, 1H), 4.35 - 4.09 (m, 9H), 3.98 (d, J = 0.7 Hz, 3H), 2.42 (t, J = 3.0 Hz, 1H).

³¹P NMR (162 MHz, D₂O) δ -10.24, -21.26.

### Example 72

### Synthesis of Compound 2037

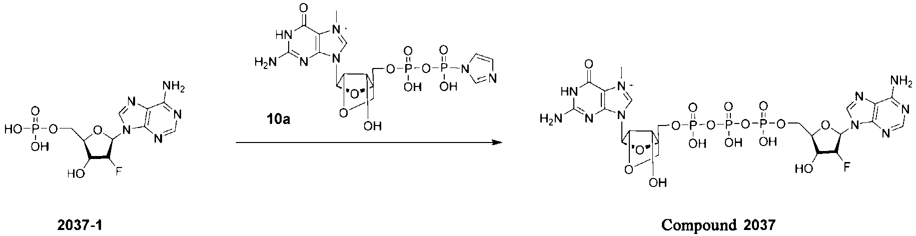

The compound ***10a*** (250 mg) was added to 16 mL of an aqueous solution with a pH of 7.0, containing 0.2 mol/L N-methylmorpholine and 0.2 mol/L manganous chloride, and a compound ***2037-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2037**,* which was an ammonium salt in the form of white powder (88 mg).

¹H NMR (400 MHz, D₂O) δ 8.42 - 8.05 (m, 2H), 6.55 - 6.15 (m, 2H), 5.28 (dddd, J = 46.4, 7.0, 3.0, 0.7 Hz, 1H), 4.64 (t, J = 2.8 Hz, 1H), 4.55 - 4.19 (m, 7H), 4.00 (dd, J = 12.9, 0.7 Hz, 5H).

³¹P NMR (162 MHz, D₂O) δ -10.24, -21.26.

### Example 73

### Synthesis of Compound 2038

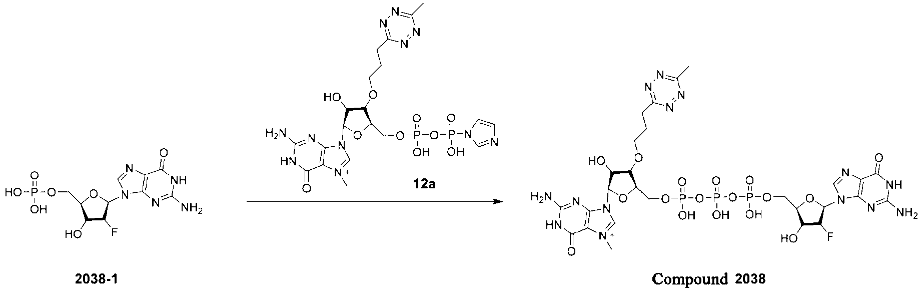

A compound ***12a*** (250 mg) was added to 16 mL of an aqueous solution with a pH of 7.0, containing 0.2 mol/L N-methylmorpholine and 0.2 mol/L manganous chloride, and a compound ***2038-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2038,*** which was an ammonium salt in the form of white powder (88 mg).

¹H NMR (400 MHz, D₂O) δ 8.02 (d, J = 0.7 Hz, 1H), 6.43 (ddt, J = 25.2, 1.6, 0.8 Hz, 1H), 6.27 - 5.99 (m, 1H), 5.24 (dddd, J = 46.5, 5.1, 1.7, 0.6 Hz, 1H), 4.86 - 4.45 (m, 2H), 4.36 - 4.07 (m, 7H), 3.98 (d, J = 0.7 Hz, 3H), 3.83 - 3.48 (m, 2H), 3.09 - 2.90 (m, 2H), 2.79 (s, 3H), 2.55 - 2.18 (m, 2H).

³¹P NMR (162 MHz, D₂O) δ -10.24, -21.26.

### Example 74

### Synthesis of Compound 2039

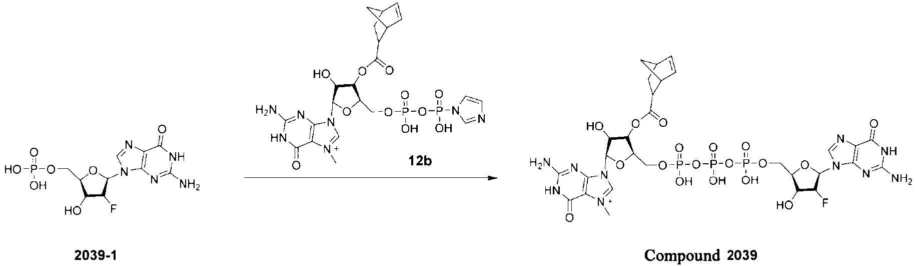

A compound ***12b*** (250 mg) was added to 16 mL of an aqueous solution with a pH of 7.0, containing 0.2 mol/L N-methylmorpholine and 0.2 mol/L manganous chloride, and a compound ***2039-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2039,*** which was an ammonium salt in the form of white powder (88 mg).

¹H NMR (400 MHz, D₂O) δ 8.02 (d, J = 0.7 Hz, 1H), 6.43 (ddd, J = 25.2, 1.6, 0.8 Hz, 1H), 6.27 - 5.70 (m, 3H), 5.42 - 5.07 (m, 1H), 5.00 - 4.81 (m, 2H), 4.65 - 4.43 (m, 2H), 4.37 - 4.10 (m, 5H), 3.98 (d, J = 0.7 Hz, 3H), 3.38 (ttdd, J = 5.3, 4.2, 1.8, 1.0 Hz, 1H), 3.11 - 2.53 (m, 2H), 2.14 - 1.79 (m, 2H), 1.62 - 1.17 (m, 2H).

³¹P NMR (162 MHz, D₂O) δ -10.24, -21.26.

### Example 75

### Synthesis of Compound 2040

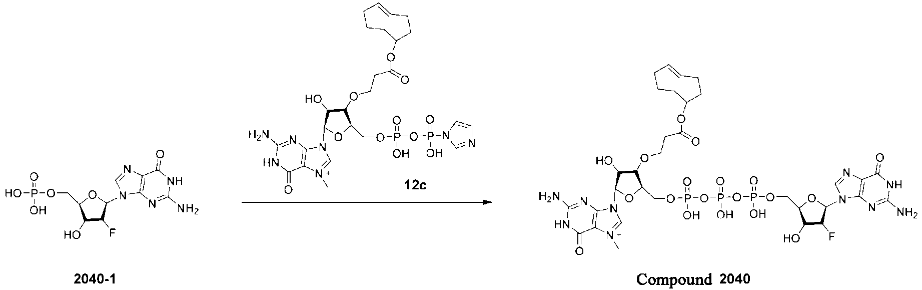

A compound ***12c*** (250 mg) was added to 16 mL of an aqueous solution with a pH of 7.0, containing 0.2 mol/L N-methylmorpholine and 0.2 mol/L manganous chloride, and a compound ***2040-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2040,*** which was an ammonium salt in the form of white powder (88 mg).

¹H NMR (400 MHz, D₂O) δ 8.02 (d, J = 0.7 Hz, 1H), 6.43 (ddd, J = 25.2, 1.5, 0.8 Hz, 1H), 6.26 - 6.03 (m, 1H), 5.69 - 5.39 (m, 2H), 5.31 - 5.07 (m, 1H), 4.84 - 4.67 (m, 2H), 4.63 - 4.46 (m, 1H), 4.28 - 4.06 (m, 7H), 3.98 (d, J = 0.7 Hz, 3H), 3.89 - 3.69 (m, 2H), 2.58 (td, J = 7.2, 2.9 Hz, 2H), 2.26 - 1.11 (m, 10H).

³¹P NMR (162 MHz, D₂O) δ -10.25, -21.23.

### Example 76

### Synthesis of Compound 2050

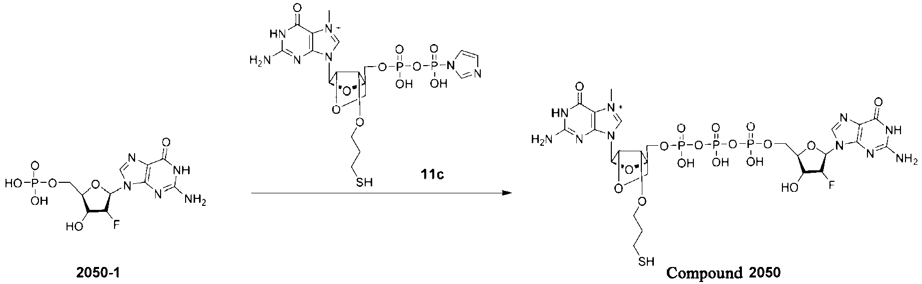

The compound ***11c*** (250 mg) was added to 16 mL of an aqueous solution with a pH of 7.0, containing 0.2 mol/L N-methylmorpholine and 0.2 mol/L manganous chloride, and a compound ***2050-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2050,*** which was an ammonium salt in the form of white powder (88 mg).

¹H NMR (400 MHz, D₂O) δ 8.02 (d, J = 0.7 Hz, 1H), 6.65 - 6.28 (m, 2H), 5.39 - 5.11 (m, 1H), 4.87 (t, J = 3.1 Hz, 1H), 4.65 - 4.47 (m, 1H), 4.39 - 4.21 (m, 5H), 4.15 (d, J = 3.1 Hz, 1H), 4.02 (s, 2H), 3.98 (d, J = 0.7 Hz, 3H), 3.66 (td, J = 5.3, 2.9 Hz, 2H), 2.96 - 2.52 (m, 2H), 1.85 (tt, J = 6.4, 5.3 Hz, 2H).

³¹P NMR (162 MHz, D₂O) δ -10.21, -21.25.

### Example 77

### Synthesis of Compound 2051

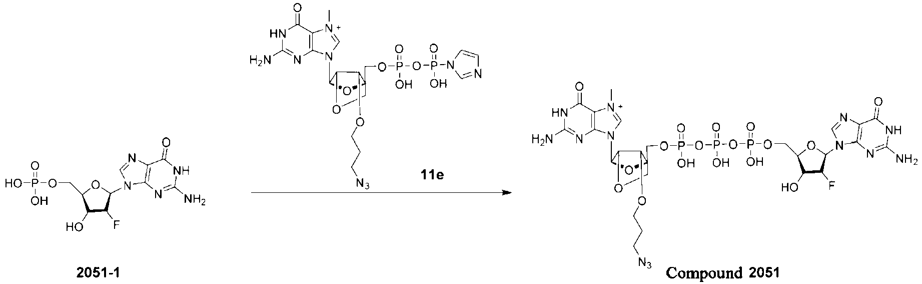

The compound ***11e*** (250 mg) was added to 16 mL of an aqueous solution with a pH of 7.0, containing 0.2 mol/L N-methylmorpholine and 0.2 mol/L manganous chloride, and a compound ***2051-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2051,*** which was an ammonium salt in the form of white powder (88 mg).

¹H NMR (400 MHz, D₂O) δ 7.95 (d, J = 0.7 Hz, 1H), 6.71 - 6.22 (m, 2H), 5.31 (dddd, J = 46.3, 5.0, 1.6, 0.7 Hz, 1H), 4.67 - 4.45 (m, 2H), 4.42 - 4.21 (m, 2H), 4.13 (qt, J = 3.1, 0.8 Hz, 1H), 4.06 - 3.85 (m, 7H), 3.76 - 3.53 (m, 3H), 3.36 (t, J = 6.2 Hz, 2H), 1.95 (tt, J = 6.3, 5.3 Hz, 2H).

³¹P NMR (162 MHz, D₂O) δ -10.34, -21.36.

### Example 78

### Synthesis of Compound 2054

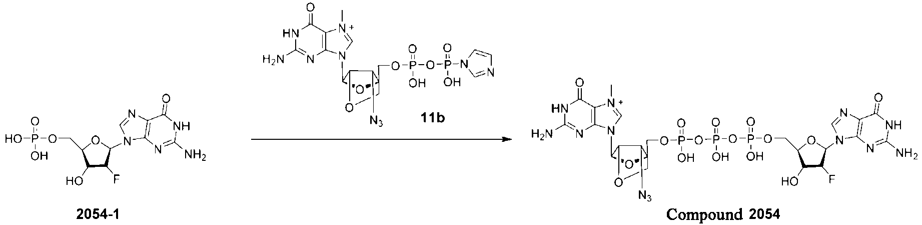

The compound ***11b*** (250 mg) was added to 16 mL of an aqueous solution with a pH of 7.0, containing 0.2 mol/L N-methylmorpholine and 0.2 mol/L manganous chloride, and a compound ***2054-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2054,*** which was an ammonium salt in the form of white powder (88 mg).

¹H NMR (400 MHz, D₂O) δ 7.95 (d, J = 0.7 Hz, 1H), 6.49 (dd, J = 2.0, 0.7 Hz, 1H), 6.44 - 6.19 (m, 1H), 5.31 (dddd, J = 46.4, 6.9, 3.5, 0.8 Hz, 1H), 4.81 (dd, J = 4.8, 2.0 Hz, 1H), 4.58 (dd, J = 11.3, 8.5 Hz, 1H), 4.42 (dddd, J = 25.2, 7.1, 3.2, 0.7 Hz, 1H), 4.36 - 4.23 (m, 3H), 4.14 - 3.99 (m, 3H), 3.92 (d, J = 0.6 Hz, 3H), 3.83 (dd, J = 11.3, 8.5 Hz, 1H).

³¹P NMR (162 MHz, D₂O) δ -10.24, -21.16.

### Example 79

### Synthesis of Compound 2064

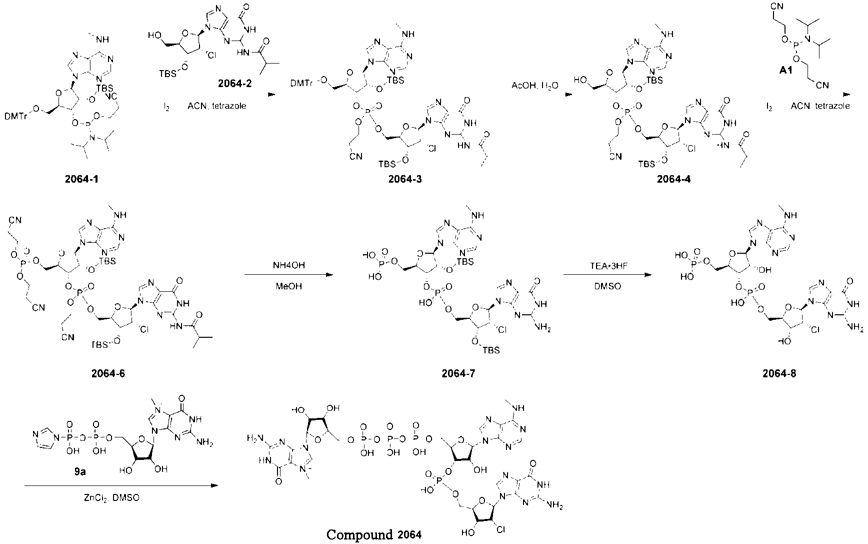

A compound ***2064-2*** (2.38 g) was added to a solution of tetrazole (1.76 g) in acetonitrile (63 mL) in a three-necked flask, and the atmosphere was replaced with argon three times. Next, at room temperature of 25°C, a material ***2064-1*** (5 g) was dissolved in 10 mL of acetonitrile and added to the above solution. The resulting solution was stirred at room temperature of 25°C for 1 hour. No obvious heat release was found, and TLC monitoring showed that the material ***2064-1*** disappeared. Then, into the solution, a solution of iodine in pyridine/tetrahydrofuran/water (0.5 mmol/mL, pyridine:tetrahydrofuran:water = 1:8:1) was added dropwise until the solution no longer faded. After that, the reaction solution was stirred for another 0.5 hours, and TLC monitoring showed that the oxidation was completed. After adding an aqueous solution (10 mL) of saturated sodium sulfite to the reaction solution for quenching, 50 mL of water was further added for dilution, and the mixture was extracted with dichloromethane (50 mL x 2). The organic phases were combined and washed once with water (50 mL), and concentrated to obtain a light yellow oily product ***2064-3*** (8 g, crude product).

The compound ***2064-3*** (8 g, crude product) was dissolved in 40 mL of acetic acid and 10 mL of water, and the reaction solution was stirred at 25°C for 16 hours. TLC monitoring showed that the compound ***2064-3*** disappeared and a spot with large polarity was generated. The reaction solution was directly concentrated in vacuo. After concentration, appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white solid product ***2064-4*** (2.8 g, 51% overall yield over two-step) was obtained.

28 mL of a tetrazole solution in acetonitrile (0.4 mmol/mL) was prepared. The compound ***2064-4*** (2.8 g) was added to the above solution, and then the compound ***A1*** (3 g) was added to the solution at room temperature of 25°C. The atmosphere was replaced with nitrogen three times, and the reaction solution was stirred at room temperature of 25°C for 1 hour. TLC monitoring showed that the reaction was completed. The reaction solution was cooled to below 10°C in an ice-water bath, and a solution of iodine in pyridine/tetrahydrofuran/water (0.5 mmol/mL, pyridine:tetrahydrofuran:water = 1:8:1) was added dropwise until the reaction solution no longer faded. TLC monitoring showed that the oxidation reaction was completed. The reaction solution was quenched by adding 10 mL of an aqueous saturated solution of sodium sulfite, diluted with water, and extracted three times with ethyl acetate. The organic phases were combined and dried over anhydrous sodium sulfate and filtered. Appropriate amounts of silica gel and DCM were added and mixed with the sample, followed by purification (40 g normal phase column, EA, 10 min, DCM: MeOH, 10% to 20%, for 20 min, flow rate: 30 mL/min). After concentration, a white foamy solid compound ***2064-6*** (2.6 g, 78.2% yield) was obtained.

The compound ***2064-6*** (2.6 g) was dissolved in methanol (30 mL) and concentrated aqueous ammonia (30 mL) was added. The resulting solution was stirred at room temperature of 25°C for 60 hours. TLC monitoring showed that the raw material ***2064-6*** was completely reacted. The reaction solution was concentrated in vacuo and concentrated again with methanol to obtain a light yellow oily liquid compound ***2064-7*** (2.4 g, crude product), which was directly used for the next step.

The compound ***2064-7*** (2.4 g, crude product) was dissolved in DMSO (3 mL) and triethylamine trihydrofluoride (3.5 mL) was added. The reaction solution was stirred at 50°C for 1 hour. TLC monitoring showed that the raw material ***2064-7*** was completely reacted. The reaction solution was diluted to 50 mL with water, and the pH was adjusted to 5.5 with 1 N NaOH aqueous solution. The mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a target compound, amine salt ***2064-8*** (0.8 g, 33.7% yield), which was a white solid.

The compound ***9a*** (200 mg) was added to 16 mL of an aqueous solution with a pH of 7.0, containing 0.2 mol/L N-methylmorpholine and 0.2 mol/L manganous chloride, and ***2064-8*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2064,*** which was an ammonium salt in the form of white powder (65 mg).

¹H NMR (400 MHz, D₂O) δ 8.33 (s, 1H), 8.29 (s, 1H), 8.04 (s, 1H), 6.25 (t, J = 0.7 Hz, 1H), 6.16 (dt, J = 3.0, 0.7 Hz, 1H), 6.11 (dq, J = 3.0, 0.7 Hz, 1H), 4.80 (ddd, J = 3.8, 2.9, 0.7 Hz, 1H), 4.74 (dddd, J = 7.9, 3.9, 2.9, 0.7 Hz, 1H), 4.61 (ddd, J = 5.2, 2.8, 0.7 Hz, 1H), 4.55 - 4.43 (m, 1H), 4.41 - 4.32 (m, 2H), 4.30 - 4.09 (m, 9H), 3.98 (d, J = 0.7 Hz, 3H), 3.08 (s, 3H).

³¹P NMR (162 MHz, D₂O) δ -0.80, -11.55, -23.03.

### Example 80

### Synthesis of Compound 2078

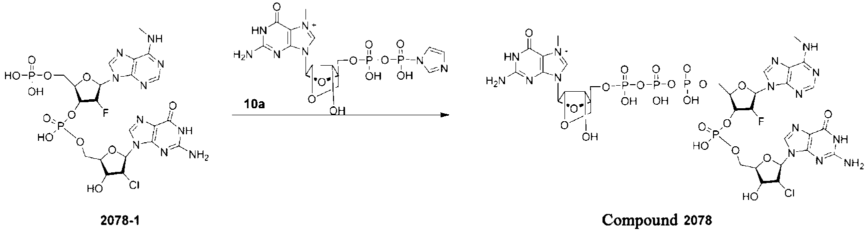

The compound ***10a*** (200 mg) was added to 16 mL of an aqueous solution with a pH of 7.0, containing 0.2 mol/L N-methylmorpholine and 0.2 mol/L manganous chloride, and ***2078-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2078,*** which was an ammonium salt in the form of white powder (55 mg).

¹H NMR (400 MHz, D₂O) δ 8.39 (s, 1H), 8.28 (s, 1H), 8.19 (s, 1H), 6.49 (dq, J = 1.5, 0.7 Hz, 1H), 6.32 - 6.09 (m, 2H), 5.30 (ddd, J = 2.6, 1.8, 0.7 Hz, 1H), 5.25 (ddd, J = 3.6, 2.9, 0.7 Hz, 1H), 4.72 - 4.60 (m, 2H), 4.56 - 4.46 (m, 2H), 4.44 - 4.36 (m, 2H), 4.33 (d, J = 2.8 Hz, 1H), 4.27 - 4.07 (m, 6H), 4.05 - 3.96 (m, 4H), 3.11 (s, 3H).

³¹P NMR (162 MHz, D₂O) δ -0.90, -11.50, -22.95.

### Example 81

### Synthesis of Compound 2092

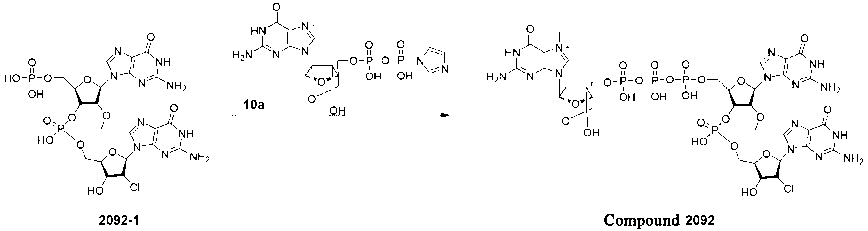

The compound ***10a*** (200 mg) was added to 16 mL of an aqueous solution with a pH of 7.0, containing 0.2 mol/L N-methylmorpholine and 0.2 mol/L manganous chloride, and ***2092-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2092*,** which was an ammonium salt in the form of white powder (65 mg).

¹H NMR (400 MHz, D₂O) δ 8.19 (s, 1H), 8.08 (s, 1H), 6.37 - 6.19 (m, 2H), 6.09 (dd, J = 2.8, 0.8 Hz, 1H), 4.97 (ddd, J = 4.4, 2.9, 0.7 Hz, 1H), 4.76 - 4.70 (m, 1H), 4.64 (t, J = 2.8 Hz, 1H), 4.55 - 4.45 (m, 3H), 4.44 - 4.35 (m, 2H), 4.33 (d, J = 2.8 Hz, 1H), 4.26 - 4.06 (m, 6H), 4.05 - 3.97 (m, 4H), 3.39 (s, 3H).

³¹P NMR (162 MHz, D₂O) δ -0.95, -11.70, -22.75.

### Example 82

### Synthesis of Compound 2098

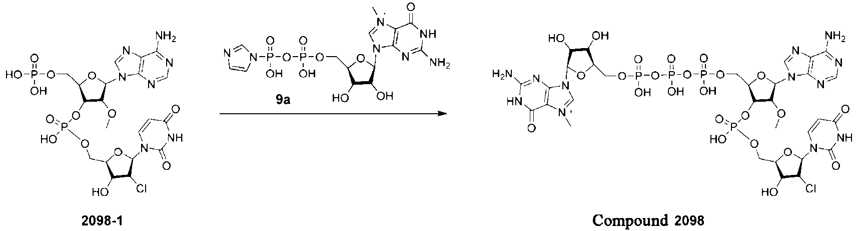

The compound ***9a*** (200 mg) was added to 16 mL of an aqueous solution with a pH of 7.0, containing 0.2 mol/L N-methylmorpholine and 0.2 mol/L manganous chloride, and ***2098-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2098,*** which was an ammonium salt in the form of white powder (70 mg).

¹H NMR (400 MHz, D₂O) δ 8.42 (s, 1H), 8.18 (s, 1H), 7.93 (d, J = 7.9 Hz, 1H), 6.15 (dq, J = 4.1, 0.7 Hz, 1H), 6.10 (dd, J = 2.9, 0.7 Hz, 1H), 6.01 (ddd, J = 2.7, 1.7, 0.8 Hz, 1H), 5.91 (d, J = 7.8 Hz, 1H), 5.02 - 4.88 (m, 1H), 4.77 (dddd, J = 5.9, 4.3, 1.5, 0.7 Hz, 1H), 4.55 (ddd, J = 5.3, 2.9, 0.6 Hz, 1H), 4.48 (qt, J = 3.3, 0.8 Hz, 1H), 4.36 (ddd, J = 7.3, 2.8, 0.6 Hz, 1H), 4.30 - 4.04 (m, 10H), 4.02 (s, 3H), 3.39 (s, 3H).

³¹P NMR (162 MHz, D₂O) δ -0.95, -11.70, -22.75.

### Example 83

### Synthesis of Compound 2110

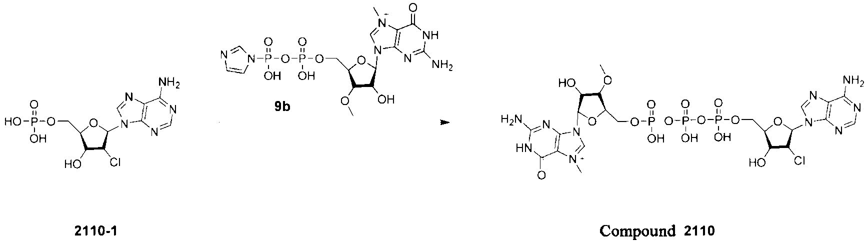

The compound ***9b*** (200 mg) was added to 16 mL of an aqueous solution with a pH of 7.0, containing 0.2 mol/L N-methylmorpholine and 0.2 mol/L manganous chloride, and ***2110-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2110,*** which was an ammonium salt in the form of white powder (70 mg).

¹H NMR (400 MHz, D₂O) δ 8.41 (s, 1H), 8.18 (s, 1H), 6.31 (dd, J = 1.4, 0.8 Hz, 1H), 6.13 (dd, J = 2.5, 0.8 Hz, 1H), 4.72 - 4.53 (m, 2H), 4.35 - 4.06 (m, 8H), 4.02 (s, 3H), 3.38 (s, 3H).

³¹P NMR (162 MHz, D₂O) δ -10.24, -21.26.

### Example 84

### Synthesis of Compound 2123

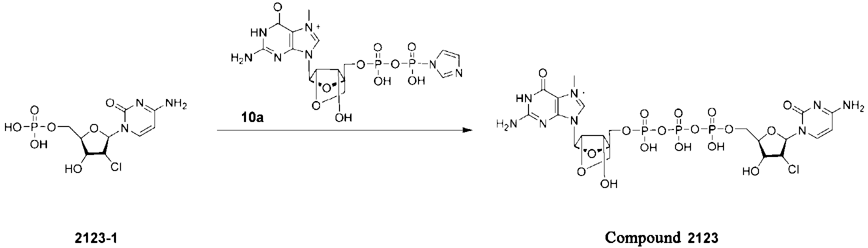

The compound ***10a*** (200 mg) was added to 16 mL of an aqueous solution with a pH of 7.0, containing 0.2 mol/L N-methylmorpholine and 0.2 mol/L manganous chloride, and ***2123-1*** (200 mg) was added to the solution. The reaction solution was stirred at room temperature of 25°C for 16 hours. TLC monitoring showed that a product was generated. The reaction solution was added to a disodium EDTA solution (1.4 g, 80 mL of water was added) pre-cooled to 0°C, and the mixture was loaded onto a DEAE Sephadex column. The product was eluted by linear gradient elution using an eluent, i.e., 0 M to 1.0 M ammonium bicarbonate aqueous solution. Most of the water in the obtained fraction was concentrated in vacuo, and the remaining liquid was freeze-dried to obtain a product, compound ***2123,*** which was an ammonium salt in the form of white powder (50 mg).

¹H NMR (400 MHz, D₂O) δ 7.62 (dd, J = 7.4, 1.8 Hz, 1H), 6.24 (dd, J = 2.7, 0.6 Hz, 1H), 6.12 (ddt, J = 2.6, 1.6, 0.7 Hz, 1H), 5.96 (d, J = 7.3 Hz, 1H), 4.64 (t, J = 2.8 Hz, 1H), 4.49 (d, J = 11.6 Hz, 1H), 4.42 (ddd, J = 5.3, 2.4, 0.7 Hz, 1H), 4.39 (d, J = 11.6 Hz, 1H), 4.33 (d, J = 2.8 Hz, 1H), 4.27 (td, J = 5.1, 0.7 Hz, 1H), 4.21 - 4.05 (m, 4H), 4.04 - 3.96 (m, 4H).

³¹P NMR (162 MHz, D₂O) δ-10.70, -20.75.

### Example 85

### Capped mRNA synthesis efficiency detection

a) A plasmid was linearized and a DNA template was purified.
b) Capping analogs of the present invention and Comparative Example 1 Trilink CleanCap, cap analogs of Comparative Example 2 and Comparative Example 3 were used to synthesize mRNA by *in vitro* transcription, respectively. Comparative Example 1 was commercially purchased, and Comparative Example 2 and Comparative Example 3 were obtained according to the method disclosed in WO2022/036858.

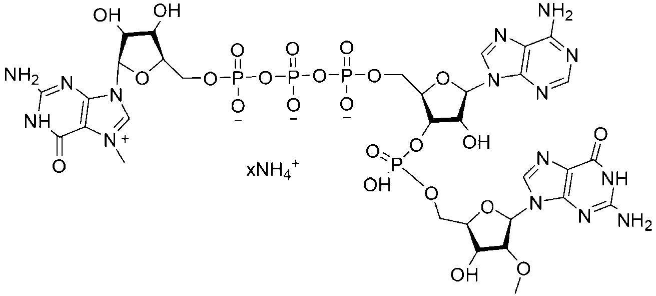

### Comparative Example 1

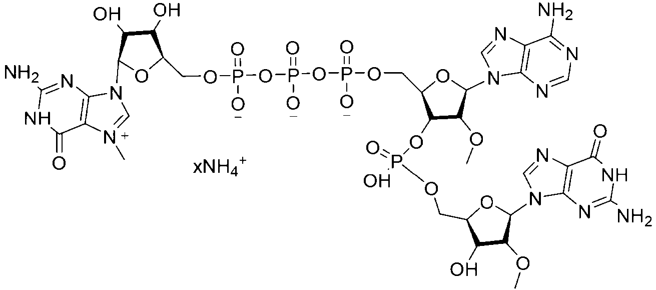

### Comparative Example 2

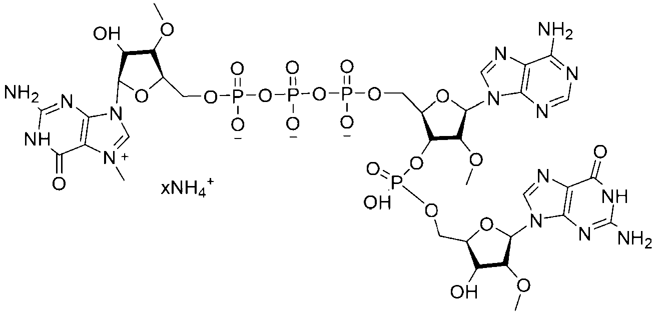

### Comparative Example 3

The minimum reaction system for preparing mRNA used in the present invention is shown in ***Table 1.***

**Table 1 In vitro transcription system**

| **Components** | **Dosage** |
|---|---|
| T7 RNA polymerase | 4 µL (200 U) |
| RNase inhibitor | 0.5 µL (20 U) |
| Inorganic pyrophosphatase | 1 µL (0.5 U) |
| 100mM ATP | 1.5 µL |
| 100mM CTP | 1.5 µL |
| 100mM GTP | 1.5 µL |
| 100mM ψTP | 1.5 µL |
| 100mM Cap analog | 1.5 µL |
| 10 × buffer | 2 µL |
| DNA Template | 1 µL |
| Water | 4 µL |
| Total | 20 µL |

c) During the experiment, the above reagents were fully mixed and incubated at 37°C. After 4 hours, deoxyribonuclease (DNase) was added and incubated for 30 minutes to remove the DNA template. After digestion, LiCl solution was added, precooled and centrifuged at 1,6000 rpm for 15 minutes to remove the supernatant. 70% ethanol was added and centrifuged to remove the supernatant again, and then a certain amount of enzyme-free water was added for storage. The purified mRNA sample was then quantitatively detected using Nanodrop One, as shown in Table 2 below. The experimental results show that the product yield in mRNA synthesis by the halogenated cap analogs in the present invention is improved compared with the comparative examples.

**Table 2 amount (mg) of final product obtained per 1 mL of mRNA synthesis reaction system**

| | | | | | | | | | |
|---|---|---|---|---|---|---|---|---|---|
| **Compound number** | **8** | **68** | **168** | **173** | **433** | **458** | **468** | **33** | **Comparative Example 2** |
| **Product amount** | **4.7** | **5.8** | **5.6** | **5.2** | **4.9** | **4.8** | **5.3** | **5.3** | **4.2** |
| **Compound number** | **58** | **76** | **101** | **158** | **408** | **1982** | **2017** | **2037** | **2058** |
| **Product amount** | **5.5** | **5.4** | **5.3** | **4.9** | **5.7** | **5.8** | **5.8** | **5.5** | **4.6** |

d) After the purified mRNA was treated with enzyme cleavage, oligonucleotide fragments of different sizes were separated and identified by liquid chromatography-mass spectrometry (LC-MS), providing accurate molecular weight information of the enzyme-cleaved fragments. Combined with the theoretical enzyme-cleaved molecular weight, and attribution, the capping efficiency of the sample can be obtained.

The capping efficiency of mRNA synthesized by the cap analogs of the present invention was between 90% and 98%. From the purified capped mRNA, the compounds of the present invention all showed good capping efficiency.

### Example 86

### Evaluation of Expression Efficiency of Green Fluorescent Protein mRNA with Different Capping Analogs in Different Cells

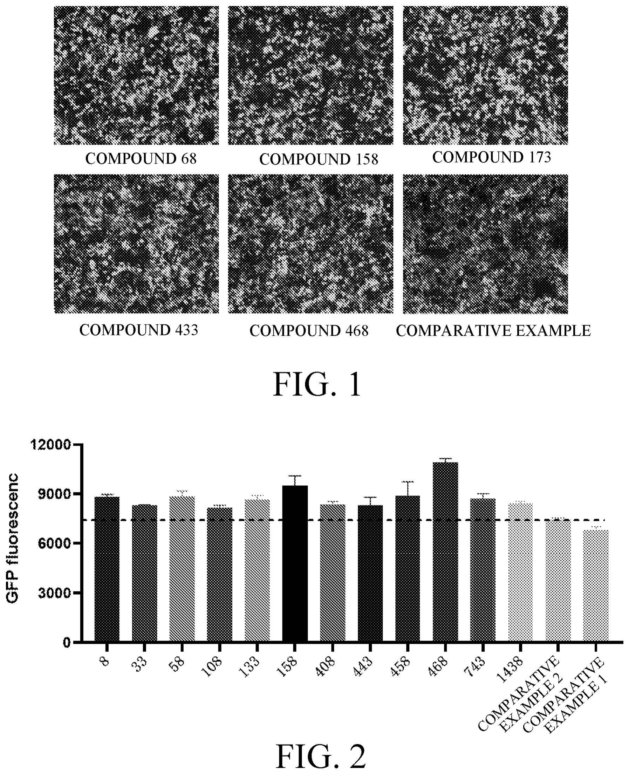

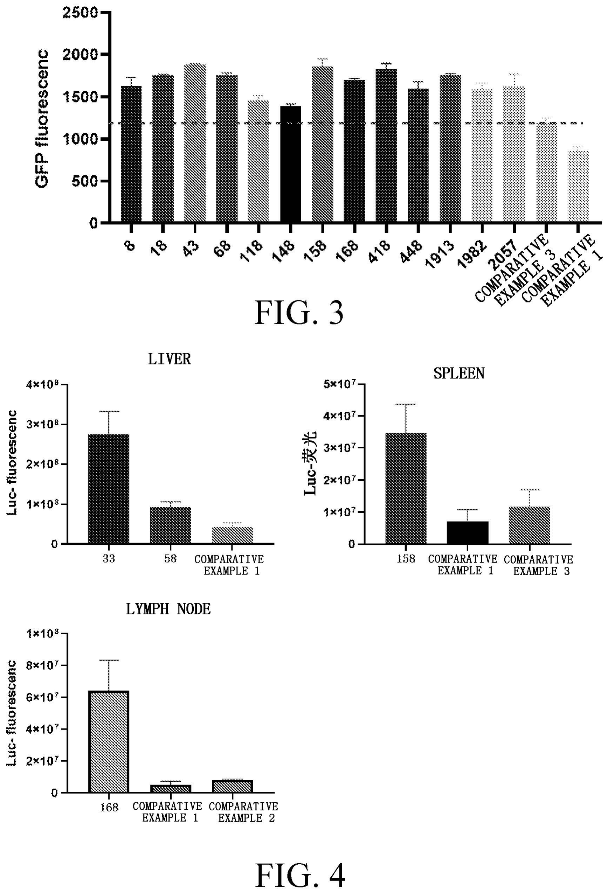

The present invention tested the expression efficiency of different capped green fluorescent protein mRNA in HEK293T and HepG2 cells. A green fluorescent protein GFP coding sequence was used as a DNA template, and the cap analogs of the present invention were used as materials for *in vitro* transcription of mRNA. Then, different mRNA products were transfected into cells, and finally fluorescent proteins in the cells were detected by flow cytometry.
a) The different cells described above were plated at 2 × 10⁵ cells (96-well plate).
b) 300 µL of mRNA buffer was mixed evenly with 6 µg of RNA, and then 6 µL of a transfection reagent (JetMESENG-ER) was added and mixed evenly. After standing for 10 min, the mixture was added to cells in each well, and a transfection medium (Opti-MEM) was supplemented to reach 2 mL/well. The cells were cultured at 37°C and 5% CO₂ for 6 h.
c) After the transfection medium was replaced with a fresh complete medium and cultured for 24 h under the same conditions, the GFP fluorescence intensity was observed using a fluorescence microscope. The results are shown in FIG. 1, from which it can be clearly seen that the mRNA expression efficiency in the present invention is higher than that of the comparative examples.
d) After the transfected cells were cultured for 24 h, the cells were treated and then detected by flow cytometry (CytoFLEX S series). The detected fluorescence intensity was proportional to the translation efficiency of the target protein. The results of the analysis are shown in FIGS. 2 and 3.

**FIG. 2** is a fluorescence intensity analysis chart of mRNA coding green fluorescent protein and with different cap analogs in HEK293T cells, the horizontal axis is compound digital number of the cap analogs, and the vertical axis is a fluorescence intensity value detected by flow cytometer. Compared with Comparative Examples 1 and 2, the fluorescence intensity values of the cap analogs in the present invention in HEK293T cells were more prominent. For example, an average fluorescence intensity value of a compound 468 was 1.5 times that of Comparative Example 2 and 1.6 times that of Comparative Example 1, and an average fluorescence intensity value of a compound 158 was 1.3 times that of Comparative Example 2 and 1.4 times that of Comparative Example 1.

**FIG. 3** is a statistical graph of the fluorescence intensity of mRNA with different cap analogs in Hep G2 cells. In comparison, the efficiency of mRNA translation and protein expression of the cap analogs of the present invention in Hep G2 cells is higher than that of the comparative examples. The protein expression levels of fluorinated nucleoside dimer cap analogs such as a compound 1982 and fluorinated nucleoside tetramer cap analogs such as 2057 were also significantly improved.

### Example 87

### Test of Expression Efficiency of mRNA Synthesized by Different Cap Analogs in Mice

a) The cap analogs of the present invention were used to prepare mRNA of encoding luciferase, and the obtained mRNA was diluted into a citric acid buffer at pH 4.0. Cationic lipids DLin-MC3-DMA, DSPC, cholesterol, and PEG lipids (DMG-PEG2000) were dissolved in ethanol at a molar ratio of 50:10:38.5:1.5.
b) Two 5 mL syringes were filled with 3 mL mRNA buffer and 1 mL lipid solution, respectively and then loaded on a microfluidic injection pump, and the flow rate of the injection pump was set. The collected product was placed in a dialysis bag and ultrafiltered and concentrated to an ideal concentration. Then, lipid nanoparticles were filtered through a 0.22 µm sterile filter and stored for use.
c) Luciferase mRNA-lipid nanoparticles containing 5 µg of mRNA were injected into female Balb/c mice aged 6 to 8 weeks via tail vein injection. Each kind of Luciferase mRNA-lipid nanoparticle was injected into 5 mice for parallel experiments. A luciferase substrate was injected 24 hours later and detected using a PerkinElmer small animal imaging system. The luminescence intensity was proportional to the translation efficiency of the effective target protein. The relative fluorescence intensity of mRNA in different organs of mice was shown in FIG. 4. The expression efficiency of mRNA with different halogenated caps analog of the present invention in different organs was significantly higher than that of the comparative examples.

## Claims

1. A compound for capping the 5' end of a nucleic acid, or a pharmaceutically acceptable salt, solvate, or stereoisomer thereof, wherein the compound has a structure of Formula (I): 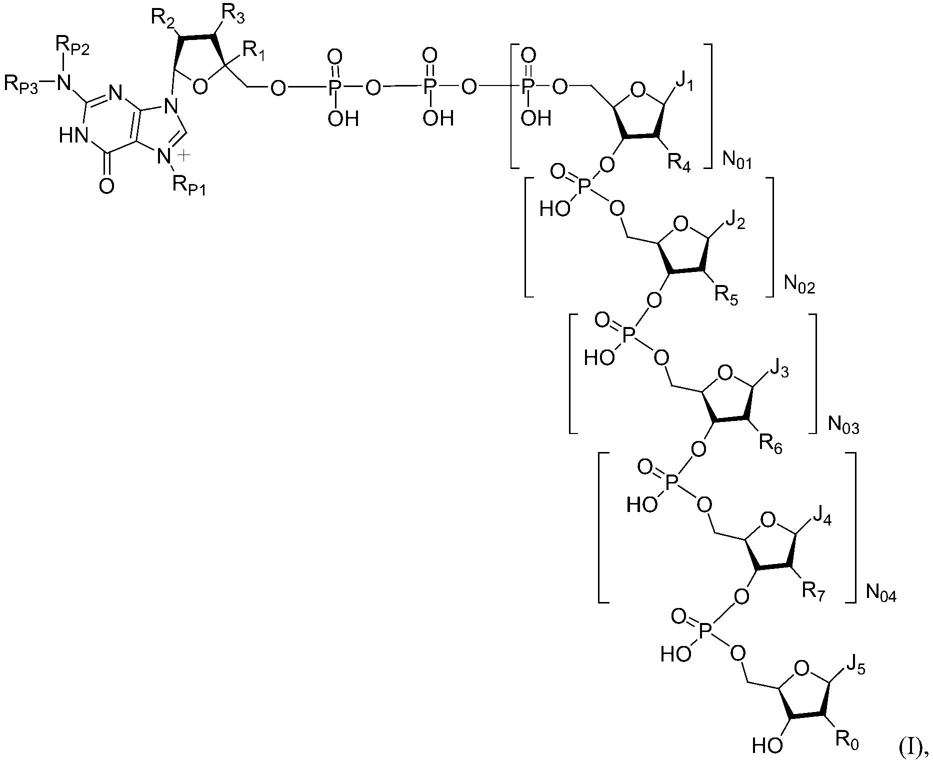 wherein
R₀ is any one selected from the group consisting of F, Cl, Br and I,
R₁ is a group selected from the group consisting of -H, -OH, C₁₋₄ alkyl and C₁₋₄ alkoxy,
R₂ is any one selected from the group consisting of -H, -OH, C₁₋₆ alkyl and C₁₋₆ alkoxy,
optionally, R₁ and R₂ are connected to form a ring by a chemical bond, and -R₁-R₂- is any one of -(CH₂)_{q}-O-, -O-(CH₂)_{q}- and -(CH₂)ₘ-O-(CH₂)ₙ-, wherein q, m, and n are each independently 1, 2 or 3,
R₃ is any one of H, -OH, -SH, -N₃, -NH₂, halogen, -CN, C₁₋₆ alkoxy, -O(CH₂)ₛCN, -SR₃ₐ, - O(CH₂)ₚR_{3b}, OCOR_{3c}, O(CH₂)ₚCOR_{3c}, -O(CH₂)ₜSH, -O(CH₂)ₚOH, -O(CH₂)ₚN₃, and - O(CH₂)ₚNH₂, wherein t, p, and s are each independently any integer from 1 to 6; R₃ₐ is C₁₋₆ alkyl; R_{3b} is C₆₋₁₂ aryl optionally substituted with one or more R_{3d}, or C₅₋₁₂ heteroaryl optionally substituted with one or more R_{3d}; R_{3c} is C₁₋₁₀ alkyl optionally substituted with one or more R_{3d}, C₁₋₁₀ alkenyl optionally substituted with one or more R_{3d}, C₅₋₁₂ cycloalkyl optionally substituted with one or more R_{3d}, or C₅₋₁₂ cycloalkenyl optionally substituted with one or more R_{3d}; wherein R₃ is optionally substituted with one or more R₃ₑ; and R_{3d} and R₃ₑ are selected from the group consisting of alkyl, alkenyl, alkoxy, halogen, cyano, amino, nitro, -OH, and -SH,
R₄, R₅, R₆, and R₇ are each independently any one selected from the group consisting of -H, -OH, -OCH₃, halogen, -CN, and -SH,
N₀₁, N₀₂, N₀₃, and N₀₄ are each independently selected from 0 or 1,
J₁, J₂, J₃, J₄, and J₅ are each independently selected from natural or modified pyrimidine nucleotide bases, or natural or modified purine nucleotide bases,
R_{P1} is C₁ to C₆ alkyl, preferably C₁ to C₃ alkyl, which is optionally substituted with -SH, - N₃, C₂ to C₆ alkenyl or C₂ to C₆ alkynyl,
R_{P2} and R_{P3} are each independently selected from the group consisting of H, C₁ to C₆ alkyl, C₂ to C₆ alkenyl, C₂ to C₆ alkynyl, cycloalkyl, cycloalkenyl, cycloalkynyl, a PEG group, COR_{P4}, and SO₂R_{P4}, wherein these groups are each optionally substituted with -CN, -N₃, -SH, or alkynyl, R_{P4} is selected from the group consisting of H and C₁ to C₆ alkyl, and R_{P2} and R_{P3} are optionally connected to form a ring,
with the proviso that when N₀₁, N₀₂, N₀₃, and N₀₄ are all 0, J₅ is a guanine base, and when R₂ is -OH, R₃ is not a methoxy group.

2. The compound or a pharmaceutically acceptable salt, solvate, or stereoisomer thereof according to claim 1, the compound having a structure of Formula (I'): 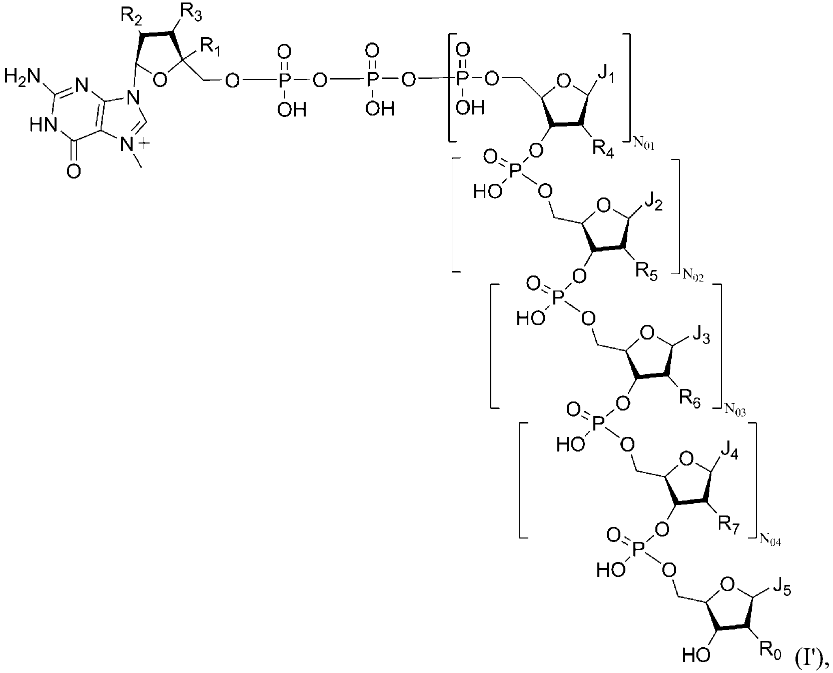 wherein, each group in Formula (I') has a meaning the same as that described in claim 1.

3. The compound or a pharmaceutically acceptable salt, solvate, or stereoisomer thereof according to claim 1 or 2, wherein
at least one of J₁, J₂, J₃, J₄, and J₅ is a modified nucleotide base, preferably a modified purine nucleotide base, more preferably a methyl-modified purine nucleotide base, and still more preferably 6-N-methyladenine.

4. The compound or a pharmaceutically acceptable salt, solvate, or stereoisomer thereof according to any one of claims 1 to 3, wherein
R₃ is any one of -H, -OH, -SH, -N₃, -NH₂, halogen, -CN, C₁₋₆ alkoxy, -O(CH₂)ₚCN, -SR₃ₐ, -O(CH₂)ₚR_{3b}, OCOR_{3c}, O(CH₂)ₚCOR_{3c}, -O(CH₂)ₚSH, -O(CH₂)ₚOH, -O(CH₂)ₚN₃, and - O(CH₂)ₚNH₂, wherein t, p, and s are each independently any integer from 1 to 6, preferably 1 to 4; R₃ₐ is C₁₋₄ alkyl; R_{3b} is C₆₋₁₀ aryl optionally substituted with one or more R_{3d}, or C₅₋₁₀ heteroaryl optionally substituted with one or more R_{3d}; R_{3c} is C₅₋₁₀ cycloalkyl optionally substituted with one or more R_{3d}, or C₅₋₁₀ cycloalkenyl optionally substituted with one or more R_{3d}, wherein R₃ is optionally substituted with one or more R₃ₑ; and R_{3d} and R₃ₑ are selected from the group consisting of C₁₋₄ alkyl, C₂₋₄ alkenyl, C₁₋₄ alkoxy, halogen, cyano, amino, nitro, -OH, and -SH,
preferably, R₃ is any one of -H, -OH, -SH, -N₃, -NH₂, halogen, -CN, C₁₋₃ alkoxy, - O(CH₂)ₚCN, -SR₃ₐ, -O(CH₂)ₚR_{3b}, OCOR_{3c}, O(CH₂)ₚCOR_{3c}, -O(CH₂)ₚSH, -O(CH₂)ₚOH, - O(CH₂)ₚN₃, and -O(CH₂)ₚNH₂, wherein p and s are each independently any integer from 1 to 3, t is any integer from 1 to 4, R₃ₐ is methyl or ethyl, R_{3b} is C₅₋₁₀ heteroaryl optionally substituted with one or two R_{3d}, R_{3c} is C₅₋₁₀ cycloalkyl optionally substituted with one or two R_{3d}, or C₅₋₁₀ cycloalkenyl optionally substituted with one or two R_{3d}, wherein R₃ is optionally substituted with one or more R₃ₑ, and R_{3d} and R₃ₑ are selected from the group consisting of C₁₋₄ alkyl, C₂₋₄ alkenyl, C₁₋₄ alkoxy, halogen, cyano, amino, nitro, -OH, and -SH,
more preferably, R_{3b} is C₅ or C₆ heteroaryl optionally substituted with C₁₋₄ alkyl, for example, tetrazinyl optionally substituted with C₁₋₄ alkyl, and R_{3c} is C₅₋₁₀ cycloalkenyl optionally substituted with C₁₋₄ alkyl, halogen, cyano, amino, or nitro, for example, norbornenyl or cyclooctenyl optionally substituted with C₁₋₄ alkyl, halogen, cyano, amino, or nitro, and for example, unsubstituted norbornenyl or cyclooctenyl.

5. The compound or a pharmaceutically acceptable salt, solvate, or stereoisomer thereof according to any one of claims 1 to 4, wherein
the compound has a structure of Formula (Ia), Formula (Ib), or Formula (Ic): 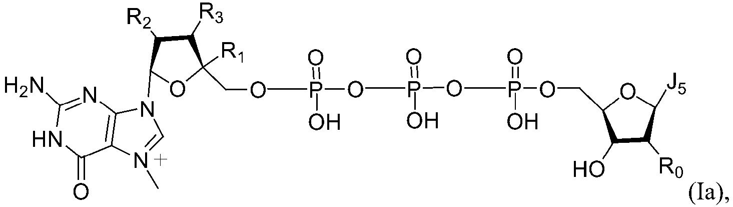 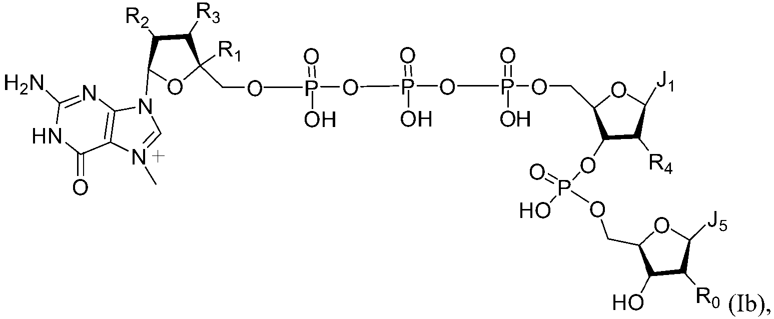 or

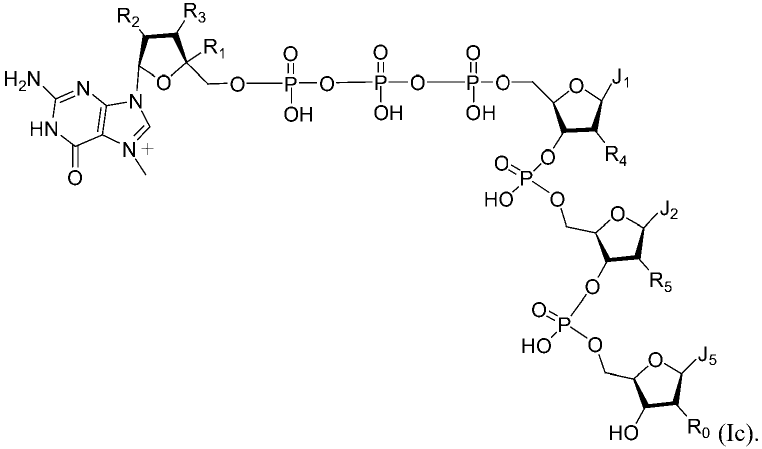

6. The compound or a pharmaceutically acceptable salt, solvate, or stereoisomer thereof according to claim 5, wherein
R₀ is -F or -Cl, and/or
R₄ and R₅ are each independently any one selected from the group consisting of H, OH, OCH₃, F, Cl, -CN, and -SH, and preferably any one selected from the group consisting of H, OH, OCH₃, and F.

7. The compound or a pharmaceutically acceptable salt, solvate, or stereoisomer thereof according to any one of claims 1 to 4, wherein the compound having a structure of Formula (Id): 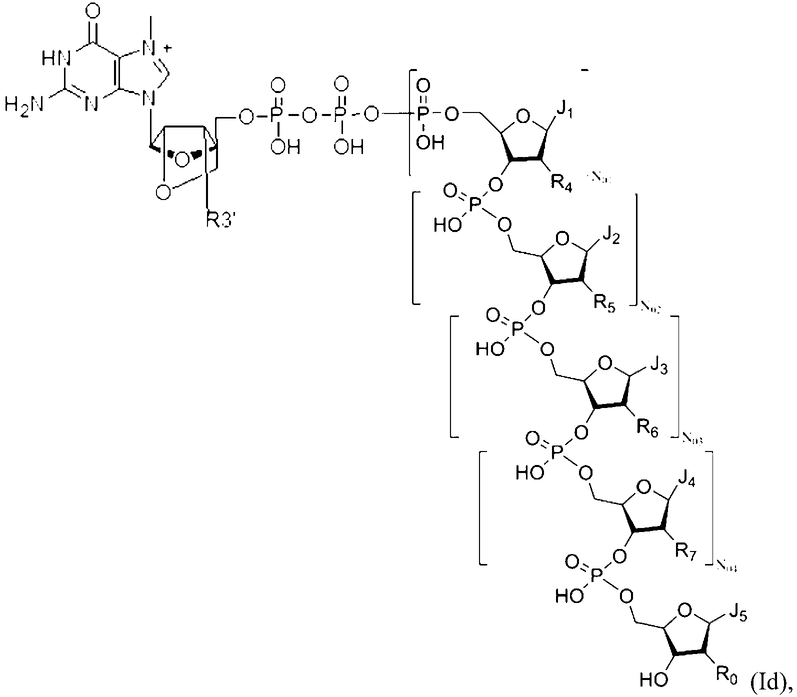 wherein
R₃' has a meaning the same as that of R₃ defined in claim 1 or 4, and
the remaining groups have the meanings the same as those described in claims 1 to 4.

8. The compound or a pharmaceutically acceptable salt, solvate, or stereoisomer thereof according to claim 7, wherein
the compound has a structure of Formula (Ie), Formula (If), or Formula (Ig): 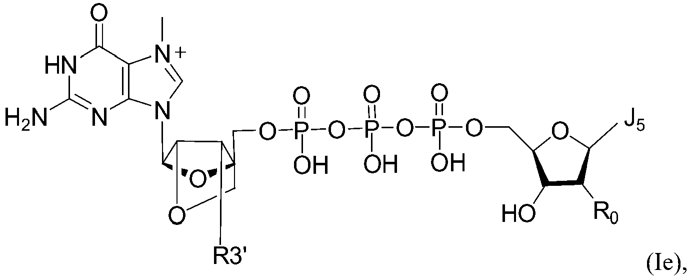 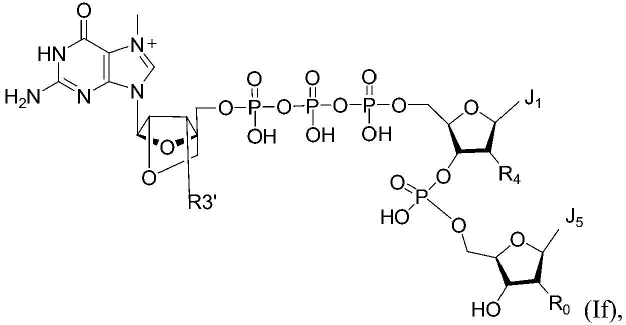 or

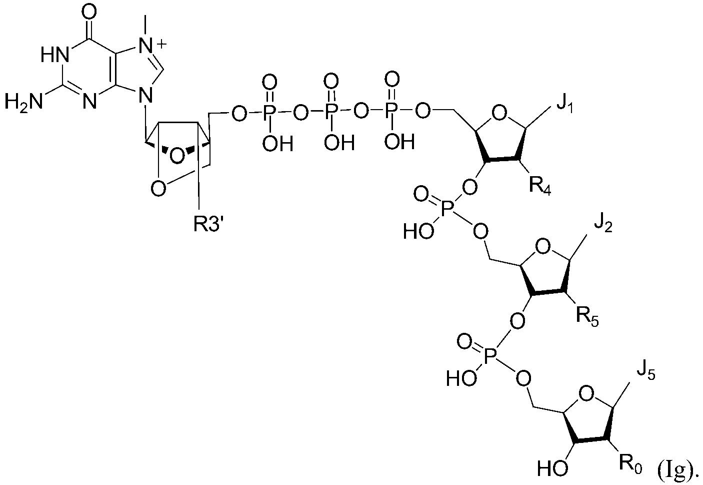

9. The compound or a pharmaceutically acceptable salt, solvate, or stereoisomer thereof according to claim 8, wherein
R₀ is -F or -Cl, and/or
R₄ and R₅ are each independently any one selected from the group consisting of H, OH, OCH₃, F, Cl, -CN, and -SH, and preferably any one selected from the group consisting of H, OH, OCH₃, and F.

10. The compound or a pharmaceutically acceptable salt, solvate, or stereoisomer thereof according to claim 1, wherein
the compound has one of the structures shown in Table 1 of the description.

11. The compound or a solvate or stereoisomer thereof according to any one of claims 1 to 10 wherein
the compound is present in the form of a pharmaceutically acceptable salt, and preferably in the form of a triethylamine salt, a sodium salt, a potassium salt, an ammonium salt, or tris(hydroxymethyl)aminomethane hydrochloride.

12. Use of the compound according to any one of claims 1 to 11 as an *in vitro* co-transcriptional RNA-capping reagent.

13. An RNA molecule, comprising:
the compound according to any one of claims 1 to 11 as a cap structure or a cap structure fragment.

14. A pharmaceutical composition, comprising:
the RNA molecule according to claim 13; and
a pharmaceutically acceptable carrier.

15. A method for synthesizing an RNA molecule, the method comprising:
incubating the compound according to any one of claims 1 to 11 with a polynucleotide template in order to perform a template-based transcription.

16. A transcriptional RNA-capping reaction system, comprising:
a polynucleotide template;
the compound according to any one of claims 1 to 11;
NTPs; and
an RNA polymerase.
